# EUROPEAN PATENT SPECIFICATION

(11) **EP 3 894 402 B1**
(45) Date of publication and mention of the grant of the patent: **05.07.2023**
(21) Application number: 19828596.7
(22) Date of filing: 11.12.2019
(51) Int. Cl.: C07D 405/12, A61K 31/352, A61K 31/4025, A61K 31/4523, A61K 31/5355, C07D 407/12, C07D 413/12, C07D 309/32, A61P 31/12

(54) **N-CONTAINING CHROMEN-4-ONE DERIVATIVES FOR THE TREATMENT AND PROPHYLAXIS OF HEPATITIS B VIRUS INFECTION**
N-ENTHALTENDE CHROMEN-4-ON-DERIVATE ZUR BEHANDLUNG UND PROPHYLAXE VON HEPATITIS-B-VIRUSINFEKTIONEN
DÉRIVÉS DE CHROMEN-4-ONE CONTENANT N POUR LE TRAITEMENT ET LA PROPHYLAXIE D'UNE INFECTION PAR LE VIRUS DE L'HÉPATITE B

(30) Priority: 14.12.2018 WO PCT/CN2018/121081
(43) Date of publication of application: 20.10.2021
(73) Proprietor: F. Hoffmann-La Roche AG, 4070 Basel (CH)
(72) Inventor: FENG, Song, Shanghai, 201203 (CN); ZHENG, Jiamin, Shanghai, 201203 (CN); LIU, Yongfu, Shanghai, 201203 (CN); CHEN, Dongdong, Shanghai, 201203 (CN); SHEN, Hong, Shanghai, 201203 (CN); TAN, Xuefei, Shanghai, 201203 (CN)
(74) Representative: Jochnowitz, Evan
(86) International application number: PCT/EP2019/084552
(87) International publication number: WO 2020/120528

(56) References cited:
- WO-A1-2017/202798
- ZHANG FAN ET AL: "A review of non-nucleoside anti-hepatitis B virus agents", EUROPEAN JOURNAL OF MEDICINAL CHEMISTRY, ELSEVIER, AMSTERDAM, NL, vol. 75, 30 January 2014 (2014-01-30), pages 267-281, XP028625376, ISSN: 0223-5234, DOI: 10.1016/J.EJMECH.2014.01.046

## Description

The present invention relates to organic compounds useful for therapy and/or prophylaxis of HBV infection in a mammal, and in particular to cccDNA (covalently closed circular DNA) inhibitors useful for treating HBV infection.

### FIELD OF THE INVENTION

The present invention relates to N-containing chromen-4-one derivatives having pharmaceutical activity, their manufacture, pharmaceutical compositions containing them and their potential use as medicaments.

The present invention relates to compounds of formula (I) 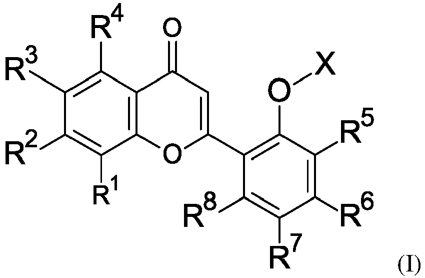 wherein R¹ to R⁸ and X are as described below, or a pharmaceutically acceptable salt thereof.

Hepatitis B virus (HBV) infection is one of the most prevalent viral infections and is a leading cause of chronic hepatitis. It is estimated that worldwide, around 2 billion people have evidence of past or present infection with HBV. Over 250 million individuals are currently chronically infected with HBV and are therefore at high risk to develop liver fibrosis, cirrhosis and hepatocellular carcinoma (HCC). There are data to indicate ~800,000 deaths per year are directly linked to HBV infection (Lozano, R. et al., Lancet (2012), 380 (9859), 2095-2128; Goldstein, S.T. et al., Int J Epidemiol (2005), 34 (6), 1329-1339).

Many countries in the world administer hepatitis B immunization starting at birth or in early childhood, which has greatly reduced the incidence and prevalence of hepatitis B in most endemic regions over the past few decades. However, the vaccination has no impact on people who were infected before the widespread use of the vaccine in developing end-stage liver disease or HCC (Chen, D.S., J Hepatol (2009), 50 (4), 805-816). Vaccination at birth of infants born to HBV positive mothers is usually not sufficient for protecting vertical transmission and combination with hepatitis B immune globulin is needed (Li, X.M. et al., World J Gastroenterol (2003), 9 (7), 1501-1503).

Eur J Med Chem. 2014 Mar 21;75:267-81 discloses that Hepatitis B Virus is the most common cause of chronic liver disease worldwide.

WO 2017/202798 discloses novel xanthone derivatives having pharmaceutical activity, their manufacture, pharmaceutical compositions containing them and their potential use as medicaments.

Currently FDA-approved treatments for chronic hepatitis B include two type 1 interferons (IFN) which are IFNalfa-2b and pegylated IFN alfa-2a and six nucleos(t)ide analogues (NAs) which are lamivudine (3TC), tenofovir disoproxil fumarate (TDF), adefovir (ADV), telbivudine (LdT), entecavir (ETV), and vemlidy (tenofovir alafenamide (TAF)). IFN treatment is finite, but it is known to have severe side effects, and only a small percentage of patients showed a sustained virological response, measured as loss of hepatitis B surface antigen (HBsAg). NAs are inhibitors of the HBV reverse transcriptase, profoundly reduce the viral load in vast majority of treated patients, and lead to improvement of liver function and reduced incidence of liver failure and hepatocellular carcinoma. However, the treatment of NAs is infinite (Ahmed, M. et al., Drug Discov Today (2015), 20 (5), 548-561; Zoulim, F. and Locarnini, S., Gastroenterology (2009), 137 (5), 1593-1608 e1591-1592).

HBV chronic infection is caused by persistence of covalently closed circular (ccc)DNA, which exists as an episomal form in hepatocyte nuclei. cccDNA serves as the template for viral RNA transcription and subsequent viral DNA generation. Only a few copies of cccDNA per liver cell can establish or re-initiate viral replication. Therefore, a complete cure of chronic hepatitis B will require elimination of cccDNA or permanently silencing of cccDNA. However, cccDNA is intrinsically very stable and currently available therapeutics could not eliminate cccDNA or permanently silence cccDNA (Nassal, M., Gut (2015), 64 (12), 1972-1984; Gish, R.G. et al., Antiviral Res (2015), 121, 47-58; Levrero, M. et al., J Hepatol (2009), 51 (3), 581-592.). The current SoC could not eliminate the cccDNA which are already present in the infected cells. There is an urgent need to discover and develop new anti-HBV reagents to eliminate or permanently silence cccDNA, the source of chronicity (Ahmed, M. et al., Drug Discov Today (2015), 20 (5), 548-561; Nassal, M., Gut (2015), 64 (12), 1972-1984).

### SUMMARY OF THE INVENTION

Objects of the present invention are compounds of formula (I), their manufacture, medicaments based on a compound in accordance with the invention and their production as well as the the compounds of formula (I) for use as cccDNA inhibitors and for use in the treatment or prophylaxis of HBV infection. The compounds of formula (I) show superior anti-HBV activity. In addition, the compounds of formula (I) also show good PK profiles.

The present invention relates to a compound of formula (I) 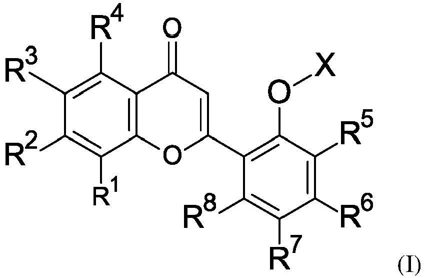 wherein
- R¹: is halogen;
- R²: is selected from H, OH, halogen, C₁₋₆alkyl, haloC₁₋₆alkyl and C₁₋₆alkoxy;
- R³: is selected from H, OH, halogen, C₁₋₆alkyl, haloC₁₋₆alkyl and C₁₋₆alkoxy;
- R⁴: is selected from H, OH, halogen, C₁₋₆alkyl, haloC₁₋₆alkyl and C₁₋₆alkoxy;
- R⁵: is selected from H, OH, halogen, C₁₋₆alkyl, haloC₁₋₆alkyl and C₁₋₆alkoxy;
- R⁶: is selected from H, OH, halogen, C₁₋₆alkyl, haloC₁₋₆alkyl and C₁₋₆alkoxy;
- R⁷: is selected from H, OH, halogen, C₁₋₆alkyl, haloC₁₋₆alkyl and C₁₋₆alkoxy;
- R⁸: is selected from H, OH, halogen, C₁₋₆alkyl, haloC₁₋₆alkyl and C₁₋₆alkoxy;
- X: is 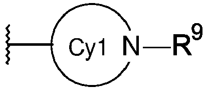 or -L-Y; wherein
Cy1 is N-containing heterocyclyl;
R⁹ is selected from C₃₋₇cycloalkylsulfonyl, carboxycarbonyl, carboxyphenylC₁₋₆alkyl and carboxyC₃₋₇cycloalkyl;
L is selected from C₁₋₆alkyl, C₁₋₆alkoxyC₁₋₆alkyl, C₃₋₇cycloalkyl, C₃₋₇cycloalkylC₁₋₆alkyl and C₁₋₆alkylheterocyclylC₁₋₆alkyl; wherein C₁₋₆alkyl is unsubstituted or substituted by OH;
Y is -NR¹⁰R¹¹ or 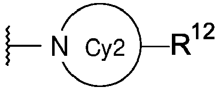 ; wherein
Cy2 is N-containing heterocyclyl; wherein N-containing heterocyclyl is unsubstituted or substituted by one or two or three substituents independently selected from halogen, C₁₋₆alkyl and OH;
R¹⁰ is selected from H, C₁₋₆alkyl, C₃₋₇cycloalkyl, C₁₋₆alkylsulfonyl, C₃₋₇cycloalkylsulfonyl, carboxyC₁₋₆alkyl, phenylsulfonyl and C₁₋₆alkylcarbonyl;
R¹¹ is selected from carboxyC₃₋₇cycloalkyl, carboxyC₁₋₆alkyl, carboxycarbonyl, carboxyheterocyclyl, carboxyC₃₋₇cycloalkylC₁₋₆alkyl, heterocyclyl, C₁₋₆alkylsulfonylC₁₋₆alkyl, aminosulfonylC₁₋₆alkyl, C₃₋₇cycloalkylsulfonyl, C₁₋₆alkoxycarbonylcarbonyl, phenylsulfonyl, C₁₋₆alkylcarbonyl, C₁₋₆alkoxycarbonyl, C₁₋₆alkylaminosulfonyl, aminocarbonylcarbonyl, C₃₋₇cycloalkylaminocarbonylcarbonyl, C₃₋₇cycloalkylsulfonylaminocarbonylcarbonyl, hydroxyheterocyclylcarbonylcarbonyl, carboxyC₁₋₆alkylaminocarbonyl, C₁₋₆alkylphenylsulfonylaminocarbonyl, aminocarbonyl, aminosulfonyl and aminocarbonylC₁₋₆alkyl;
R¹² is selected from H, carboxy, carboxyC₁₋₆alkyl, C₁₋₆alkylsulfonylaminocarbonyl, C₃₋₇cycloalkylsulfonylaminocarbonyl, C₁₋₆alkyl(C₁₋₆alkylsulfonyl)amino, C₁₋₆alkylsulfonyl, C₁₋₆alkylcarbonyl, C₁₋₆alkylsulfinyl, C₁₋₆alkylsulfonimidoyl, aminocarbonyl, carboxycarbonyl and heterocyclyl;
or a pharmaceutically acceptable salt thereof.

### DETAILED DESCRIPTION OF THE INVENTION

### DEFINITIONS

As used herein, the term "C₁₋₆alkyl" alone or in combination signifies a saturated, linear- or branched chain alkyl group containing 1 to 6, particularly 1 to 4 carbon atoms, for example methyl, ethyl, propyl, isopropyl, butyl, isobutyl, *tert*-butyl and the like. Particular "C₁₋₆alkyl" groups are methyl, ethyl, propyl, isopropyl and isobutyl. Most particular "C₁₋₆alkyl" group is methyl.

The term "C₁₋₆alkoxy" alone or in combination signifies a group C₁₋₆alkyl-O-, wherein the "C₁₋₆alkyl" is as defined above; for example methoxy, ethoxy, propoxy, *iso*-propoxy, *n*-butoxy, *iso*-butoxy, 2-butoxy, *tert*-butoxy, pentoxy, hexyloxy and the like. Particular "C₁₋₆alkoxy" groups are methoxy, ethoxy and *iso*-butoxy.

The term "C₃₋₇cycloalkyl" denotes to a saturated carbon mono or bicyclic ring or a saturated spiro- linked bicyclic carbon ring or a bridged carbon ring, containing from 3, 4, 5, 6, or 7 carbon atoms, particularly from 3 to 6 carbon atoms, for example, cyclopropyl, cyclobutyl, cyclopentyl, cyclohexyl, cycloheptyl, bicyclo[1.1.1]pentanyl and the like. Particular "C₃₋₇cycloalkyl" group is cyclopropyl or cyclobutyl.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group is replaced by same or different halogen atoms, particularly fluoro atoms. Examples of haloC₁₋₆alkyl include monochloro-, difluoro-or trifluoro-methyl, -ethyl or - propyl, for example difluoromethyl and trifluoromethyl.

The term "haloC₁₋₆alkoxy" denotes a C₁₋₆alkoxy group wherein at least one of the hydrogen atoms of the C₁₋₆alkoxy group is replaced by same or different halogen atoms, particularly fluoro atoms. Examples of haloC₁₋₆alkoxy include monofluoro-, difluoro- or trifluoro-methoxy, -ethoxy or -propoxy, for example trifluoromethoxy.

The term "heterocyclyl" refers to any mono-, bi-, tricyclic or spiro, saturated or unsaturated, aromatic (heteroaryl) or non-aromatic (e.g., heterocycloalkyl), ring system, having 3 to 20 ring atoms, where the ring atoms are carbon, and at least one atom in the ring or ring system is a heteroatom selected from nitrogen, sulfur or oxygen. If any ring atom of a cyclic system is a heteroatom, that system is a heterocyclyl, regardless of the point of attachment of the cyclic system to the rest of the molecule. In one example, heterocyclyl includes 3-11 ring atoms ("members") and includes monocycles, bicycles, tricycles and spiro ring systems, wherein the ring atoms are carbon, where at least one atom in the ring or ring system is a heteroatom selected from nitrogen, sulfur or oxygen. In one example, heterocyclyl includes 3- to 7-membered monocycles having 1, 2, 3 or 4 heteroatoms selected from nitrogen, sulfur or oxygen. In another example, heterocyclyl includes 4-, 5- or 6-membered monocycles having 1, 2, 3 or 4 heteroatoms selected from nitrogen, sulfur or oxygen. In one example, heterocyclyl includes 8- to 12-membered bicycles having 1, 2, 3, 4, 5 or 6 heteroatoms selected from nitrogen, sulfur or oxygen. In another example, heterocyclyl includes 9- or 10-membered bicycles having 1, 2, 3, 4, 5 or 6 heteroatoms selected from nitrogen, sulfur or oxygen. Examplary heterocyclyls are oxetanyl, pyrrolidinyl, morpholinyl, azetidinyl, piperidyl, azabicyclo[3.2.1]octanyl, oxopyrrolidinyl, piperazinyl, thiomorpholinyl, dioxothiazinanyl, oxoimidazolidinyl, dioxoimidazolidinyl, tetrahydropyranyl and 2H-tetrazolyl.

The term "carbonyl" alone or in combination refers to the group -C(O)-.

The term "sulfonyl" alone or in combination refers to the group -S(O)₂-.

The term "sulfonimidoyl" alone or in combination refers to the group -S(O)(NH)-, whose formula is

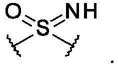

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula (I) and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as *p*-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435. Particular are the sodium salts of the compounds of formula (I).

Compounds of the general formula (I) which contain one or several chiral centers can either be present as racemates, diastereomeric mixtures, or optically active single isomers. The racemates can be separated according to known methods into the enantiomers. Particularly, diastereomeric salts which can be separated by crystallization are formed from the racemic mixtures by reaction with an optically active acid such as e.g. D- or L-tartaric acid, mandelic acid, malic acid, lactic acid or camphorsulfonic acid.

### HBV cccDNA INHIBITORS

The present invention provides (i) a compound having the general formula (I): 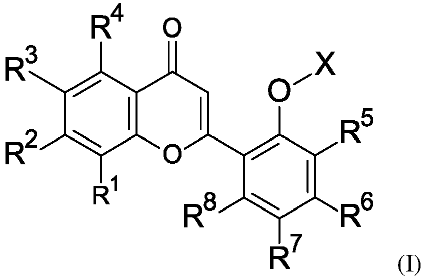 wherein
- R¹: is halogen;
- R²: is selected from H, OH, halogen, C₁₋₆alkyl, haloC₁₋₆alkyl and C₁₋₆alkoxy;
- R³: is selected from H, OH, halogen, C₁₋₆alkyl, haloC₁₋₆alkyl and C₁₋₆alkoxy;
- R⁴: is selected from H, OH, halogen, C₁₋₆alkyl, haloC₁₋₆alkyl and C₁₋₆alkoxy;
- R⁵: is selected from H, OH, halogen, C₁₋₆alkyl, haloC₁₋₆alkyl and C₁₋₆alkoxy;
- R⁶: is selected from H, OH, halogen, C₁₋₆alkyl, haloC₁₋₆alkyl and C₁₋₆alkoxy;
- R⁷: is selected from H, OH, halogen, C₁₋₆alkyl, haloC₁₋₆alkyl and C₁₋₆alkoxy;
- R⁸: is selected from H, OH, halogen, C₁₋₆alkyl, haloC₁₋₆alkyl and C₁₋₆alkoxy;
- X: is 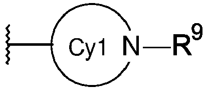 or -L-Y; wherein
Cy1 is N-containing heterocyclyl;
R⁹ is selected from C₃₋₇cycloalkylsulfonyl, carboxycarbonyl, carboxyphenylC₁₋₆alkyl and carboxyC₃₋₇cycloalkyl;
L is selected from C₁₋₆alkyl, C₁₋₆alkoxyC₁₋₆alkyl, C₃₋₇cycloalkyl, C₃₋₇cycloalkylC₁₋₆alkyl and C₁₋₆alkylheterocyclylC₁₋₆alkyl; wherein C₁₋₆alkyl is unsubstituted or substituted by OH;
Y is -NR¹⁰R¹¹ or 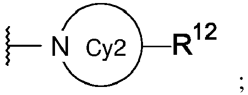 wherein
Cy2 is N-containing heterocyclyl; wherein N-containing heterocyclyl is unsubstituted or substituted by one or two or three substituents independently selected from halogen, C₁₋₆alkyl and OH;
R¹⁰ is selected from H, C₁₋₆alkyl, C₃₋₇cycloalkyl, C₁₋₆alkylsulfonyl, C₃₋₇cycloalkylsulfonyl, carboxyC₁₋₆alkyl, phenylsulfonyl and C₁₋₆alkylcarbonyl;
R¹¹ is selected from carboxyC₃₋₇cycloalkyl, carboxyC₁₋₆alkyl, carboxycarbonyl, carboxyheterocyclyl, carboxyC₃₋₇cycloalkylC₁₋₆alkyl, heterocyclyl, C₁₋₆alkylsulfonylC₁₋₆alkyl, aminosulfonylC₁₋₆alkyl, C₃₋₇cycloalkylsulfonyl, C₁₋₆alkoxycarbonylcarbonyl, phenylsulfonyl, C₁₋₆alkylcarbonyl, C₁₋₆alkoxycarbonyl, C₁₋₆alkylaminosulfonyl, aminocarbonylcarbonyl, C₃₋₇cycloalkylaminocarbonylcarbonyl, C₃₋₇cycloalkylsulfonylaminocarbonylcarbonyl, hydroxyheterocyclylcarbonylcarbonyl, carboxyC₁₋₆alkylaminocarbonyl, C₁₋₆alkylphenylsulfonylaminocarbonyl, aminocarbonyl, aminosulfonyl and aminocarbonylC₁₋₆alkyl;
R¹² is selected from H, carboxy, carboxyC₁₋₆alkyl, C₁₋₆alkylsulfonylaminocarbonyl, C₃₋₇cycloalkylsulfonylaminocarbonyl, C₁₋₆alkyl(C₁₋₆alkylsulfonyl)amino, C₁₋₆alkylsulfonyl, C₁₋₆alkylcarbonyl, C₁₋₆alkylsulfinyl, C₁₋₆alkylsulfonimidoyl, aminocarbonyl, carboxycarbonyl and heterocyclyl;
or a pharmaceutically acceptable salt thereof.

A further embodiment of the present invention is (ii) a compound of formula (I) according to (i), wherein
- R¹: is halogen;
- R²: is H;
- R³: is selected from H, halogen and C₁₋₆alkoxy;
- R⁴: is selected from H and haloC₁₋₆alkyl;
- R⁵: is H;
- R⁶: is selected from H, halogen, C₁₋₆alkyl, haloC₁₋₆alkyl and C₁₋₆alkoxy;
- R⁷: is selected from H, halogen, C₁₋₆alkyl and C₁₋₆alkoxy;
- R⁸: is selected from H, halogen and C₁₋₆alkoxy;
- X: is 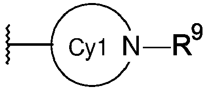 or -L-Y; wherein
Cy1 is selected from piperidyl, pyrrolidinyl and azetidinyl;
R⁹ is selected from C₃₋₇cycloalkylsulfonyl, carboxycarbonyl, carboxyphenylC₁₋₆alkyl and carboxyC₃₋₇cycloalkyl;
L is selected from C₁₋₆alkyl, C₁₋₆alkoxyC₁₋₆alkyl, C₃₋₇cycloalkyl, C₃₋₇cycloalkylC₁₋₆alkyl and C₁₋₆alkyloxetanylC₁₋₆alkyl; wherein C₁₋₆alkyl is unsubstituted or substituted by OH;
Y is -NR¹⁰R¹¹ or 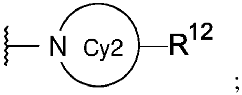 wherein
Cy2 is selected from pyrrolidinyl, morpholinyl, azetidinyl, piperidyl, azabicyclo[3.2.1]octanyl, oxopyrrolidinyl, piperazinyl, thiomorpholinyl, dioxothiazinanyl, oxoimidazolidinyl and dioxoimidazolidinyl; wherein pyrrolidinyl is unsubstituted or substituted by one or two substituents independently selected from halogen, C₁₋₆alkyl and OH;
R¹⁰ is selected from H, C₁₋₆alkyl, C₃₋₇cycloalkyl, C₁₋₆alkylsulfonyl, C₃₋₇cycloalkylsulfonyl, carboxyC₁₋₆alkyl, phenylsulfonyl and C₁₋₆alkylcarbonyl;
R¹¹ is selected from carboxyC₃₋₇cycloalkyl, carboxyC₁₋₆alkyl, carboxycarbonyl, carboxytetrahydropyranyl, carboxyC₃₋₇cycloalkylC₁₋₆alkyl, dioxothiazinanyl, C₁₋₆alkylsulfonylC₁₋₆alkyl, aminosulfonylC₁₋₆alkyl, C₃₋₇cycloalkylsulfonyl, C₁₋₆alkoxycarbonylcarbonyl, phenylsulfonyl, C₁₋₆alkylcarbonyl, C₁₋₆alkoxycarbonyl, C₁₋₆alkylaminosulfonyl, aminocarbonylcarbonyl, C₃₋₇cycloalkylaminocarbonylcarbonyl, C₃₋₇cycloalkylsulfonylaminocarbonylcarbonyl, hydroxypyrrolidinylcarbonylcarbonyl, carboxyC₁₋₆alkylaminocarbonyl, C₁₋₆alkylphenylsulfonylaminocarbonyl, aminocarbonyl, aminosulfonyl and aminocarbonylC₁₋₆alkyl;
R¹² is selected from H, carboxy, carboxyC₁₋₆alkyl, C₁₋₆alkylsulfonylaminocarbonyl, C₃₋₇cycloalkylsulfonylaminocarbonyl, C₁₋₆alkyl(C₁₋₆alkylsulfonyl)amino, C₁₋₆alkylsulfonyl, C₁₋₆alkylcarbonyl, C₁₋₆alkylsulfinyl, C₁₋₆alkylsulfonimidoyl, aminocarbonyl, carboxycarbonyl and 2H-tetrazolyl;
or a pharmaceutically acceptable salt thereof.

A further embodiment of the present invention is (iii) a compound of formula (I) according to (i), wherein
- R¹: is Cl.,
- R²: is H;
- R³: is selected from H, F and methoxy;
- R⁴: is selected from H and CF₃;
- R⁵: is H;
- R⁶: is selected from H, Cl, Br, methyl, CF₃, methoxy, ethoxy and isobutoxy;
- R⁷: is selected from H, Br, methyl and methoxy;
- R⁸: is selected from H, F and methoxy;
- X: is 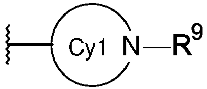 or -L-Y; wherein
Cy1 is selected from piperidyl, pyrrolidinyl and azetidinyl;
R⁹ is selected from cyclopropylsulfonyl, carboxycarbonyl, carboxyphenylmethyl and carboxycyclobutyl;
L is selected from ethyl, propyl, isobutyl, ethoxyethyl, cyclobutyl, cyclopropylmethyl and methyloxetanylmethyl; wherein propyl is unsubstituted or substituted one time by OH;
Y is -NR¹⁰R¹¹ or 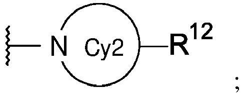 wherein
Cy2 is selected from pyrrolidinyl, morpholinyl, azetidinyl, piperidyl, azabicyclo[3.2.1]octanyl, oxopyrrolidinyl, piperazinyl, thiomorpholinyl, dioxothiazinanyl, oxoimidazolidinyl and dioxoimidazolidinyl; wherein pyrrolidinyl is unsubstituted or substituted by one or two substituents independently selected from F, methyl, isopropyl and OH;
R¹⁰ is selected from H, methyl, cyclopropyl, ethylsulfonyl, cyclopropylsulfonyl, carboxymethyl, phenylsulfonyl and methylcarbonyl;
R¹¹ is selected from carboxycyclobutyl, carboxycyclopentyl, carboxyisobutyl, carboxycarbonyl, carboxydimethylbutyl, carboxybicyclo[1.1.1]pentanyl, carboxycyclohexyl, carboxyethyl, carboxyisopropyl, carboxytetrahydropyranyl, carboxycyclohexylethyl, dioxothiazinanyl, methylsulfonylethyl, aminosulfonylethyl, cyclopropylsulfonyl, ethoxycarbonylcarbonyl, phenylsulfonyl, methylcarbonyl, ethylcarbonyl, methoxycarbonyl, ethoxycarbonyl, methylaminosulfonyl, carboxymethyl, ethylaminosulfonyl, aminocarbonylcarbonyl, cyclopropylaminocarbonylcarbonyl, cyclopropylsulfonylaminocarbonylcarbonyl, hydroxypyrrolidinylcarbonylcarbonyl, carboxyethylaminocarbonyl, carboxymethylaminocarbonyl, methylphenylsulfonylaminocarbonyl, aminocarbonyl, aminosulfonyl, aminocarbonylmethyl and aminocarbonylethyl;
R¹² is selected from H, carboxy, carboxymethyl, methylsulfonylaminocarbonyl, cyclopropylsulfonylaminocarbonyl, methyl(methylsulfonyl)amino, methylsulfonyl, methylcarbonyl, methylsulfinyl, methylsulfonimidoyl, aminocarbonyl, carboxycarbonyl and 2H-tetrazolyl;
or a pharmaceutically acceptable salt thereof.

A further embodiment of the present invention is (iv) a compound of formula (I) according to (i), or a pharmaceutically acceptable salt thereof, wherein R³ is selected from H.

A further embodiment of the present invention is (v) a compound of formula (I) according to (i), or a pharmaceutically acceptable salt thereof, wherein R⁶ is selected from halogen, C₁₋₆alkyl and haloC₁₋₆alkyl.

A further embodiment of the present invention is (vi) a compound of formula (I) according to (i), or a pharmaceutically acceptable salt thereof, wherein R⁶ is selected from Br, methyl and CF₃.

A further embodiment of the present invention is (vii) a compound of formula (I) according to (i), or a pharmaceutically acceptable salt thereof, R⁷ is selected from H and C₁₋₆alkoxy.

A further embodiment of the present invention is (viii) a compound of formula (I) according to (i), or a pharmaceutically acceptable salt thereof, wherein R⁷ is selected from H and methoxy.

A further embodiment of the present invention is (ix) a compound of formula (I) according to (viii), or a pharmaceutically acceptable salt thereof, wherein X is -L-Y.

A further embodiment of the present invention is (x) a compound of formula (I) according to (i), or a pharmaceutically acceptable salt thereof, wherein L is selected from C₁₋₆alkyl and C₁₋₆alkoxyC₁₋₆alkyl; wherein C₁₋₆alkyl is unsubstituted or substituted by OH.

A further embodiment of the present invention is (xi) a compound of formula (I) according to (i), or a pharmaceutically acceptable salt thereof, wherein L is selected from ethyl, propyl and ethoxyethyl; wherein propyl is unsubstituted or substituted one time by OH.

A further embodiment of the present invention is (xii) a compound of formula (I) according to (i), or a pharmaceutically acceptable salt thereof, wherein Y is 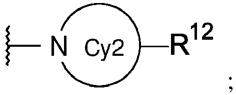 wherein Cy2 is selected from pyrrolidinyl and morpholinyl.

A further embodiment of the present invention is (xiii) a compound of formula (I) according to (i), or a pharmaceutically acceptable salt thereof, wherein R¹² is selected from carboxy, C₁₋₆alkylsulfonylaminocarbonyl and C₁₋₆alkylsulfonyl.

A further embodiment of the present invention is (xiv) a compound of formula (I) according to (i), or a pharmaceutically acceptable salt thereof, wherein R¹² is selected from carboxy, methylsulfonylaminocarbonyl and methylsulfonyl.

A further embodiment of the present invention is (xv) a compound of formula (I) according to (i), or a pharmaceutically acceptable salt thereof, wherein Y is -NHR¹¹.

A further embodiment of the present invention is (xvi) a compound of formula (I) according to (i), or a pharmaceutically acceptable salt thereof, wherein R¹¹ is selected from carboxyC₃₋₇cycloalkyl, carboxycarbonyl, carboxyC₁₋₆alkylaminocarbonyl, aminocarbonyl and aminosulfonyl.

A further embodiment of the present invention is (xvii) a compound of formula (I) according to (i), or a pharmaceutically acceptable salt thereof, wherein R¹¹ is selected from carboxycyclobutyl, carboxycyclopentyl, carboxycarbonyl, carboxyethylaminocarbonyl, aminocarbonyl and aminosulfonyl.

A further embodiment of the present invention is (xviii) a compound of formula (II) according to (i), or a pharmaceutically acceptable salt thereof, 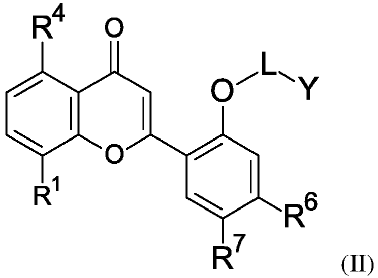
- R¹: is halogen;
- R⁴: is selected from H and haloC₁₋₆alkyl;
- R⁶: is selected from halogen, C₁₋₆alkyl and haloC₁₋₆alkyl;
- R⁷: is selected from H and C₁₋₆alkoxy;
- L: is selected from C₁₋₆alkyl and C₁₋₆alkoxyC₁₋₆alkyl; wherein C₁₋₆alkyl is unsubstituted or substituted by OH;
- Y: is -NHR¹¹ or 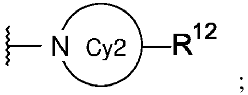 wherein
Cy2 is selected from pyrrolidinyl and morpholinyl;
R¹¹ is selected from carboxyC₃₋₇cycloalkyl, carboxycarbonyl, carboxyC₁₋₆alkylaminocarbonyl, aminocarbonyl and aminosulfonyl;
R¹² is selected from carboxy, C₁₋₆alkylsulfonylaminocarbonyl and C₁₋₆alkylsulfonyl.

A further embodiment of the present invention is (xix) a compound of formula (II) according to (i), or a pharmaceutically acceptable salt thereof, wherein
- R¹: is Cl.,
- R⁴: is selected from H and CF₃;
- R⁶: is selected from Br, methyl and CF₃;
- R⁷: is selected from H and methoxy;
- L: is selected from ethyl, propyl and ethoxyethyl; wherein propyl is unsubstituted or substituted one time by OH;
- Y: is -NHR¹¹ or 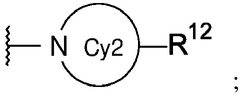 wherein
Cy2 is selected from pyrrolidinyl and morpholinyl;
R¹¹ is selected from carboxycyclobutyl, carboxycyclopentyl, carboxycarbonyl, carboxyethylaminocarbonyl, aminocarbonyl and aminosulfonyl;
R¹² is selected from carboxy, methylsulfonylaminocarbonyl and methylsulfonyl.

In another embodiment (xx) of the present invention, particular compounds of the present invention are selected from:
1-[3-[5-bromo-2-(8-chloro-4-oxo-chromen-2-yl)phenoxylpropyl]pyrrolidine-3-carboxylic acid;
4-[3-[5-bromo-2-(8-chloro-4-oxo-chromen-2-yl)phenoxy]propyl]morpholine-2-carboxylic acid;
(2R)-1-[3-[5-bromo-2-(8-chloro-4-oxo-chromen-2-yl)phenoxy]propyl]pyrrolidine-2-carboxylic acid;
(2S)-1-[3-[5-bromo-2-(8-chloro-4-oxo-chromen-2-yl)phenoxy]propyl]pyrrolidine-2-carboxylic acid;
(3R)-1-[3-[5-bromo-2-(8-chloro-4-oxo-chromen-2-yl)phenoxy]propyl]pyrrolidine-3-carboxylic acid;
3-[3-[2-(8-chloro-4-oxo-chromen-2-yl)-5-(trifluoromethyl)phenoxy]propylamino]cyclobutanecarboxylic acid;
3-[3-[2-(8-chloro-4-oxo-chromen-2-yl)-5-(trifluoromethyl)phenoxy]propylamino]cyclopentanecarboxylic acid;
2-[1-[3-[2-(8-chloro-4-oxo-chromen-2-yl)-5-(trifluoromethyl)phenoxy]propyl]pyrrolidin-3-yl]acetic acid;
(2R)-2-[3-[2-(8-chloro-4-oxo-chromen-2-yl)-5-(trifluoromethyl)phenoxy]propylamino]-3-methyl-butanoic acid;
2-[[3-[[2-(8-chloro-4-oxo-chromen-2-yl)-5-(trifluoromethyl)phenoxy]methyl]oxetan-3-yl]methylamino]-2-oxo-acetic acid;
2-[3-[2-(8-chloro-4-oxo-chromen-2-yl)-5-(trifluoromethyl)phenoxy]propylamino]-4,4-dimethyl-pentanoic acid;
1-[3-[2-(8-chloro-4-oxo-chromen-2-yl)-5-(trifluoromethyl)phenoxy]propyl]pyrrolidine-3-carboxylic acid;
(3R)-1-[3-[2-(8-chloro-4-oxo-chromen-2-yl)-5-(trifluoromethyl)phenoxy]propyl]pyrrolidine-3-carboxylic acid;
(3S)-1-[3-[2-(8-chloro-4-oxo-chromen-2-yl)-5-(trifluoromethyl)phenoxy]propyl]pyrrolidine-3-carboxylic acid;
(3S)-1-[3-[2-(8-chloro-4-oxo-chromen-2-yl)-5-ethoxy-phenoxy]propyl]pyrrolidine-3-carboxylic acid;
(3R)-1-[3-[5-bromo-2-(8-chloro-4-oxo-chromen-2-yl)-4-methoxy-phenoxy]propyl]pyrrolidine-3-carboxylic acid;
(3S)-1-[3-[5-bromo-2-(8-chloro-4-oxo-chromen-2-yl)-4-methoxy-phenoxy]propyl]pyrrolidine-3-carboxylic acid;
4-[3-[2-(8-chloro-4-oxo-chromen-2-yl)-5-(trifluoromethyl)phenoxy]propyl] morpholine-2-carboxylic acid;
1-[2-[5-bromo-2-(8-chloro-4-oxo-chromen-2-yl)phenoxy]ethyl]azetidine-3-carboxylic acid;
1-[2-[5-bromo-2-(8-chloro-4-oxo-chromen-2-yl)phenoxy]ethyl]pyrrolidine-2-carboxylic acid;
1-[2-[5-bromo-2-(8-chloro-4-oxo-chromen-2-yl)phenoxy]ethyl]pyrrolidine-3-carboxylic acid;
1-[2-[5-bromo-2-(8-chloro-4-oxo-chromen-2-yl)phenoxy]ethyl]piperidine-4-carboxylic acid;
4-[2-[5-bromo-2-(8-chloro-4-oxo-chromen-2-yl)phenoxy]ethyl]morpholine-2-carboxylic acid;
(3S,4S)-1-[2-[5-bromo-2-(8-chloro-4-oxo-chromen-2-yl)phenoxy]ethyl]-4-methyl-pyrrolidine-3-carboxylic acid;
8-[2-[5-bromo-2-(8-chloro-4-oxo-chromen-2-yl)phenoxy]ethyl]-8-azabicyclo[3.2.1]octane-3-carboxylic acid;
3-[2-[5-bromo-2-(8-chloro-4-oxo-chromen-2-yl)phenoxy]ethylamino]bicyclo[1.1.1]pentane-1-carboxylic acid;
1-[2-[5-bromo-2-(8-chloro-4-oxo-chromen-2-yl)phenoxy]ethyl]-4,4-difluoro-pyrrolidine-2-carboxylic acid;
1-[2-[2-(8-chloro-4-oxo-chromen-2-yl)phenoxy]ethyl]piperidine-4-carboxylic acid;
(2R)-1-[2-[2-(8-chloro-4-oxo-chromen-2-yl)phenoxy]ethyl]pyrrolidine-2-carboxylic acid;
1-[2-[2-(8-chloro-4-oxo-chromen-2-yl)phenoxy]ethyl]pyrrolidine-3-carboxylic acid;
(2S)-1-[2-[2-(8-chloro-4-oxo-chromen-2-yl)phenoxy]ethyl]pyrrolidine-2-carboxylic acid;
1-[2-[2-(8-chloro-4-oxo-chromen-2-yl)phenoxylethyl]azetidine-3-carboxylic acid;
(3S)-1-[2-[2-(8-chloro-4-oxo-chromen-2-yl)-5-(trifluoromethyl)phenoxy]ethyl]pyrrolidine-3-carboxylic acid;
4-[2-[2-(8-chloro-4-oxo-chromen-2-yl)-5-(trifluoromethyl)phenoxylethyl]morpholine-2-carboxylic acid;
(2R)-2-[2-[2-(8-chloro-4-oxo-chromen-2-yl)-5-(trifluoromethyl)phenoxy]ethylamino]-3-methylbutanoic acid;
(2S)-2-[2-[2-(8-chloro-4-oxo-chromen-2-yl)-5-(trifluoromethyl)phenoxy]ethylamino]-3-methylbutanoic acid;
3-[2-[2-(8-chloro-4-oxo-chromen-2-yl)-5-(trifluoromethyl)phenoxy]ethylamino]cyclobutanecarboxylic acid;
(3R)-1-[2-[2-(8-chloro-4-oxo-chromen-2-yl)-5-isobutoxy-phenoxy]ethyl]pyrrolidine-3-carboxylic acid;
(3S)-1-[2-[2-(8-chloro-4-oxo-chromen-2-yl)-5-isobutoxy-phenoxy]ethyl]pyrrolidine-3-carboxylic acid;
(3R)-1-[2-[5-bromo-2-[8-chloro-4-oxo-5-(trifluoromethyl)chromen-2-yl]phenoxy]ethyl]pyrrolidine-3-carboxylic acid;
(3S)-1-[2-[5-bromo-2-[8-chloro-4-oxo-5-(trifluoromethyl)chromen-2-yl]phenoxy]ethyl]pyrrolidine-3-carboxylic acid;
1-[2-[2-(8-chloro-6-fluoro-4-oxo-chromen-2-yl)phenoxy]ethyl]pyrrolidine-3-carboxylic acid;
(2R)-1-[2-[5-bromo-2-(8-chloro-4-oxo-chromen-2-yl)-4-methyl-phenoxy]ethyl]pyrrolidine-2-carboxylic acid;
(2S)-1-[2-[5-chloro-2-(8-chloro-4-oxo-chromen-2-yl)-4-methyl-phenoxy]ethyl]pyrrolidine-2-carboxylic acid;
3- [3-[5-chloro-2-(8-chloro-4-oxo-chromen-2-yl)-4-methyl-phenoxy]propylamino]cyclobutanecarboxylic acid;
(3R)-1-[2-[2-[5-bromo-2-(8-chloro-4-oxo-chromen-2-yl)phenoxylethoxylethyl]pyrrolidine-3-carboxylic acid;
(2R)-1-[2-[2-[5-bromo-2-(8-chloro-4-oxo-chromen-2-yl)-4-methyl-phenoxy]ethoxy]ethyl]pyrrolidine-2-carboxylic acid;
4- [2- [2- [5-bromo-2-(8-chloro-4-oxo-chromen-2-yl)phenoxyl ethoxyl ethyl] morpholine-2-carboxylic acid;
(3R)-1-[2-[2-[2-(8-chloro-4-oxo-chromen-2-yl)-5-methoxy-4-methyl-phenoxy]ethoxy]ethyl]pyrrolidine-3-carboxylic acid;
(3S)-1-[2-[2-[2-(8-chloro-4-oxo-chromen-2-yl)-5-methoxy-4-methyl-phenoxy]ethoxy]ethyl]pyrrolidine-3-carboxylic acid;
4-[2-[5-bromo-2-(8-chloro-4-oxo-chromen-2-yl)phenoxy]ethyl]morpholine-3-carboxylic acid;
3-[2-[2-(8-chloro-4-oxo-chromen-2-yl)-5-methyl-phenoxy]ethylamino]cyclobutanecarboxylic acid;
1-[2-[2-(8-chloro-4-oxo-chromen-2-yl)-5-methyl-phenoxy]ethyl]azetidine-3-carboxylic acid;
cis-4-[2-[5-bromo-2-(8-chloro-4-oxo-chromen-2-yl)phenoxy] ethylamino] cyclohexanecarboxylic acid;
trans-4-[2-[5-bromo-2-(8-chloro-4-oxo-chromen-2-yl)phenoxy]ethylamino]cyclohexanecarboxylic acid;
2-[2-[2-(8-chloro-4-oxo-chromen-2-yl)-5-(trifluoromethyl)phenoxy]ethylamino]propanoic acid;
2-[2-[2-(8-chloro-4-oxo-chromen-2-yl)-5-methyl-phenoxy]ethylamino]-2-methyl-propanoic acid;
3-[3-[2-(8-chloro-4-oxo-chromen-2-yl)-5-methyl-phenoxy]propylamino]cyclobutanecarboxylic acid;
cis-4-[3-[5-bromo-2-(8-chloro-4-oxo-chromen-2-yl)phenoxy]propylamino]tetrahydropyran-2-carboxylic acid;
1-[2-[5-chloro-2-(8-chloro-4-oxo-chromen-2-yl)-4-methyl-phenoxy]ethyl]azetidine-3-carboxylic acid;
3-[3-[2-(8-chloro-4-oxo-chromen-2-yl)-5-(trifluoromethyl)phenoxy]propylamino]butanoic acid;
2-[3-[2-(8-chloro-4-oxo-chromen-2-yl)-5-(trifluoromethyl)phenoxy]propylamino]-3-cyclohexyl-propanoic acid;
(3R)-1-[2-[5-bromo-2-(8-chloro-4-oxo-chromen-2-yl)phenoxy]ethyl]-N-methylsulfonyl-pyrrolidine-3-carboxamide;
(3R)-1-[3-[2-(8-chloro-4-oxo-chromen-2-yl)-5-(trifluoromethyl)phenoxy]propyl]-N-methylsulfonyl-pyrrolidine-3-carboxamide;
(3S)-1-[3-[2-(8-chloro-4-oxo-chromen-2-yl)-5-(trifluoromethyl)phenoxy]propyl]-N-methylsulfonyl-pyrrolidine-3-carboxamide;
(3R)-1-[3-[2-(8-chloro-4-oxo-chromen-2-yl)-5-(trifluoromethyl)phenoxy]propyl]-N-cyclopropylsulfonyl-pyrrolidine-3-carboxamide;
4-[2-[2-(8-chloro-4-oxo-chromen-2-yl)-5-(trifluoromethyl)phenoxy]ethyl]-N-methylsulfonyl-morpholine-2-carboxamide;
(3S)-1-[2-[5-bromo-2-(8-chloro-4-oxo-chromen-2-yl)phenoxy]ethyl]-N-methylsulfonyl-pyrrolidine-3-carboxamide;
(3R)-1-[2-[2-(8-chloro-4-oxo-chromen-2-yl)-5-(trifluoromethyl)phenoxy]ethyl]-N-methylsulfonyl-pyrrolidine-3-carboxamide;
(3S)-1-[2-[2-(8-chloro-4-oxo-chromen-2-yl)-5-(trifluoromethyl)phenoxy]ethyl]-N-methylsulfonyl-pyrrolidine-3-carboxamide;
1-[2-[2-(8-chloro-4-oxo-chromen-2-yl)-5-(trifluoromethyl)phenoxy]ethyl]-N-cyclopropylsulfonyl-pyrrolidine-2-carboxamide;
1-[2-[2-(8-chloro-4-oxo-chromen-2-yl)-5-(trifluoromethyl)phenoxy]ethyl]-N-methylsulfonyl-pyrrolidine-2-carboxamide;
1-[2-[5-bromo-2-(8-chloro-4-oxo-chromen-2-yl)phenoxy]ethyl]-N-methylsulfonyl-pyrrolidine-2-carboxamide;
1-[2-[5-bromo-2-(8-chloro-4-oxo-chromen-2-yl)phenoxy]ethyl]pyrrolidin-2-one;
N-[1-[2-[5-bromo-2-(8-chloro-4-oxo-chromen-2-yl)phenoxy]ethyl]pyrrolidin-3-yl]-N-methyl-methane sulfonamide;
8-chloro-2-[2-[3-[(3R,4S)-3,4-dihydroxypyrrolidin- 1-yl]propoxy] -4-(trifluoromethyl)phenyl] chromen-4-one;
8-chloro-2-[2-[3-[(1,1-dioxothian-4-yl)amino]propoxy]-4-(trifluoromethyl)phenyl]chromen-4-one;
8-chloro-2-[2-[3-(2-methylsulfonylethylamino)propoxy]-4-(trifluoromethyl)phenyl]chromen-4-one;
2-[3-[2-(8-chloro-4-oxo-chromen-2-yl)-5-(trifluoromethyl)phenoxy]propylamino]ethanesulfonamide;
8-chloro-2-[2-[3-(4-methylsulfonylpiperazin-1-yl)propoxy]-4-(trifluoromethyl)phenyl]chromen-4-one;
2-[2-[2-(4-acetylpiperazin-1-yl)ethoxy]-4-bromo-phenyl]-8-chloro-chromen-4-one;
1-[2-[5-bromo-2-(8-chloro-4-oxo-chromen-2-yl)phenoxy]ethyl]pyrrolidine-3-carboxamide;
2-[4-bromo-2-[2-(3-methylsulfonylpyrrolidin-1-yl)ethoxy]phenyl]-8-chloro-chromen-4-one;
4-[2-[5-bromo-2-(8-chloro-4-oxo-chromen-2-yl)phenoxy]ethyl]thiomorpholine-3-carboxamide;
N-[3-[2-(8-chloro-4-oxo-chromen-2-yl)-5-(trifluoromethyl)phenoxy]-2-hydroxypropyl]cyclopropanesulfonamide;
8-chloro-2-[2-[2-[4-(1H-tetrazol-5-yl)-1-piperidyl]ethoxy]-4-(trifluoromethyl)phenyl]chromen-4-one;
8-chloro-2-[2-[3-(3-methylsulfonylpyrrolidin-1-yl)propoxy]-4-(trifluoromethyl)phenyl]chromen-4-one;
2-[4-bromo-2-[2-(1,1-dioxo-1,4-thiazinan-4-yl)ethoxy]phenyl]-8-chloro-chromen-4-one;
2-[4-bromo-2-[2-(2-morpholinoethoxy)ethoxy]phenyl]-8-chloro-chromen-4-one;
1-[2-[2-(8-chloro-4-oxo-chromen-2-yl)-5-methyl-phenoxy]ethyl]imidazolidin-2-one;
1-[2-[2-(8-chloro-4-oxo-chromen-2-yl)-5-methyl-phenoxy]ethyl]-3-methyl-imidazolidin-2-one;
8-chloro-2-[2-[2-(4-methylsulfinyl-1-piperidyl)ethoxy]-4-(trifluoromethyl)phenyl]chromen-4-one;
8-chloro-2-[2-[2-[4-(methylsulfonimidoyl)-1-piperidyl]ethoxy]-4-(trifluoromethyl)phenyl] chromen-4-one;
ethyl 2-[3-[2-(8-chloro-4-oxo-chromen-2-yl)-5-methyl-phenoxy]propylamino]-2-oxo-acetate;
N-[2-[2-(8-chloro-4-oxo-chromen-2-yl)-5-(trifluoromethyl)phenoxy]ethyl]cyclopropanesulfonamide;
N-[3-[2-(8-chloro-4-oxo-chromen-2-yl)-5-(trifluoromethyl)phenoxy]propyl]benzenesulfonamide;
N-[3-[2-(8-chloro-4-oxo-chromen-2-yl)-5-(trifluoromethyl)phenoxy]propyl] acetamide;
N-[3-[2-(8-chloro-4-oxo-chromen-2-yl)-5-(trifluoromethyl)phenoxy]propyl]propanamide;
ethyl 2-[3-[2-(8-chloro-4-oxo-chromen-2-yl)-5-(trifluoromethyl)phenoxy]propylamino]-2-oxo-acetate;
ethyl 2-[2-[2-(8-chloro-4-oxo-chromen-2-yl)-5-(trifluoromethyl)phenoxy]ethylamino]-2-oxo-acetate;
ethyl 2-[2-[4-bromo-2-(8-chloro-4-oxo-chromen-2-yl)-5-methoxy-phenoxy]ethylamino]-2-oxo-acetate;
ethyl 2-[2-[2-(8-chloro-6-methoxy-4-oxo-chromen-2-yl)-5-methyl-phenoxy]ethylamino]-2-oxo-acetate;
ethyl 2-[2-[2-(8-chloro-4-oxo-chromen-2-yl)-5-methoxy-phenoxy]ethylamino]-2-oxo-acetate; methyl N-[2-[5-bromo-2-(8-chloro-4-oxo-chromen-2-yl)phenoxy]ethyl]carbamate;
ethyl N-[2-[5-bromo-2-(8-chloro-4-oxo-chromen-2-yl)phenoxy]ethyl]carbamate;
8-chloro-2-[4-methyl-2-[3-(methylsulfamoylamino)propoxy]phenyl]chromen-4-one;
2-[4-bromo-2-[(1-cyclopropylsulfonyl-4-piperidyl)oxy]phenyl]-8-chloro-chromen-4-one;
ethyl 2-[2-[2-(8-chloro-4-oxo-chromen-2-yl)-5-methyl-phenoxy]ethylamino]-2-oxo-acetate;
2-[[1-[[2-(8-chloro-4-oxo-chromen-2-yl)-5-(trifluoromethyl)phenoxy]methyl]cyclopropyl]amino]-2-oxo-acetic acid;
2-[2-[2-(8-chloro-4-oxo-chromen-2-yl)-5-methoxy-phenoxy]ethylamino]-2-oxo-acetic acid;
2-[2-[2-(8-chloro-4-oxo-chromen-2-yl)-3-fluoro-5-methyl-phenoxy]ethylamino]-2-oxo-acetic acid;
2-[2-[5-bromo-2-(8-chloro-4-oxo-chromen-2-yl)phenoxy]ethylamino]-2-oxo-acetic acid;
2-[2-[2-(8-chloro-4-oxo-chromen-2-yl)-4,5-dimethoxy-phenoxy]ethylamino]-2-oxo-acetic acid;
2-[[2-[2-(8-chloro-4-oxo-chromen-2-yl)-5-(trifluoromethyl)phenoxy]-1,1-dimethylethyl]amino]-2-oxo-acetic acid;
2-[3-[2-(8-chloro-6-methoxy-4-oxo-chromen-2-yl)-5-(trifluoromethyl)phenoxy]propylamino]-2-oxo-acetic acid;
2-[3-[2-(8-chloro-4-oxo-chromen-2-yl)-5-(trifluoromethyl)phenoxy]propylamino]-2-oxo-acetic acid;
2-[2-[2-(8-chloro-4-oxo-chromen-2-yl)-3-methoxy-5-methyl-phenoxy]ethylamino]-2-oxo-acetic acid;
2-[2-[2-(8-chloro-4-oxo-chromen-2-yl)-5-(trifluoromethyl)phenoxy]ethylamino]-2-oxo-acetic acid;
2-[3-[2-(8-chloro-4-oxo-chromen-2-yl)-5-methyl-phenoxy]propylamino]-2-oxo-acetic acid;
2-[2-[2-(8-chloro-4-oxo-chromen-2-yl)-5-methyl-phenoxy]ethylamino]-2-oxo-acetic acid;
2-[2-[4-bromo-2-(8-chloro-4-oxo-chromen-2-yl)-5-methoxy-phenoxy]ethylamino]-2-oxo-acetic acid;
2-[2-[2-(8-chloro-4-oxo-chromen-2-yl)-5-(trifluoromethyl)phenoxy]ethylamino]acetic acid;
3-[2-[2-(8-chloro-4-oxo-chromen-2-yl)-5-(trifluoromethyl)phenoxy]ethylamino]propanoic acid;
2-[3-[2-(8-chloro-4-oxo-chromen-2-yl)-5-(trifluoromethyl)phenoxy]propylamino]acetic acid;
2-oxo-2-[(3S)-3-[2-(8-chloro-4-oxo-chromen-2-yl)-5-(trifluoromethyl)phenoxy]pyrrolidin-1-yl]acetic acid;
2-[3-[2-(8-chloro-4-oxo-chromen-2-yl)-5-(trifluoromethyl)phenoxy] azetidin-1-yl]-2-oxo-acetic acid;
3-[[4-[5-bromo-2-(8-chloro-4-oxo-chromen-2-yl)phenoxy]-1-piperidyl]methyl]benzoic acid;
2-[4-[2-(8-chloro-4-oxo-chromen-2-yl)-5-(trifluoromethyl)phenoxy]-1-piperidyl]-2-oxo-acetic acid;
2-oxo-2-[ (3R)-3-[2-(8-chloro-4-oxo-chromen-2-yl)-5-(trifluoromethyl)phenoxy]pyrrolidin-1-yl]acetic acid;
3-[4-[5-bromo-2-(8-chloro-4-oxo-chromen-2-yl)phenoxy]-1-piperidyl]cyclobutanecarboxylic acid;
2-[(3S)-3-[2-(8-chloro-4-oxo-chromen-2-yl)-5-methyl-phenoxy]pyrrolidin-1-yl]-2-oxo-acetic acid;
2-[[3-[2-(8-chloro-4-oxo-chromen-2-yl)-5-(trifluoromethyl)phenoxy]cyclobutyl]amino]-2-oxo-acetic acid;
2-[3-[2-(8-chloro-4-oxo-chromen-2-yl)-5-(trifluoromethyl)phenoxy]propyl-methyl-amino]acetic acid;
2-[2-[2-(8-chloro-4-oxo-chromen-2-yl)-5-methyl-phenoxy]ethyl-cyclopropyl-amino]-2-oxo-acetic acid;
8-chloro-2-[2-[2-[ethylsulfamoyl(methyl)amino]ethoxy]-4-methyl-phenyl]-4-oxo-chromene;
ethyl 2-[2-[2-(8-chloro-4-oxo-chromen-2-yl)-5-methyl-phenoxy]ethyl-methyl-amino]-2-oxo-acetate;
ethyl 2-[3-[2-(8-chloro-4-oxo-chromen-2-yl)-5-(trifluoromethyl)phenoxy]propyl-ethylsulfonyl-amino]-2-oxo-acetate;
3-[3-[2-(8-chloro-4-oxo-chromen-2-yl)-5-(trifluoromethyl)phenoxy]propyl-cyclopropylsulfonyl-amino]cyclobutanecarboxylic acid;
2-[carboxymethyl-[3-[2-(8-chloro-4-oxo-chromen-2-yl)-5-(trifluoromethyl)phenoxy]propyl] amino] acetic acid;
cis-3-[benzenesulfonyl-[3-[2-(8-chloro-4-oxo-chromen-2-yl)-5-(trifluoromethyl)phenoxy]propyl]amino]cyclobutanecarboxylic acid;
trans-3-[benzenesulfonyl-[3-[2-(8-chloro-4-oxo-chromen-2-yl)-5-(trifluoromethyl)phenoxy]propyl]amino]cyclobutanecarboxylic acid;
2-[benzenesulfonyl-[3-[2-(8-chloro-4-oxo-chromen-2-yl)-5-(trifluoromethyl)phenoxy]propyl] amino] acetic acid;
2-[acetyl-[3-[2-(8-chloro-4-oxo-chromen-2-yl)-5-(trifluoromethyl)phenoxy]propyl] amino] acetic acid;
2-[3-[2-(8-chloro-4-oxo-chromen-2-yl)-5-(trifluoromethyl)phenoxy]propyl-cyclopropylsulfonyl-amino]acetic acid;
N'-[2-[2-(8-chloro-4-oxo-chromen-2-yl)-5-(trifluoromethyl)phenoxy]ethyl]oxamide;
N'-[2-[2-(8-chloro-4-oxo-chromen-2-yl)-5-methoxy-phenoxy]ethyl]oxamide;
N-[2-[2-(8-chloro-4-oxo-chromen-2-yl)-5-(trifluoromethyl)phenoxy]ethyl]-N'-cyclopropyl-oxamide;
N-[3-[2-(8-chloro-4-oxo-chromen-2-yl)-5-(trifluoromethyl)phenoxy]propyl]-N'-cyclopropylsulfonyl-oxamide;
N-[2-[2-(8-chloro-4-oxo-chromen-2-yl)-5-methyl-phenoxylethyl]-2-[(3S)-3-hydroxypyrrolidin-1-yl]-2-oxo-acetamide;
(2S)-2-[2-[2-(8-chloro-4-oxo-chromen-2-yl)-5-methyl-phenoxy]ethylcarbamoylamino]propanoic acid;
(5S)-3-[2-[2-(8-chloro-4-oxo-chromen-2-yl)-5-methyl-phenoxy]ethyl]-5-isopropyl-imidazolidine-2,4-dione;
(5S)-3-[2-[2-(8-chloro-4-oxo-chromen-2-yl)-5-(trifluoromethyl)phenoxy]ethyl]-5-isopropyl-imidazolidine-2,4-dione;
2-[2-[2-(8-chloro-4-oxo-chromen-2-yl)-5-methyl-phenoxy]ethylcarbamoylamino]acetic acid;
(5S)-3-[2-[2-(8-chloro-4-oxo-chromen-2-yl)-5-(trifluoromethyl)phenoxy]ethyl]-5-methyl-imidazolidine-2,4-dione;
1-[3-[2-(8-chloro-4-oxo-chromen-2-yl)-5-methyl-phenoxy]propyl]-3-(p-tolylsulfonyl)urea;
3-[2-(8-chloro-4-oxo-chromen-2-yl)-5-(trifluoromethyl)phenoxy]propylurea;
1-[2-[2-(8-chloro-4-oxo-chromen-2-yl)-5-(trifluoromethyl)phenoxy]ethyl]-3-(p-tolylsulfonyl)urea;
8-chloro-4-oxo-2-[2-[2-(sulfamoylamino)ethoxy]-4-(trifluoromethyl)phenyl]chromene;
8-chloro-4-oxo-2-[2-[3-(sulfamoylamino)propoxy]-4-(trifluoromethyl)phenyl]chromene;
8-chloro-2-[4-methyl-2-[2-(sulfamoylamino)ethoxy]phenyl]-4-oxo-chromene;
2-[3-[2-[2-(8-chloro-4-oxo-chromen-2-yl)-5-(trifluoromethyl)phenoxy]ethyl]-2-oxo-imidazolidin-1-yl]-2-oxo-acetic acid;
(3S)-1-[3-[2-(8-chloro-4-oxo-chromen-2-yl)-5-(trifluoromethyl)phenoxy]-2-hydroxy-propyl]pyrrolidine-3-carboxylic acid;
(3S)-1-[3-[5-bromo-2-(8-chloro-4-oxo-chromen-2-yl)phenoxy]-2-hydroxy-propyl]pyrrolidine-3-carboxylic acid;
(3R)-1-[3-[5-bromo-2-(8-chloro-4-oxo-chromen-2-yl)phenoxy]-2-hydroxy-propyl]pyrrolidine-3-carboxylic acid;
2-[[3-[2-(8-chloro-4-oxo-chromen-2-yl)-5-(trifluoromethyl)phenoxy]-2-hydroxy-propyl]amino]-2-oxo-acetic acid;
2-[2-[2-(8-chloro-4-oxo-chromen-2-yl)-5-(trifluoromethyl)phenoxy]ethylamino]acetamide;
2-[2-[2-(8-chloro-4-oxo-chromen-2-yl)-5-(trifluoromethyl)phenoxy]ethylamino]propanamide; and
1-[3-[5-bromo-2-(8-chloro-4-oxo-chromen-2-yl)phenoxy]propyl]-5-oxo-pyrrolidine-3-carboxylic acid;
or a pharmaceutically acceptable salt thereof.

In another embodiment (xxi) of the present invention, particular compounds of the present invention are selected from:
1-[3-[5-bromo-2-(8-chloro-4-oxo-chromen-2-yl)phenoxy]propyl]pyrrolidine-3-carboxylic acid;
4-[3-[5-bromo-2-(8-chloro-4-oxo-chromen-2-yl)phenoxy]propyl]morpholine-2-carboxylic acid;
3-[3-[2-(8-chloro-4-oxo-chromen-2-yl)-5-(trifluoromethyl)phenoxy]propylamino]cyclobutanecarboxylic acid;
3-[3-[2-(8-chloro-4-oxo-chromen-2-yl)-5-(trifluoromethyl)phenoxy]propylamino]cyclopentanecarboxylic acid;
(3R)-1-[3-[2-(8-chloro-4-oxo-chromen-2-yl)-5-(trifluoromethyl)phenoxy]propyl]pyrrolidine-3-carboxylic acid;
(3S)-1-[3-[2-(8-chloro-4-oxo-chromen-2-yl)-5-(trifluoromethyl)phenoxy]propyl]pyrrolidine-3-carboxylic acid;
(3S)-1-[3-[5-bromo-2-(8-chloro-4-oxo-chromen-2-yl)-4-methoxy-phenoxy]propyl]pyrrolidine-3-carboxylic acid;
4-[3-[2-(8-chloro-4-oxo-chromen-2-yl)-5-(trifluoromethyl)phenoxy]propyl] morpholine-2-carboxylic acid;
1-[2-[5-bromo-2-(8-chloro-4-oxo-chromen-2-yl)phenoxy]ethyl]pyrrolidine-2-carboxylic acid;
4-[2-[5-bromo-2-(8-chloro-4-oxo-chromen-2-yl)phenoxy]ethyl]morpholine-2-carboxylic acid;
(3R)-1-[2-[5-bromo-2-[8-chloro-4-oxo-5-(trifluoromethyl)chromen-2-yl]phenoxy]ethyl]pyrrolidine-3-carboxylic acid;
(3R)-1-[2-[2-[5-bromo-2-(8-chloro-4-oxo-chromen-2-yl)phenoxy]ethoxy]ethyl]pyrrolidine-3-carboxylic acid;
4-[2-[5-bromo-2-(8-chloro-4-oxo-chromen-2-yl)phenoxy]ethyl]morpholine-3-carboxylic acid;
(3R)-1- [2-[5-bromo-2-(8-chloro-4-oxo-chromen-2-yl)phenoxy]ethyl]-N-methylsulfonyl-pyrrolidine-3-carboxamide;
1-[2-[2-(8-chloro-4-oxo-chromen-2-yl)-5-(trifluoromethyl)phenoxy]ethyl]-N-methylsulfonyl-pyrrolidine-2-carboxamide;
2-[4-bromo-2-[2-(3-methylsulfonylpyrrolidin-1-yl)ethoxy]phenyl]-8-chloro-chromen-4-one;
(2S)-2-[2-[2-(8-chloro-4-oxo-chromen-2-yl)-5-methyl-phenoxy]ethylcarbamoylamino]propanoic acid;
3-[2-(8-chloro-4-oxo-chromen-2-yl)-5-(trifluoromethyl)phenoxy]propylurea;
8-chloro-4-oxo-2-[2-[3-(sulfamoylamino)propoxy]-4-(trifluoromethyl)phenyl]chromene;
(3R)-1-[3-[5-bromo-2-(8-chloro-4-oxo-chromen-2-yl)phenoxy] -2-hydroxy-propyl]pyrrolidine-3-carboxylic acid; and
2-[[3-[2-(8-chloro-4-oxo-chromen-2-yl)-5-(trifluoromethyl)phenoxy]-2-hydroxy-propyl]amino]-2-oxo-acetic acid;
or a pharmaceutically acceptable salt thereof.

### SYNTHESIS

The compounds of the present invention can be prepared by any conventional means. Suitable processes for synthesizing these compounds as well as their starting materials are provided in the schemes below and in the subsequent examples. All substituents, in particular, R¹ to R¹², L, Cy1, Cy2, X and Y are defined above unless otherwise indicated. Furthermore, and unless explicitly otherwise stated, all reactions, reaction conditions, abbreviations and symbols have the meanings well known to a person of ordinary skill in organic chemistry. 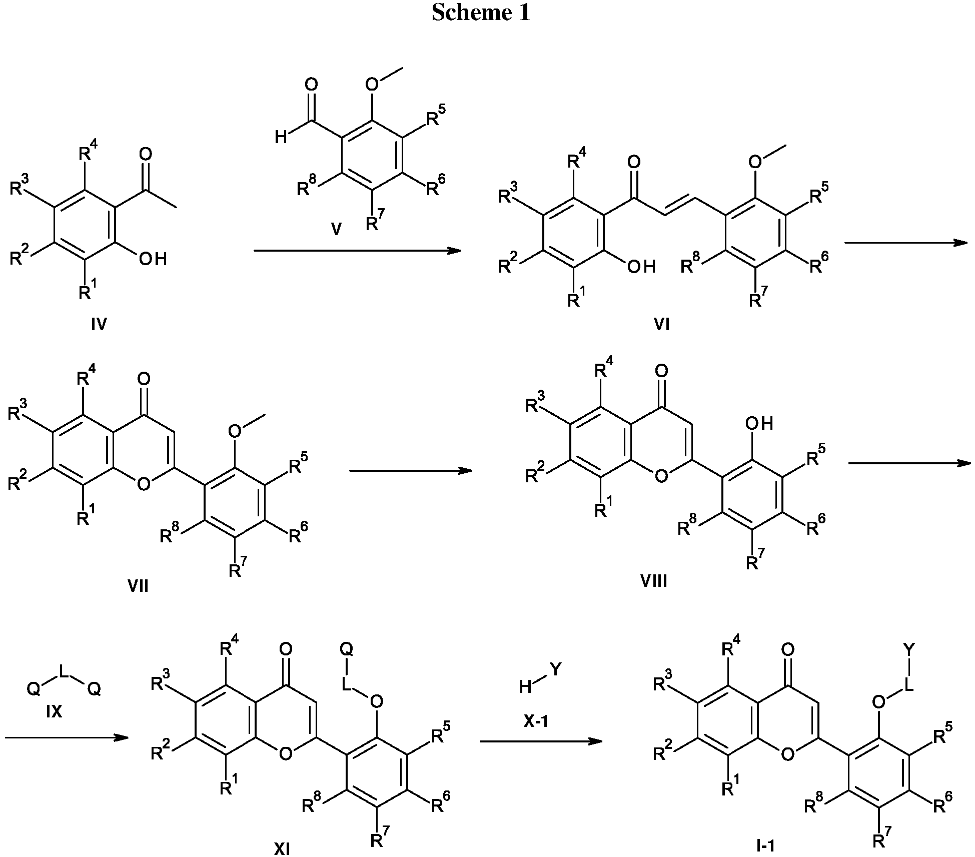 wherein Q is halogen or OMs.

Condensation of ketone **IV** with aldehyde **V** in the presence of a base, such as KOH, in a suitable solvent, such as ethanol, affords α,β-unsaturated carbonyl intermediate **VI.** Cyclization of α,β-unsaturated carbonyl intermediate **VI** in the presence of a suitable Lewis acid, such as I₂, KI or NaI, in a suitable solvent, such as DMSO, affords flavone derivative **VII.** Demethylation of flavone derivative **VII** with a suitable Lewis acid, such as BBr₃, in a suitable solvent, such as dichloromethane, affords compound of formula **VIII.** Substitution of compounds of formula **VIII** with compounds of formula **IX** in the presence of a suitable base, such as K₂CO₃, in a suitable solvent, such as DMF, affords compounds of formula **XI.** Substitution of compounds of formula **XI** with compounds of formula **X-1** in the presence of a suitable base, such as K₂CO₃, in a suitable solvent, such as DMF, affords compounds of formula **I-1.**

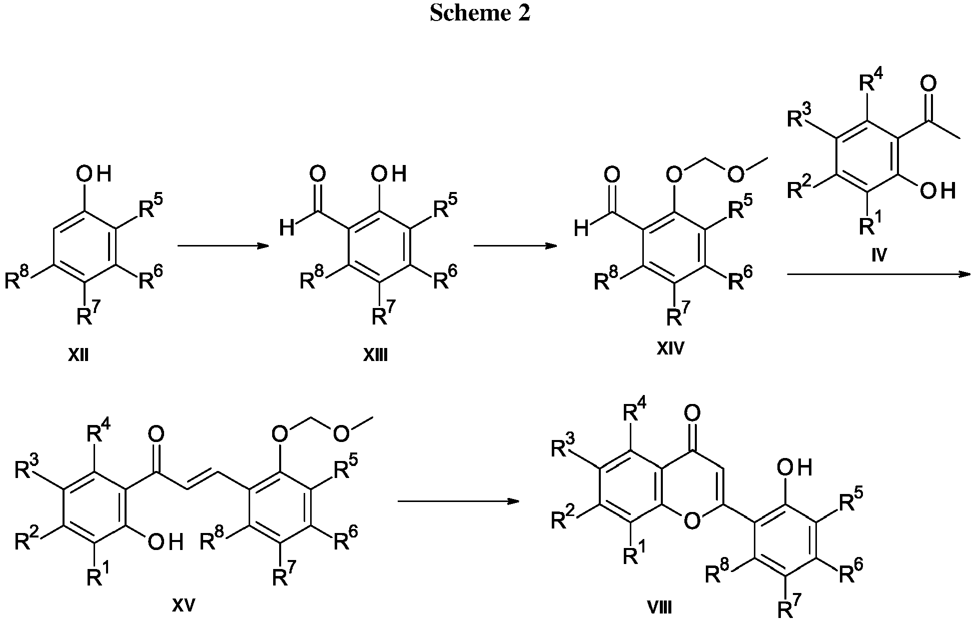

The compounds of formula **VIII** can also be prepared according to the Scheme 2. Formylation of compounds of formula **XII** with formaldehyde in the presence of a suitable base, such as TEA, with a suitable Lewis acid, such as MgCl₂, in a suitable solvent, such as ACN, affords aldehyde derivative **XIII.** Protection of aldehyde derivative **XIII** with bromo(methoxy)methane in the presence of a suitable base, such as NaH, in a suitable solvent, such as THF, affords aldehyde derivative **XIV.** Condensation of compounds of formula **XIV** with substituted ketone **IV** in the presence of a base, such as KOH, in a suitable solvent, such as ethanol, affords α,β-unsaturated carbonyl intermediate **XV.** Cyclization of intermediate **XV** in the presence of a suitable Lewis acid, such as I₂, KI or NaI, in a suitable solvent, such as DMSO, affords compounds of formula **VIII.** 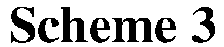 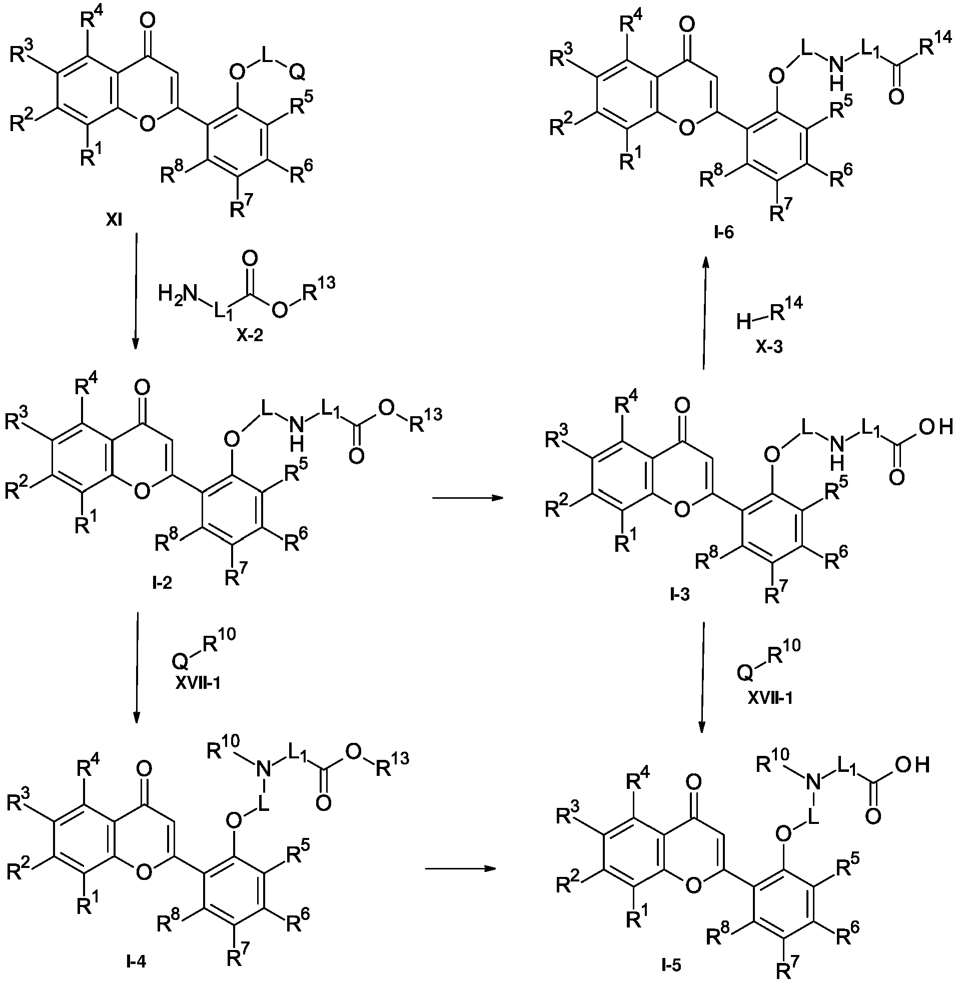 wherein Q is halogen or OMs; L₁ is C₁₋₆alkyl, carbonyl or C₃₋₇cycloalkyl; R¹³ is C₁₋₆alkyl; R¹⁴ is C₁₋₆alkylsulfonylamino, C₃₋₇cycloalkylsulfonylamino, C₃₋₇cycloalkylamino or hydroxyheterocyclyl.

Substitution of compounds of formula **XI** with amine **X-2** in the presence of a suitable base, such as NaHCO₃, in a suitable solvent, such as DMF, affords compounds of formula **I-2**. Hydrolysis of compounds of formula **I-2** in the presence of a suitable base, such as LiOH, in a suitable solvent, such as THF/water; or a suitable Lewis acid, such as TFA, in a suitable solvent, such as dichloromethane, affords compounds of formula **I-3**.

Treatment of compounds of formula **I-2** with compounds of formula **XVII-1** in the presence of a suitable base, such as TEA, in a suitable solvent, such as dichloromethane, affords compounds of formula **I-4**. The following hydrolysis of compounds of formula **I-4** in the presence of a suitable base, such as LiOH, in a suitable solvent, such as THF and water, affords compounds of formula **I-5**. The compounds of formula **I-5** can also be prepared in the other route in scheme 3 via **I-3**. Treatment of compounds of formula **I-3** with compounds of formula **XVII-1** in the presence of a suitable base, such as DIPEA, in a suitable solvent, such as dichloromethane, affords compounds of formula **I-5**.

Condensation of compounds of formula **I-3** with compounds of formula **X-3** in the presence of a condensation reagent, such as HATU, in a suitable solvent, such as dichloromethane, affords compound of formula **I-6**. 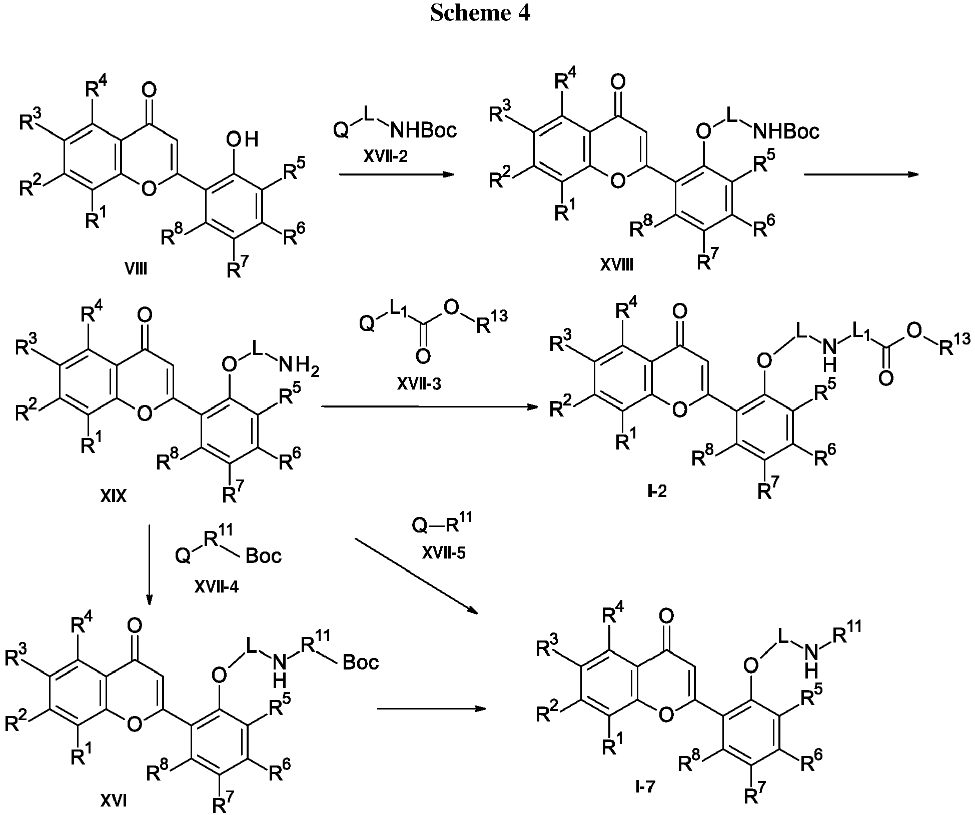 wherein Q is halogen or OMs; L₁ is C₁₋₆alkyl, carbonyl or C₃₋₇cycloalkyl; R¹³ is C₁₋₆alkyl.

The compounds of formula **I-2** can also be prepared according to the Scheme 4. Substitution of compounds of formula **VIII** with compounds of formula **XVII-2** in the presence of a suitable base, such as K₂CO₃, in a suitable solvent, such as DMF, affords compounds of formula **XVIII.** Boc deprotection of compounds of formula **XVIII** with a suitable Lewis acid, such as TFA, in a suitable solvent, such as dichloromethane, affords compounds of formula **XIX.** Treatment of compounds of formula **XIX** with compounds of formula **XVII-3** in the presence of a suitable base, such as DIPEA, in a suitable solvent, such as dichloromethane, affords compounds of formula **I-2**.

Substitution of compounds of formula **XIX** with compounds of formula **XVII-4** in the presence of a suitable base, such as DIPEA, in a suitable solvent, such as dichloromethane, affords compounds of formula **XVI.** Deprotection of compounds of formula **XVI** in the presence of a Lewis acid, such as TFA, in a suitable solvent, such as dichloromethane, affords compounds of formula **I-7**. Compounds of formula **I-7** can also be prepared from the substitution of compounds of formula **XIX** with halide **XVII-5** in the presence of a suitable base, such as DIPEA, in a suitable solvent, such as dichloromethane. 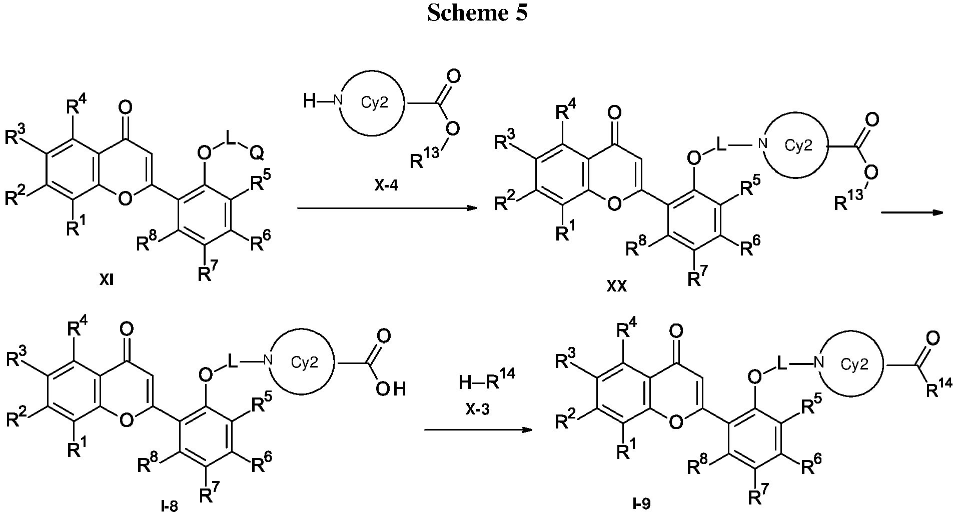 Wherein Q is halogen or OMs; R¹⁴ is C₁₋₆alkylsulfonylamino, C₃₋₇cycloalkylsulfonylamino, C₃₋₇cycloalkylamino or hydroxyheterocyclyl.

Substitution of compounds of formula **XI** with compounds of formula **X-4** in the presence of a suitable base, such as NaHCO₃, in a suitable solvent, such as DMF, affords flavone derivative **XX.** Hydrolysis of the compounds of formula **XX** in the presence of a suitable base, such as LiOH, in a suitable solvent, such as THF/water, or a suitable Lewis acid, such as TFA, in a suitable solvent, such as dichloromethane, affords carboxylic acid **I-8**. Condensation of compounds of formula **I-8** with compounds of formula **X-3** in the presence of a suitable condensation reagent, such as HATU, with a suitable base, such as NaH, in a suitable solvent, such as THF, affords amide derivative of formula **I-9**. 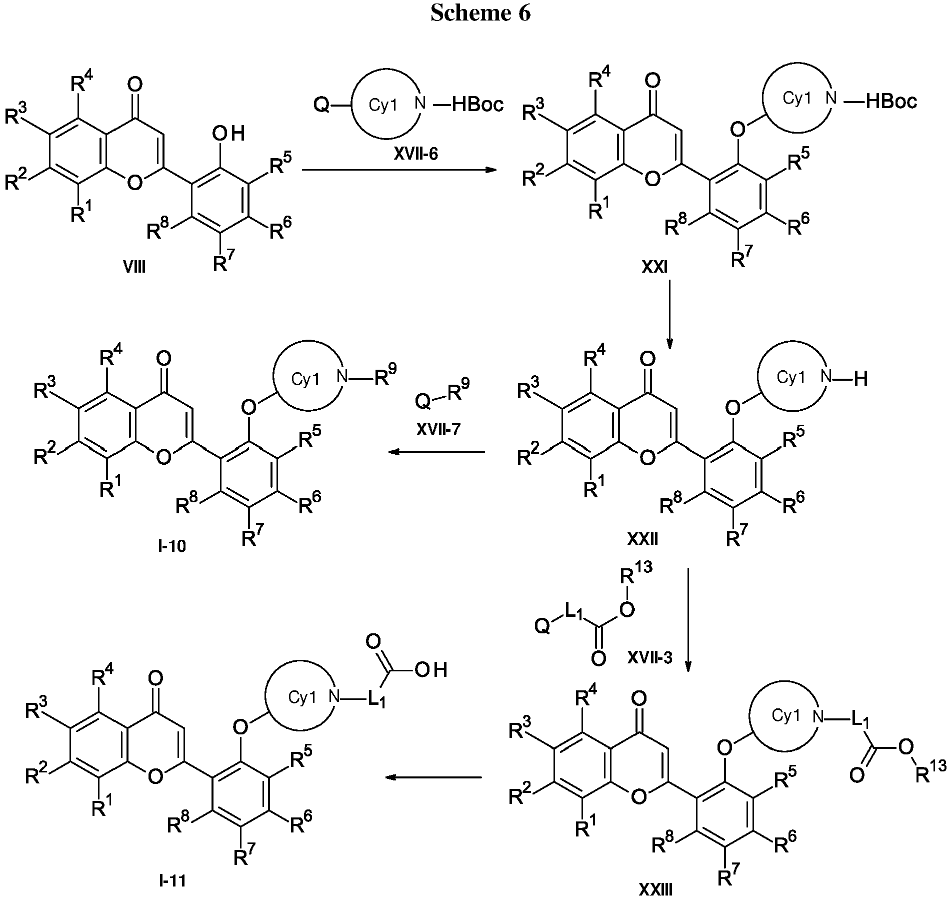 wherein Q is halogen or OMs; L₁ is C₁₋₆alkyl, carbonyl or C₃₋₇cycloalkyl; R¹³ is C₁₋₆alkyl.

Substitution of compounds of formula **VIII** with compounds of formula **XVII-6** in the presence of a suitable base, such as K₂CO₃, in a suitable solvent, such as DMF, affords compounds of formula **XXI.** Deprotection of compounds of formula **XXI** with a suitable Lewis acid, such as TFA, in a suitable solvent, such as dichloromethane, affords compounds of formula **XXII.** Treatment of compounds of formula **XXII** with compounds of formula **XVII-7** in the presence of a suitable base, such as DIPEA, in a suitable solvent, such as dichloromethane, affords compounds of formula **I-10**.

Treatment of compounds of formula **XXII** with compounds of formula **XVII-3** in the presence of a suitable base, such as DIPEA, in a suitable solvent, such as dichloromethane, affords compounds of formula **XXIII.** Hydrolysis of compounds of formula **XXIII** in the presence of a suitable base, such as LiOH, in a suitable solvent, such as THF/water, affords compounds of formula **I-11**. 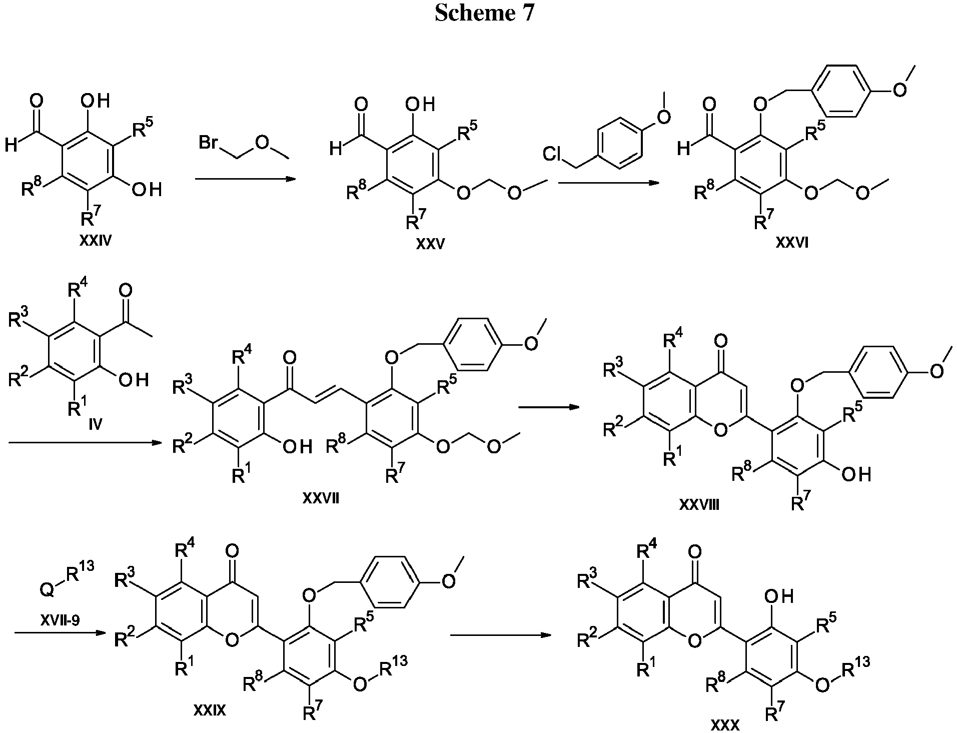 Wherein Q is halogen or OMs; R¹³ is C₁₋₆alkyl.

Protection of 4-hydroxy group of compounds of formula **XXIV** with bromomethyl methyl ether in the presence of a suitable base, such as DIPEA, in a suitable solvent, such as dichloromethane, affords compounds of formula **XXV.** Protection of 2-hydroxy group of compounds of formula **XXV** with 4-methoxybenzyl chloride in the presence of a suitable base, such as K₂CO₃, in a suitable solvent, such as DMF, affords compounds of formula **XXVI.** Condensation of ketone **IV** with aldehyde **XXVI** in the presence of a base, such as KOH, in a suitable solvent, such as ethanol, affords α,β-unsaturated carbonyl intermediate **XXVII.** Cyclization of α,β-unsaturated carbonyl intermediate **XXVII** in the presence of a suitable Lewis acid, such as I₂, KI or NaI, in a suitable solvent, such as DMSO, affords flavone derivative **XXVIII.** Alkylation of compounds of formula **XXVIII** with compounds of formula **XVII-9** in the presence of a base, such as KOH, in a suitable solvent, such as ethanol, affords compounds of formula **XXIX.** Deprotection of compounds of formula **XXIX** in the presence of Lewis acid, such as TFA, affords phenol **XXX.** 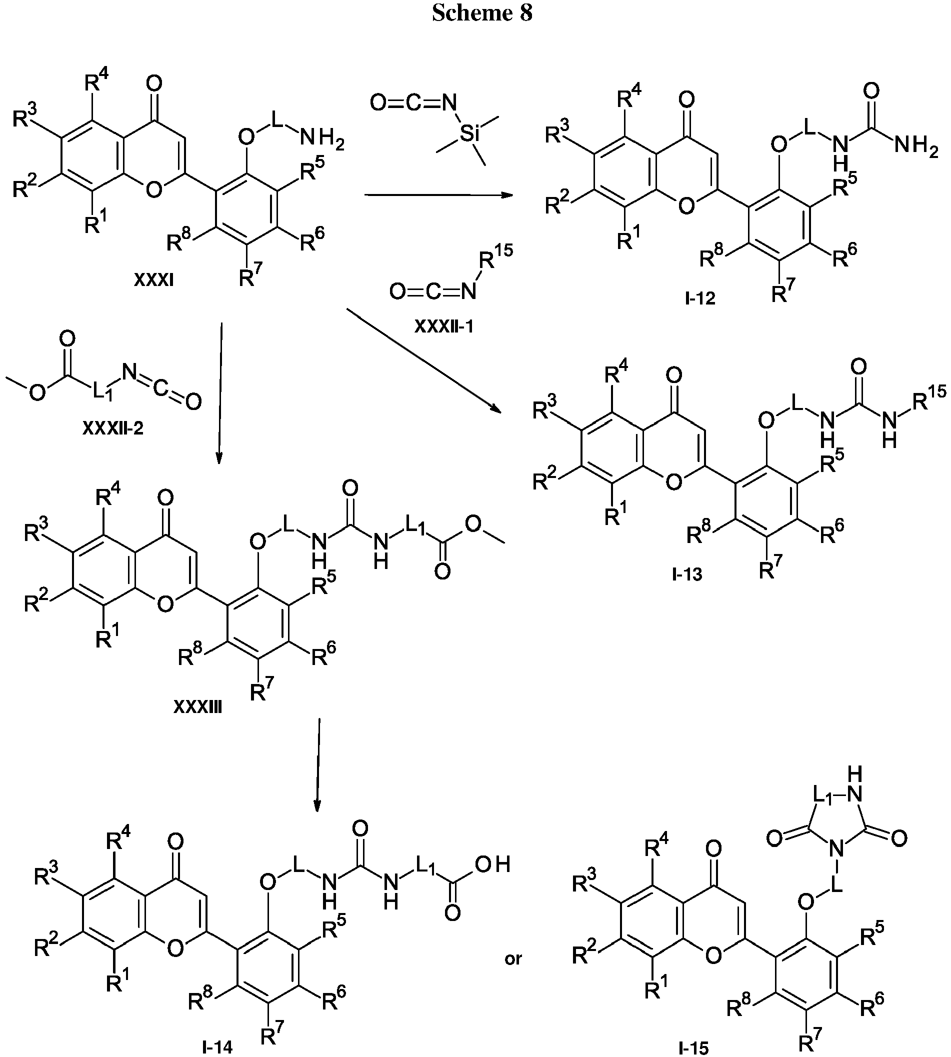 wherein Q is halogen or OMs; L₁ is C₁₋₆alkyl, carbonyl or C₃₋₇cycloalkyl; R¹⁵ is C₁₋₆alkylphenylsulfonyl.

Treatment of compounds of formula **XXXI** with isocyanato(trimethyl)silane in the presence of a suitable base, such as DIPEA, in a suitable solvent, such as dichloromethane, affords compounds of formula **I-12**. Treatment of compounds of formula **XXXI** with compounds of formula **XXXII-1** in the presence of a suitable base, such as DIPEA, in a suitable solvent, such as dichloromethane, affords compounds of formula **I-13**. Treatment of compounds of formula **XXXI** with compounds of formula **XXXII-2** in the presence of a suitable base, such as DIPEA, in a suitable solvent, such as dichloromethane, affords compounds of formula **XXXIII.** Deprotection of compounds of formula **XXXIII** in the presence of a base, such as sodium hydroxide, in a suitable solvent, such as THF, affords compounds of formula **I-14** and in situ-cyclization compounds of formula **I-15**. 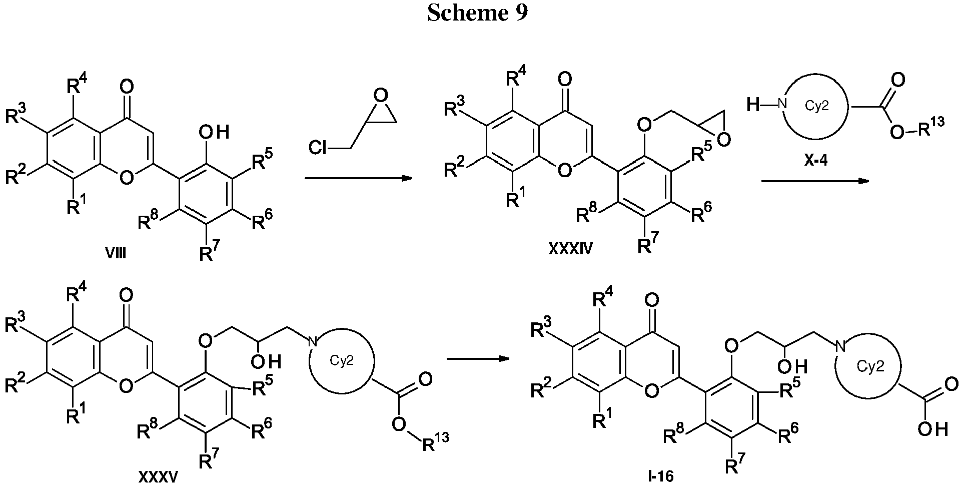 wherein R¹³ is C₁₋₆alkyl.

Substitution of compounds of formula **VIII** with 2-(chloromethyl)oxirane in the presence of a suitable base, such as K₂CO₃, in a suitable solvent, such as DMF, affords compounds of formula **XXXIV.** Ring open of the epoxide of compounds of formula **XXXIV** in the presence of a suitable base, such as K₂CO₃, in a suitable solvent, such as DMF, affords compounds of formula **XXXV.** Hydrolysis of compounds of formula **XXXV** in the presence of a suitable base, such as LiOH, in a suitable solvent, such as THF/water, affords compounds of formula **I-16**. 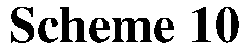 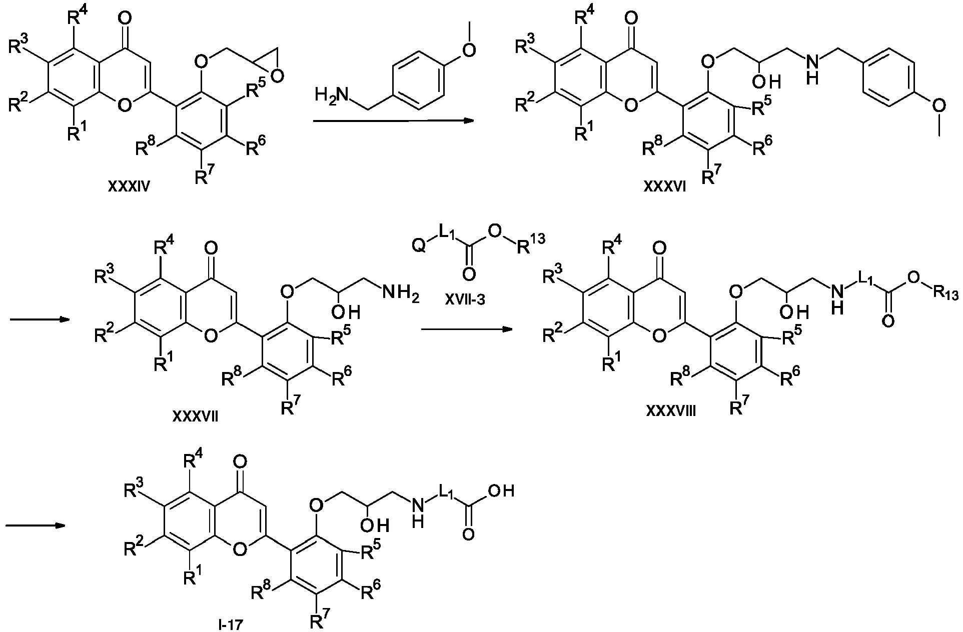 wherein L₁ is C₁₋₆alkyl, carbonyl or C₃₋₇cycloalkyl; R¹³ is C₁₋₆alkyl.

Ring open of compounds of formula **XXXIV** with (4-methoxyphenyl)methanamine in the presence of a suitable base, such as K₂CO₃, in a suitable solvent, such as DMF, affords compounds of formula **XXXVI.** Deprotection of compounds of formula **XXXVI** in the presence of suitable Lewis acid, such as TFA, in a suitable solvent, such as dichloromethane, affords compounds of formula **XXXVII.** Substitution of compounds of formula **XXXVII** with compounds of formula **XVII-3** in the presence of a suitable base, such as K₂CO₃, in a suitable solvent, such as DMF, affords compounds of formula **XXXVIII.** Hydrolysis of compounds of formula **XXXVIII** in the presence of a suitable base, such as LiOH, in a suitable solvent, such as THF/water, affords compounds of formula **I-17**.

This invention also relates to a process for the preparation of a compound of formula (I) comprising at least one of the following steps:
(a) Substitution of a compound of formula (XI), 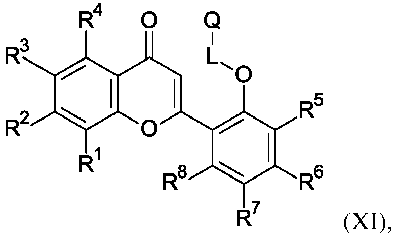 with a compound of formula (X-1), 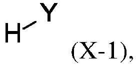 in the presence of a base;
(b) Substitution of a compound of formula (XI) with amine (X-2), 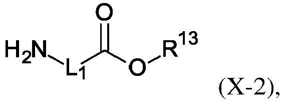 in the presence of a base;
(c) Hydrolysis of a compound of formula (1-2), 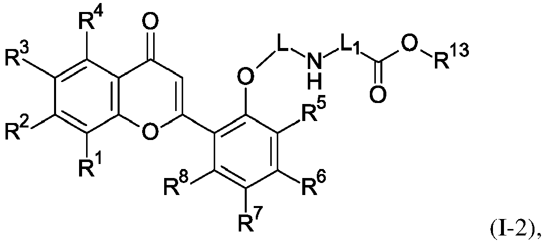 in the presence of a base;
(d) Substitution of a compound of formula (1-2) with a compound of formula (XVII-1), 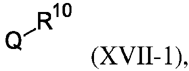 in the presence of a base;
(e) Hydrolysis of a compound of formula (1-4), 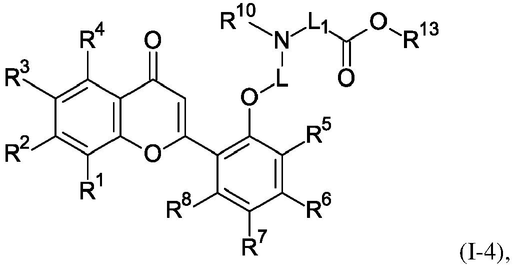 in the presence of a base or a Lewis acid;
(f) Treatment of a compound of formula (1-3), 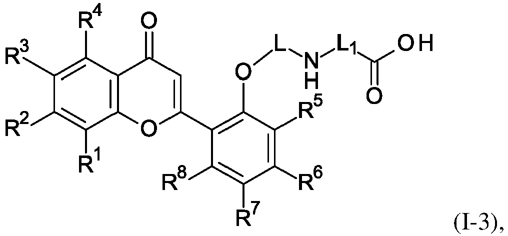 with a compound of formula (XVII-1) in the presence of a base;
(g) Condensation of a compound of formula (1-3), with a compound of formula (X-3), 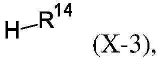 in the presence of a condensation reagent;
(h) Substitution of a compound of formula (XIX), 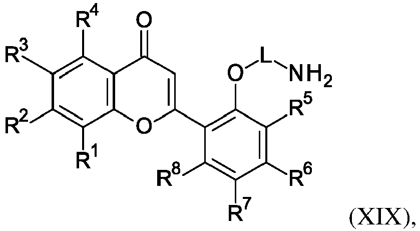 with a compound of formula (XVII-3), 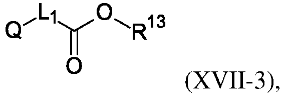 in the presence of a base;
(i) Substitution of a compound of formula (XIX), with a compound of formula (XVII-5), Q-R¹¹ (XVII-5), in the presence of a base;
(j) Deprotection of a compound of formula (XVI), 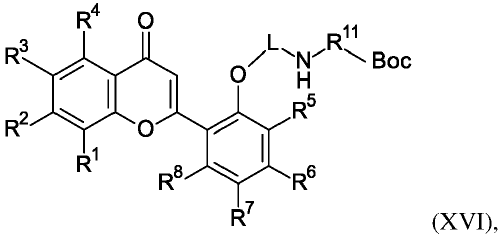 in the presence of a Lewis acid;
(k) Hydrolysis of a compound of formula (XX), 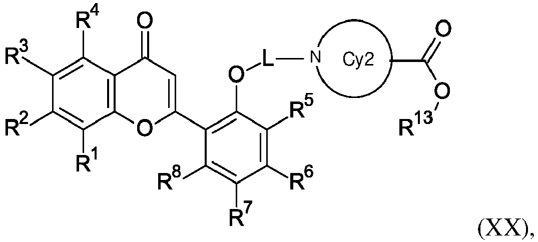 in the presence of a base or a Lewis acid;
(l) Condensation of a compound of formula (1-8), 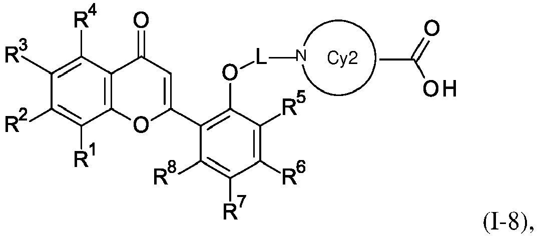 with a compound of formula (X-3), in the presence of a condensation reagent and a base;
(m) Treatment of a compound of formula (XXII), 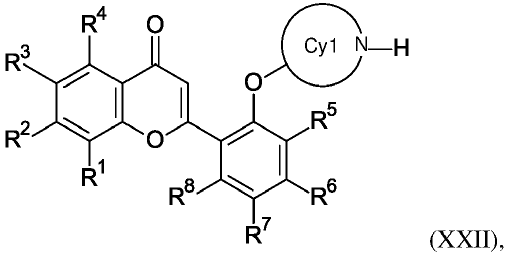 with a compound of formula (XVII-7); 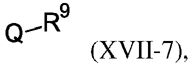 in the presence of a base;
(n) Hydrolysis of a compound of formula (XXIII), 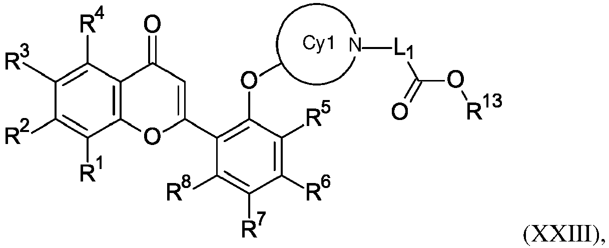 in the presence of a base;
(o) Treatment of a compound of formula (XXXI), 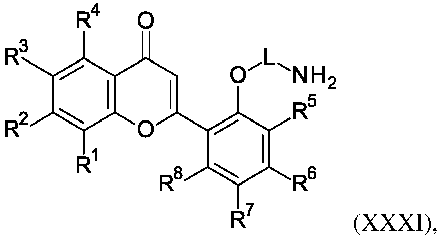 with isocyanato(trimethyl)silane in the presence of a base;
(p) Treatment of a compound of formula (XXXI) with a compound of formula (XXXII-1); 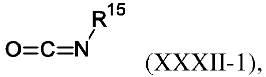 in the presence of a base;
(q) Deprotection of a compounds of formula (XXXIII), 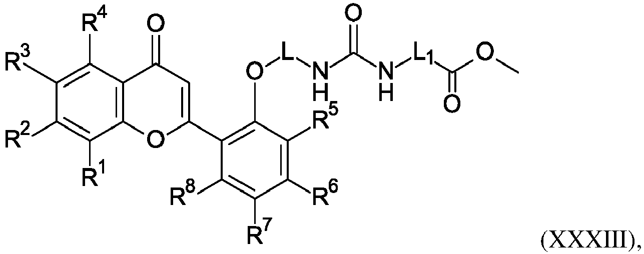 in the presence of a base;
(r) Hydrolysis of a compound of formula (XXXV), 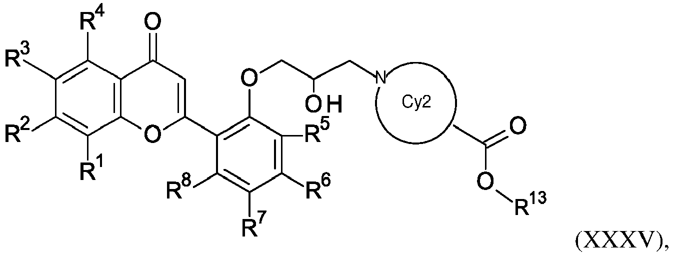 in the presence of a base;
(s) Hydrolysis of a compound of formula (XXXVIII), 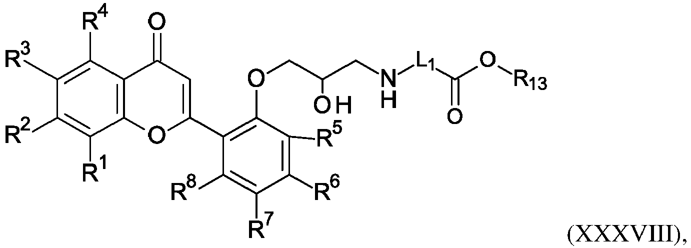 in the presence of a base;
wherein R¹ to R¹², L, Cy1, Cy2 and Y are defined above; wherein Q is halogen or Oms; L₁ is C₁₋₆alkyl, carbonyl or C₃₋₇cycloalkyl; R¹³ is C₁₋₆alkyl; R¹⁴ is C₁₋₆alkylsulfonylamino, C₃₋₇cycloalkylsulfonylamino, C₃₋₇cycloalkylamino or hydroxyheterocyclyl; R¹⁵ is C₁₋₆alkylphenylsulfonyl.
The base in step (a) can be for example K₂CO₃;
The base in step (b) can be for example NaHCOs;
The base in step (c) can be for example LiOH;
The base in step (d) can be for example TEA;
The base in step (e) can be for example LiOH;
The Lewis acid in step (e) can be for example TFA;
The base in step (f) can be for example DIPEA;
The condensation reagent in step (g) can be for example HATU;
The base in step (h) can be for example DIPEA;
The base in step (i) can be for example DIPEA;
The Lewis acid in step (j) can be for example TFA;
The base in step (k) can be for example LiOH;
The Lewis acid in step (k) can be for example TFA;
The condensation reagent in step (1) can be for example HATU;
The base in step (1) can be for example NaH;
The condensation reagent in step (m) can be for example LiOH;
The base in step (m) can be for example DIPEA;
The base in step (n) can be for example LiOH;
The base in step (o) can be for example DIPEA;
The base in step (p) can be for example DIPEA;
The base in step (q) can be for example sodium hydroxide;
The base in step (r) can be for example LiOH;
The base in step (s) can be for example LiOH.

A compound of formula (I) or (II) when manufactured according to the above process is also an object of the invention.

The compound of this invention also shows good safety and PK profile.

### PHARMACEUTICAL COMPOSITIONS AND ADMINISTRATION

The invention also relates to a compound of formula (I) or (II) for use as therapeutically active substance. Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) or (II) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) or (II) is formulated in an acetate buffer, at pH 5. In another embodiment, the compounds of formula (I) or (II) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to inhibit cccDNA in HBV patients, consequently lead to the reduction of HBsAg and HBeAg (HBV e antigen) in serum. For example, such amount may be below the amount that is toxic to normal cells, or the mammal as a whole.

In one example, the pharmaceutically effective amount of the compound of the invention administered parenterally per dose will be in the range of about 0.1 to 100 mg/kg, alternatively about 0.1 to 50 mg/kg of patient body weight per day, with the typical initial range of compound used being 0.3 to 15 mg/kg/day. In another embodiment, oral unit dosage forms, such as tablets and capsules, preferably contain from about 25 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 25 to 500 mg of the compound of the invention compounded with about 90 to 30 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 400 mg, of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of Formula (I) or (II), or pharmaceutically acceptable salt or enantiomer or diastereomer thereof.

In a further embodiment includes a pharmaceutical composition comprising a compound of formula (I) or (II), or pharmaceutically acceptable salt or enantiomer or diastereomer thereof, together with a pharmaceutically acceptable carrier or excipient.

Another embodiment includes a pharmaceutical composition comprising a compound of formula (I) or (II), or pharmaceutically acceptable salt or enantiomer or diastereomer thereof for use in the treatment of HBV infection.

### INDICATIONS AND METHODS OF TREATMENT

The references to methods of treatment and use of compounds for treatment in the subsequent paragraphs of this description are to be interpreted as references to the compounds, pharmaceutical compositions and medicaments of the present invention for use in a method for treatment of the human (or animal) body by therapy.

The compounds of the invention can inhibit cccDNA and have anti-HBV activity. Accordingly, the compounds of the invention are useful for the treatment or prophylaxis of HBV infection.

The invention relates to the use of a compound of formula (I) or (II) for the inhibition of cccDNA.

The invention also relates to the use of a compound of formula (I) or (II) for the inhibition of HBeAg.

The invention further relates to the use of a compound of formula (I) or (II) for the inhibition of HBsAg.

The invention relates to the use of a compound of formula (I) or (II) for the inhibition of HBV DNA.

The invention relates to the use of a compound of formula (I) or (II) for use in the treatment or prophylaxis of HBV infection.

The use of a compound of formula (I) or (II) for the preparation of medicaments useful in the treatment or prophylaxis diseases that are related to HBV infection is an object of the invention.

The invention relates in particular to the use of a compound of formula (I) or (II) for the preparation of a medicament for the treatment or prophylaxis of HBV infection.

The application discloses a method for the treatment or prophylaxis of HBV infection, which method comprises administering an effective amount of a compound of Formula (I) or (II), or enantiomers, diastereomers, prodrugs or pharmaceutically acceptable salts thereof.

### EXAMPLES

The invention will be more fully understood by reference to the following examples. They should not, however, be construed as limiting the scope of the invention. Abbreviations used herein are as follows:
- ACN:: acetonitrile
- BBr₃:: boron tribromide
- DMAP:: 4-dimethylaminopyridine
- DMF:: *N*, *N*-dimethylformamide
- IC₅₀:: the molar concentration of an inhibitor, which produces 50% of the maximum possible response for that inhibitor.
- FBS:: fetal bovine serum
- H₂O₂:: hydrogen peroxide
- HPLC:: high performance liquid chromatography
- MS (ESI):: mass spectroscopy (electron spray ionization)
- Ms:: methylsulfonyl
- obsd.:: observed
- PE:: petroleum ether
- DCM:: dichloromethane
- EtOAc:: ethyl acetate
- AcOH_{:}: acetic acid
- THF:: tetrahydrofuran
- TFA:: trifluoroacetic acid
- DIPEA:: N, N-Diisopropylethylamine
- TEA:: Triethylamine
- HATU:: 1-[Bis(dimethylamino)methylene]-1H-1,2,3-triazolo[4,5-b]pyridinium 3-oxid hexafluorophosphate
- δ:: chemical shift

### GENERAL EXPERIMENTAL CONDITIONS

Intermediates and final compounds were purified by flash chromatography using one of the following instruments: i) Biotage SP1 system and the Quad 12/25 Cartridge module, ii) column chromatography on silica gel combi-flash chromatography instrument. Silica gel Brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

LC/MS spectra were obtained using a Waters UPLC-SQD Mass. Standard LC/MS conditions were as follows (running time 3 minutes):
Acidic condition: A: 0.1% formic acid and 1% acetonitrile in H₂O; B: 0.1% formic acid in acetonitrile;
   Basic condition: A: 0.05% NH₃·H₂O in H₂O; B: acetonitrile.
Mass spectra (MS): generally only ions which indicate the parent mass are reported, and unless otherwise stated the mass ion quoted is the positive mass ion (M+H)⁺.
NMR Spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

### Intermediate 1: 8-chloro-2-[2-hydroxy-4-(trifluoromethyl)phenyl]chromen-4-one

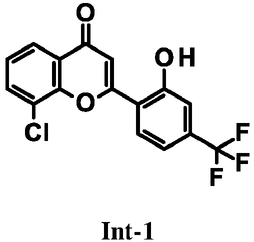

### Step 1: Preparation of (E)-1-(3-chloro-2-hydroxy-phenyl)-3-[2-methoxy-4-(trifluoromethyl)phenyl]prop-2-en-1-one

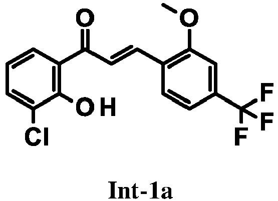

A mixture of 1-(3-chloro-2-hydroxy-phenyl)ethanone (2.5 g, 14.7 mmol, CAS registry number: 3226-34-4, Vendor: Bide Pharmatech, Catalog number: BD11027), 2-methoxy-4-(trifluoromethyl)benzaldehyde (2 g, 14.7 mmol, CAS registry number: 132927-09-4, Vendor: Alfa Aesar, Catalog number: H26797) and KOH (1.64 g, 29.3 mmol) in EtOH (25 mL) was stirred at 100 °C for 3 hours. After the reaction was completed, the mixture was adjusted to pH~4 by addition of 2N HCl and the resulting suspension was filtered. The solid was collected and concentrated *in vacuo* to give (*E*)-1-(3-chloro-2-hydroxy-phenyl)-3-[2-methoxy-4-(trifluoromethyl)phenyl]prop-2-en-1-one (3.3 g, yield: 78%) as a yellow solid, which was used in the next step without further purification. MS obsd. (ESI⁺) [(M+H)⁺]: 357.2.

### Step 2: Preparation of 8-chloro-2-[2-methoxy-4-(trifluoromethyl)phenyl]chromen-4-one

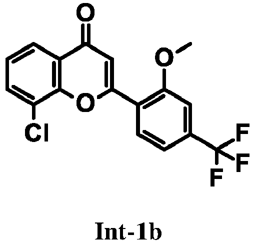

To a solution of (*E*)-1-(3-chloro-2-hydroxy-phenyl)-3-[2-methoxy-4-(trifluoromethyl)phenyl]prop-2-en-1-one (5.3 g, 18.4 mmol) in DMSO (60 mL) was added Iodine (466 mg, 1.84 mmol). The reaction mixture was stirred at 140 °C for 3 hours. After the reaction was completed, the reaction mixture was cooled to room temperature, quenched with sat. NaHSO₃ solution (10 mL). The mixture was diluted with water (100 mL) and the resulting suspension was filtered. The solid was collected and dried *in vacuo* to give 8-chloro-2-[2-methoxy-4-(trifluoromethyl)phenyl]chromen-4-one (5 g, yield: 94.9%) as a yellow solid, which was used in the next step without further purification. MS obsd. (ESI⁺) [(M+H)⁺]: 355.1.

### Step 3: Preparation of 8-chloro-2-[2-hydroxy-4-(trifluoromethyl)phenyl]chromen-4-one

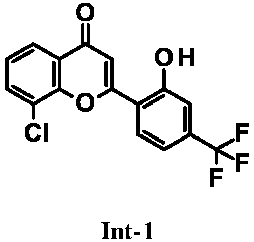

To a solution of 8-chloro-2-[2-methoxy-4-(trifluoromethyl)phenyl]chromen-4-one (5 g, 14.0 mmol) in dichloromethane (40 mL) was added BBr₃ (1M solution in dichloromethane, 69.8 mL, 69.8 mmol) at room temperature. The mixture was stirred at room temperature overnight. After the reaction was completed, the mixture was concentrated *in vacuo* and then the residue was suspended in sat. NH₄Cl solution (30 mL). The solid was collected by filtration and dried *in vacuo* to give 8-chloro-2-[2-hydroxy-4-(trifluoromethyl)phenyl]chromen-4-one (4.1 g, yield: 86%) as a yellow solid, which was used in the next step without further purification. MS obsd. (ESI⁺) [(M+H)⁺]: 341.2.

### Intermediate 2: 8-chloro-2-(2-hydroxy-4-methyl-phenyl)chromen-4-one

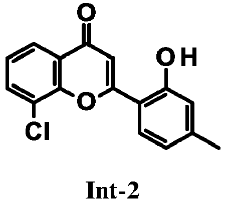

**Int-2** was prepared in analogy to the procedure described for the preparation of compound **Int-1** by using 2-methoxy-4-methyl-benzaldehyde as the starting material instead of 2-methoxy-4-(trifluoromethyl)benzaldehyde in **Step 1.** MS obsd. (ESI⁺) [(M+H)⁺]: 287.1.

### Intermediate 3: 8-chloro-2-(2-hydroxyphenyl)chromen-4-one

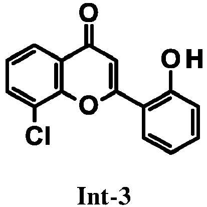

**Int-3** was prepared in analogy to the procedure described for the preparation of compound **Int-1** by using 2-hydroxybenzaldehyde as the starting material instead of 2-methoxy-4-(trifluoromethyl)benzaldehyde in **Step 1.** MS obsd. (ESI⁺) [(M+H)⁺]: 273.2.

### Intermediate 4: 2-(4-bromo-2-hydroxy-phenyl)-8-chloro-chromen-4-one

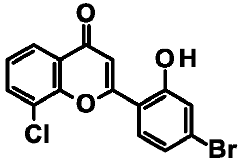

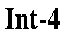

**Int-4** was prepared in analogy to the procedure described for the preparation of compound **Int-1** by using 4-bromo-2-hydroxy-benzaldehyde as the starting material instead of 2-methoxy-4-(trifluoromethyl)benzaldehyde in **Step 1.** MS obsd. (ESI⁺) [(M+H)⁺]: 351.2.

### Intermediate 5: 8-chloro-2-(4-chloro-2-hydroxy-5-methyl-phenyl)chromen-4-one

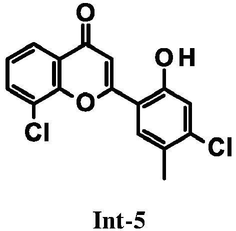

### Step 1: Preparation of 4-chloro-2-hydroxy-5-methyl-benzaldehyde

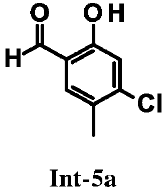

To a solution of 3-chloro-4-methyl-phenol (10.0 g, 70.1 mmol, CAS registry number: 615-62-3, Vendor: Bide Pharmatech, Catalog number: BD85862) in ACN (200 mL) were added formaldehyde (8.42 g, 280.54 mmol), TEA (39.1 mL, 280.5 mmol) and magnesium chloride (27.0 mL, 210.4 mmol) and the mixture was stirred at 80 °C for 16 hours. After the reaction was completed, the reaction was quenched with 1M HCl (500 mL) and extracted with EtOAc (150 mL) three times. The combined organic layer was washed with brine, dried over Na₂SO₄ and concentrated *in vacuo* to give 4-chloro-2-hydroxy-5-methyl-benzaldehyde (11.3 g, yield: 94.5%) as brown oil, which was used in the next step without further purification.

### Step 2: Preparation of 4-chloro-2-(methoxymethoxy)-5-methyl-benzaldehyde

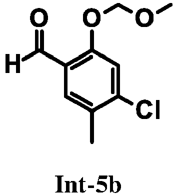

To a solution of 4-chloro-2-hydroxy-5-methyl-benzaldehyde (9.6 g, 56.28 mmol) in THF (100 mL) cooled at 0 °C was added sodium hydride (3.38 g, 84.41 mmol) in small portions. After addition, the mixture was stirred at 0 °C for 30 minutes and then to the resulting mixture was added bromomethyl methyl ether (10.55 g, 84.41 mmol) dropwise. The reaction mixture was stirred at 0 °C for another 2 hours. After the reaction was complete, the mixture was poured into ice-water (200 mL) slowly and extracted with EtOAc (80 mL) three times. The combined organic layer was washed with brine, dried over Na₂SO₄ and concentrated *in vacuo* to give 4-chloro-2-(methoxymethoxy)-5-methyl-benzaldehyde (12 g, yield: 99.3%) as a white solid, which was used in the next step directly. MS obsd. (ESI⁺)[(M+H)⁺]: 215.1.

### Step 3: Preparation of (E)-1-(3-chloro-2-hydroxy-phenyl)-3-[4-chloro-2 - (methoxymethoxy)-5-methyl-phenyl]prop-2-en-1-one

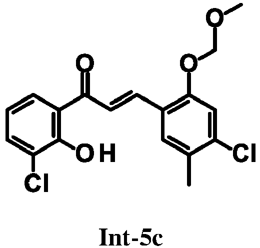

To a solution of 4-chloro-2-(methoxymethoxy)-5-methyl-benzaldehyde (6.0 g, 27.95 mmol) and 1-(3-chloro-2-hydroxy-phenyl)ethanone (4.77 g, 27.95 mmol) in EtOH (300 mL) was added KOH (15.68 g, 279.52 mmol). The mixture was stirred at 35 °C for 16 hours. After the reaction was completed, the mixture was poured into 0.5M HCl (200 mL) and the resulting suspension was filtered. The filter cake was collected and dried *in vacuo* to give (*E*)-1-(3-chloro-2-hydroxyphenyl)-3-[4-chloro-2-(methoxymethoxy)-5-methyl-phenyl]prop-2-en-1-one (10 g, 27.23 mmol, yield: 97.4%) as a yellow solid, which was used in the next step without further purification. MS obsd. (ESI⁺)[(M+H)⁺]: 367.0.

### Step 4: Preparation of 8-chloro-2-[4-chloro-2-(methoxymethoxy)-5-methylphenyl]chromen-4-one

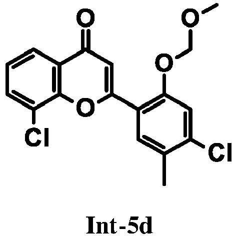

To a solution of (*E*)-1-(3-chloro-2-hydroxy-phenyl)-3-[4-chloro-2-(methoxymethoxy)-5-methyl-phenyl]prop-2-en-1-one (10.0 g, 27.23 mmol) in DMSO (250 mL) was added iodine (345.58 mg, 1.36 mmol). The reaction mixture was stirred at 140 °C under N₂ for 2 hours. After the reaction was complete, the mixture was poured into ice-water and the resulting suspension was filtered. The solid was washed with water and then dried to give 8-chloro-2-[4-chloro-2-(methoxymethoxy)-5-methyl-phenyl]chromen-4-one (8.7 g, yield: 87.5%) as a yellow solid, which was used in the next step without further purification. MS obsd. (ESI⁺)[(M+H)⁺]: 365.0.

### Step 5: Preparation of 8-chloro-2-(4-chloro-2-hydroxy-5-methyl-phenyl)chromen-4-one

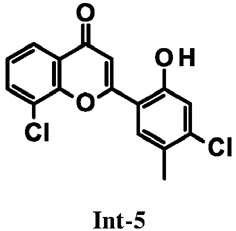

To a solution of 8-chloro-2-[4-chloro-2-(methoxymethoxy)-5-methyl-phenyl]chromen-4-one (3.4 g, 9.31 mmol) in dichloromethane (10 mL) was added trifluoroacetic acid (10.0 mL, 129.8 mmol). The reaction mixture was stirred at room temperature for 16 hours. After the reaction was completed, the reaction mixture was concentrated *in vacuo* to give 8-chloro-2-(4-chloro-2-hydroxy-5-methyl-phenyl)chromen-4-one (2.7 g, yield: 90.3%) as a brown solid, which was used in the next step directly. MS obsd. (ESI⁺) [(M+H)⁺]: 320.9.

### Intermediate 6: 2-(4-bromo-2-hydroxy-5-methyl-phenyl)-8-chloro-chromen-4-one

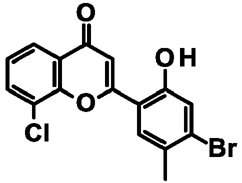

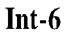

**Int-6** was prepared in analogy to the procedure described for the preparation of compound **Int-5** by using 3-bromo-4-methyl-phenol as the starting material instead of 3-chloro-4-methylphenol in **Step 1.**

### Intermediate 7: 2-(4-bromo-2-hydroxy-5-methoxy-phenyl)-8-chloro-chromen-4-one

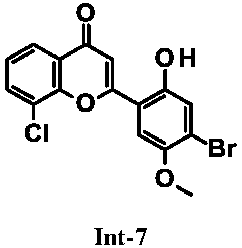

**Int-7** was prepared in analogy to the procedure described for the preparation of compound **Int-5** by using 4-bromo-2-hydroxy-5-methoxy-benzaldehyde as the starting material instead of 4-chloro-2-hydroxy-5-methyl-benzaldehyde in **Step 2.** MS obsd. (ESI⁺) [(M+H)⁺]: 381.1.

### Intermediate 8: 8-chloro-2-(2-hydroxy-4,5-dimethoxy-phenyl)chromen-4-one

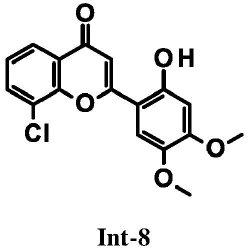

**Int-8** was prepared in analogy to the procedure described for the preparation of compound **Int-5** by using 2-hydroxy-4,5-dimethoxy-benzaldehyde (CAS registry number: 14382-91-3, Vendor: Accela ChemBio Inc., Catalog number: SY025559) as the starting material instead of 4-chloro-2-hydroxy-5-methyl-benzaldehyde in **Step 2.** MS obsd. (ESI⁺) [(M+H)⁺]: 333.2.

### Intermediate 9: 8-chloro-2-(2-hydroxy-4-methoxy-5-methyl-phenyl)chromen-4-one

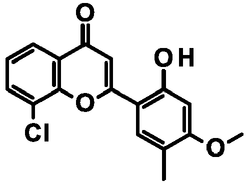

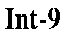

**Int-9** was prepared in analogy to the procedure described for the preparation of compound **Int-5** by using 3-methoxy-4-methyl-phenol as the starting material instead of 3-chloro-4-methylphenol in **Step 1.**

### Intermediate 10: 8-chloro-2-(2-hydroxy-4-methoxy-phenyl)chromen-4-one

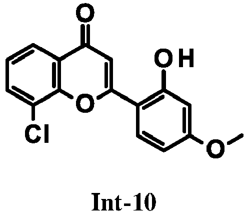

**Int-10** was prepared in analogy to the procedure described for the preparation of compound **Int-5** by using 2-hydroxy-4-methoxy-benzaldehyde (CAS registry number: 673-22-3, Vendor: Accela ChemBio Inc., Catalog number: SY012912) as the starting material instead of 4-chloro-2-hydroxy-5-methyl-benzaldehyde in **Step 2.** MS obsd. (ESI⁺) [(M+H)⁺]: 302.9.

### Intermediate 11: 2-(5-bromo-2-hydroxy-4-methoxy-phenyl)-8-chloro-chromen-4-one

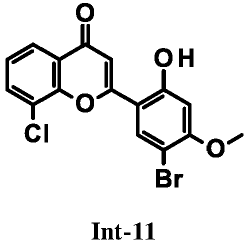

**Int-11** was prepared in analogy to the procedure described for the preparation of compound **Int-5** by using 5-bromo-2-hydroxy-4-methoxybenzaldehyde as the starting material instead of 4-chloro-2-hydroxy-5-methyl-benzaldehyde in **Step 2.** MS obsd. (ESI⁺)[(M+H)⁺]: 381.1.

### Intermediate 12: 8-chloro-2-(2-hydroxy-5-methoxy-4-methyl-phenyl)chromen-4-one

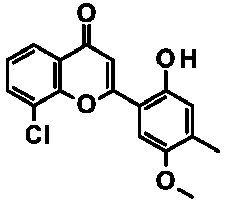

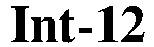

**Int-12** was prepared in analogy to the procedure described for the preparation of compound **Int-5** by using 4-methoxy-3-methyl-phenol as the starting material instead of 3-chloro-4-methyl-phenol in **Step 1.** MS obsd. (ESI⁺) [(M+H)]⁺: 317.2.

### Intermediate 13: 8-chloro-2-(4-ethoxy-2-hydroxy-phenyl)chromen-4-one

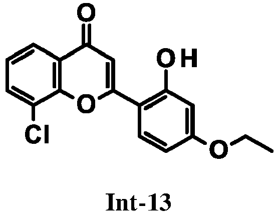

### Step 1: Preparation of 2-hydroxy-4-(methoxymethoxy)benzaldehyde

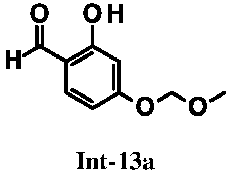

A 500 mL of flask fitted with magnetic stirrer was charged with 2,4-dihydroxybenzaldehyde (5.52 g, 39.97 mmol, CAS registry number: 95-01-2, Vendor: TCI Shanghai, Catalog number: 0564) and DIPEA (10.3 g, 79.93 mmol) in THF (40 mL) and DCM (240 mL). The solution was cooled to 0 °C and then bromomethyl methyl ether (5.0 g, 39.97 mmol) was added. The reaction mixture was allowed to warm to room temperature and stirred for 16 hours. After the starting material was consumed, the reaction mixture was concentrated *in vacuo* to afford 2-hydroxy-4-(methoxymethoxy)benzaldehyde (4.8 g, yield: 65.9%) as a white solid. MS obsd. (ESI⁺)[(M+H)⁺]: 183.1.

### Step 2: Preparation of 4-(methoxymethoxy)-2-[(4-methoxyphenyl)methoxy]benzaldehyde

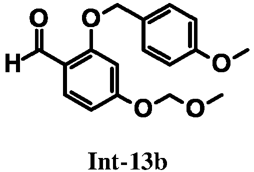

To a solution of 2-hydroxy-4-(methoxymethoxy)benzaldehyde (4.8 g, 26.35 mmol) and 4-methoxybenzylchloride (4.29 mL, 31.62 mmol) in DMF (100 mL) was added K₂CO₃ (7.27 g, 52.7 mmol). The reaction mixture was stirred at 70 °C for 3 hours. After completion, the reaction was concentrated *in vacuo* and purified by flash column (eluting with EtOAc : PE = 0 to 25%) to give 4-(methoxymethoxy)-2-[(4-methoxyphenyl)methoxy]benzaldehyde (7.0 g, yield: 84.4%) as a white solid. MS obsd. (ESI⁺)[(M+Na)⁺]: 325.1.

### Step 3: Preparation of (E)-1-(3-chloro-2-hydroxy-phenyl)-3-[4-(methoxymethoxy)-2-[(4-methoxyphenyl)methoxy]phenyl]prop-2-en-1-one

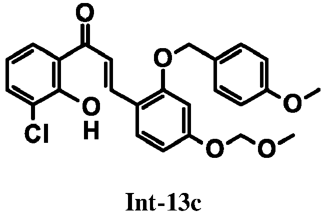

To a solution of 1-(3-chloro-2-hydroxy-phenyl)ethanone (2.82 g, 16.54 mmol) and KOH (7.41 g, 132.31 mmol) in ethanol (500 mL) was added 5-(methoxymethoxy)-2-[(4-methoxyphenyl)methoxy]benzaldehyde (5.0 g, 16.54 mmol). The reaction mixture was stirred at 50 °C for 16 hours. After the starting material was consumed, the reaction mixture was acidified to pH = 2 by adding 1N HCl (6 mL) to form a suspension. The suspension was filtered and the filter cake was collected and dried to give (*E*)-1-(3-chloro-2-hydroxy-phenyl)-3-[4-(methoxymethoxy)-2-[(4-methoxyphenyl)methoxy]phenyl]prop-2-en-1-one (6.0 g, yield: 79.8%) as a yellow solid. MS obsd. (ESI⁺)[(M+Na)⁺]: 477.1.

### Step 4: Preparation of 8-chloro-2-[4-hydroxy-2-[(4-methoxyphenyl)methoxy]phenyl]chromen-4-one

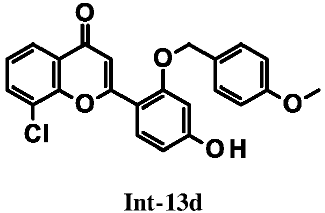

To a solution of (*E*)-1-(3-chloro-2-hydroxy-phenyl)-3-[4-(methoxymethoxy)-2-[(4-methoxyphenyl)methoxy]phenyl]prop-2-en-1-one (6.0 g, 13.19 mmol) in DMSO (200 mL) was added iodine (167 mg, 0.66 mmol). The reaction mixture was stirred at 140 °C for 3 hours. Then, the reaction mixture was poured into water (100 mL) and the solid was precipitated out. The mixture was filtered and the filter cake was washed with aq. Na₂SO₃ solution (10 mL) and dried to give 8-chloro-2-[4-hydroxy-2-[(4-methoxyphenyl)methoxy]phenyl]chromen-4-one (5.0 g, yield: 92.7% as a dark brown solid. MS obsd. [(M+H)⁺]: (ESI⁺): 409.2.

### Step 5: Preparation of 8-chloro-2-[4-ethoxy-2-[(4-methoxyphenyl)methoxy]phenyl]chromen-4-one

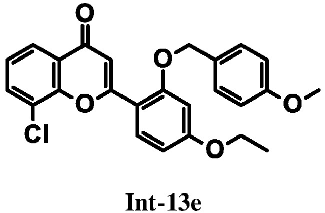

To a solution of 8-chloro-2-[4-hydroxy-2-[(4-methoxyphenyl)methoxy]phenyl]chromen-4-one (3.0 g, 7.34 mmol) and iodoethane (1.17 mL, 14.68 mmol) in DMF (25 mL) was added K₂CO₃ (2.03 g, 14.68 mmol). The reaction mixture was stirred at room temperature. After stirring for 3 hours, the reaction was quenched by adding brine (40 mL). The mixture was extracted with EtOAc (40 mL) twice. The organic layer was combined and concentrated *in vacuo* to give the crude product. The crude product was purified by flash column (eluting with EtOAc : PE = 0 to 40%) to give 8-chloro-2-[4-ethoxy-2-[(4-methoxyphenyl)methoxy]phenyl]chromen-4-one (1.5 g, yield: 46.8%) as a yellow solid. MS obsd. (ESI⁺)[(M+H)⁺]: 437.1.

### Step 6: Preparation of 8-chloro-2-(4-ethoxy-2-hydroxy-phenyl)chromen-4-one

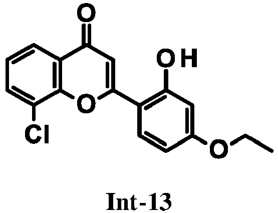

A solution of 8-chloro-2-[4-ethoxy-2-[(4-methoxyphenyl)methoxy]phenyl]chromen-4-one (1.5 g, 3.43 mmol) in TFA (10.0 mL, 3.43 mmol) was stirred at 110 °C for 2 hours. After completion, the reaction mixture was cooled in the ice/water bath and then concentrated *in vacuo* to give 8-chloro-2-(4-ethoxy-2-hydroxy-phenyl)chromen-4-one (1.0 g, yield: 92.0%) as a yellow solid. MS obsd. (ESI⁺)[(M+H)⁺]: 317.0.

### Intermediate 14: 8-chloro-2-(4-ethoxy-2-hydroxy-phenyl)chromen-4-one

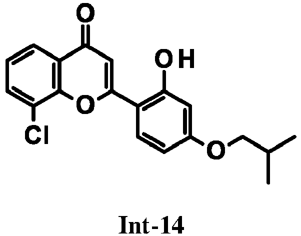

**Int-14** was prepared in analogy to the procedure described for the preparation of compound **Int-13** by using 1-iodo-2-methyl-propane as the reagent instead of iodoethane in **Step 5.** MS obsd. (ESI⁺) [(M+H)⁺]: 345.2.

### Intermediate 15: 8-chloro-6-fluoro-2-(2-hydroxyphenyl)chromen-4-one

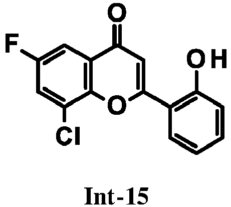

### Step 1: Preparation of 1-(3-chloro-5-fluoro-2-hydroxy-phenyl)ethanone

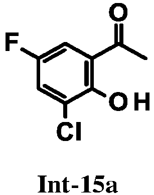

A solution of 2-chloro-4-fluorophenol (4.5 g, 30.71 mmol, CAS registry number: 1996-41-4, Vendor: Bide Pharmatech, Catalog number: BD19192), acetic anhydride (4.7 g, 46.06 mmol) and sulfuric acid (1.51 g, 15.35 mmol) was stirred at room temperature for 2 hours. After completion, the reaction mixture was diluted with water (50 mL). The aqueous layer was extracted by EtOAc (50 mL) twice, and the combined organic layer was concentrated *in vacuo.* Then, aluminum chloride (6.1 g, 46.06 mmol) was added to the resulting residue and stirred at 120 °C for 8 hours. The reaction mixture was diluted with water (150 mL), and the aqueous layer was extracted by EtOAc (100 mL) twice. The combined organic layer was concentrated *in vacuo* to give the crude product. The crude product was purified by flash column (eluting with EtOAc : PE = 3% to 10%) to give 1-(3-chloro-5-fluoro-2-hydroxy-phenyl)ethanone (3.8 g, yield: 65.62%) as a light yellow solid. MS obsd. (ESI⁺) [(M-H)⁻]: 187.0.

### Step 2-5: Preparation of 8-chloro-6-fluoro-2-(2-hydroxyphenyl)chromen-4-one

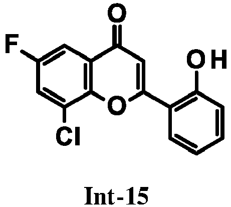

**Int-15** was prepared in analogy to the procedure described for the preparation of compound **Int-5** by using 2-methoxybenzaldehyde as the starting materials instead of 4-chloro-2-hydroxy-5-methyl-benzaldehyde in **Step 2** and using **Int-15a** instead of 1-(3-chloro-2-hydroxy-phenyl)ethanone in **Step 3.** MS obsd. (ESI⁺)[(M+H)⁺]: 291.2.

### Intermediate 16: 8-chloro-2-(2-hydroxy-4-methyl-phenyl)-6-methoxy-chromen-4-one

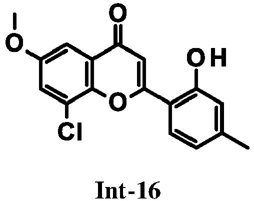

### Step 1: Preparation of 1-(3-chloro-2-hydroxy-5-methoxy-phenyl)ethanone

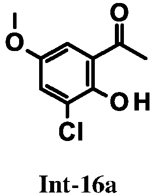

To a solution of 2-hydroxy-5-methoxyacetophenone (2.0 g, 12.04 mmol, CAS registry number: 705-15-7, Vendor: Bide Pharmatech, Catalog number: BD11251) in DMF (15 mL) was added N-chlorosuccinimide (2.41 g, 18.05 mmol). Then, the reaction mixture was stirred for 2 hours. After the starting material was consumed, the reaction mixture was poured into ice water (100 mL) and extracted with EtOAc (50 mL) three times. The combined organic layer was washed with brine (50 mL) twice, dried over anhydrous MgSO₄, filtered and concentrated *in vacuo* to give the crude product. The crude product was purified by flash column (eluting with EtOAc : PE = 10%) to give 1-(3-chloro-2-hydroxy-5-methoxy-phenyl)ethanone (2.3 g, yield: 95.3%) as a light brown solid. (ESI⁺)[(M+H)⁺]: 201.0.

### Step 2-5: Preparation of 8-chloro-2-(2-hydroxy-4-methyl-phenyl)-6-methoxy-chromen-4-one

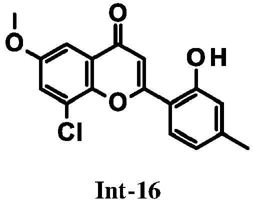

**Int-16** was prepared in analogy to the procedure described for the preparation of compound **Int-5** by using 2-hydroxy-4-methyl-benzaldehyde as the starting materials instead of 4-chloro-2-hydroxy-5-methyl-benzaldehyde in **Step 2** and using **Int-16a** instead of 1-(3-chloro-2-hydroxy-phenyl)ethanone in **Step 3.** MS obsd. (ESI⁺)[(M+H)⁺]: 291.2.

### Intermediate 17: 2-(4-bromo-2-hydroxy-phenyl)-8-chloro-5-(trifluoromethyl)chromen-4-one

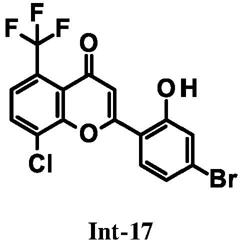

### Step 1: Preparation of 1-(6-bromo-3-chloro-2-hydroxy-phenyl)ethanone

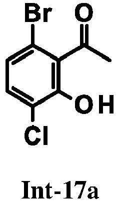

To a mixture of 5-bromo-2-chlorophenol (6.0 g, 28.92 mmol) and acetyl acetate (4.1 mL, 43.38 mmol) was added four drops of concentrated sulfuric acid (0.2 mL) at room temperature. The reaction was stirred at room temperature for 2 hours. Then, the reaction mixture was poured into water (50 mL) under stirring and layers were separated. The aqueous layer was extracted with EtOAc (50 mL) three times. The combined organic layer was washed with brine, dried over anhydrous Na₂SO₄ and concentrated *in vacuo.* Into the above residue cooled in an ice-water bath was then added with powdered anhydrous trichloroalumane (5.78 g, 43.38 mmol). The resulting mixture was heated to 120 °C and stirred at this temperature for 5 hours. After completion, the reaction mixture was quenched with crushed ice and extracted with EtOAc (90 mL) three times. The combined organic phase was concentrated *in vacuo* to give the crude product as light brown solid, which was purified by flash chromatography to give 1-(6-bromo-3-chloro-2-hydroxyphenyl)ethanone (623 mg, yield: 8.63%) as light yellow liquid. MS obsd. (ESI⁺)[(M+H)⁺]: 248.0.

### Step 2: Preparation of 1-[6-bromo-3-chloro-2-[(4-methoxyphenyl)methoxy]phenyl]ethanone

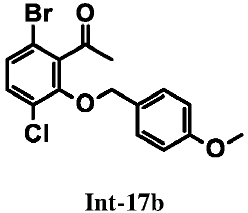

To a mixture of 1-(6-bromo-3-chloro-2-hydroxy-phenyl)ethanone (300 mg, 1.2 mmol) and 4-methoxybenzylchloride (0.2 mL, 1.44 mmol) in DMF (20 mL) was added potassium carbonate (415 mg, 3.01 mmol). The reaction mixture was stirred at 80 °C for 3 hours. Then, the reaction mixture was quenched with water and extracted with EtOAc (50 mL) three times. The combined organic layer was concentrated *in vacuo* to give the crude product. The crude product was purified by flash column (eluting with PE:EtOAc = 10:1) to give 1-[6-bromo-3-chloro-2-[(4-methoxyphenyl)methoxy]phenyl]ethanone (303 mg, yield: 68.17%) as a white solid. MS obsd. (ESI⁺)[(M+Na)⁺]: 391.0.

### Step 3: Preparation of 1-[3-chloro-2-hydroxy-6-(trifluoromethyl)phenyl]ethanone

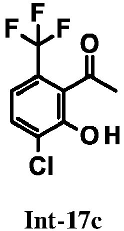

To a solution of 1-[6-bromo-3-chloro-2-[(4-methoxyphenyl)methoxy]phenyl]ethanone (300 mg, 0.810 mmol) and iodocopper (773 mg, 4.06 mmol) in DMF (8 mL) was added methyl 2,2-difluoro-2-fluorosulfinyl-acetate (715 mg, 4.06 mmol). The reaction mixture was stirred at 110 °C for 18 hours. The reaction mixture was quenched with water (10 mL) to form a suspension. The suspension was filtered, and the filter cake was washed with EtOAc (10 mL) three times. The filtrate was collected and layers were separated. The aqueous layer was extracted with EtOAc (10 mL) twice. The organic layer was combined, dried over anhydrous Na₂SO₄, filtered and concentrated *in vacuo.* The crude product was purified by flash chromatography (eluting with EtOAc : PE = 10%) to give 1-[3-chloro-2-hydroxy-6-(trifluoromethyl)phenyl]ethanone (135 mg, yield: 69.7%) as a colorless oil. MS obsd. (ESI⁺)[(M+Na)⁺]: 391.0.

### Step 4-7: Preparation of 2-(4-bromo-2-hydroxy-phenyl)-8-chloro-5-(trifluoromethyl)chromen-4-one

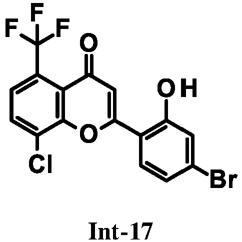

**Int-17** was prepared in analogy to the procedure described for the preparation of compound Int-5 by using 2-hydroxy-4-bromo-benzaldehyde as the starting materials instead of 4-chloro-2-hydroxy-5-methyl-benzaldehyde in **Step 2** and using **Int-17c** instead of 1-(3-chloro-2-hydroxy-phenyl)ethanone in **Step 3.** MS obsd. (ESI⁺)[(M+H)⁺]: 419.0.

### Intermediate 18: 8-chloro-2-(2-fluoro-6-hydroxy-4-methyl-phenyl)chromen-4-one

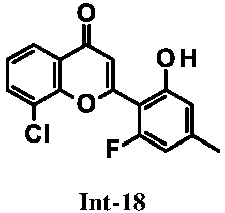

### Step 1: Preparation of 2-(4-bromo-2-fluoro-6-methoxy-phenyl)-8-chloro-chromen-4-one

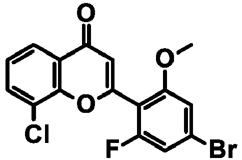

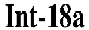

**Int-18a** was prepared in analogy to the procedure described for the preparation of compound **Int-1b** by using 4-bromo-2-fluoro-6-methoxybenzaldehyde (CAS registry number: 856767-09-4, Vendor: Bide Pharmatech, Catalog number: BD259901) as the starting material instead of 2-methoxy-4-(trifluoromethyl)benzaldehyde in **Step 1.** MS obsd. (ESI+) [(M+H)+]: 382.9.

### Step 2: Preparation of 8-chloro-2-(2-fluoro-6-methoxy-4-methyl-phenyl)chromen-4-one

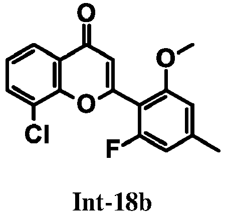

To a solution of 2-(4-bromo-2-fluoro-6-methoxy-phenyl)-8-chloro-chromen-4-one (300.0 mg, 0.8 mmol), trimethylboroxine (196.4 mg, 1.56 mmol) and K₂CO₃ (324.3 mg, 2.35 mmol) in dioxane (10 mL) under N₂ was added Pd(dppf)₂Cl₂ (57.8 mg, 0.08mmol). The reaction was stirred at 110 °C under N₂ for 1 hour. After the reaction was completed, the mixture was cooled to room temperature and concentrated *in vacuo.* The crude product was purified by flash column (eluting with EtOAc : PE = 0 to 30%) to give 8-chloro-2-(2-fluoro-6-methoxy-4-methylphenyl)chromen-4-one (150 mg, yield: 57.2%) as an off-white solid. MS obsd. (ESI+) [(M+H)+]: 319.0.

### Step 3: Preparation of 8-chloro-2-(2-fluoro-6-hydroxy-4-methyl-phenyl)chromen-4-one

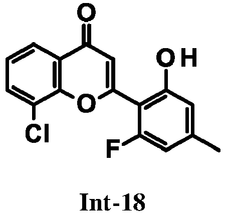

To a solution of 8-chloro-2-(2-fluoro-6-methoxy-4-methyl-phenyl)chromen-4-one (0.15 g, 0.47 mmol) in dichloromethane (10 mL) was added BBr₃ (1M solution in dichloromethane, 7 mL, 7 mmol) at room temperature. The mixture was stirred at room temperature overnight. After the reaction was completed, the mixture was concentrated *in vacuo* and then the residue was suspended in sat. NH₄Cl solution (10 mL). The solid was collected by filtration and dried *in vacuo* to give 8-chloro-2-[2-hydroxy-4-(trifluoromethyl)phenyl]chromen-4-one (0.124 g, yield: 86%) as a yellow solid, which was used in the next step without further purification. MS obsd. (ESI⁺) [(M+H)]⁺: 305.0.

### Intermediate 19: 8-chloro-2-(2-hydroxy-6-methoxy-4-methyl-phenyl)chromen-4-one

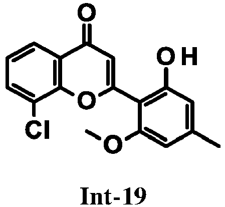

### Step 1: Preparation of 4-bromo-2-methoxy-6-[(4-methoxyphenyl)methoxy]benzaldehyde

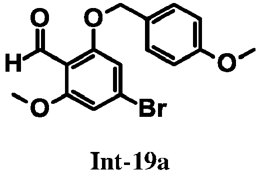

To a solution of 4-bromo-2-hydroxy-6-methoxy-benzaldehyde (1.4 g, 6.06 mmol) in 4-methoxybenzylchloride (0.99 mL, 7.27 mmol) was added potassium carbonate (2.5 g, 18.18 mmol). The reaction was stirred at 80 °C for 2 hours. After completion, the reaction was quenched by adding water (50 mL) and extracted with EtOAc (50 mL) three times. The combined organic layer was washed with brine, dried over anhydrous sodium sulfate and concentrated *in vacuo* to give the crude product. The crude product was purified by flash column (EtOAc : PE = 15%) to give 4-bromo-2-methoxy-6-[(4-methoxyphenyl)methoxy]benzaldehyde (1.9 g, yield: 93%) as a yellow solid. MS obsd. (ESI⁺): 373.0.

### Step 2: Preparation of 2-methoxy-6-[(4-methoxyphenyl)methoxy]-4-methyl-benzaldehyde

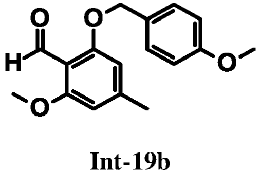

To a solution of 4-bromo-2-methoxy-6-[(4-methoxyphenyl)methoxy]benzaldehyde (1.2 mg, 3.42 mmol) in 1,4-dioxane (16.79 mL) was added trimethylboroxine (857 mg, 6.83 mmol) and [1,1'-bis(diphenylphosphino)ferrocene]dichloropalladium(II) (250 mg, 0.340 mmol) under N₂ at room temperature. Then, the reaction was heated at 110 °C for 16 hours. After the reaction was completed, the reaction was quenched by adding ice-water (50 mL) and extracted with EtOAc (50 mL) three times. The combined organic layer was washed with brine, dried over anhydrous sodium sulfate and concentrated *in vacuo* to give the crude product. The crude product was purified by flash column (EA:PE = 50%) to give 2-methoxy-6-[(4-methoxyphenyl)methoxy]-4-methyl-benzaldehyde (700 mg, yield: 67.9%) as a yellow solid. MS obsd. (ESI⁺)[(M+H)⁺]: 287.1.

### Step 3: Preparation of 2-hydroxy-6-methoxy-4-methyl-benzaldehyde

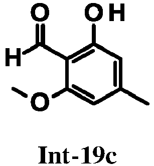

To a solution of 2-methoxy-6-[(4-methoxyphenyl)methoxy]-4-methyl-benzaldehyde (650 mg, 2.27 mmol) in DCM (9 mL) was added sodium hydroxide (181 mg, 4.54 mmol). The reaction mixture was stirred at room temperature for 30 minutes, then trifluoroacetic acid (0.17 mL, 2.27 mmol) was added. After stirring for another 1 hour, the reaction was quenched by adding water (50 mL) and extracted with EtOAc (50 mL) three times. The combined organic layer was washed with brine, dried over anhydrous sodium sulfate and concentrated *in vacuo* to give the crude product. The crude product was purified by flash column (EtOAc : PE = 20%) to give 2-hydroxy-6-methoxy-4-methyl-benzaldehyde (290 mg, yield: 76.87%) as a white solid. MS obsd. (ESI⁺)[(M+H)⁺]: 167.1.

### Step 4: Preparation of 2-methoxy-6-(methoxymethoxy)-4-methyl-benzaldehyde

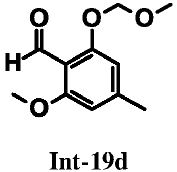

A solution of 2-hydroxy-6-methoxy-4-methyl-benzaldehyde (293 mg, 1.77 mmol) in THF (135 mL) was added bromomethyl methyl ether (441 mg, 3.53 mmol) and sodium hydride (51 mg, 2.12 mmol) at 0 °C for 30 minutes. Then, the reaction solution was diluted with water (50 mL) and extracted with EtOAc (50 mL) three times. The combined organic layer was washed with brine, dried over anhydrous sodium sulfate and concentrated *in vacuo* to give the crude product. The crude product was purified by flash column (EtOAc : PE = 10%) to give 2-methoxy-6-(methoxymethoxy)-4-methyl-benzaldehyde (250 mg, yield: 67.3%) as colorless liquid. MS obsd. (ESI⁺) [(M+H)⁺]: 211.1.

### Step 4: Preparation of (E)-1-(3-chloro-2-hydroxy-phenyl)-3-[2-methoxy-6-(methoxymethoxy)-4-methyl-phenyl]prop-2-en-1-one

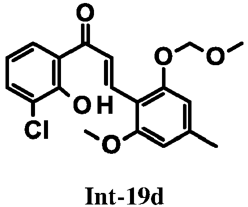

A solution of 1-(3-chloro-2-hydroxy-phenyl)ethanone (203 mg, 1.19 mmol) in THF (50 mL) was added potassium hydroxide (667 mg, 11.89 mmol) and 2-methoxy-6-(methoxymethoxy)-4-methyl-benzaldehyde (250 mg, 1.19 mmol) at 35 °C for 16 hours. After the reaction was completed, the reaction was adjusted to pH 6-7 by adding 1M HCl. Then, the reaction mixture was partitioned between water (50 mL) and EtOAc (50 mL). The aqueous layer was extracted with EtOAc (50 mL) twice and the combined organic layer was washed with brine, dried over anhydrous sodium sulfate and concentrated *in vacuo* to give the crude product. The crude product was purified by flash column (EtOAc : PE = 10%) to give (*E*)-1-(3-chloro-2-hydroxy-phenyl)-3-[2-methoxy-6-(methoxymethoxy)-4-methyl-phenyl]prop-2-en-1-one (320 mg, yield: 70.8%) as yellow oil. MS obsd. (ESI⁺)[(M+H)⁺]: 363.1.

### Step 5: Preparation of 8-chloro-2-(2-hydroxy-6-methoxy-4-methyl-phenyl)chromen-4-one

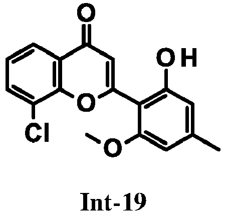

A solution of (*E*)-1-(3-chloro-2-hydroxy-phenyl)-3-[2-methoxy-6-(methoxymethoxy)-4-methyl-phenyl]prop-2-en-1-one (320 mg, 0.880 mmol) and iodine (11.19 mg, 0.040 mmol) in DMSO (2.32 mL) was stirred at 140 °C for 4 hours. Then, the reaction mixture was diluted with water (20 mL) to form a suspension. The suspension was filtered and the filter cake was washed with water (50 mL) and dried to give 8-chloro-2-(2-hydroxy-6-methoxy-4-methylphenyl)chromen-4-one (130 mg, yield: 44.2%) as a white solid. MS obsd. [(M+H)⁺] (ESI⁺): 317.1.

### Example 001: 1-[3-[5-bromo-2-(8-chloro-4-oxo-chromen-2-yl)phenoxylpropyllpyrrolidine-3-carboxylic acid

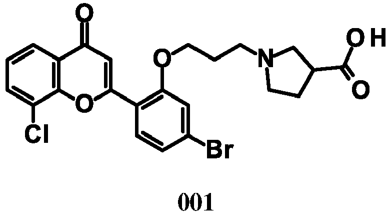

### Step 1: Preparation of 2-[4-bromo-2-(3-bromopropoxy)phenyl]-8-chloro-chromen-4-one

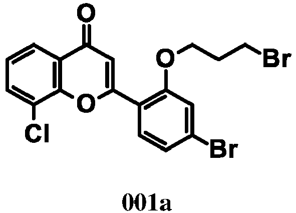

To a solution of 2-(4-bromo-2-hydroxy-phenyl)-8-chloro-chromen-4-one (300 mg, 0.853 mmol, as the **"CORE"** in Table 1) and 1,3-dibromopropane (861 mg, 4.27 mmol, as the **"LINKER"** in Table 1) in DMF was added K₂CO₃ (236 mg, 1.71 mmol). The reaction mixture was stirred at 70 °C for 2 hours. After the reaction was completed, the mixture was diluted by EtOAc and partitioned between EtOAc (10 mL) and water (30 mL). The organic layer was separated out and the aqueous layer was extracted with EtOAc (30 mL) twice. The combined organic layer was concentrated *in vacuo* and the resulting residue was purified by ISCO (eluting with EtOAc : PE = 0 to 20%) to give 2-[4-bromo-2-(3-bromopropoxy)phenyl]-8-chloro-chromen-4-one (200 mg, yield: 49.6%) as a yellow solid. MS obsd. (ESI⁺) [(M+H)]⁺: 471.3.

### Step 2: Preparation of methyl 1-[3-[5-bromo-2-(8-chloro-4-oxo-chromen-2-yl)phenoxy]propyl]pyrrolidine-3-carboxylate

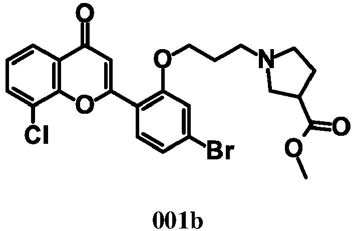

To a solution of 2-[4-bromo-2-(3-bromopropoxy)phenyl]-8-chloro-chromen-4-one (300 mg, 0.635 mmol) and methyl pyrrolidine-3-carboxylate hydrochloride (105 mg, 0.635 mmol, as the **"AMINE"** in Table 1) in DMF was added NaHCO₃ (53.3 mg, 0.635 mmol). The mixture was stirred at 50 °C overnight. After the reaction was completed, the reaction mixture was diluted with EtOAc and partitioned between EtOAc (30 mL) and water (20 mL). The organic layer was separated out and the aqueous phase was extracted with EtOAc (30 mL) twice. The combined organic layer was concentrated *in vacuo* to give methyl 1-[3-[5-bromo-2-(8-chloro-4-oxo-chromen-2-yl)phenoxy]propyl]pyrrolidine-3-carboxylate (100 mg, yield: 30.2%) as a yellow solid. (ESI⁺) [(M+H)]⁺: 521.9.

### Step 3: Preparation of 1-[3-[5-bromo-2-(8-chloro-4-oxo-chromen-2-yl)phenoxy]propyl]pyrrolidine-3-carboxylic acid

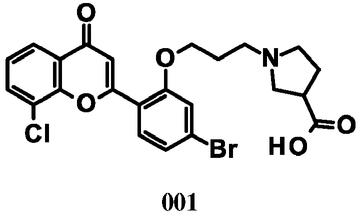

To the solution of methyl 1-[3-[5-bromo-2-(8-chloro-4-oxo-chromen-2-yl)phenoxy]propyl]pyrrolidine-3-carboxylate (100 mg, 0.192 mmol) in DMF was added LiOH (13.8 mg, 0.576 mmol). The reaction mixture was stirred at room temperature till completion. Then, the reaction mixture was neutralized by acetic acid and then concentrated *in vacuo.* The crude material was purified by prep-HPLC to give 1-[3-[5-bromo-2-(8-chloro-4-oxo-chromen-2-yl)phenoxy]propyl]pyrrolidine-3-carboxylic acid (33 mg, yield: 31.2%) as a white powder. (ESI⁺) [(M+H)]⁺: 508.0. **Example 001:** ¹H NMR (DMSO-*d₆*, 400MHz) δ ppm 8.02 (dd, *J* = 7.9, 1.5 Hz, 2H), 7.87 (d, *J* = 8.4 Hz, 1H), 7.39-7.55 (m, 3H), 7.01 (s, 1H), 4.28 (br t, *J* = 6.1 Hz, 2H), 2.64-2.84 (m, 4H), 2.54-2.60 (m, 2H), 2.32-2.42 (m, 1H), 1.87-1.98 (m, 4H).

The following Examples **002** to **061** were prepared in analogy to the procedure described for the preparation of Example **001,** replacing 2-(4-bromo-2-hydroxy-phenyl)-8-chloro-chromen-4-one with **"CORE",** 1,3-dibromopropane with **"LINKER"** in **Step 1,** methyl pyrrolidine-3-carboxylate hydrochloride with **"AMINE"** by the reagent indicated in Table 1.

**Table 1: Compounds synthesis and characterization**

| Examp le No. | Compounds Name and Structure | **CORE, LINKER** and **AMINE** | ¹H NMR and (ESI⁺) |
|---|---|---|---|
| **002** | **4-[3-[5-bromo-2-(8-chloro-4-oxo-chromen-2-yl)phenoxy]propyl]morpholi ne-2-carboxylic acid** | **CORE: Int-4** | ¹H NMR (DMSO-*d₆*, 400MHz) δ ppm 8.01 (d, *J* = 7.9 Hz, 2H), 7.88 (d, *J* = 8.4 Hz, 1H), 7.42-7.55 (m, 3H), 7.03 (s, 1H), 4.18-4.38 (m, 3H), 3.85-4.08 (m, 2H), 3.47-3.81 (m, 2H), 2.87-3.16 (m, 2H), 1.99-2.26 (m, 2H), 1.17-1.33 ppm (m, 2H). MS obsd. (ESI⁺) [(M+H)⁺]: 521.9. |
| | | **LINKER:** 1,3-dibromopropane | |
| | | **AMINE:** methyl morpholine-2-carboxylate | |
| | 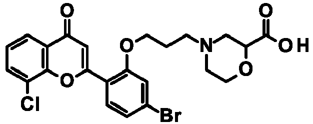 | | |
| **003** | **(2R)-1-[3-[5-bromo-2-(8-chloro-4-oxo-chromen-2-yl)phenoxy]propyl]pyrrolidin e-2-carboxylic acid** | **CORE: Int-4** | ¹H NMR (DMSO-*d₆*, 400MHz) δ ppm 8.15-8.20 (m, 1H), 7.98-8.04 (m, 1H), 7.82-7.90 (m, 1H), 7.41-7.55 (m, 2H), 6.96-7.06 (m, 1H), 6.48-6.61 (m, 1H), 4.13-4.34 (m, 3H), 3.60-3.70 (m, 1H), 2.63-2.89 (m, 3H), 2.27-2.38 (m, 1H), 2.08-2.17 (m, 1H), 1.85-1.98 (m, 1H), 1.52-1.74 (m, 3H). MS obsd. (ESI⁺) [(M+H)⁺]: 506.0. |
| | | **LINKER:** 1,3-dibromopropane | |
| | | **AMINE:** methyl (2R)-pyrrolidine-2-carboxylate | |
| | 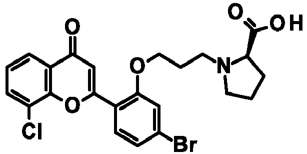 | | |
| **004** | **(2S)-1-[3-[5-bromo-2-(8-chloro-4-oxo-chromen-2-yl)phenoxy]propyl]pyrrolidin e-2-carboxylic acid** | **CORE: Int-4** | ¹H NMR (DMSO-*d₆*, 400MHz) δ ppm 8.20-8.24 (m, 1H), 7.98-8.03 (m, 2H), 7.85-7.89 (m, 1H), 7.40-7.55 (m, 3H), 7.02 (s, 1H), 4.16-4.33 (m, 4H), 2.69-2.87 (m, 2H), 2.08-2.17 (m, 2H), 1.85-2.04 (m, 2H), 1.53-1.73 (m, 2H). MS obsd. (ESI⁺) [(M+H)⁺]: 506.0. |
| | | **LINKER:** 1,3-dibromopropane | |
| | | **AMINE:** methyl (2S)-pyrrolidine-2-carboxylate | |
| | 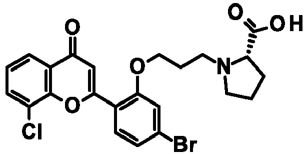 | | |
| **005** | **(3R)-1-[3-[5-bromo-2-(8-chloro-4-oxo-chromen-2-yl)phenoxy]propyl]pyrrolidin e-3-carboxylic acid** | **CORE: Int-4** | ¹H NMR (DMSO-*d₆*, 400MHz) δ ppm 8.01 (dq, *J* = 7.8, 1.5 Hz, 2H), 7.88 (d, *J* = 8.4 Hz, 1H), 7.47-7.56 (m, 2H), 7.42 (dd, *J* = 8.4, 1.7 Hz, 1H), 7.01-7.10 (m, 1H), 4.24 (t, *J* = 6.2 Hz, 2H), 2.64-2.84 (m, 4H), 2.54-2.60 (m, 2H), 2.32-2.42 (m, 1H), 1.87-1.98 (m, 4H). MS obsd. (ESI⁺) [(M+H)⁺]: 505.9. |
| | | **LINKER:** 1,3-dibromopropane | |
| | | **AMINE:** methyl (3R)-pyrrolidine-3-carboxylate | |
| | 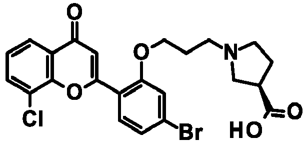 | | |
| **006** | **3-[3-[2-(8-chloro-4-oxo-chromen-2-yl)-5-(trifluoromethyl)phenoxy]pr opylamino] cyclobutanecarbo xylic acid** | **CORE: Int-1** | ¹H NMR (DMSO-*d₆*, 400MHz) δ ppm 12.19-12.69 (br s, 1H), 8.65-8.99 (m, 1H), 7.90-8.18 (m, 3H), 7.44-7.65 (m, 3H), 6.94-7.05 (m, 1H), 4.26-4.46 (m, 2H), 3.71-3.94 (m, 1H), 2.74-3.20 (m, 4H), 2.33-2.45 (m, 3H), 2.00-2.14 (m, 2H). MS obsd. (ESI⁺) [(M+H)⁺]: 495.9. |
| | | **LINKER:** 1,3-dibromopropane | |
| | | **AMINE:** methyl 3-aminocyclobuta necarboxylate hydrochloride | |
| | 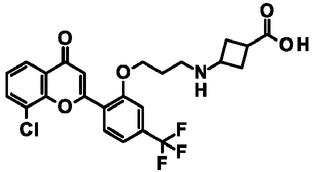 | | |
| **007** | **3-[3-[2-(8-chloro-4-oxo-chromen-2-yl)-5-(trifluoromethyl)phenoxy]pr opylamino]cyclopentanecarb oxylic acid** | **CORE: Int-1** | ¹H NMR (DMSO-*d₆*, 400MHz) δ ppm 12.26-12.53 (br s, 1H), 8.64 (br s, 1H), 8.02-8.13 (m, 3H), 7.51-7.63 (m, 2H), 7.02 (s, 1H), 4.31-4.43 (m, 2H), 3.76-3.98 (m, 1H), 3.00 - 3.11 (m, 2H), 2.70-2.81 (m, 1H), 2.06-2.29 (m, 3H), 1.90-2.01 (m, 1H), 1.80-1.87 (m, 2H), 1.69-1.78 (m, 1H), 1.56-1.70 (m, 1H). MS obsd. (ESI⁺) [(M+H)⁺]: 509.9. |
| | | **LINKER:** 1,3-dibromopropane | |
| | | **AMINE:** methyl 3-aminocyclopent anecarboxylate hydrochloride | |
| | 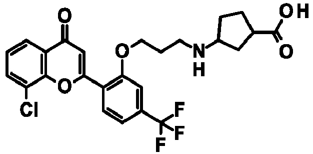 | | |
| **008** | **2-[1-[3-[2-(8-chloro-4-oxo-chromen-2-yl)-5-(trifluoromethyl)phenoxy]pr opyl]pyrrolidin-3-yl]acetic acid** | **CORE: Int-1** | ¹H NMR (METHANOL-d4, 400MHz) δ ppm 8.24 (d, *J* =8.6 Hz, 1H), 8.10 (dd, *J* =7.9, 1.3 Hz, 1H), 7.94 (dd, *J* =7.8, 1.3 Hz, 1H), 7.46-7.55 (m, 3H), 7.27 (s, 1H), 4.30-4.42 (m, 4H), 3.52 (dd, *J* =11.6, 7.6 Hz, 1H), 3.35-3.44 (m, 1H), 3.16-3.31 (m, 1H), 2.93 (dd, *J* =11.4, 8.7 Hz, 1H), 2.49-2.73 (m, 3H), 2.17-2.37 (m, 3H), 1.67 (dq, *J* =13.2, 8.8 Hz, 1H). MS obsd. (ESI⁺) [(M+H)⁺]: 510.2. |
| | | **LINKER:** 1,3-dibromopropane | |
| | | **AMINE:** methyl 2-pyrrolidin-3-ylacetate hydrochloride | |
| | 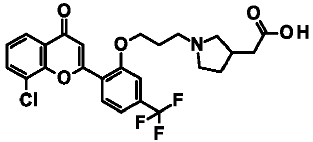 | | |
| **009** | **(2R)-2-[3-[2-(8-chloro-4-oxo-chromen-2-yl)-5-(trifluoromethyl)phenoxy]pr opylamino] -3-methylbutanoic acid** | **CORE: Int-1** | ¹H NMR (DMSO-*d₆*, 400MHz) δ ppm 8.09-8.16 (m, 1H), 8.04 (s, 2H), 7.57 (s, 3H), 7.05 (s, 1H), 4.31-4.39 (m, 2H), 2.70-2.95 (m, 2H), 1.87-2.11 (m, 3H), 1.19-1.29 (m, 1H), 0.80-0.92 (m, 6H). MS obsd. (ESI⁺) [(M+H)⁺]: 498.1. |
| | | **LINKER:** 1,3-dibromopropane | |
| | | **AMINE:** methyl (2R)-2-amino-3-methylbutanoate | |
| | 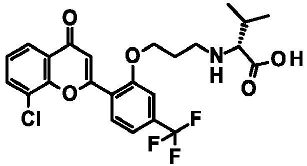 | | |
| **010** | **2-[[3-[[2-(8-chloro-4-oxo-chromen-2-yl)-5-(trifluoromethyl)phenoxy] me thyl]oxetan-3-yl]methylamino]-2-oxo-acetic acid** | **CORE: Int-1** | ¹H NMR (DMSO-*d₆*, 400MHz) δ ppm 8.55-8.79 (m, 1H), 8.10 (d, *J* = 8.3 Hz, 1H), 7.96-8.05 (m, 2H), 7.56-7.67 (m, 2H), 7.51 (t, *J* = 7.9 Hz, 1H), 7.06 (s, 2H), 4.43 (d, *J* =7.1 Hz, 4H), 4.35 (s, 2H), 3.52 (br d, *J* =6.1 Hz, 2H). (ESI⁺) [(M+H)⁺]: 512.0. |
| | | **LINKER:** 3,3-bis(bromomethy l)oxetane | |
| | | **AMINE:** ethyl 2-amino-2-oxo-acetate | |
| | 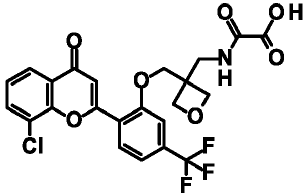 | | |
| **011** | **2-[3-[2-(8-chloro-4-oxo-chromen-2-yl)-5-(trifluoromethyl)phenoxy]pr opylamino]-4,4-dimethyl-pentanoic acid** | **CORE: Int-1** | ¹H NMR (DMSO-*d₆*, 400MHz) δ ppm 7.96-8.23 (m, 3H), 7.44-7.65 (m, 3H), 7.04 (s, 1H), 4.35 (br s, 2H), 2.90 (br d, *J* =0.7 Hz, 2H), 2.07 (s, 4H), 1.58-1.73 (m, 1H), 0.87 (s, 9H). MS obsd. (ESI⁺) [(M+H)⁺]: 526.2. |
| | | **LINKER:** 1,3-dibromopropane | |
| | | **AMINE:** methyl 2-amino-4,4-dimethyl-pentanoate | |
| | 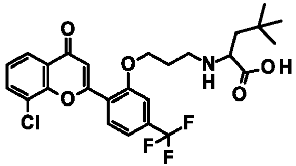 | | |
| **012** | **1-[3-[2-(8-chloro-4-oxo-chromen-2-yl)-5-(trifluoromethyl)phenoxy]pr opyl]pyrrolidine-3-carboxylic acid** | **CORE: Int-1** | ¹H NMR (DMSO-d6, 400MHz) δ ppm 8.09-8.16 (m, 1H), 7.98-8.07 (m, 2H), 7.47-7.64 (m, 3H), 7.02-7.13 (m, 1H), 4.26-4.40 (m, 2H), 2.72-3.16 (m, 7H), 1.93-2.16 (m, 4H). MS obsd. (ESI+) [(M+H)+]: 496.1 |
| | | **LINKER:** 1,3-dibromopropane | |
| | | **AMINE:** methyl pyrrolidine-3-carboxylate hydrochloride | |
| | 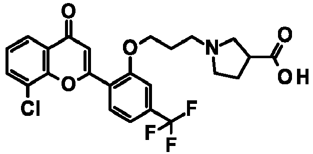 | | |
| **013** | **(3R)-1-[3-[2-(8-chloro-4-oxo-chromen-2-yl)-5-(trifluoromethyl)phenoxy]pr opyl]pyrrolidine-3-carboxylic acid** | **CORE: Int-1** | ¹H NMR (DMSO-d6, 400MHz) δ ppm 8.09-8.16 (m, 1H), 7.98-8.07 (m, 2H), 7.47-7.64 (m, 3H), 7.02-7.13 (m, 1H), 4.26-4.40 (m, 2H), 2.72-3.16 (m, 7H), 1.93-2.16 (m, 4H). MS obsd. (ESI+) [(M+H)+]: 496.1 |
| | | **LINKER:** 1,3-dibromopropane | |
| | | **AMINE:** methyl (3R)-pyrrolidine-3-carboxylate | |
| | 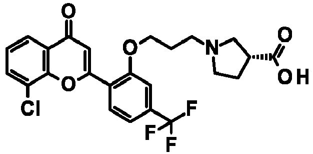 | | |
| **014** | **(3S)-1-[3-[2-(8-chloro-4-oxo-chromen-2-yl)-5-(trifluoromethyl)phenoxy]pr opyl]pyrrolidine-3-carboxylic acid** | **CORE: Int-1** | ¹H NMR (DMSO-d6, 400MHz) δ ppm 8.07-8.15 (m, 1H), 7.98-8.06 (m, 2H), 7.47-7.60 (m, 3H), 7.05-7.11 (m, 1H), 4.25-4.36 (m, 2H), 2.83-2.93 (m, 1H), 2.67-2.74 (m, 1H), 2.52-2.59 (m, 4H), 2.37-2.45 (m, 1H), 1.86-2.02 (m, 4H). MS obsd. (ESI+) [(M+H)+]: 496.1 |
| | | **LINKER:** 1,3-dibromopropane | |
| | | **AMINE:** methyl (3S)-pyrrolidine-3-carboxylate | |
| | 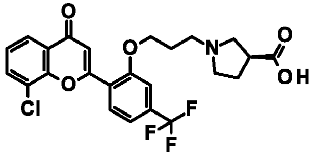 | | |
| **015** | **(3S)-1-[3-[2-(8-chloro-4-oxo-chromen-2-yl)-5-ethoxy-phenoxy]propyl]pyrrolidine-3-carboxylic acid** | **CORE: Int-13** | ¹H NMR (DMSO-*d₆*, 400MHz) δ ppm 7.97 (s, 3H), 7.43-7.51 (m, 1H), 7.02-7.09 (m, 1H), 6.72-6.81 (m, 2H), 4.10-4.25 (m, 4H), 2.86-2.97 (m, 1H), 2.70-2.78 (m, 1H), 2.53-2.64 (m, 5H), 1.87-2.02 (m, 4H), 1.31-1.42 (m, 3H). MS obsd. (ESI⁺) [(M+H)⁺]: 472.1. |
| | | **LINKER:** 1,3-dibromopropane | |
| | | **AMINE:** methyl (3S)-pyrrolidine-3-carboxylate | |
| | 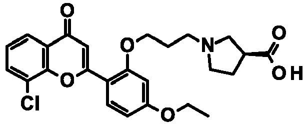 | | |
| **016** | **(3R)-1-[3-[5-bromo-2-(8-chloro-4-oxo-chromen-2-yl)-4-methoxy-phenoxy]propyl]pyrrolidine-3-carboxylic acid** | **CORE: Int-7** | ¹H NMR (DMSO-*d₆*, 400MHz) δ ppm 7.97-8.04 (m, 2H), 7.58-7.64 (m, 1H), 7.45-7.57 (m, 2H), 7.03-7.09 (m, 1H), 4.16-4.24 (m, 2H), 3.90 (s, 3H), 2.75-3.11 (m, 7H), 1.95-2.10 (m, 4H). MS obsd. (ESI⁺) [(M+H)⁺]: 536.1. |
| | | **LINKER:** 1,3-dibromopropane | |
| | | **AMINE:** methyl (3R)-pyrrolidine-3-carboxylate | |
| | 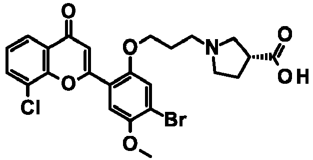 | | |
| **017** | **(3S)-1-[3-[5-bromo-2-(8-chloro-4-oxo-chromen-2-yl)-4-methoxy-phenoxy]propyl]pyrrolidine-3-carboxylic acid** | **CORE: Int-7** | ¹H NMR (DMSO-*d₆*, 400MHz) δ ppm 7.96-8.05 (m, 2H), 7.60-7.63 (m, 1H), 7.45-7.55 (m, 2H), 7.04-7.11 (m, 1H), 4.13-4.21 (m, 2H), 3.88 (s, 3H), 2.65-2.94 (m, 3H), 2.31-2.46 (m, 3H), 1.84-1.98 (m, 5H). MS obsd. (ESI⁺) [(M+H)+]: 536.1. |
| | | **LINKER:** 1,3-dibromopropane | |
| | | **AMINE:** methyl (3S)-pyrrolidine-3-carboxylate | |
| | 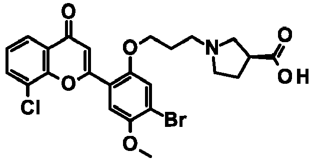 | | |
| **018** | **4-[3-[2-(8-chloro-4-oxo-chromen-2-yl)-5-(trifluoromethyl)phenoxy]pr opyl]morpholine-2-carboxylic acid** | **CORE: Int-1** | ¹H NMR (DMSO-*d₆*, 400MHz) δ ppm 8.13 (d, *J* = 7.8 Hz, 1H), 8.04 (d, *J* = 7.8 Hz, 2H), 7.45-7.64 (m, 3H), 7.02-7.10 (m, 1H), 4.21-4.46 (m, 3H), 3.89-4.16 (m, 2H), 3.63-3.84 (m, 2H), 3.21-3.22 (m, 2H), 2.89-3.15 (m, 2H), 2.09-2.26 (m, 2H). MS obsd. (ESI⁺) [(M+H)⁺]: 512.0. |
| | | **LINKER:** 1,3-dibromopropane | |
| | | **AMINE:** methyl morpholine-3-carboxylate | |
| | 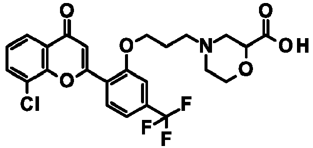 | | |
| **019** | **1-[2-[5-bromo-2-(8-chloro-4-oxo-chromen-2-yl)phenoxy]ethyl]azetidine-3-carboxylic acid** | **CORE: Int-4** | ¹H NMR (DMSO-*d₆*, 400MHz) δ ppm 7.98 (dd, *J* = 8.0, 1.5 Hz, 1H), 7.80-7.94 (m, 2H), 7.39-7.49 (m, 2H), 7.35 (dd, *J* = 8.4, 1.8 Hz, 1H), 7.09 (s, 1H), 3.79-3.93 (m, 2H), 3.60-3.71 (m, 2H), 3.42-3.55 (m, 2H), 3.10-3.15 (m, 1H), 3.02-3.07 (m, 2H). MS obsd. (ESI⁺) [(M+H)⁺]: 478.3. |
| | | **LINKER:** 1,2-dibromoethane | |
| | | **AMINE:** methyl azetidine-3-carboxylate hydrochloride | |
| | 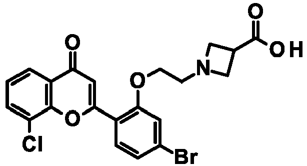 | | |
| **020** | **1-[2-[5-bromo-2-(8-chloro-4-oxo-chromen-2-yl)phenoxy]ethyl]pyrrolidine-2-carboxylic acid** | **CORE: Int-4** | ¹H NMR (METHANOL-*d₄*, 400MHz) δ ppm 7.96-8.02 (m, 1H), 7.79-7.86 (m, 2H), 7.28-7.41 (m, 3H), 7.07-7.13 (m, 1H), 4.33-4.48 (m, 2H), 3.49-3.92 (m, 4H), 2.97-3.12 (m, 1H), 2.24-2.36 (m, 1H), 1.68-2.03 (m, 3H). MS obsd. (ESI⁺) [(M+H)⁺]: 492.1. |
| | | **LINKER:** 1,2-dibromoethane | |
| | | **AMINE:** methyl pyrrolidine-2-carboxylate hydrochloride | |
| | 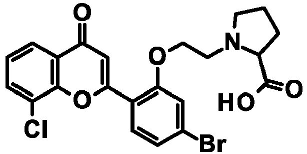 | | |
| **021** | **1-[2-[5-bromo-2-(8-chloro-4-oxo-chromen-2-yl)phenoxy]ethyl]pyrrolidine-3-carboxylic acid** | **CORE: Int-4** | ¹H NMR (DMSO-*d₆*, 400MHz) δ ppm 8.02 (d, *J* = 7.8 Hz, 2H), 7.84 (d, *J* = 8.4 Hz, 1H), 7.60 (d, *J* = 1.7 Hz, 1H), 7.45-7.54 (m, 2H), 7.00 (s, 1H), 4.45-4.55 (m, 2H), 3.57-3.69 (m, 2H), 3.03-3.23 (m, 1H), 1.87-2.28 (m, 4H), 1.12-1.33 (m, 2H). MS obsd. (ESI⁺) [(M+H)⁺]: 492.0. |
| | | **LINKER:** 1,2-dibromoethane | |
| | | **AMINE:** methyl pyrrolidine-3-carboxylate hydrochloride | |
| | 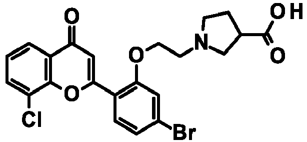 | | |
| **022** | **1-[2-[5-bromo-2-(8-chloro-4-oxo-chromen-2-yl)phenoxy]ethyl]piperidine-4-carboxylic acid** | **CORE: Int-4** | ¹H NMR (DMSO-*d₆*, 400MHz) δ ppm 7.99 (dd, *J* = 8.1, 1.5 Hz, 1H), 7.80-7.87 (m, 2H), 7.34-7.43 (m, 2H), 7.28-7.34 (m, 1H), 7.09 (s, 1H),4.35 (t, *J* = 5.1 Hz, 2H), 3.09-3.14 (m, 2H), 2.58-2.70 (m, 2H), 2.18-2.30 (m, 1H), 1.82-1.98 (m, 2H), 1.62-1.78 (m, 4H). MS obsd. (ESI⁺) [(M+H)⁺]: 506.4. |
| | | **LINKER:** 1,2-dibromoethane | |
| | | **AMINE:** methyl piperidine-4-carboxylate hydrochloride | |
| | 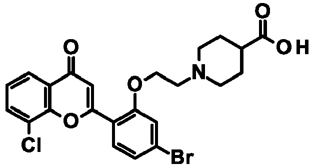 | | |
| **023** | **4-[2-[5-bromo-2-(8-chloro-4-oxo-chromen-2-yl)phenoxy]ethyl]morpholine -2-carboxylic acid** | **CORE: Int-4** | ¹H NMR (DMSO-*d₆*, 400MHz) δ ppm 7.91-7.97 (m, 2H), 7.83-7.88 (m, 1H), 7.48 (d, *J* = 1.6 Hz, 1H), 7.33-7.45 (m, 3H), 4.26 (br t, *J* = 5.4 Hz, 2H), 4.08 (br dd, *J* = 8.6, 2.3 Hz, 1H), 3.73-3.82 (m, 1H), 3.51-3.58 (m, 1H), 2.86-2.92 (m, 1H), 2.72 (br d, *J* = 6.7 Hz, 2H), 2.63 (br d, *J* = 12.8 Hz, 1H), 2.15-2.27 (m, 2H). MS obsd. (ESI⁺) [(M+H)⁺]: 506.0. |
| | | **LINKER:** 1,2-dibromoethane | |
| | | **AMINE:** methyl morpholine-2-carboxylate hydrochloride | |
| | 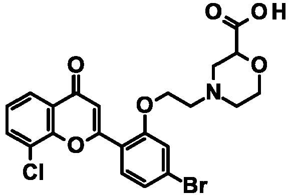 | | |
| **024** | **(3S,4S)-1-[2-[5-bromo-2-(8-chloro-4-oxo-chromen-2-yl)phenoxy]ethyl]-4-methyl-pyrrolidine-3-carboxylic acid** | **CORE: Int-4** | ¹H NMR (DMSO-*d₆*, 400MHz) δ ppm 9.96-10.31 (br s, 1H), 8.02 (d, *J* = 7.9 Hz, 2H), 7.83 (d, *J* = 8.4 Hz, 1H), 7.57-7.65 (m, 1H), 7.42-7.55 (m, 2H), 6.96 (s, 1H), 4.48-4.54 (m, 2H), 3.74-3.90 (m, 2H), 3.59-3.74 (m, 3H), 2.65-2.98 (m, 2H), 2.25-2.44 (m, 1H), 1.04 (d, J = 6.6 Hz, 3H). MS obsd. (ESI⁺) [(M+H)⁺]: 506.1. |
| | | **LINKER:** 1,2-dibromoethane | |
| | | **AMINE:** methyl (3S,4S)-4-methylpyrrolidin e-3-carboxylate hydrochloride | |
| | 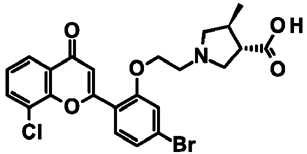 | | |
| **025** | **8-[2-[5-bromo-2-(8-chloro-4-oxo-chromen-2-yl)phenoxy]ethyl]-8-azabicyclo[3.2.1]octane-3-carboxylic acid** | **CORE: Int-4** | ¹H NMR (DMSO-*d₆*, 400MHz) δ ppm 12.34-12.64 (m, 1H), 7.93-8.15 (m, 2H), 7.83 (d, *J* = 8.3 Hz, 1H), 7.38-7.64 (m, 3H), 6.85-7.04 (m, 1H), 4.47-4.65 (m, 2H), 3.97-4.15 (m, 3H), 2.63-2.82 (m, 2H), 2.04-2.24 (m, 2H), 1.93-2.04 (m, 2H), 1.72-1.90 (m, 4H). MS obsd. (ESI⁺) [(M+H)⁺]: 532.2. |
| | | **LINKER:** 1,2-dibromoethane | |
| | | **AMINE:** methyl 8-azabicyclo[3.2.1 ]octane-3-carboxylate hydrochloride | |
| | 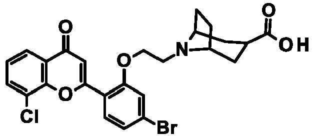 | | |
| **026** | **3-[2-[5-bromo-2-(8-chloro-4-oxo-chromen-2-yl)phenoxy]ethylamino]bicycl o[1.1.1]pentane-1-carboxylic acid** | **CORE: Int-4** | ¹H NMR (DMSO-*d₆*, 400MHz) δ ppm 8.03 (d, *J* = 7.8 Hz, 2H), 7.87 (d, *J* = 8.4 Hz, 1H), 7.44-7.61 (m, 3H), 7.07 (s, 1H), 4.35-4.46 (m, 2H), 2.54-2.64 (m, 2H), 2.11-2.29 (m, 6H). MS obsd. (ESI⁺) [(M+H)⁺]: 504.2. |
| | | **LINKER:** 1,2-dibromoethane | |
| | | **AMINE:** methyl 3-aminobicyclo[1. 1.1]pentane-1-carboxylate hydrochloride | |
| | 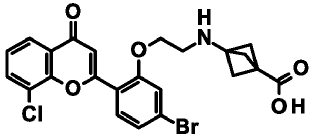 | | |
| **027** | **1-[2-[5-bromo-2-(8-chloro-4-oxo-chromen-2-yl)phenoxy]ethyl]-4,4-difluoro-pyrrolidine-2-carboxylic acid** | **CORE: Int-4** | ¹H NMR (DMSO-*d₆*, 400MHz) δ ppm 8.02 (t, *J* = 6.6 Hz, 2H), 7.94 (d, *J* = 8.6 Hz, 1H), 7.59 (d, *J* = 1.5 Hz, 1H), 7.51 (d, *J* = 7.8 Hz, 2H), 7.14 (s, 1H), 4.70 (br t, *J* = 8.3 Hz, 2H), 4.56-4.63 (m, 2H), 4.46-4.54 (m, 2H), 3.57-3.66 (m, 1H), 2.77-2.89 (m, 1H), 2.61-2.73 ppm (m, 1H). MS obsd. (ESI⁺) [(M+H)⁺]: 528.0. |
| | | **LINKER:** 1,2-dibromoethane | |
| | | **AMINE:** methyl 4,4-difluoropyrrolidi ne-3-carboxylate hydrochloride | |
| | 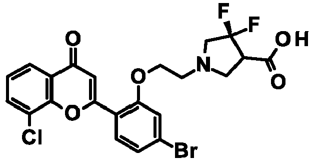 | | |
| **029** | **1-[2-[2-(8-chloro-4-oxo-chromen-2-yl)phenoxy]ethyl]piperidine-4-carboxylic acid** | **CORE: Int-3** | ¹H NMR (DMSO-*d₆*, 400MHz) δ ppm 8.00-8.06 (m, 2H), 7.86-7.94 (m, 1H), 7.61-7.69 (m, 1H), 7.48-7.55 (m, 1H), 7.31-7.36 (m, 1H), 7.22-7.30 (m, 1H), 6.93-6.99 (m, 1H), 4.50-4.59 (m, 2H), 3.52-3.65 (m, 3H), 3.01-3.16 (m, 2H), 2.36-2.49 (m, 2H), 1.91-2.03 (m, 2H), 1.63-1.85 (m, 2H). MS obsd. (ESI⁺) [(M+H)⁺]: 428.1. |
| | | **LINKER:** 1,2-dibromoethane | |
| | | **AMINE:** methyl piperidine-4-carboxylate hydrochloride | |
| | 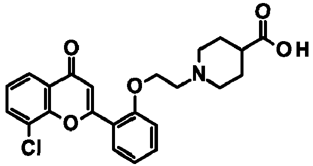 | | |
| **030** | **(2R)-1-[2-[2-(8-chloro-4-oxo-chromen-2-yl)phenoxy]ethyl]pyrrolidine-2-carboxylic acid** | **CORE: Int-3** | ¹H NMR (METHANOL-*d₄*, 400MHz) δ ppm 8.06-8.12 (m, 1H), 7.96-8.03 (m, 1H), 7.87-7.94 (m, 1H), 7.55-7.64 (m, 1H), 7.42-7.51 (m, 1H), 7.18-7.29 (m, 2H), 7.10-7.15 (m, 1H), 4.47-4.59 (m, 2H), 3.98-4.08 (m, 1H), 3.66-3.89 (m, 3H), 3.16-3.28 (m, 1H), 2.33-2.47 (m, 1H), 1.98-2.16 (m, 2H), 1.80-1.95 (m, 1H). MS obsd. (ESI⁺) [(M+H)⁺]: 414.1. |
| | | **LINKER:** 1,2-dibromoethane | |
| | | **AMINE:** methyl (2R)-pyrrolidine-2-carboxylate hydrochloride | |
| | 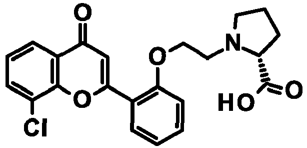 | | |
| **031** | **1-[2-[2-(8-chloro-4-oxo-chromen-2-yl)phenoxy]ethyl]pyrrolidine-3-carboxylic acid** | **CORE: Int-3** | ¹H NMR (DMSO-*d₆*, 400MHz) δ ppm 7.95-8.03 (m, 3H), 7.55-7.61 (m, 1H), 7.46-7.53 (m, 1H), 7.26-7.33 (m, 2H), 7.14-7.23 (m, 1H), 4.21-4.28 (m, 2H), 2.77-2.93 (m, 4H), 2.57-2.73 (m, 3H), 1.82-1.95 (m, 2H). MS obsd. (ESI⁺) [(M+H)⁺]: 414.1. |
| | | **LINKER:** 1,2-dibromoethane | |
| | | **AMINE:** methyl pyrrolidine-3-carboxylate hydrochloride | |
| | 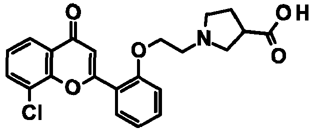 | | |
| **032** | **(2S)-1-[2-[2-(8-chloro-4-oxo-chromen-2-yl)phenoxy]ethyl]pyrrolidine-2-carboxylic acid** | **CORE: Int-3** | ¹H NMR (METHANOL-*d₄*, 400MHz) δ ppm 8.06-8.12 (m, 1H), 7.96-8.03 (m, 1H), 7.87-7.94 (m, 1H), 7.55-7.64 (m, 1H), 7.42-7.51 (m, 1H), 7.18-7.29 (m, 2H), 7.10-7.15 (m, 1H), 4.47-4.59 (m, 2H), 3.98-4.08 (m, 1H), 3.66-3.89 (m, 3H), 3.16-3.28 (m, 1H), 2.33-2.47 (m, 1H), 1.98-2.16 (m, 2H), 1.80-1.95 (m, 1H). MS obsd. (ESI⁺) [(M+H)⁺]: 414.1. |
| | | **LINKER:** 1,2-dibromoethane | |
| | | **AMINE:** methyl (2S)-pyrrolidine-2-carboxylate hydrochloride | |
| | 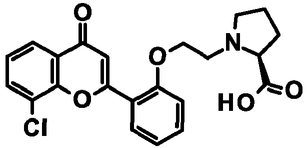 | | |
| **033** | **1-[2-[2-(8-chloro-4-oxo-chromen-2-yl)phenoxy]ethyl]azetidine-3-carboxylic acid** | **CORE: Int-3** | ¹H NMR (DMSO-*d₆*, 400MHz) δ ppm 7.92-8.05 (m, 3H), 7.54-7.62 (m, 1H), 7.45-7.54 (m, 1H), 7.14-7.29 (m, 3H), 4.07-4.15 (m, 2H), 3.39-3.46 (m, 2H), 3.17-3.24 (m, 2H), 3.07-3.17 (m, 1H), 2.76-2.86 (m, 2H). MS obsd. (ESI⁺) [(M+H)⁺]: 400.1. |
| | | **LINKER:** 1,2-dibromoethane | |
| | | **AMINE:** methyl azetidine-3-carboxylate hydrochloride | |
| | 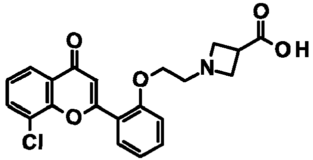 | | |
| **034** | **(3S)-1-[2-[2-(8-chloro-4-oxo-chromen-2-yl)-5-(trifluoromethyl)phenoxy]eth yl]pyrrolidine-3-carboxylic acid** | **CORE: Int-1** | ¹H NMR (DMSO-*d₆*, 400MHz) δ ppm 8.06-8.13 (m, 1H), 7.99-8.06 (m, 2H), 7.58-7.67 (m, 2H), 7.48-7.56 (m, 1H), 7.06-7.14 (m, 1H), 4.51-4.60 (m, 2H), 3.03-3.42 (m, 7H), 1.93-2.21 (m, 2H). MS obsd. (ESI⁺) [(M+H)⁺]: 482.1. |
| | | **LINKER:** 1,2-dibromoethane | |
| | | **AMINE:** methyl (3S)-pyrrolidine-3-carboxylate hydrochloride | |
| | 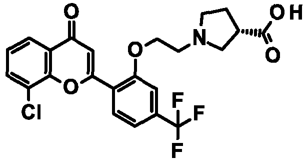 | | |
| **035** | **4-[2-[2-(8-chloro-4-oxo-chromen-2-yl)-5-(trifluoromethyl)phenoxy]eth yl]morpholine-2-carboxylic acid** | **CORE: Int-1** | ¹H NMR (DMSO-*d₆*, 400MHz) δ ppm 8.03-8.12 (m, 1H), 7.89-8.00 (m, 2H), 7.50-7.60 (m, 2H), 7.32-7.50 (m, 1H), 7.16-7.32 (m, 1H), 4.40-4.52 (m, 2H), 4.11-4.27 (m, 1H), 3.78-3.92 (m, 1H), 3.57-3.66 (m, 2H), 2.87-3.11 (m, 3H), 2.50-2.80 (m, 2H). MS obsd. (ESI⁺) [(M+H)⁺]: 498.1. |
| | | **LINKER:** 1,2-dibromoethane | |
| | | **AMINE:** methyl morpholine-2-carboxylate hydrochloride | |
| | 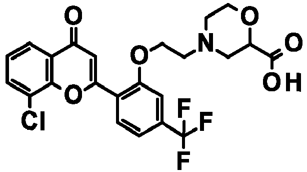 | | |
| **036** | **(2R)-2-[2-[2-(8-chloro-4-oxo-chromen-2-yl)-5-(trifluoromethyl)phenoxy]eth ylamino]-3-methyl-butanoic acid** | **CORE: Int-1** | ¹H NMR (DMSO-*d₆*, 400MHz) δ ppm 8.11 (d, *J* = 8.0 Hz, 1H), 8.01 (d, *J* = 7.9 Hz, 2H), 7.56 (d, *J* = 10.1 Hz, 2H), 7.51 (t, *J* = 7.9 Hz, 1H), 7.16 (s, 1H), 4.35 (t, *J* = 5.1 Hz, 2H), 3.16 - 3.05 (m, 1H), 3.00 (d, *J* = 5.2 Hz, 1H), 2.95 - 2.81 (m, 1H), 1.85 (dq, *J* = 13.2, 6.6 Hz, 1H), 0.83 (t, *J* = 6.8 Hz, 6H). MS obsd. (ESI⁺) [(M+H)⁺] : 484.1. |
| | | **LINKER:** 1,2-dibromoethane | |
| | | **AMINE:** methyl (2R)-2-amino-3-methylbutanoate | |
| | 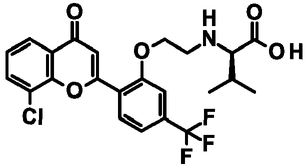 | | |
| **037** | **(2S)-2-[2-[2-(8-chloro-4-oxo-chromen-2-yl)-5-(trifluoromethyl)phenoxy]eth ylamino]-3-methyl-butanoic acid** | **CORE: Int-1** | ¹H NMR (DMSO-*d₆*, 400MHz) δ ppm 8.11 (d, *J* = 8.1 Hz, 1H), 8.05 - 7.97 (m, 2H), 7.57 (d, *J* = 7.5 Hz, 2H), 7.51 (t, *J* = 7.9 Hz, 1H), 7.14 (s, 1H), 4.39 (t, *J* = 5.2 Hz, 2H), 3.21 - 3.12 (m, 2H), 3.06 - 2.94 (m, 1H), 1.92 (dq, *J* = 13.4, 6.6 Hz, 1H), 0.84 (d, *J* = 6.8 Hz, 6H). MS obsd. (ESI⁺) [(M+H)⁺]: 484.1. |
| | | **LINKER:** 1,2-dibromoethane | |
| | | **AMINE:** methyl (2S)-2-amino-3-methylbutanoate | |
| | 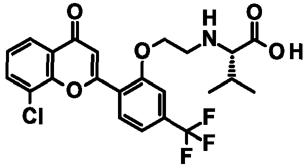 | | |
| **038** | **3-[2-[2-(8-chloro-4-oxo-chromen-2-yl)-5-(trifluoromethyl)phenoxy]ethylamino]cyclobutanecarboxyl ic acid** | **CORE: Int-1** | ¹H NMR (DMSO-*d₆*, 400MHz) δ ppm 8.12 (dd, *J* = 7.7, 5.3 Hz, 1H), 8.05 (dd, *J* = 7.9, 1.8 Hz, |
| | | **LINKER:** 1,2-dibromoethane | |
| | | **AMINE:** | 2H), 7.59-7.68 (m, 2H), 7.54 (td, *J* = 7.9, 1.6 Hz, 1H), 7.10 (d, *J* = 12.7 Hz, 1H), 4.51 (q, *J* = 4.3 Hz, 2H), 3.84-3.93 (m, 1H), 3.64-3.73 (m, 1H), 2.95-3.09 (m, 1H), 2.78 (br t, *J* = 8.6 Hz, 1H), 2.18-2.43 ppm (m, 4H). MS obsd. (ESI⁺) [(M+H)⁺]: 482.6. |
| | | methyl 3-aminocyclobuta necarboxylate hydrochloride | |
| | 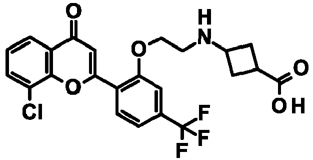 | | |
| **039** | **(3R)-1-[2-[2-(8-chloro-4-oxo-chromen-2-yl)-5-isobutoxy-phenoxy]ethyl]pyrrolidine-3-carboxylic acid** | **CORE: Int-14** | ¹H NMR (DMSO-*d₆*, 400MHz) δ ppm 7.93-8.00 (m, 3H), 7.43-7.50 (m, 1H), 7.24-7.28 (m, 1H), 6.74-6.83 (m, 2H), 4.23-4.31 (m, 2H), 3.84-3.92 (m, 2H), 2.85-2.96 (m, 5H), 2.63-2.75 (m, 2H), 2.01-2.12 (m, 1H), 1.88-1.99 (m, 2H), 1.01 (d, *J* =6.72 Hz, 6H). MS obsd. (ESI⁺) [(M+H)⁺]: 486.1. |
| | | **LINKER:** 1,2-dibromoethane | |
| | | **AMINE:** methyl (3R)-pyrrolidine-3-carboxylate hydrochloride | |
| | 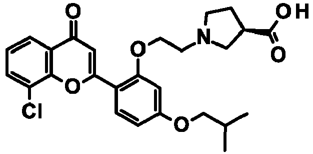 | | |
| **040** | **(3S)-1-[2-[2-(8-chloro-4-oxo-chromen-2-yl)-5-isobutoxy-phenoxy]ethyl]pyrrolidine-3-carboxylic acid** | **CORE: Int-14** | ¹H NMR (DMSO-*d₆*, 400MHz) δ ppm 7.97 (d, *J* =7.95 Hz, 3H), 7.42-7.50 (m, 1H), 7.26 (s, 1H), 6.80 (s, 2H), 4.21-4.30 (m, 2H), 3.84-3.91 (m, 2H), 2.81-2.99 (m, 5H), 2.63-2.75 (m, 2H), 1.87-2.11 (m, 3H), 1.01 (d, *J* =6.72 Hz, 6H). MS obsd. (ESI⁺) [(M+H)⁺]: 486.1. |
| | | **LINKER:** 1,2-dibromoethane | |
| | | **AMINE:** methyl (3S)-pyrrolidine-3-carboxylate hydrochloride | |
| | 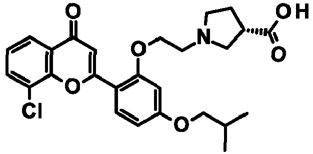 | | |
| **041** | **(3R)-1-[2-[5-bromo-2-[8-chloro-4-oxo-5-(trifluoromethyl)chromen-2-yl] phenoxy] ethyl] pyrrolidine - 3-carboxylic acid** | **CORE: Int-17** | ¹H NMR (DMSO-*d₆*, 400MHz) δ ppm 8.14-8.19 (m, 1H), 7.85-7.93 (m, 2H), 7.54-7.60 (m, 1H), 7.43-7.48 (m, 1H), 7.14-7.22 (m, 1H), 4.38-4.47 (m, 2H), 2.87-3.14 (m, 7H), 1.94-2.15 (m, 2H). MS obsd. (ESI⁺) [(M+H)⁺]: 560.1. |
| | | **LINKER:** 1,2-dibromoethane | |
| | | **AMINE:** methyl (3R)-pyrrolidine-3-carboxylate hydrochloride | |
| | 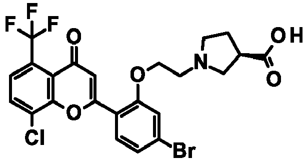 | | |
| **042** | **(3S)-1-[2-[5-bromo-2-[8-chloro-4-oxo-5-(trifluoromethyl)chromen-2-yl] phenoxy] ethyl] pyrrolidine - 3-carboxylic acid** | **CORE: Int-17** | ¹H NMR (DMSO-*d₆*, 400MHz) δ ppm 8.13-8.19 (m, 1H), 7.91-7.95 (m, 1H), 7.84-7.90 (m, 1H), 7.52-7.58 (m, 1H), 7.41-7.46 (m, 1H), 7.31-7.37 (m, 1H), 4.25-4.34 (m, 2H), 2.78-2.99 (m, 5H), 2.60-2.74 (m, 2H), 1.84-2.03 (m, 2H). MS obsd. (ESI⁺) [(M+H)⁺]: 560.1. |
| | | **LINKER:** 1,2-dibromoethane | |
| | | **AMINE:** methyl (3S)-pyrrolidine-3-carboxylate hydrochloride | |
| | 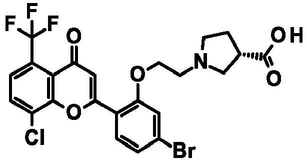 | | |
| **043** | **1-[2-[2-(8-chloro-6-fluoro-4-oxo-chromen-2-yl)phenoxy]ethyl]pyrrolidine-3-carboxylic acid** | **CORE: Int-15** | ¹H NMR (DMSO-*d₆*, 400MHz) δ ppm 8.11 (dd, *J* =3.00, 8.13 Hz, 1H), 7.94-8.00 (m, 1H), 7.68-7.75 (m, 1H), 7.53-7.65 (m, 1H), 7.30 (s, 2H), 7.15-7.24 (m, 1H), 4.19-4.30 (m, 2H), 2.78-2.96 (m, 4H), 2.59-2.74 (m, 3H), 1.87-1.99 (m, 2H). MS obsd. (ESI⁺) [(M+H)⁺]: 432.1. |
| | | **LINKER:** 1,2-dibromoethane | |
| | | **AMINE:** methyl pyrrolidine-3-carboxylate hydrochloride | |
| | 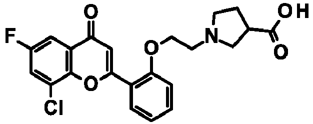 | | |
| **044** | **(2R)-1-[2-[5-bromo-2-(8-chloro-4-oxo-chromen-2-yl)-4-methyl-phenoxy]ethyl]pyrrolidine-2-carboxylic acid** | **CORE: Int-6** | ¹H NMR (DMSO-*d₆*, 400MHz) δ ppm 7.95-8.02 (m, 2H), 7.80-7.85 (m, 1H), 7.45-7.53 (m, 2H), 7.09-7.13 (m, 1H), 4.26-4.35 (m, 2H), 3.40-3.49 (m, 1H), 3.03-3.32 (m, 3H), 2.62-2.74 (m, 1H), 2.37 (s, 3H), 2.00-2.14 (m, 1H), 1.61-1.89 (m, 3H). MS obsd. (ESI⁺) [(M+H)⁺]: 506.1. |
| | | **LINKER:** 1,2-dibromoethane | |
| | | **AMINE:** methyl (2R)-pyrrolidine-2-carboxylate hydrochloride | |
| | 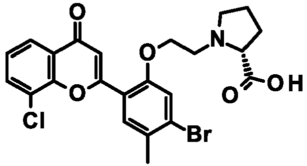 | | |
| **045** | **(2S)-1-[2-[5-chloro-2-(8-chloro-4-oxo-chromen-2-yl)-4-methyl-phenoxy]ethyl]pyrrolidine-2-carboxylic acid** | **CORE: Int-5** | ¹H NMR (DMSO-*d₆*, 400MHz) δ ppm 7.95-8.08 (m, 2H), 7.73-7.89 (m, 1H), 7.47-7.53 (m, 1H), 7.38-7.42 (m, 1H), 7.02-7.07 (m, 1H), 4.33-4.40 (m, 2H), 3.19-3.31 (m, 2H), 2.80-2.91 (m, 2H), 2.38 (s, 3H), 2.11-2.22 (m, 1H), 1.67-1.92 (m, 4H). MS obsd. (ESI⁺) [(M+H)⁺]: 462.1 |
| | | **LINKER:** 1,2-dibromoethane **AMINE:** methyl (2S)-pyrrolidine-2-carboxylate hydrochloride | |
| | 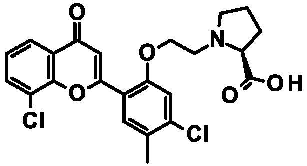 | | |
| **046** | **3-[3-[5-chloro-2-(8-chloro-4-oxo-chromen-2-yl)-4-methyl-phenoxy]propylamino]cyclob utanecarboxylic acid** | **CORE: Int-5** | ¹H NMR (DMSO-*d₆*, 400MHz) δ ppm 7.97-8.07 (m, 2H), 7.81-7.92 (m, 1H), 7.47-7.56 (m, 1H), 7.35-7.40 (m, 1H), 6.90-6.96 (m, 1H), 4.20-4.30 (m, 2H), 3.54-3.85 (m, 1H), 2.81-3.12 (m, 4H), 2.2-2.37 (m, 5H), 2.18-2.28 (m, 1H), 2.01-2.13 (m, 2H). MS obsd. (ESI⁺) [(M+H)⁺]: 476.1. |
| | | **LINKER:** 1,3-dibromopropane | |
| | | **AMINE:** methyl 3-aminocyclobuta necarboxylate hydrochloride | |
| | 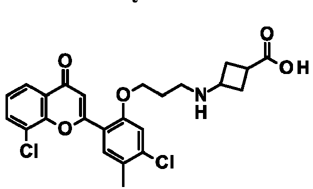 | | |
| **047** | **(3R)-1-[2-[2-[5-bromo-2-(8-chloro-4-oxo-chromen-2-yl)phenoxy]ethoxy]ethyl]pyrr olidine-3-carboxylic acid** | **CORE: Int-4** | ¹H NMR (DMSO-*d₆*, 400MHz) δ ppm 9.66-9.92 (m, 1H), 7.90-8.06 (m, 3H), 7.42-7.58 (m, 3H), 7.29 (s, 1H), 4.35-4.46 (m, 2H), 3.86-3.97 (m, 2H), 3.71-3.84 (m, 3H), 3.54-3.66 (m, 2H), 3.24-3.39 (m, 2H), 3.00-3.24 (m, 2H), 1.91-2.37 ppm (m, 2H). MS obsd. (ESI⁺) [(M+H)⁺]: 536.2. |
| | | **LINKER:** 1-bromo-2-(2-bromoethoxy)et hane **AMINE:** methyl (3R)-pyrrolidine-3-carboxylate hydrochloride | |
| | 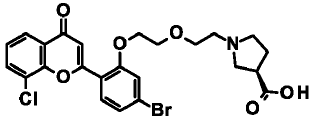 | | |
| **048** | **(2R)-1-[2-[2-[5-bromo-2-(8-chloro-4-oxo-chromen-2-yl)-4-methyl-phenoxy]ethoxy]ethyl]pyrroli dine-2-carboxylic acid** | **CORE: Int-6** | ¹H NMR (DMSO-*d₆*, 400MHz) δ ppm 7.98-8.07 (m, 2H), 7.90-7.96 (m, 1H), 7.45-7.59 (m, 2H), 7.23-7.30 (m, 1H), 4.24-4.44 (m, 2H), 3.51-3.93 (m, 8H), 3.08-3.29 (m, 1H), 2.31-2.41 (m, 4H), 1.68-2.08 (m, 3H). MS obsd. (ESI⁺) [(M+H)⁺]: 550.1. |
| | | **LINKER:** 1-bromo-2-(2-bromoethoxy)et hane | |
| | | **AMINE:** methyl (2R)-pyrrolidine-2-carboxylate hydrochloride | |
| | 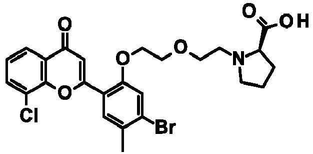 | | |
| **049** | **4-[2-[2-[5-bromo-2-(8-chloro-4-oxo-chromen-2-yl)phenoxy]ethoxy]ethyl]mor pholine-2-carboxylic acid** | **CORE: Int-4** | ¹H NMR (DMSO-*d₆*, 400MHz) δ ppm 7.88-8.08 (m, 3H), 7.42-7.59 (m, 3H), 7.30 (s, 1H), 4.30-4.50 (m, 3H), 3.98-4.10 (m, 1H), 3.81-3.96 (m, 4H), 3.64-3.79 (m, 3H), 3.28 (br d, *J* = 7.8 Hz, 2H), 2.88-3.22 ppm (m, 2H). MS obsd. (ESI+) [(M+H)+]: 552.1. |
| | | **LINKER:** 1-bromo-2-(2-bromoethoxy)et hane | |
| | 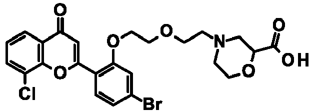 | | |
| | | **AMINE:** methyl morpholine-2-carboxylate hydrochloride | |
| **050** | **(3R)-1-[2-[2-[2-(8-chloro-4-oxo-chromen-2-yl)-5-methoxy-4-methyl-phenoxy]ethoxy]ethyl]pyrroli dine-3-carboxylic acid** | **CORE: Int-9** | ¹H NMR (DMSO-*d₆*, 400MHz) δ ppm 7.94-8.03 (m, 2H), 7.76-7.87 (m, 1H), 7.43-7.53 (m, 1H), 7.27-7.33 (m, 1H), 6.79-6.87 (m, 1H), 4.37-4.42 (m, 2H), 3.85-3.96 (m, 5H), 3.57-3.87 (m, 5H), 3.04-3.40 (m, 3H), 2.18-2.40 (m, 6H). MS obsd. (ESI⁺) [(M+H)⁺]: 502.1. |
| | | **LINKER:** 1-bromo-2-(2-bromoethoxy)et hane | |
| | 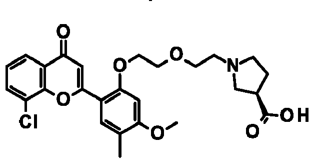 | **AMINE:** methyl (3R)-pyrrolidine-3-carboxylate hydrochloride | |
| **051** | **(3S)-1-[2-[2-[2-(8-chloro-4-oxo-chromen-2-yl)-5-methoxy-4-methyl-phenoxy]ethoxy]ethyl]pyrroli dine-3-carboxylic acid** | **CORE: Int-9** | ¹H NMR (DMSO-*d₆*, 400MHz) δ ppm 7.94-8.03 (m, 2H), 7.82 (s, 1H), 7.48 (s, 1H), 7.29 (s, 1H), 6.83 (s, 1H), 4.33-4.44 (m, 2H), 3.6-3.93 (m, 10H), 3.06-3.39 (m, 3H), 1.95-2.28 (m, 6H). MS obsd. (ESI⁺) [(M+H)⁺]: 502.1. |
| | | **LINKER:** 1-bromo-2-(2-bromoethoxy)et hane | |
| | 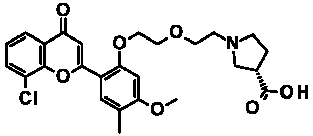 | **AMINE:** methyl (3S)-pyrrolidine-3-carboxylate hydrochloride | |
| **052** | **4-[2-[5-bromo-2-(8-chloro-4-oxo-chromen-2-yl)phenoxy]ethyl]morpholine -3-carboxylic acid** | **CORE: Int-4** | ¹H NMR (DMSO-*d₆*, 400MHz) δ ppm 8.02 (ddd, *J* = 9.6, 8.0, 1.5 Hz, 2H), 7.90-7.98 (m, 1H), 7.60 (d, *J* = 1.7 Hz, 1H), 7.44-7.55 (m, 2H), 7.14 (s, 1H), 4.60-4.68 (m, 2H), 4.47-4.57 (m, 3H), 4.06 (dd, *J* = 12.5, 3.8 Hz, 1H), 3.76-3.92 (m, 2H), 3.59-3.70 (m, 1H), 3.26-3.31 (m, 1H), 3.06-3.17 (m, 1H). MS obsd. (ESI⁺) [(M+H)⁺]: 508.1. |
| | | **LINKER:** 1,2-dibromoethane | |
| | | **AMINE:** methyl morpholine-3-carboxylate hydrochloride | |
| | 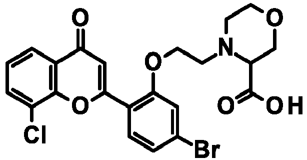 | | |
| **053** | **3-[2-[2-(8-chloro-4-oxo-chromen-2-yl)-5-methyl-phenoxy]ethylamino]cyclobut anecarboxylic acid** | **CORE: Int-2** | ¹H NMR (DMSO-*d₆*, 400MHz) δ ppm 7.99-8.06 (m, 2H), 7.80-7.89 (m, 1H), 7.44-7.55 (m, 1H), 7.12-7.18 (m, 1H), 6.99-7.11 (m, 2H), 4.34-4.43 (m, 2H), 4.04-4.15 (m, 1H), 3.86-3.94 (m, 1H), 3.65-3.77 (m, 1H), 3.15-3.19 (m, 2H), 2.98-3.10 (m, 1H), 2.73-2.88 (m, 1H), 2.42 (s,3H), 2.21-2.31 (m, 1H). MS obsd. (ESI⁺) [(M+H)⁺]: 428.1 |
| | | **LINKER:** 1,2-dibromoethane | |
| | | **AMINE:** methyl 3-aminocyclobuta ne-1-carboxylate | |
| | 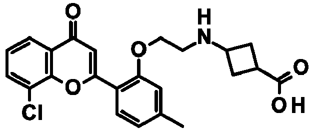 | | |
| **054** | **1-[2-[2-(8-chloro-4-oxo-chromen-2-yl)-5-methyl-phenoxy]ethyl]azetidine-3-carboxylic acid** | **CORE: Int-2** | ¹H NMR (DMSO-*d₆*, 400MHz) δ ppm 7.96-8.01 (m, 2H), 7.84-7.89 (m, 1H), 7.45-7.51 (m, 1H), 7.15-7.19 (m, 1H), 7.07-7.12 (m, 1H), 6.99-7.04 (m, 1H), 4.07-4.13 (m, 2H), 3.41-3.46 (m, 2H), 3.19-3.24 (m, 1H), 3.11-3.18 (m, 2H), 2.78-2.84 (m, 2H), 2.42 (s, 3H). MS obsd. (ESI⁺) [(M+H)⁺]: 414.1. |
| | | **LINKER:** 1,2-dibromoethane | |
| | | **AMINE:** methyl azetidine-3-carboxylate hydrochloride | |
| | 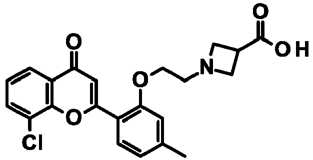 | | |
| **055** | **cis-4-[2-[5-bromo-2-(8-chloro-4-oxo-chromen-2-yl)phenoxy]ethylamino]cyclo hexanecarboxylic acid** | **CORE: Int-4** | ¹H NMR (DMSO-*d₆*, 400MHz) δ ppm 7.90-7.96 (m, 2H), 7.80 (d, *J* = 8.4 Hz, 1H), 7.40-7.48 (m, 2H), 7.35 (dd, *J* = 8.3, 1.7 Hz, 1H), 7.05 (s, 1H), 4.14-4.20 (m, 2H), 2.88 (br t, *J* = 5.4 Hz, 2H), 2.52-2.57 (m, 2H), 2.20-2.25 (m, 1H), 1.65-1.78 (m, 2H), 1.42-1.53 (m, 2H), 1.23-1.41 (m, 3H). MS obsd. (ESI⁺) [(M+H)⁺]: 520.1. |
| | | **LINKER:** 1,2-dibromoethane **AMINE:** methyl cis-4-aminocyclohexa ne-1-carboxylate hydrochloride | |
| | 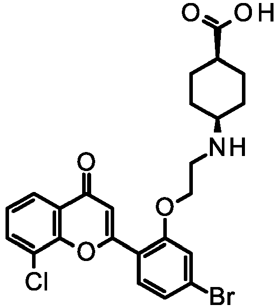 | | |
| **056** | **trans-4-[2-[5-bromo-2-(8-chloro-4-oxo-chromen-2-yl)phenoxy]ethylamino]cyclo hexanecarboxylic acid** | **CORE: Int-4** | ¹H NMR (DMSO-*d₆*, 400MHz) δ ppm 7.94 (d, *J* = 7.9 Hz, 2H), 7.81 (d, *J* = 8.4 Hz, 1H), 7.29-7.49 (m, 3H), 7.13 (s, 1H), 4.16 (br t, *J* = 5.1 Hz, 2H), 2.92 (br t, *J* = 4.8 Hz, 2H), 2.30-2.37 (m, 1H), 1.95-2.08 (m, 1H), 1.72-1.87 (m, 4H), 1.11-1.27 (m, 2H), 0.81-1.01 (m, 2H). MS obsd. (ESI⁺) [(M+H)⁺]: 520.1. |
| | | **LINKER:** 1,2-dibromoethane | |
| | | **AMINE:** | |
| | 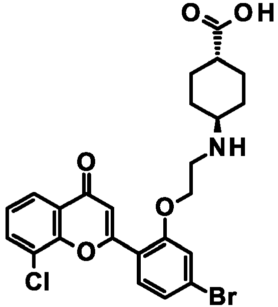 | methyl trans-4-aminocyclohexa ne-1-carboxylate hydrochloride | |
| **057** | **2-[2-[2-(8-chloro-4-oxo-chromen-2-yl)-5-(trifluoromethyl)phenoxy]eth ylamino]propanoic acid** | **CORE: Int-1** | ¹H NMR (DMSO-*d₆*, 400MHz) δ ppm 8.11 (d, *J* =8.1 Hz, 1H), 8.03 (d, *J* =7.9 Hz, 2H), 7.59-7.65 (m, 2H), 7.53 (t, *J* =7.9 Hz, 1H), 7.13 (s, 1H), 4.49 (br t, *J* =4.6 Hz, 2H), 2.07 (m, 2H), 1.49 (d, *J* =6.7 Hz, 1H), 1.32 (br d, *J* =7.1 Hz, 3H). MS obsd. (ESI⁺) [(M+H)⁺]: 456.3. |
| | | **LINKER:** 1,2-dibromoethane | |
| | | **AMINE:** ethyl 2-aminopropanoat e | |
| | 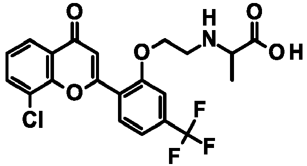 | | |
| **058** | **2-[2-[2-(8-chloro-4-oxo-chromen-2-yl)-5-methyl-phenoxy]ethylamino]-2-methyl-propanoic acid** | **CORE: Int-2** | ¹H NMR (DMSO-*d₆*, 400MHz) δ ppm 7.98-8.03 (m, 2H), 7.84-7.88 (m, 1H), 7.47-7.52 (m, 1H), 7.13-7.16 (m, 1H), 7.06-7.11 (m, 2H), 4.37-4.43 (m, 2H), 2.99-3.21 (m, 2H), 2.41 (s, 3H), 1.47 (s, 6H). MS obsd. (ESI⁺) [(M+H)⁺]: 416.1. |
| | | **LINKER:** 1,2-dibromoethane | |
| | | **AMINE:** methyl 2-amino-2-methylpropanoate | |
| | 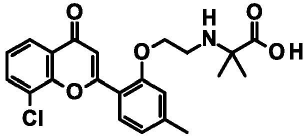 | | |
| **059** | **3-[3-[2-(8-chloro-4-oxo-chromen-2-yl)-5-methyl-phenoxy]propylamino]cyclob utanecarboxylic acid** | **CORE: Int-2** | ¹H NMR (DMSO-*d₆*, 400MHz) δ ppm 7.98-8.03 (m, 2H), 7.84-7.88 (m, 1H), 7.47-7.52 (m, 1H), 7.13-7.16 (m, 1H), 7.06-7.11 (m, 2H), 4.37-4.43 (m, 2H), 2.99-3.21 (m, 2H), 2.41 (s, 3H), 1.47 (s, 6H). MS obsd. (ESI⁺) [(M+H)⁺]: 416.1, |
| | | **LINKER:** 1,3-dibromopropane | |
| | | **AMINE:** methyl 3-aminocyclobuta ne-1-carboxylate | |
| | 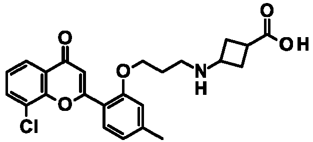 | | |
| **060** | **cis-4-[3-[5-bromo-2-(8-chloro-4-oxo-chromen-2-yl)phenoxy]propylamino]tetr ahydropyran-2-carboxylic acid** | **CORE: Int-4** | ¹H NMR (DMSO-*d₆*, 400MHz) δ ppm 7.97-8.04 (m, 2H), 7.83-7.89 (m, 1H), 7.41-7.55 (m, 3H), 6.99 (s, 1H), 4.27-4.36 (m, 2H), 3.86-4.01 (m, 2H), 3.56-3.81 (m, 1H), 3.38-3.43 (m, 1H), 2.95-3.12 (m, 2H), 2.08-2.30 (m, 3H), 1.71-1.93 (m, 1H), 1.36-1.57 (m, 2H). MS obsd. (ESI⁺) [(M+H)⁺]: 536.1. |
| | | **LINKER:** 1,3-dibromopropane | |
| | | **AMINE:** methyl cis-3-aminocyclohexa ne-1-carboxylate hydrochloride | |
| | 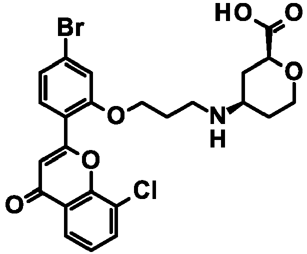 | | |
| **061** | **1-[2-[5-chloro-2-(8-chloro-4-oxo-chromen-2-yl)-4-methyl-phenoxy]ethyl]azetidine-3-carboxylic acid** | **CORE: Int-5** | ¹H NMR (DMSO-*d₆*, 400MHz) δ ppm 7.97-8.05 (m, 2H), 7.80-7.88 (m, 1H), 7.47-7.56 (m, 1H), 7.39-7.46 (m, 1H), 6.88-6.96 (m, 1H), 4.36-4.46 (m, 2H), 4.15-4.30 (m, 4H), 3.62-3.70 (m, 2H), 2.34-2.41 (m, 4H). MS obsd. (ESI⁺) [(M+H)⁺]: 448.1. |
| | | **LINKER:** 1,2-dibromoethane | |
| | | **AMINE:** methyl azetidine-3-carboxylate hydrochloride | |
| | 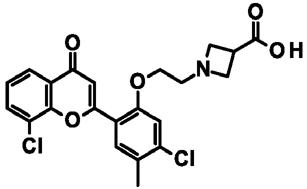 | | |

### Example 062: 3-[3-[2-(8-chloro-4-oxo-chromen-2-yl)-5-(trifluoromethyl)phenoxy]propylamino]butanoic acid

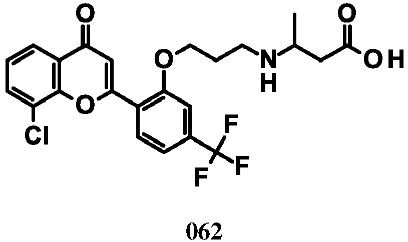

### Step 1: Preparation of 2-[2-(3-bromopropoxy)-4-(trifluoromethyl)phenyl]-8-chloro-chromen-4-one

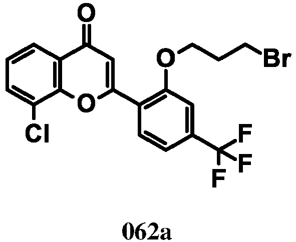

To a solution of 8-chloro-2-[2-hydroxy-4-(trifluoromethyl)phenyl]chromen-4-one (500 mg, 1.47 mmol), 1,3-dibromopropane (1.19 g, 5.87 mmol) in DMF was added K₂CO₃ (203 mg, 1.47 mmol). The reaction mixture was stirred at 50 °C overnight. After the reaction was completed, the reaction mixture was diluted and partitioned between EtOAc (10 mL) and water (30 mL). The organic layer was separated out and the aqueous phase was extracted with EtOAc (30 mL) twice. The combined organic layer was concentrated *in vacuo* to give the crude product. The crude product was purified by ISCO (eluting with EtOAc : PE = 0 to 20%) to give 2-[2-(3-bromopropoxy)-4-(trifluoromethyl)phenyl]-8-chloro-chromen-4-one (680 mg, yield: 100%) as a yellow solid. MS obsd. (ESI⁺) [(M+H)]⁺: 463.0.

### Step 2: Preparation of tert-butyl 3-[3-[2-(8-chloro-4-oxo-chromen-2-yl)-5-(trifluoromethyl)phenoxy]propylamino]butanoate

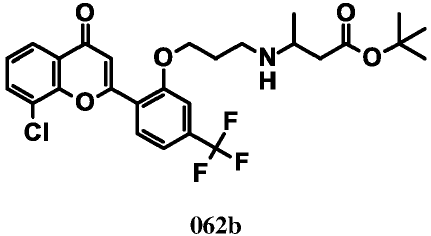

To a solution of 2-[2-(3-bromopropoxy)-4-(trifluoromethyl)phenyl]-8-chloro-chromen-4-one (94 mg, 204 µmol) and tert-butyl 3-aminobutanoate (64.8 mg, 407 µmol) in DMF was added K₂CO₃ (141 mg, 1.02 mmol). The mixture was stirred at 90 °C overnight. After the reaction was completed, the reaction mixture was diluted with EtOAc and partitioned between EtOAc (30 mL) and water (20 mL). The organic layer was separated out and the aqueous phase was extracted with EtOAc (30 mL) twice. The combined organic layer was concentrated *in vacuo* to give tert-butyl 3-[3-[2-(8-chloro-4-oxo-chromen-2-yl)-5-(trifluoromethyl)phenoxy]propylamino]butanoate (110 mg, yield: 100%) as a yellow solid. (ESI⁺) [(M+H)]⁺: 540.3.

### Step 3: Preparation of 3-[3-[2-(8-chloro-4-oxo-chromen-2-yl)-5-(trifluoromethyl)phenoxy]propylamino]butanoic acid

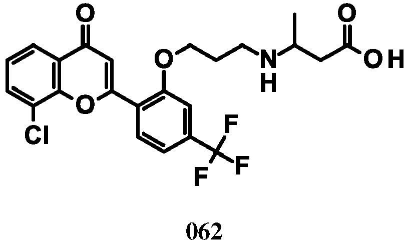

To a flask charged with tert-butyl 3-[3-[2-(8-chloro-4-oxo-chromen-2-yl)-5-(trifluoromethyl)phenoxy]propylamino]butanoate (110 mg, 204 µmol) dissolved in DCM (2.04 mL) was added TFA (0.1 mL, 204 µmol). The reaction was stirred at room temperature for 2 hours and then concentrated *in vacuo* to give yellow oil, which was purified by prep-HPLC to give 3-[3-[2-(8-chloro-4-oxo-chromen-2-yl)-5-(trifluoromethyl)phenoxy]propylamino]butanoic acid (6.5 mg, yield: 6.26%) as a white powder. (ESI⁺) [(M+H)]⁺: 484.2.

Example 062: ¹H NMR (DMSO-*d₆*, 400MHz) δ ppm 8.12 (m, 1H), 7.94-8.06 (m, 2H), 7.46-7.69 (m, 3H), 6.95-7.13 (m, 1H), 4.36 (m, 2H), 4.22-4.28 (m, 2H), 2.07 (m, 2H), 1.24 (m, 3H), 1.08 (m, 2H), 0.80-0.92 (m, 1H).

### Example 063:

### 2-[3-[2-(8-chloro-4-oxo-chromen-2-yl)-5-(trifluoromethyl)phenoxy]propylamino]-3-cyclohexyl-propanoic acid

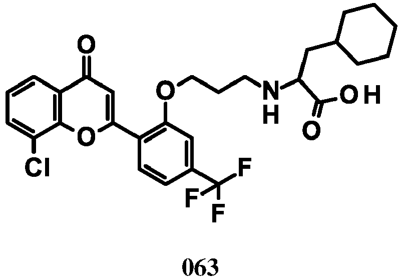

**063** was prepared in analogy to the procedure described for the preparation of compound **062** by using tert-butyl 2-amino-3-cyclohexyl-propanoate as the starting material instead of tert-butyl 3-aminobutanoate in **Step 2.** MS obsd. (ESI⁺) [(M+H)]⁺: 552.6.

**Example 063:** ¹H NMR (DMSO-*d₆*, 400MHz) δ ppm 8.12 (d, *J* =8.8 Hz, 1H), 7.96-8.07 (m, 2H), 7.41-7.68 (m, 3H), 7.03 (s, 1H), 4.29-4.47 (m, 2H), 2.98-3.10 (m, 2H), 2.12-2.27 (m, 2H), 2.07 (s, 1H), 1.69-1.79 (m, 2H), 1.49-1.65 (m, 4H), 1.40 (m, 1H), 1.04-1.19 (m, 4H), 0.73-0.92 (m, 2H).

### Example 064: (3R)-1-[2-[5-bromo-2-(8-chloro-4-oxo-chromen-2-yl)phenoxy]ethyl]-N-methylsulfonyl-pyrrolidine-3-carboxamide

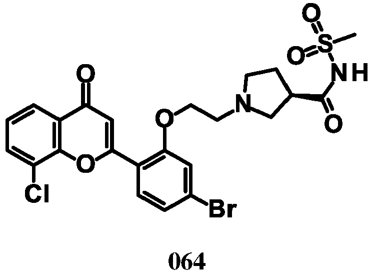

### Step 1: Preparation of 2-[4-bromo-2-(2-bromoethoxy)phenyl]-8-chloro-chromen-4-one

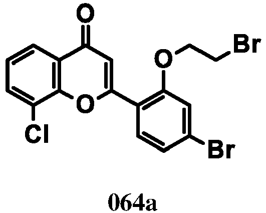

To a mixture of 2-(4-bromo-2-hydroxy-phenyl)-8-chloro-chromen-4-one (500 mg, 1.42 mmol, as the **"CORE"** in Table 2) and 1,2-dibromoethane (1.07 g, 5.69 mmol, as the **"LINKER"** in Table 2) in DMF (15 mL) was added K₂CO₃ (197 mg, 1.42 mmol). The reaction was stirred at 50 °C overnight. After completion, the reaction mixture was quenched with 4N HCl (20 mL) and extracted with EtOAc (30 mL) three times. The combined organic layer was washed with brine, dried over anhydrous Na₂SO₄ and concentrated *in vacuo* to give 2-[4-bromo-2-(2-bromoethoxy)phenyl]-8-chloro-chromen-4-one (450 mg, yield: 69%) as a yellow solid. MS obsd. (ESI⁺) [(M+H)⁺]: 459.0.

### Step 2: Preparation of methyl (3R)-1-[2-[5-bromo-2-(8-chloro-4-oxo-chromen-2-yl)phenoxy]ethyl]pyrrolidine-3-carboxylate

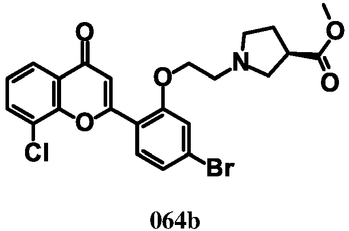

A mixture of 2-[4-bromo-2-(2-bromoethoxy)phenyl]-8-chloro-chromen-4-one (250 mg, 545 µmol), methyl (3R)-pyrrolidine-3-carboxylate hydrochloride (200 mg, 5.49 mmol, as the "CYC" in Table 2) and sodium bicarbonate (275 mg, 3.27 mmol) in ethanol (10 mL) was stirred at 90 °C overnight. After completion, the reaction mixture was diluted with water (20 mL) and extracted with EtOAc (30 mL) three times. The combined organic layer was washed with brine, dried over anhydrous Na₂SO₄ and concentrated *in vacuo* to give methyl (3R)-1-[2-[5-bromo-2-(8-chloro-4-oxo-chromen-2-yl)phenoxy]ethyl] pyrrolidine-3-carboxylate (200 mg, yield: 72.4%) as a yellow oil. MS obsd. (ESI⁺) [(M+H)⁺]: 508.1.

### Step 3: Preparation of (3R)-1-[2-[5-bromo-2-(8-chloro-4-oxo-chromen-2-yl)phenoxy]ethyl]pyrrolidine-3-carboxylic acid

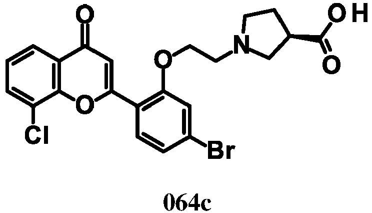

To a mixture of methyl (3R)-1-[2-[5-bromo-2-(8-chloro-4-oxo-chromen-2-yl)phenoxy]ethyl]pyrrolidine-3-carboxylate (200 mg, 395 µmol) in THF (5 mL) and water (5 mL) was added 4M HCl (5 mL, 20 mmol) at room temperature. The reaction mixture was then stirred at 50 °C for 2 hours. After the reaction was completed, the reaction mixture was concentrated *in vacuo* to give (3R)-1-[2-[5-bromo-2-(8-chloro-4-oxo-chromen-2-yl)phenoxy]ethyl]pyrrolidine-3-carboxylic acid (190 mg, yield: 97.7 %) as a white foam .MS obsd. (ESI⁺) [(M+H)⁺]: 492.0.

### Step 4: Preparation of (3R)-1-[2-[5-bromo-2-(8-chloro-4-oxo-chromen-2-yl)phenoxy]ethyl]-N-methylsulfonyl-pyrrolidine-3-carboxamide

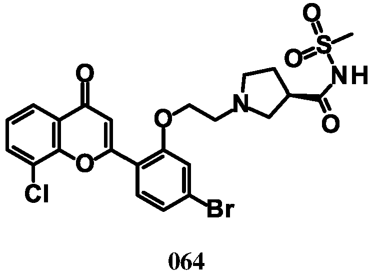

To a mixture of (3R)-1-[2-[5-bromo-2-(8-chloro-4-oxo-chromen-2-yl)phenoxy]ethyl]-N-methylsulfonyl-pyrrolidine-3-carboxamide (120 mg, 237 µmol), methanesulfonamide (45 mg, 474 µmol, as the **"SA"** in Table 2), DIPEA (306 mg, 414 µl, 2.37 mmol), DMAP (28.9 mg, 237 µmol) in DCM (5 mL) was added HATU (90.8 mg, 474 µmol). The reaction mixture was then stirred at room temperature for 12 hours. After completion, the reaction mixture was concentrated *in vacuo* to give the crude product. The crude product was purified by prep-HPLC to give (3R)-1-[2-[5-bromo-2-(8-chloro-4-oxo-chromen-2-yl)phenoxy]ethyl]-N-methylsulfonyl-pyrrolidine-3-carboxamide (13 mg, yield: 9.4%) as a white foam. MS obsd. (ESI⁺) [(M+H)⁺]: 569.1.

**Example 064:** ¹H NMR (DMSO-*d₆*, 400MHz) δ ppm 7.96-8.06 (m, 2H), 7.82-7.90 (m, 1H), 7.55-7.61 (m, 1H), 7.42-7.54 (m, 2H), 7.08-7.17 (m, 1H), 4.34-4.46 (m, 2H), 3.17 (m, 4H), 2.88-3.11 (m, 5H), 1.87-2.15 (m, 3H).

The following compounds **065** to **071** were prepared in analogy to the procedure described for the preparation of Example **064,** replacing 2-(4-bromo-2-hydroxy -phenyl)-8-chloro-chromen-4-one with **"CORE",** 1,2-dibromoethane with **"LINKER"** in **Step 1,** methyl (3R)-pyrrolidine-3-carboxylate hydrochloride with **"CYC"** in **step 2,** replacing methanesulfonamide with **"SA",** by the reagent indicated in Table 2.

**Table 2: Compounds synthesis and characterization**

| Example No. | Compounds Name and Structure | **CORE, LINKER, CYC** and **SA** | ¹H NMR and (ESI⁺) |
|---|---|---|---|
| **065** | **(3R)-1-[3-[2-(8-chloro-4-oxo-chromen-2-yl)-5-(trifluoromethyl)phenoxy]pr opyl]-N-methylsulfonyl-pyrrolidine-3-carboxamide** | **CORE:Int-1** | ¹H NMR (DMSO-*d₆*, 400MHz) δ ppm 8.09-8.17 (m, 1H), 7.99-8.08 (m, 2H), 7.45-7.65 (m, 3H), 7.02-7.08 (m, 1H), 4.28-4.40 (m, 2H), 3.72-3.88 (m, 1H), 3.28-3.45 (m, 8H), 2.35-2.46 (m, 1H), 2.11-2.25 (m, 3H), 1.85-2.04 (m, 1H). MS obsd. (ESI⁺) [(M+H)⁺]: 573.1. |
| | | **LINKER:** 1,3-dibromopropane | |
| | | **CYC:** methyl (3R)-pyrrolidine-3-carboxylate | |
| | 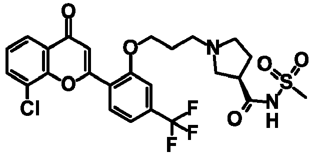 | **SA:** methanesulfonamid e | |
| **066** | **(3S)-1-[3-[2-(8-chloro-4-oxo-chromen-2-yl)-5-(trifluoromethyl)phenoxy]pr opyl]-N-methylsulfonyl-pyrrolidine-3-carboxamide** | **CORE:Int-1** | ¹H NMR (DMSO-*d₆*, 400MHz) δ ppm 8.09-8.17 (m, 1H), 7.98-8.09 (m, 2H), 7.49-7.67 (m, 3H), 7.01-7.08 (m, 1H), 4.29-4.40 (m, 2H), 3.60-3.85 (m, 2H), 3.20-3.37 (m, 8H), 3.02-3.14 (m, 1H), 2.10-2.26 (m, 3H). MS obsd. (ESI⁺) [(M+H)⁺]: 573.1. |
| | | **LINKER:** 1,3-dibromopropane | |
| | | **CYC:** methyl (3S)-pyrrolidine-3-carboxylate | |
| | 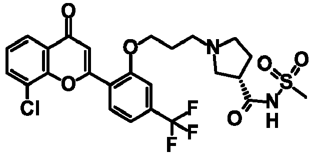 | **SA:** methanesulfo namide | |
| **067** | **(3R)-1-[3-[2-(8-chloro-4-oxo-chromen-2-yl)-5-(trifluoromethyl)phenoxy]pr opyl]-N-cyclopropylsulfonyl-pyrrolidine-3-carboxamide** | **CORE:Int-1** | ¹H NMR (DMSO-*d₆*, 400MHz) δ ppm 8.25 (s, 1H), 8.12 (d, *J* = 8.4 Hz, 1H), 8.03 (d, *J* = 7.9 Hz, 2H), 7.48-7.58 (m, 3H), 7.08 (s, 1H), 4.27-4.34 (m, 2H), 2.81-2.88 (m, 2H), 2.72-2.79 (m, 2H), 2.60-2.72 (m, 6H), 1.77-2.04 (m, 6H). MS obsd. (ESI⁺) [(M+H)⁺]: 599.3 |
| | | **LINKER:** 1,3-dibromopropane | |
| | | CYC: methyl (3R)-pyrrolidine-3-carboxylate | |
| | 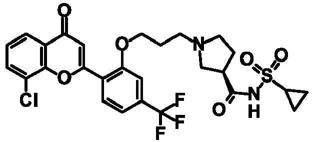 | | |
| | | **SA:** cyclopropanesulfon amide | |
| **068** | **4-[2-[2-(8-chloro-4-oxo-chromen-2-yl)-5-(trifluoromethyl)phenoxy]eth yl]-N-methylsulfonyl-morpholine-2-carboxamide** | **CORE: Int-1** | ¹H NMR (DMSO-*d₆*, 400MHz) δ ppm 11.31-11.81 (m, 1H), 8.16-8.24 (m, 1H), 7.95-8.06 (m, 2H), 7.45-7.66 (m, 4H), 4.41 (br t, *J* = 5.2 Hz, 2H), 4.13 (dd, *J* = 9.8, 2.6 Hz, 1H), 3.86 (br d, *J* = 11.1 Hz, 1H), 3.63-3.72 (m, 1H), 3.16 (s, 3H), 3.05 (br d, *J* = 11.0 Hz, 1H), 2.84 (br t, *J* = 5.2 Hz, 2H), 2.77 (br d, *J* = 11.5 Hz, 1H), 2.07-2.32(m, 2H). MS obsd. (ESI⁺) [(M+H)⁺]: 575.2. |
| | | **LINKER:** 1,2-dibromoethane | |
| | 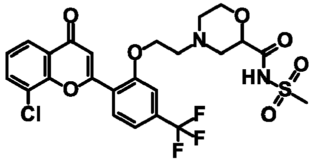 | **CYC:** methyl morpholine-2-carboxylate hydrochloride **SA:** methanesulfonamid e | |
| **069** | **(3S)-1-[2-[5-bromo-2-(8-chloro-4-oxo-chromen-2-yl)phenoxy]ethyl]-N-methylsulfonyl-pyrrolidine-3-carboxamide** | **CORE:Int-4** | ¹H NMR (DMSO-*d₆*, 400MHz) δ ppm 8.12-8.15 (m, 1H), 7.95-8.04 (m, 2H), 7.80-7.91 (m, 1H), 7.41-7.58 (m, 3H), 7.11-7.19 (m, 1H), 4.32-4.45 (m, 2H), 3.12-3.20 (m, 6H), 2.81-3.08 (m, 3H), 1.88-2.12 (m, 3H). MS obsd. (ESI⁺) [(M+H)⁺]: 569.1. |
| | | **LINKER:** 1,2-dibromoethane **CYC:** methyl (3S)-pyrrolidine-3-carboxylate **SA:** methanesulfonamid e | |
| | 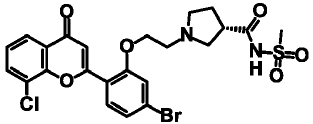 | | |
| **070** | **(3R)-1-[2-[2-(8-chloro-4-oxo-chromen-2-yl)-5-(trifluoromethyl)phenoxy]eth yl]-N-methylsulfonyl-pyrrolidine-3-carboxamide** | **CORE:Int-1** | ¹H NMR (DMSO-*d₆*, 400MHz) δ ppm 12.15 (m, 1H), 7.98-8.14 (m, 3H), 7.61-7.70 (m, 2H), 7.46-7.59 (m, 1H), 6.99-7.05 (m, 1H), 4.51-4.65 (m, 3H), 3.55-3.90 (m, 4H), 3.13-3.21 (m, 5H), 1.79-2.24 (m, 2H). MS obsd. (ESI⁺) [(M+H)⁺]: 559.1. |
| | | **LINKER:** 1,2-dibromoethane | |
| | | **CYC:** methyl (3R)-pyrrolidine-3-carboxylate | |
| | 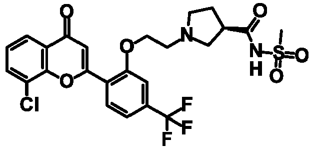 | | |
| | | **SA:** methanesulfonamid e | |
| **071** | **(3S)-1-[2-[2-(8-chloro-4-oxo-chromen-2-yl)-5-(trifluoromethyl)phenoxy]eth yl]-N-methylsulfonyl-pyrrolidine-3-carboxamide** | **CORE:Int-1** | ¹H NMR (DMSO-*d₆*, 400MHz) δ ppm 8.08-8.17 (m, 2H), 7.97-8.06 (m, 2H), 7.57-7.65 (m, 2H), 7.46-7.56 (m, 1H), 7.15-7.24 (m, 1H), 4.33-4.49 (m, 2H), 3.11-3.18 (m, 5H), 2.80-3.07 (m, 5H), 1.87-2.11 (m, 2H). MS obsd. (ESI⁺) [(M+H)⁺]: 559.1. |
| | | **LINKER:** 1,2-dibromoethane | |
| | | **CYC:** methyl (3S)-pyrrolidine-3-carboxylate **SA:** methanesulfonamid e | |
| | 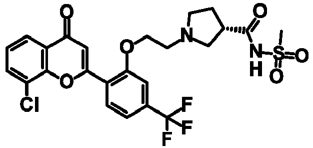 | | |

### Example 072:

### 1-[2-[2-(8-chloro-4-oxo-chromen-2-yl)-5-(trifluoromethyl)phenoxy]ethyl]-N-cyclopropylsulfonyl-pyrrolidine-2-carboxamide

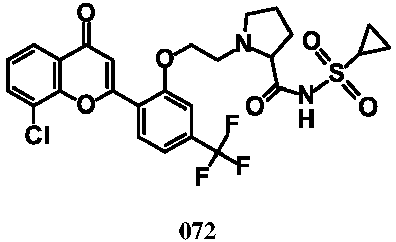

### Step 1: Preparation of 2-[4-bromo-2-(2-bromoethoxy)phenyl]-8-chloro-chromen-4-one

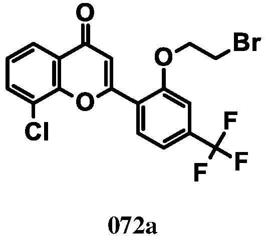

To a solution of 8-chloro-2-[2-hydroxy-4-(trifluoromethyl)phenyl]chromen-4-one (680 mg, 2 mmol, as the **"CORE"** in Table 3) in DMF (20 mL) was added 1,2-dibromoethane (3.75 g, 19.96 mmol, as the **"LINKER"** in Table 3) and potassium carbonate (827 mg, 5.99 mmol). The reaction was heated at 80 °C and stirred for 5 hours. After the starting material was consumed, the reaction mixture was quenched with water (150 mL) and extracted with EtOAc (50 mL) three times. The combined organic layer was concentrated *in vacuo* to give 2-[2-(2-bromoethoxy)-4-(trifluoromethyl)phenyl]-8-chloro-chromen-4-one (800 mg, yield: 89.5%) as an off-white solid. MS obsd. (ESI⁺)[(M+H)⁺]: 446.9.

### Step 2: Preparation of tert-butyl 1-[2-[2-(8-chloro-4-oxo-chromen-2-yl)-5-(trifluoromethyl)phenoxy]ethyl]pyrrolidine-2-carboxylate

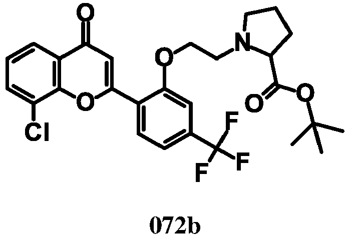

To a solution of 2-[2-(2-bromoethoxy)-4-(trifluoromethyl)phenyl]-8-chloro-chromen-4-one (700 mg, 1.56 mmol) and tert-butyl pyrrolidine-2-carboxylate (348.12 mg, 2.03 mmol, as the "CYC" in Table 3) in DMF (7 mL) and ethanol (7 mL) were added NaHCO₃ (394.12 mg, 4.69 mmol) and potassium iodide (26 mg, 0.160 mmol). The reaction mixture was stirred at 90 °C for 5 hours. After the reaction was completed, the reaction mixture was concentrated *in vacuo* to give tert-butyl 1-[2-[2-(8-chloro-4-oxo-chromen-2-yl)-5-(trifluoromethyl)phenoxy]ethyl]pyrrolidine-2-carboxylate (0.5 g, yield: 59.4%) as a light yellow solid. MS obsd. (ESI⁺)[(M+H)⁺]: 538.2.

### Step 3: Preparation of 1-[2-[2-(8-chloro-4-oxo-chromen-2-yl)-5-(trifluoromethyl)phenoxy]ethyl]pyrrolidine-2-carboxylic acid

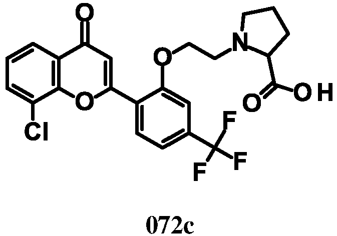

To a solution of tert-butyl 1-[2-[2-(8-chloro-4-oxo-chromen-2-yl)-5-(trifluoromethyl)phenoxy]ethyl]pyrrolidine-2-carboxylate (460 mg, 0.860 mmol) in DCM (4 mL) was added trifluoroacetic acid (2 mL, 26 mmol). The reaction was stirred at room temperature for 5 hours. After completion, the reaction mixture was concentrated *in vacuo* to give 1-[2-[2-(8-chloro-4-oxo-chromen-2-yl)-5-(trifluoromethyl)phenoxy]ethyl]pyrrolidine-2-carboxylic acid (410 mg, yield: 99.5%) as a light yellow solid. MS obsd. (ESI⁺)[(M+H)⁺]: 482.1.

### Step 4: Preparation of 1-[2-[2-(8-chloro-4-oxo-chromen-2-yl)-5-(trifluoromethyl)phenoxy]ethyl]-N-cyclopropylsulfonyl-pyrrolidine-2-carboxamide

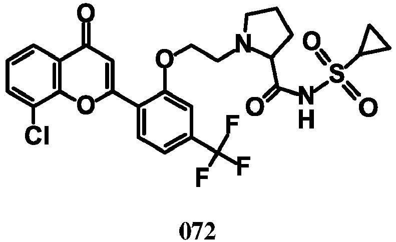

To a solution **1** of cyclopropanesulfonamide (98 mg, 0.810 mmol, as the **"SA"** in Table 3) in THF (4 mL) was added sodium hydride (37.36 mg, 0.930 mmol) dropwise at 0 °C and kept stirred at 0 °C for 1 hour. To a solution **2** of 1-[2-[2-(8-chloro-4-oxo-chromen-2-yl)-5-(trifluoromethyl)phenoxy]ethyl]pyrrolidine-2-carboxylic acid (300 mg, 0.620 mmol) in THF (4 mL) were added HATU (473 mg, 1.25 mmol) and DIPEA (0.33 mL, 1.87 mmol), and the solution was stirred at room temperature for 1 hour. Then, the solution **1** was added to the solution **2** and the resulting residue was stirred at room temperature for 16 hours. After completion, the reaction mixture was concentrated *in vacuo* to give the crude product. The crude product was purified by prep-HPLC to give 1-[2-[2-(8-chloro-4-oxo-chromen-2-yl)-5-(trifluoromethyl)phenoxy]ethyl]-N-cyclopropylsulfonyl-pyrrolidine-2-carboxamide (75 mg, yield: 19.8%) as an off-white solid. MS obsd. (ESI⁺)[(M+H)⁺]: 585.1.

**Example 072:** ¹H NMR (DMSO-*d₆*, 400MHz) δ ppm 7.97-8.14 (m, 3H), 7.47-7.65 (m, 3H), 7.07-7.16 (m, 1H), 4.44-4.57 (m, 2H), 3.06-3.65 (m, 3H), 2.71-2.97 (m, 3H), 2.15-2.31 (m, 1H), 1.66-1.91 (m, 3H), 0.74-1.00 (m, 4H).

The following compounds **073** and **074** were prepared in analogy to the procedure described for the preparation of Example **072,** replacing 8-chloro-2-[2-hydroxy-4-(trifluoromethyl)phenyl]chromen-4-one with **"CORE",** 1,2-dibromoethane with **"LINKER"** in **Step 1,** tert-butyl pyrrolidine-3-carboxylate with **"CYC"** in **Step 2,** replacing cyclopropanesulfonamide with **"SA",** by the reagent indicated in Table 3.

**Table 3: Compounds synthesis and characterization**

| Example No. | Compounds Name and Structure | **CORE, LINKER, CYC and SA** | ¹H NMR and (ESI⁺) |
|---|---|---|---|
| **073** | **1-[2-[2-(8-chloro-4-oxo-chromen-2-yl)-5-(trifluoromethyl)phenoxy]eth yl]-N-methylsulfonyl-pyrrolidine-2-carboxamide** | **CORE: Int-1** | ¹H NMR (DMSO-*d₆*, 400MHz) δ ppm 8.07-8.12 (m, 1H), 7.99-8.05 (m, 2H), 7.58-7.64 (m, 2H), 7.49-7.56 (m, 1H), 7.06-7.12 (m, 1H), 4.47-4.57 (m, 2H), 3.72-3.81 (m, 1H), 3.37-3.54 (m, 3H), 2.88-2.98 (m, 1H), 2.84 (s, 3H), 2.13-2.26 (m, 1H), 1.64-1.88 (m, 3H). MS obsd. (ESI⁺) [(M+H)⁺]: 559.1. |
| | | **LINKER:** 1,2-dibromoethane | |
| | | **CYC:** tert-butyl pyrrolidine-2-carboxylate | |
| | 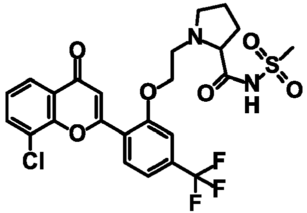 | **SA:** methanesulfonam ide | |
| **074** | **1-[2-[5-bromo-2-(8-chloro-4-oxo-chromen-2-yl)phenoxy]ethyl]-N-methylsulfonyl-pyrrolidine-2-carboxamide** | **CORE:Int-4** | ¹H NMR (DMSO-*d₆*, 400MHz) δ ppm 7.98-8.04 (m, 2H), 7.81-7.87 (m, 1H), 7.54-7.58 (m, 1H), 7.43-7.54 (m, 2H), 7.02-7.07 (m, 1H), 4.43-4.50 (m, 2H), 3.71-3.80 (m, 1H), 3.41-3.53 (m, 2H), 2.87-2.99 (m, 1H), 2.84 (s, 3H), 2.12-2.25 (m, 1H), 1.66-1.89 (m, 3H), 1.18-1.29 (m, 1H). MS obsd. (ESI⁺) [(M+H)⁺]: 569.1. |
| | | **LINKER:** 1,2-dibromoethane | |
| | | **CYC:** tert-butyl pyrrolidine-2-carboxylate | |
| | 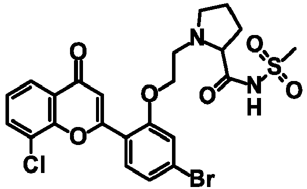 | **SA:** methanesulfonam ide | |

### Example 075: 1-[2-[5-bromo-2-(8-chloro-4-oxo-chromen-2-yl)phenoxy]ethyl]pyrrolidin-2-one

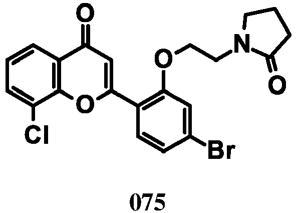

### Step 1: Preparation of 1-(2-chloroethyl)pyrrolidin-2-one

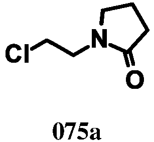

To a solution of 1-(2-hydroxyethyl)pyrrolidin-2-one (200 mg, 1.55 mmol) in DCM (15 mL) was added thionyl chloride (368 mg, 3.1 mmol) at room temperature and the reaction mixture was stirred at 60 °C for 3 hours. After the reaction was completed, the reaction mixture was washed with water and brine. The organic layer was separated out and dried over anhydrous MgSO₄, filtered and concentrated *in vacuo* to give 1-(2-chloroethyl)pyrrolidin-2-one (200 mg, yield: 65.6%) as brown oil.

### Step 2: Preparation of 1-[2-[5-bromo-2-(8-chloro-4-oxo-chromen-2-yl)phenoxy]ethyl]pyrrolidin-2-one

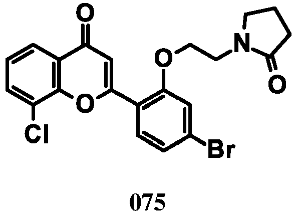

To a solution of 2-(4-bromo-2-hydroxy-phenyl)-8-chloro-chromen-4-one (200.0 mg, 0.570 mmol) and K₂CO₃ (157.24 mg, 1.14 mmol) in DMF (5 mL) was added 1-(2-chloroethyl)pyrrolidin-2-one (84 mg, 0.570 mmol) and the reaction mixture was stirred at 80 °C for 16 hours. The reaction mixture was filtered and the filtrate was concentrated *in vacuo.* The residue was purified by prep-HPLC to give 1-[2-[5-bromo-2-(8-chloro-4-oxo-chromen-2-yl)phenoxy]ethyl]pyrrolidin-2-one (42 mg, 15.68% yield) as a yellow solid. MS obsd.

(ESI⁺)[(M+H)⁺]: 462.1.

**Example 075:** ¹H NMR (DMSO-*d₆*, 400MHz) δ ppm 8.04-7.95 (m, 2H), 7.86 (d, *J* =8.4 Hz, 1H), 7.56 (d, *J* = 1.6 Hz, 1H), 7.50 (t, *J* =7.9 Hz, 1H), 7.43 (dd, *J* =1.7, 8.4 Hz, 1H), 6.97 (s, 1H), 4.35 (t, *J* =5.3 Hz, 2H), 3.59 (br t, *J* =5.3 Hz, 2H), 3.45-3.42 (m, 2H), 2.20 (t, *J* =8.1 Hz, 2H), 1.86 (m, *J* =7.5 Hz, 2H).

### Example 076: N-[1-[2-[5-bromo-2-(8-chloro-4-oxo-chromen-2-yl)phenoxy]ethyl]pyrrolidin-3-yl]-N-methyl-methanesulfonamide

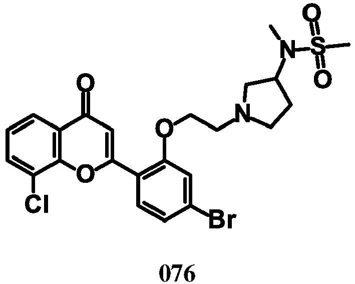

### Step 1: Preparation of 2-[4-bromo-2-(2-bromoethoxy)phenyl]-8-chloro-chromen-4-one

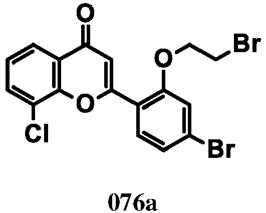

To a solution of 2-(4-bromo-2-hydroxy-phenyl)-8-chloro-chromen-4-one (500 mg, 1.42 mmol, as the **"CORE"** in Table 4) and 1,2-dibromoethane (1.07 g, 5.69 mmol, as the **"LINKER"** in Table 4) in DMF was added K₂CO₃ (197 mg, 1.42 mmol). The reaction mixture was stirred at 50 °C overnight. After the reaction was completed, the reaction mixture was partitioned between EtOAc (10 mL) and water (30 mL). The organic layer was separated out and the aquatic phase was extracted with EtOAc (30 mL) twice. The combined organic layer was concentrated *in vacuo* and the residue was purified by ISCO (eluting with EtOAc : PE = 0 to 20%) to give 2-[4-bromo-2-(2-bromoethoxy)phenyl]-8-chloro- chromen-4-one (450 mg, yield: 69%) as a yellow solid.

### Step 2: Preparation of N-[1-[2-[5-bromo-2-(8-chloro-4-oxo-chromen-2-yl)phenoxy]ethyl]pyrrolidin-3-yl]-N-methyl-methanesulfonamide

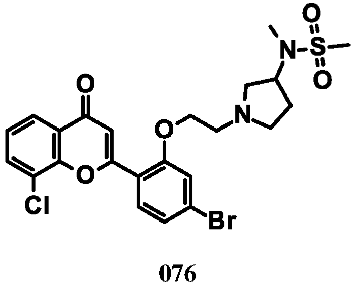

To a mixture of 2-[4-bromo-2-(2-bromoethoxy)phenyl]-8-chloro-chromen-4-one (70 mg, 153 µmol), 3-(methylsulfonyl)pyrrolidine (32.8 mg, 153 µmol, as the **"AMINE"** in Table 4) in DMF (1 mL) and ethanol (3 mL) was added sodium bicarbonate (51.3 mg, 0.611 mmol) and the reaction mixture was stirred at 50 °C for 5 hours. After the reaction was completed, the mixture was purified by prep-HPLC to give N-[1-[2-[5-bromo-2-(8-chloro-4-oxo-chromen-2-yl)phenoxy]ethyl]pyrrolidin-3-yl]-N-methyl-methanesulfonamide (33.5 mg, yield: 37.5%) as a white solid. MS obsd. (ESI⁺) [(M+2H)⁺]: 557.2.

**Example 076:** ¹H NMR (DMSO-*d₆*, 400MHz) δ ppm 8.03 (d, *J* = 7.9 Hz, 2H), 7.86 (d, *J* = 8.4 Hz, 1H), 7.61 (d, *J* = 1.7 Hz, 1H), 7.40-7.54 (m, 2H), 7.00 (s, 1H), 4.40-4.89 (m, 3H), 3.64-3.85 (m, 2H), 3.03-3.28 (m, 1H),2.90 (s, 3H), 2.41-2.62 (m,6H), 1.85-2.29 (m, 2H).

The following compounds **077** to **092** were prepared in analogy to the procedure described for the preparation of Example **076,** replacing 2-(4-bromo-2-hydroxy-phenyl)-8-chloro-chromen-4-one with **"CORE",** 1,2-dibromoethane with **"LINKER"** in **Step 1,** 3-(methylsulfonyl)pyrrolidine with **"AMINE"** in **Step 2,** by the reagent indicated in Table 4.

**Table 4: Compounds synthesis and characterization**

| Example No. | Compounds Name and Structure | **CORE, LINKER** and **AMINE** | ¹H NMR and (ESI⁺) |
|---|---|---|---|
| **077** | **8-chloro-2-[2-[3-[(3R,4S)-3,4-dihydroxypyrrolidin-1-yl]propoxy]-4-(trifluoromethyl)phenyl]chro men-4-one** | **CORE: Int-1** | H NMR (DMSO-*d₆*, 400MHz) δ ppm 8.09-8.17 (m, 1H), 7.98-8.07 (m, 2H), 7.48-7.60 (m, 3H), 7.06 (s, 1H), 4.30 (br t, *J* = 5.9 Hz, 2H), 4.00 (br s, 2H), 2.97-3.06 (m, 2H), 2.77 (br d, *J* = 6.2 Hz, 2H), 2.58 (br d, *J* = 9.5 Hz, 2H), 1.95-2.04 (m, 2H). MS obsd. (ESI⁺) [(M+H)⁺]: 484.4. |
| | | **LINKER:** 1,3-dibromopropan e **AMINE:** (3R,4S)-pyrrolidine-3,4-diol | |
| | 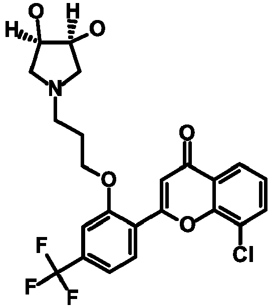 | | |
| **078** | **8-chloro-2-[2-[3-[(1,1-dioxothian-4-yl)amino]propoxy]-4-(trifluoromethyl)phenyl]chro men-4-one** | **CORE: Int-1** | ¹H NMR (DMSO-*d₆*, 400MHz) δ ppm 8.14 (d, *J* = 7.9 Hz, 1H), 8.00-8.08 (m, 2H), 7.46-7.62 (m, 3H), 7.09 (s, 1H), 4.29-4.40 (m, 2H), 3.01-3.13 (m, 2H), 2.88-3.00 (m, 2H), 2.61-2.77 (m, 3H), 1.75-2.07 (m, 4H), 1.53-1.66 (m, 2H). MS obsd. (ESI⁺) [(M+H)⁺]: 530.3. |
| | | **LINKER:** 1,3-dibromopropan e **AMINE:** 1,1-dioxothian-4-amine hydrochloride | |
| | 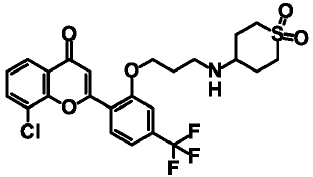 | | |
| **079** | **8-chloro-2-[2-[3-(2-methylsulfonylethylamino)pr opoxy]-4-(trifluoromethyl)phenyl]chro men-4-one** | **CORE: Int-1** | ¹H NMR (DMSO-*d₆*, 400MHz) δ ppm 8.10-8.17 (m, 1H), 8.00-8.08 (m, 2H), 7.49-7.65 (m, 3H), 7.05 (s, 1H), 4.31-4.42 (m, 2H), 3.43-3.56 (m, 4H), 3.09-3.19 (m, 5H), 2.09-2.20 (m, 2H). MS obsd. (ESI⁺) [(M+H)⁺]: 503.9. |
| | | **LINKER:** 1,3-dibromopropan e **AMINE:** 2-methylsulfonyl ethanamine | |
| | 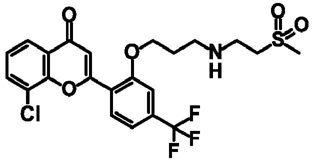 | | |
| **080** | **2-[3-[2-(8-chloro-4-oxo-chromen-2-yl)-5-(trifluoromethyl)phenoxy]pr opylamino] ethanesulfonamid e** | **CORE: Int-1** | ¹H NMR (DMSO-*d₆*, 400MHz) δ ppm 8.15 (d, *J* = 8.4 Hz, 1H), 8.03 (dd, *J* = 7.8, 3.9 Hz, 2H), 7.46-7.60 (m, 3H), 7.11 (s, 1H), 6.83 (br s, 2H), 4.33 (br t, *J* = 6.2 Hz, 2H), 3.05-3.15 (m, 2H), 2.89-2.95 (m, 2H), 2.72 (br t, *J* = 6.7 Hz, 2H), 1.95 (quin, *J* = 6.2 Hz, 2H). MS obsd. (ESI⁺) [(M+H)⁺]: 505.0. |
| | | **LINKER:** 1,3-dibromopropan e **AMINE:** 2-aminoethanesu Ifonamide | |
| | 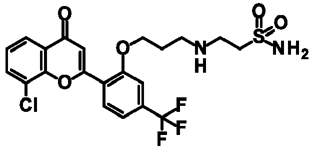 | | |
| **081** | **8-chloro-2-[2-[3-(4-methylsulfonylpiperazin-1-yl)propoxy]-4-(trifluoromethyl)phenyl]chro men-4-one** | **CORE: Int-1** | ¹H NMR (DMSO-*d₆*, 400MHz) δ ppm 8.14 (d, *J* = 8.2 Hz, 1H), 8.04 (dq, *J* = 7.9, 1.4 Hz, 2H), 7.61 (d, *J* = 8.8 Hz, 1H), 7.47-7.57 (m, 2H), 7.09 (s, 1H), 4.36 (t, *J* = 6.0 Hz, 2H), 3.53-3.80 (m, 4H), 3.05-3.31 (m, 6H), 3.00 (s, 3H), 2.23 (br s, 2H). MS obsd. (ESI⁺) [(M+H)⁺]: .544.9. |
| | | **LINKER:** 1,3-dibromopropan e **AMINE:** 1-methylsulfonyl | |
| | 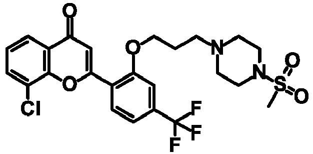 | piperazine | |
| **082** | **2-[2-[2-(4-acetylpiperazin-1-yl)ethoxy]-4-bromo-phenyl]-8-chloro-chromen-4-one** | **CORE: Int-4** | ¹H NMR (DMSO-*d₆*, 400MHz) δ ppm 7.97-8.03 (m, 2H), 7.93 (d, *J* = 8.4 Hz, 1H), 7.55 (d, *J* = 1.7 Hz, 1H), 7.47-7.53 (m, 2H), 7.43 (dd, *J* = 8.5, 1.8 Hz, 1H), 4.33 (t, *J* = 5.3 Hz, 2H), 3.47 (q, *J* = 4.9 Hz, 4H), 2.77 (t, *J* = 5.3 Hz, 2H), 2.45-2.49 (m, 2H), 2.42 (t, *J* = 5.0 Hz, 2H), 1.98 (s, 3H). MS obsd. (ESI⁺) [(M+H)⁺]: 505.0. |
| | | **LINKER:** 1,2-dibromoethane | |
| | 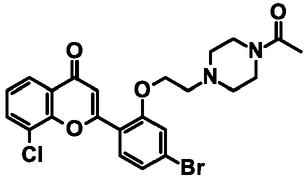 | **AMINE:** 1-piperazin-1-ylethanone | |
| **083** | **1-[2-[5-bromo-2-(8-chloro-4-oxo-chromen-2-yl)phenoxy]ethyl]pyrrolidine-3-carboxamide** | **CORE: Int-4** | ¹H NMR (DMSO-*d₆*, 400MHz) δ ppm 8.02 (d, *J* = 7.9 Hz, 2H), 7.84 (d, *J* = 8.4 Hz, 1H), 7.57-7.68 (m, 2H), 7.46-7.56 (m, 2H), 7.07-7.22 (m, 1H), 6.97 (s, 1H), 4.37-4.62 (m, 2H), 3.57-3.87 (m, 4H), 2.95-3.32 (m, 3H), 1.77-2.35 (m, 2H). MS obsd. (ESI⁺) [(M+H)⁺]: 491.1. |
| | | **LINKER:** 1,2-dibromoethane | |
| | 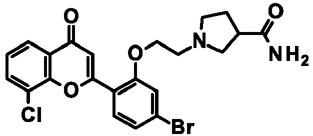 | **AMINE:** pyrrolidine-3-carboxamide | |
| **084** | **2-[4-bromo-2-[2-(3-methylsulfonylpyrrolidin-1-yl)ethoxy]phenyl]-8-chloro-chromen-4-one** | **CORE: Int-4** | ¹H NMR (DMSO-*d₆*, 400MHz) δ ppm 8.02 (d, *J* = 7.9 Hz, 2H), 7.84 (d, *J* = 8.4 Hz, 1H), 7.57-7.68 (m, 2H), 7.46-7.56 (m, 2H), 7.07-7.22 (m, 1H), 6.97 (s, 1H), 4.37-4.62 (m, 2H), 3.57-3.87 (m, 4H), 2.95-3.32 (m, 3H), 1.77-2.35 (m, 3H). MS obsd. (ESI⁺) [(M+H)⁺]: 526.2. |
| | | **LINKER:** 1,2-dibromoethane | |
| | 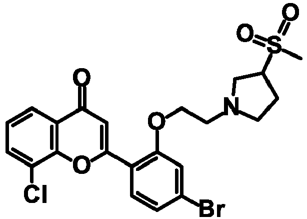 | **AMINE:** 3-methylsulfonyl pyrrolidine | |
| **085** | **4-[2-[5-bromo-2-(8-chloro-4-oxo-chromen-2-yl)phenoxy]ethyl]thiomorpho line-3-carboxamide** | **CORE: Int-4** | ¹H NMR (DMSO-*d₆*, 400MHz) δ ppm 7.95 (dd, *J* = 7.9, 2.3 Hz, 2H), 7.80 (d, *J* = 8.4 Hz, 1H), 7.35-7.54 (m, 3H), 7.03 (br s, 1H), 4.39 (br s, 2H), 3.45-3.82 (m, 4H), 2.81-2.96 (m, 3H), 2.63-2.78 (m, 1H), 2.49-2.59 (m, 1H). MS obsd. (ESI⁺) [(M+H)⁺]: 523.1. |
| | | **LINKER:** 1,2-dibromoethane | |
| | 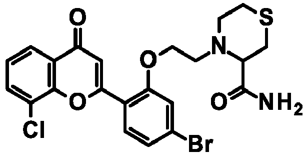 | **AMINE:** thiomorpholine -3-carboxamide | |
| **086** | **N-[3-[2-(8-chloro-4-oxo-chromen-2-yl)-5-(trifluoromethyl)phenoxy]-2-hydroxypropyl]cyclopropanesulfona mide** | **CORE: Int-1** | ¹H NMR (DMSO-*d₆*, 400MHz) δ ppm 8.16 (d, *J* = 7.9 Hz, 1H), 8.03 (dq, *J* = 7.9, 1.4 Hz, 2H), 7.55-7.64 (m, 2H), 7.52 (t, *J* = 7.9 Hz, 1H), 7.24 (s, 1H), 7.14-7.20 (m, 1H), 5.38 (br d, *J* = 4.4 Hz, 1H), 4.28-4.36 (m, 1H), 4.20-4.25 (m, 1H), 3.97 (br d, *J* = 4.9 Hz, 1H), 3.08-3.19 (m, 1H),1.93-2.09 (m, 1H), 0.84-0.92 (m, 4H). MS obsd. (ESI⁺) [(M+H)+]: 518.4. |
| | | **LINKER:** 2-(chloromethyl) oxirane **AMINE:** cyclopropanes | |
| | 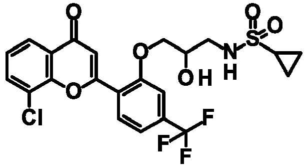 | ulfonamide | |
| **087** | **8-chloro-2-[2-[2-[4-(1H-tetrazol-5-yl)-1-piperidyl]ethoxy]-4-(trifluoromethyl)phenyl]chro men-4-one** | **CORE: Int-4** | ¹H NMR (CHLOROFORM-d, 400MHz) δ ppm 7.97-8.11 (m, 2H), 7.72 (br dd, *J* = 7.7, 1.6 Hz, 1H), 7.26-7.48 (m, 3H), 7.01 (s, 1H), 4.97-5.15 (m, 2H), 4.60-4.71 (m, 2H), 4.12 (dt, *J* = 8.4, 4.1 Hz, 4H), 3.31-3.64 (m, 1H), 3.00-3.31 (m, 2H), 2.26-2.42 (m, 1H), 1.98-2.08 (m, 2H), 1.18 (m, 2H). MS obsd. (ESI⁺) [(M+H)⁺]: 530.0. |
| | | **LINKER:** 1,2-dibromoethane **AMINE:** 4-(1H-tetrazol-5-yl)piperidine | |
| | 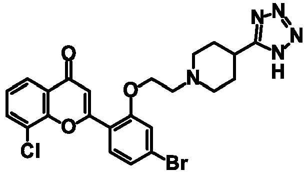 | | |
| **088** | **8-chloro-2-[2-[3-(3-methylsulfonylpyrrolidin-1-yl)propoxy]-4-(trifluoromethyl)phenyl]chro men-4-one** | **CORE: Int-1** | ¹H NMR (DMSO-*d₆*, 400MHz) δ ppm 8.10-8.16 (m, 1H), 8.03 (d, *J* = 8.1 Hz, 2H), 7.43-7.60 (m, 3H), 7.09 (s, 1H), 4.31 (t, *J* = 6.1 Hz, 2H), 3.75 (br d, *J* = 8.8 Hz, 1H), 2.91 (s, 3H), 2.75-2.85 (m, 2H), 2.52-2.62 (m, 4H), 1.88-2.15 (m, 4H). MS obsd. (ESI⁺) [(M+H)⁺]: 529.9. |
| | | **LINKER:** 1,3-dibromopropan e **AMINE:** 3-methylsulfonyl pyrrolidine | |
| | 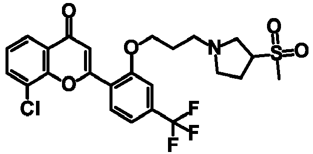 | | |
| **089** | **2-[4-bromo-2-[2-(1,1-dioxo-1,4-thiazinan-4-yl)ethoxy]phenyl]-8-chloro-chromen-4-one** | **CORE: Int-4** | ¹H NMR (DMSO-*d₆*, 400MHz) δ ppm 7.99-8.06 (m, 2H), 7.95 (d, *J* = 8.6 Hz, 1H), 7.38-7.57 (m, 4H), 4.35 (t, *J* = 5.1 Hz, 2H), 3.21 (br d, *J* = 4.5 Hz, 4H), 2.95-3.14(m, 6H). MS obsd. (ESI⁺) [(M+H)⁺]: 511.9. |
| | | **LINKER:** 1,2-dibromoethane | |
| | | **AMINE:** thiomorpholine 1,1-dioxide | |
| | 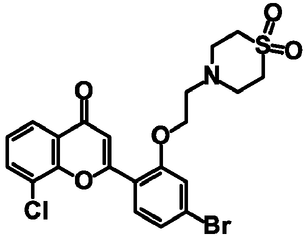 | | |
| **090** | **2-[4-bromo-2-[2-(2-morpholinoethoxy)ethoxy]ph enyl]-8-chloro-chromen-4-one** | **CORE: Int-4** | ¹H NMR (DMSO-*d₆*, 400MHz) δ ppm 9.76-10.12 (m, 1H), 7.90-8.08 (m, 3H), 7.43-7.61 (m, 2H), 7.29 (s, 1H), 4.33-4.48 (m, 2H), 3.79-3.98 (m, 6H), 3.57-3.75 (m, 2H), 3.44-3.53 (m, 4H), 2.87-3.22 (m, 2H). MS obsd. (ESI⁺) [(M+H)⁺]: 508.1. |
| | | **LINKER:** 1-bromo-2-(2-bromoethoxy)e thane | |
| | | **AMINE:** morpholine | |
| **091** | **1-[2-[2-(8-chloro-4-oxo-chromen-2-yl)-5-methyl-phenoxy]ethyl]imidazolidin-2-one** | **CORE: Int-2 AMINE:** tert-butyl N-(3-bromoethyl)car bamate | ¹H NMR (DMSO-*d₆*, 400MHz) δ ppm 7.99 (d, *J* =8.1 Hz, 2H), 7.87 (d, *J* =8.1 Hz, 1H), 7.49 (t, *J* =7.9 Hz, 1H), 7.16 (s, 1H), 7.09 (s, 1H), 7.03 (d, *J* =8.1 Hz, 1H), 6.35 (s, 1H), 4.27 (t, *J* =5.4 Hz, 2H), 3.25-3.48 (m, 4H), 3.13-3.23 (m, 2H), 2.40 (s, 3H). MS obsd. (ESI⁺) [(M+H)⁺]: 399.0. |
| | 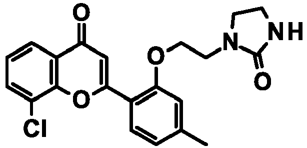 | **AMINE:** imidazolidin-2-one | |
| **092** | **1-[2-[2-(8-chloro-4-oxo-chromen-2-yl)-5-methyl-phenoxy]ethyl]-3-methyl-imidazolidin-2-one** | **CORE: Int-2** | ¹H NMR (HDMSO, 400MHz) δ ppm 7.99 (d, *J* =7.8 Hz, 2H), 7.86 (d, *J* =7.9 Hz, 1H), 7.49 (t, *J* =7.9 Hz, 1H), 7.16 (s, 1H), 7.06 (m, 2H), 4.28 (s, 2H), 3.49 (t, *J* =5.4 Hz, 2H), 3.14-3.22 (m, 4H), 2.60 (s, 3H), 2.40 (s, 3H). MS obsd. (ESI⁺) [(M+H)⁺]: 413.4. |
| | | **AMINE:** tert-butyl N-(3-bromoethyl)car bamate | |
| | 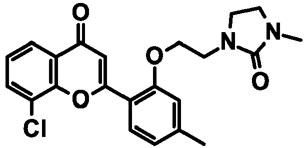 | **AMINE:** 1-methylimidazo lidin-2-one | |

### Example 093: 8-chloro-2-[2-[2-(4-methylsulfinyl-1-piperidyl)ethoxy]-4-(trifluoromethyl)phenyl]chromen-4-one and Example 094: 8-chloro-2-[2-[2-[4-(methylsulfonimidoyl)-1-piperidyl]ethoxy]-4-(trifluoromethyl)phenyl]chromen-4-one

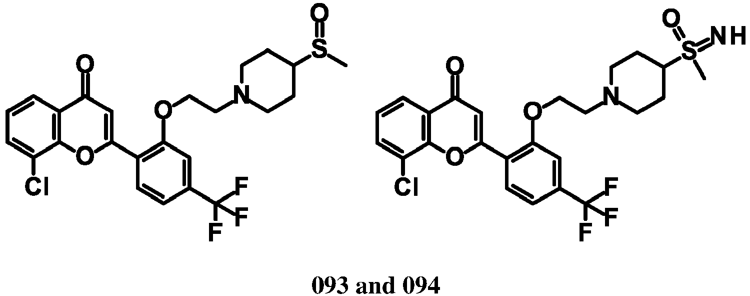

### Step 1: preparation of 8-chloro-2-[2-[2-(4-methylsulfanyl-1-piperidyl)ethoxy]-4-(trifluoromethyl)phenyl]chromen-4-one

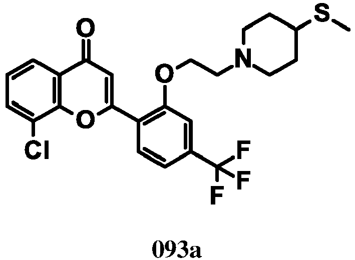

Compound **093a** was prepared in analogy to the procedure described for the preparation of example **076** by using 4-methylsulfanylpiperidine as the starting material instead of 3-(methylsulfonyl)pyrrolidine in **Step 2.** MS obsd. (ESI⁺) [(M+H)⁺]: 497.9.

### Step 2: preparation of 8-chloro-2-[2-[2-(4-methylsulfinyl-1-piperidyl)ethoxy]-4-(trifluoromethyl)phenyl]chromen-4-one and 8-chloro-2-[2-[2-[4-(methylsulfonimidoyl)-1-piperidyl]ethoxy]-4-(trifluoromethyl)phenyl]chromen-4-one

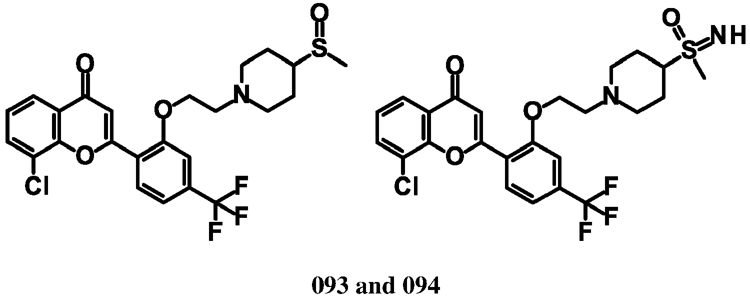

A solution of 8-chloro-2-[2-[2-(4-methylsulfanyl-1-piperidyl)ethoxy]-4-(trifluoromethyl)phenyl]chromen-4-one (40 mg, 80.3 µmol), iodobenzene diacetate (103 mg, 321 µmol) and ammonium carbomate (11.6 mg, 120 µmol) in MeOH (10 mL) was stirred at room temperature for 4 hours. After the reaction was completed, the mixture was purified by prep-HPLC to give 8-chloro-2-[2-[2-(4-methylsulfinyl-1-piperidyl)ethoxy]-4-(trifluoromethyl)phenyl]chromen-4-one (15.2 mg, yield: 35.0%) and 8-chloro-2-[2-[2-[4-(methylsulfonimidoyl)-1-piperidyl]ethoxy]-4-(trifluoromethyl)phenyl]chromen-4-one (2.7 mg, yield: 5.8%) as white solid.

**Example 093:** MS obsd. (ESI⁺)[(M+H)⁺]: 513.9. ¹H NMR (DMSO-*d*₆, 400MHz) δ ppm 8.08-8.13 (m, 1H), 8.05 (dd, *J* = 7.9, 1.3 Hz, 2H), 7.65 (t, *J* = 3.2 Hz, 2H), 7.54 (t, *J* = 7.9 Hz, 1H), 7.06 (s, 1H), 4.62 (br t, *J* = 4.6 Hz, 2H), 3.65 (br s, 2H), 3.57 (br d, *J* = 3.8 Hz, 2H), 3.21-3.31 (m, 1H), 3.04-3.13 (m, 2H), 2.98 (s, 3H), 2.20 (br d, *J* = 1.0 Hz, 2H), 1.78-1.96 (m, 2H).

**Example 094:** MS obsd. (ESI⁺)[(M+H)⁺]: 528.9. ¹H NMR (DMSO-*d*₆, 400MHz) δ ppm 9.46-9.69 (m, 1H), 8.10 (br d, *J* = 8.3 Hz, 1H), 8.00-8.07 (m, 2H), 7.61-7.68 (m, 2H), 7.54 (t, *J* = 7.9 Hz, 1H), 7.03 (br s, 1H), 4.63 (br s, 2H), 3.56-3.77 (m, 4H), 3.06-3.23 (m, 2H), 2.69-2.85 (m, 2H), 2.54-2.58 (m, 2H), 1.94-2.16 (m, 2H), 1.71-1.89 (m, 2H).

### Example 095: ethyl 2-[3-[2-(8-chloro-4-oxo-chromen-2-yl)-5-methyl-phenoxy]propylamino]-2-oxo-acetate

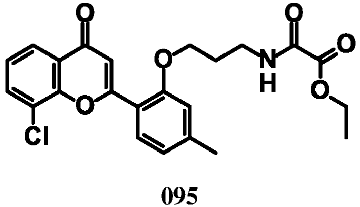

### Step 1: Preparation of tert-butyl N-[3-[2-(8-chloro-4-oxo-chromen-2-yl)-5-methyl-phenoxy]propyl]carbamate

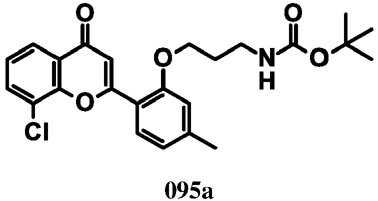

To a solution of 8-chloro-2-(2-hydroxy-4-methyl-phenyl)chromen-4-one (500 mg, 1.74 mmol, as the **"CORE"** in Table 5) and tert-butyl (3-bromopropyl)carbamate (415 mg, 1.74 mmol, as the **"LINKER"** in Table 5) in DMF (18 mL) was added K₂CO₃ (1.21 g, 8.72 mmol) and the mixture was stirred at 50 °C overnight. After the reaction was completed, the reaction mixture was partitioned between EtOAc (20 mL) and water (30 mL). The organic layer was separated out and the aqueous phase was extracted with EtOAc (30 mL) twice. The combined organic layer was concentrated *in vacuo* and the residue was purified by ISCO (eluting with EtOAc : PE = 0 to 30%) to give tert-butyl N-[3-[2-(8-chloro-4-oxo-chromen-2-yl)-5-methyl-phenoxy]propyl]carbamate (530 mg, yield: 68.5%) as a yellow solid. MS obsd. (ESI⁺) [(M+H)⁺]: 444.2.

### Step 2: Preparation of 2-[2-(3-aminopropoxy)-4-methyl-phenyl]-8-chloro-chromen-4-one

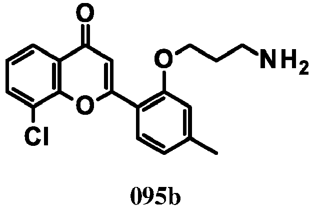

Compound tert-butyl N-[3-[2-(8-chloro-4-oxo-chromen-2-yl)-5-methyl-phenoxy]propyl]carbamate (530mg, 1.06 mmol) was dissolved in the DCM (3 mL), followed by adding TFA (1 mL, 13 mmol) dropwise. The reaction was stirred at room temperature for 2 hours. Then, the reaction mixture was concentrated *in vacuo* and coevaperated with toluene to give 2-[2-(3-aminopropoxy)-4-methyl-phenyl]-8-chloro-chromen-4-one (400 mg, yield: 97.4%) as yellow oil. MS obsd. (ESI⁺) [(M+H)⁺]: 344.2.

### Step 3: Preparation of tert-butyl 2-[3-[2-(8-chloro-4-oxo-chromen-2-yl)-5-methyl-phenoxy]propylamino]-2-oxo-acetate

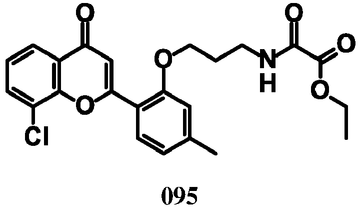

To a mixture solution of 2-[2-(3-aminopropoxy)-4-methyl-phenyl]-8-chloro-chromen-4-one (540 mg, 1.57 mmol) and DIPEA (549 µL, 3.14 mmol) in DCM (15 mL) was added ethyl 2-chloro-2-oxoacetate (197 µL, 1.73 mmol, as the **"TAIL"** in Table 5) at 0 °C. The reaction was stirred at room temperature for 2 hours. After the reaction was completed, the reaction was adjusted to pH~4 by addition of 4N HCl and then extracted with EtOAc (50 mL) three times. The combined organic layer was washed with brine, dried over anhydrous Na₂SO₄ and concentrated *in vacuo* to give 2-[3-[2-(8-chloro-4-oxo-chromen-2-yl)-5-methyl-phenoxy]propylamino]-2-oxo-acetate (90 mg, yield: 12.3%) as a light yellow foam. MS obsd. (ESI⁺) [(M+H)⁺]: 444.1.

**Example 095:** ¹H NMR (DMSO-*d*₆, 400MHz) δ ppm 8.98-9.07 (m, 1H), 7.95-8.04 (m, 2H), 7.83-7.92 (m, 1H), 7.42-7.55 (m, 1H), 6.97-7.13 (m, 3H), 4.11-4.25 (m, 4H), 3.28-3.34 (m, 2H), 2.40 (s, 3H), 1.94-2.06 (m, 2H), 1.19-1.29 (m, 3H).

The following compounds **096** to **109** were prepared in analogy to the procedure described for the preparation of Example **095,** replacing 8-chloro-2-(2-hydroxy-4-methyl-phenyl)chromen-4-one with **"CORE",** tert-butyl (3-bromopropyl)carbamate with **"LINKER"** in **Step 1,** replacing ethyl 2-chloro-2-oxoacetate with **"TAIL"** in **Step 3,** by the reagent indicated in Table 5.

**Table 5: Compounds synthesis and characterization**

| Example No. | Compounds Name and Structure | **CORE, LINKER** and **TAIL** | ¹H NMR and (ESI⁺) |
|---|---|---|---|
| **096** | **N-[2-[2-(8-chloro-4-oxo-chromen-2-yl)-5-(trifluoromethyl)phenoxy]eth yl]cyclopropanesulfonamide** | **CORE: Int-1** | ¹H NMR (METHANOL-*d₄*, 400MHz) δ ppm 8.16 (d, *J* =8.7 Hz, 1H), 8.00 (dd, *J* =8.0, 1.5 Hz, 1H), 7.83 (dd, *J* =7.8, 1.5 Hz, 1H), 7.32-7.52 (m, 3H), 7.25 (s, 1H), 4.51 (m, 1H), 4.27 (t, *J* =5.3 Hz, 2H), 3.53 (t, *J* =5.3 Hz, 2H), 2.45-2.56 (m, 1H), 0.80-0.97 (m, 4H). MS obsd. (ESI⁺) [(M+H)⁺]: 488.0. |
| | | **LINKER:** tert-butyl N-(2-bromoethyl)car bamate | |
| | 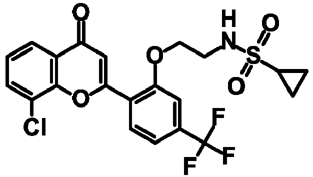 | | |
| | | **TAIL:** cyclopropanes ulfonyl chloride | |
| **097** | **N-[3-[2-(8-chloro-4-oxo-chromen-2-yl)-5-(trifluoromethyl)phenoxy]pr opyl]benzenesulfonamide** | **CORE: Int-1** | ¹H NMR (DMSO-*d*₆, 400MHz) δ ppm 9.39-9.67 (m, 1H), 8.09 (d, *J* =8.3 Hz, 1H), 8.03 (dt, *J* =7.9, 1.9 Hz, 1H), 7.67-7.75 (m, 3H), 7.39-7.62 (m, 6H), 6.97 (s, 1H), 4.23 (t, *J* =6.1 Hz, 2H), 2.93 (d, *J* =6.1 Hz, 2H), 1.88 (br t, *J* =6.4 Hz, 2H). MS obsd. (ESI⁺) [(M+H)⁺]: 538.2. |
| | | **LINKER:** tert-butyl N-(3-bromopropyl)c arbamate | |
| | | **TAIL:** benzenesulfonyl chloride | |
| | 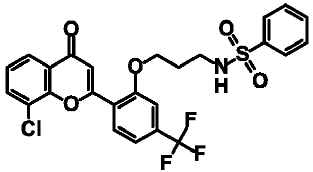 | | |
| **098** | **N-[3-[2-(8-chloro-4-oxo-chromen-2-yl)-5-(trifluoromethyl)phenoxy]pr opyl]acetamide** | **CORE: Int-1** | ¹H NMR (DMSO-*d*₆, 400MHz) δ ppm 8.13 (d, *J* =8.2 Hz, 1H), 8.02 (d, *J* =7.8 Hz, 2H), 7.53 (m, 3H), 7.09 (s, 1H), 4.27 (t, *J* =6.3 Hz, 2H), 3.21 (m, 2H), 1.93 (m, 2H), 1.78 (s, 3H). MS obsd. (ESI⁺) [(M+H)⁺]: 440.4. |
| | | **LINKER:** tert-butyl N-(3-bromopropyl)c arbamate | |
| | 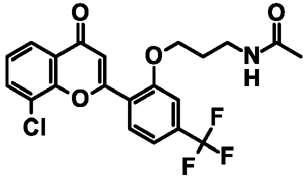 | | |
| | | **TAIL:** acetyl acetate | |
| **099** | **N-[3-[2-(8-chloro-4-oxo-chromen-2-yl)-5-(trifluoromethyl)phenoxy]pr opyl]propanamide** | **CORE: Int-1** | ¹H NMR (DMSO-*d*₆, 400MHz) δ ppm 8.13 (d, *J* =8.2 Hz, 1H), 8.03 (dd, *J* =7.9, 1.4 Hz, 1H), 7.77-7.92 (m, 1H), 7.44-7.63 (m, 3H), 7.10 (s, 1H), 4.27 (t, *J* =6.3 Hz, 2H), 3.21 (br d, *J* =6.0 Hz, 2H), 2.04 (d, *J* =7.6 Hz, 2H), 1.92 (t, *J* =6.5 Hz, 2H), 0.96 (t, *J* =7.6 Hz, 3H). MS obsd. (ESI⁺) [(M+H)⁺]: 454.2. |
| | | **LINKER:** tert-butyl N-(3-bromopropyl)c arbamate | |
| | 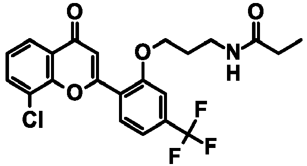 | | |
| | | **TAIL:** propanoyl propanoate | |
| **100** | **ethyl 2-[3-[2-(8-chloro-4-oxo-chromen-2-yl)-5-(trifluoromethyl)phenoxy]pr opylamino] -2-oxo-acetate** | **CORE: Int-1** | ¹H NMR (DMSO-*d*₆, 400MHz) δ ppm 9.02 (t, *J* = 5.7 Hz, 1H), 8.12 (d, *J* = 8.1 Hz, 1H), 8.08 - 7.94 (m, 2H), 7.67 - 7.42 (m, 3H), 7.10 (s, 1H), 4.27 (t, *J* = 6.1 Hz, 2H),4.17 (q, *J* = 7.1 Hz, 2H), 3.34 - 3.30 (m,2H), 2.07 - 1.95 (m, 2H), 1.24 (t, *J* = 7.1 Hz, 3H). MS obsd. (ESI⁺) [(M+H)⁺]: 498.0. |
| | | **LINKER:** tert-butyl N-(3-bromopropyl)c arbamate | |
| | 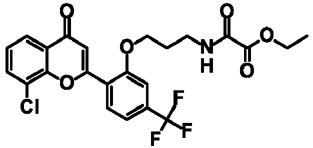 | | |
| | | **TAIL:** ethyl 2-chloro-2-oxo-acetate | |
| **101** | **ethyl 2-[2-[2-(8-chloro-4-oxo-chromen-2-yl)-5-(trifluoromethyl)phenoxy]eth ylamino]-2-oxo-acetate** | **CORE: Int-1** | ¹H NMR (DMSO-*d*₆, 400MHz) δ ppm 9.07 (br t, *J* = 5.6 Hz, 1H), 8.11 (d, *J* = 7.9 Hz, 1H), 8.01 (dq, *J* = 7.8, 1.7 Hz, 2H), 7.63 (s, 1H), 7.55-7.59 (m, 1H), 7.44-7.54 (m, 1H), 7.02 (s, 1H), 4.42 (t, *J* = 5.7 Hz, 2H), 4.17 (q, *J* = 7.1 Hz, 2H), 3.57 (q, *J* = 5.7 Hz, 2H), 1.21(t, *J* = 7.2 Hz, 3H). MS obsd. (ESI⁺) [(M+H)⁺]: 484.1. |
| | | **LINKER:** tert-butyl N-(3-bromoethyl)car bamate | |
| | 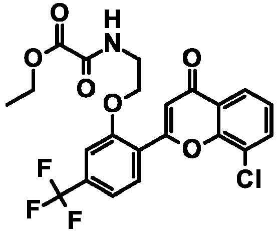 | **TAIL:** ethyl 2-chloro-2-oxo-acetate | |
| **102** | **ethyl 2-[2-[4-bromo-2-(8-chloro-4-oxo-chromen-2-yl)-5-methoxy-phenoxy]ethylamino]-2-oxo-acetate** | **CORE: Int-11** | ¹H NMR (DMSO-*d*₆, 400MHz) δ ppm 9.13 (t, *J* = 5.5 Hz, 1H), 8.11 (s, 1H), 8.04 -7.92 (m, 2H), 7.48 (t, *J* = 7.9 Hz, 1H), 6.99 (d, *J* = 8.2 Hz, 2H), 4.40 (t, *J* = 5.8 Hz, 2H), 4.18 (q, *J* = 7.1 Hz, 2H), 4.00 (s, 3H), 3.59 (q, *J* = 5.7 Hz, 2H), 1.21 (t, *J* = 7.1 Hz, 3H). MS obsd. (ESI⁺) [(M+H)⁺]: 524.0. |
| | | **LINKER:** tert-butyl N-(3-bromoethyl)car bamate | |
| | 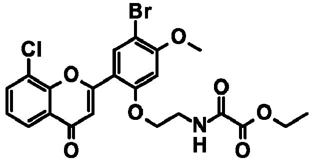 | **TAIL:** ethyl 2-chloro-2-oxo-acetate | |
| **103** | **ethyl 2-[2-[2-(8-chloro-6-methoxy-4-oxo-chromen-2-yl)-5-methyl-phenoxy]ethylamino]-2-oxo-acetate** | **CORE: Int-16** | ¹H NMR (DMSO-*d*₆, 400MHz) δ ppm 9.07 (t, *J* = 5.4 Hz, 1H), 7.83 (d, *J* = 8.0 Hz, 1H), 7.66 (d, *J* = 2.9 Hz, 1H), 7.39 (s, 1H), 7.15 (s, 1H), 7.02 (d, *J* = 8.0 Hz, 1H), 6.96 (s, 1H), 4.29 (t, *J* = 5.8 Hz, 2H), 4.19 (q, *J* = 7.1 Hz, 2H), 3.88 (s, 3H), 3.57 (dd, *J* = 11.6, 5.8 Hz, 2H), 2.40 (s, 3H), 1.22 (t, *J* = 7.1 Hz, 3H). MS obsd. (ESI⁺) [(M+H)⁺]: 460.0. |
| | | **LINKER:** tert-butyl N-(3-bromoethyl)car bamate | |
| | 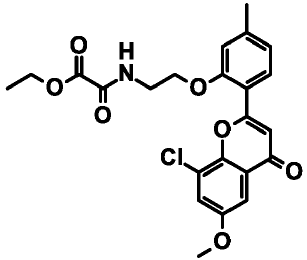 | **TAIL:** ethyl 2-chloro-2-oxo-acetate | |
| **104** | **ethyl 2-[2-[2-(8-chloro-4-oxo-chromen-2-yl)-5-methoxy-phenoxy]ethylamino]-2-oxo-acetate** | **CORE: Int-10 LINKER:** tert-butyl N-(3-bromoethyl)car bamate **TAIL:** ethyl 2-chloro-2-oxo-acetate | ¹H NMR (DMSO-*d*₆, 400MHz) δ ppm 9.09 (s, 1H), 7.96 (dd, *J* = 10.8, 8.6 Hz, 3H), 7.47 (t, *J* = 7.9 Hz, 1H), 6.97 (s, 1H), 6.82 (dd, *J* = 12.1, 3.2 Hz, 2H), 4.32 (t, *J* = 5.9 Hz, 2H), 4.19 (q, *J* = 7.1 Hz, 2H), 3.88 (s, 3H), 3.58 (dd, *J* = 11.5, 5.7 Hz, 2H), 1.22 (t, *J* = 7.1 Hz, 3H). MS obsd. (ESI⁺) [(M+H)⁺]: 446.1. |
| | 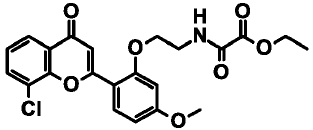 | | |
| **105** | **methyl N-[2-[5-bromo-2-(8-chloro-4-oxo-chromen-2-yl)phenoxy]ethyl]carbamate** | **CORE: Int-4** | ¹H NMR (DMSO-*d*₆, 400MHz) δ ppm 8.00 (d, *J* = 7.9 Hz, 2H), 7.86 (d, *J* = 8.4 Hz, 1H), 7.54-7.33 (m, 4H), 7.01 (s, 1H), 4.24 (t, *J* = 5.5 Hz, 2H), 3.50 (s, 3H), 3.44-3.39 (m, 2H). MS obsd. (ESI⁺) [(M+H)⁺]: 452.0. |
| | | **LINKER:** tert-butyl N-(3-bromopropyl)c arbamate | |
| | 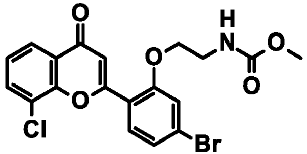 | | |
| | | **TAIL:** methyl carbonochlorid ate | |
| **106** | **ethyl N-[2-[5-bromo-2-(8-chloro-4-oxo-chromen-2-yl)phenoxy]ethyl]carbamate** | **CORE: Int-4** | ¹H NMR (DMSO-*d*₆, 400MHz) δ ppm 8.00 (d, *J* = 7.9 Hz, 2H), 7.86 (d, *J* = 8.4 Hz, 1H), 7.54 - 7.40 (m, 3H), 7.30 (s, 1H), 7.02 (s, 1H), 4.24 (t, *J* = 5.6 Hz, 2H), 3.94 (q, *J* = 7.1 Hz, 2H), 3.40 (dd, *J* = 11.0, 5.5 Hz, 2H), 1.10 (t, *J* = 7.1 Hz, 3H). MS obsd. (ESI⁺) [(M+H)⁺]: 466.0. |
| | | **LINKER:** tert-butyl N-(3-bromopropyl)c arbamate | |
| | 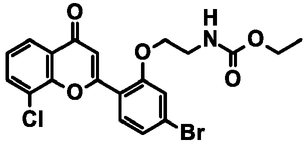 | | |
| | | **TAIL:** ethyl carbonochlorid ate | |
| **107** | **8-chloro-2-[4-methyl-2-[3-(methylsulfamoylamino)prop oxy]phenyl]chromen-4-one** | **CORE: Int-2** | ¹H NMR (DMSO-d₆) δ: 8.00 (d, *J* = 7.9 Hz, 2H), 7.89 (d, *J* =7.9 Hz, 1H), 7.49 (t, *J* =7.9 Hz, 1H), 7.13 (s, 1H), 7.07 (s, 1H), 7.03 (d, *J* =8.1 Hz, 1H), 6.95 (t, *J* =5.7 Hz, 1H), 6.69 (d, *J* =5.1 Hz, 1H), 4.23 (t, *J* =6.2 Hz, 2H), 3.01 (br d, *J* =6.1 Hz, 2H), 2.39-2.46 (m, 6H), 2.01 (br t, *J* =6.4 Hz, 2H). MS obsd. (ESI⁺) [(M+H)⁺]: 437.2. |
| | | **LINKER:** tert-butyl N-(3-bromopropyl)c arbamate **TAIL:** N-methylsulfamo yl chloride | |
| | 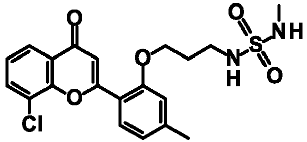 | | |
| **108** | **2-[4-bromo-2-[(1-cyclopropylsulfonyl-4-piperidyl)oxy]phenyl]-8-chloro-chromen-4-one** | **CORE: Int-4** | ¹H NMR (DMSO-*d*₆, 400MHz) δ ppm 7.96-8.06 (m, 2H), 7.85 (d, *J* = 8.4 Hz, 1H), 7.64 (d, *J* = 1.7 Hz, 1H), 7.50 (t, *J* = 7.9 Hz, 1H), 7.42 (dd, *J* = 8.4, 1.8 Hz, 1H), 7.01 (s, 1H), 4.92-5.05 (m, 1H), 3.22-3.31 (m, 4H), 2.43-2.48 (m, 1H), 1.94-2.07 (m, 2H), 1.71-1.89 (m, 2H), 0.84-0.96 (m, 4H) MS obsd. (ESI⁺) [(M+H)⁺]: 538.2. |
| | | **LINKER:** tert-butyl 4-methylsulfonyl oxypiperidine-1-carboxylate | |
| | 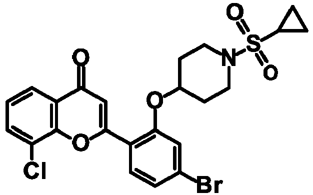 | | |
| | | **TAIL:** cyclopropanes ulfonyl chloride | |
| **109** | **ethyl 2-[2-[2-(8-chloro-4-oxo-chromen-2-yl)-5-methyl-phenoxy]ethylamino]-2-oxo-acetate** | **CORE: Int-2** | ¹H NMR (DMSO-d6, 400MHz) δ ppm 9.03-9.12 (m, 1H), 7.94-8.01 (m, 2H), 7.82-7.88 (m, 1H), 7.44-7.52 (m, 1H), 7.13-7.19 (m, 1H), 7.00-7.06 (m, 1H), 6.96-7.00 (m, 1H), 4.26-4.34 (m, 2H), 4.14-4.24 (m, 2H), 3.52-3.62 (m, 2H), 2.40 (s, 3H), 1.18-1.27 (m, 3H). MS obsd. (ESI+) [(M+H)+]: 430.1. |
| | | **LINKER:** tert-butyl N-(3-bromoethyl)car bamate | |
| | 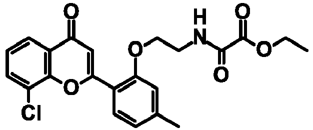 | | |
| | | **TAIL:** ethyl 2-chloro-2-oxo-acetate | |

### Example 110: 2-[[1-[[2-(8-chloro-4-oxo-chromen-2-yl)-5-(trifluoromethyl)phenoxy]methyl]cyclopropyl]amino]-2-oxo-acetic acid

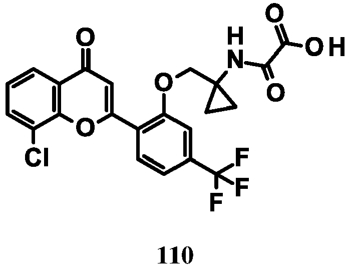

### Step 1: Preparation of tert-butyl N-[1-[[2-(8-chloro-4-oxo-chromen-2-yl)-5-(trifluoromethyl)phenoxy]methyl]cyclopropyl]carbamate

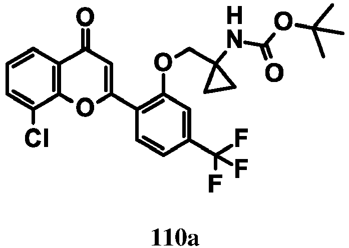

To a solution of 8-chloro-2-[2-hydroxy-4-(trifluoromethyl)phenyl]chromen-4-one (300 mg, 0.88 mmol, as the **"CORE"** in Table 6) and tert-butyl (1-(bromomethyl)cyclopropyl)carbamate (286 mg, 1.14 mmol, as the **"LINKER"** in Table 6) in DMF (4 mL) was added K₂CO₃ (609 mg, 4.4 mmol) and the mixture was stirred at 80 °C overnight. After the reaction was completed, the reaction mixture was partitioned between EtOAc (20 mL) and water (30 mL). The organic layer was separated out and the aqueous phase was extracted with EtOAc (30 mL) twice. The combined organic layer was concentrated *in vacuo* to give tert-butyl N-[1-[[2-(8-chloro-4-oxo-chromen-2-yl)-5-(trifluoromethyl)phenoxy]methyl]cyclopropyl]carbamate (200 mg, yield: 44.5%) as a yellow solid. MS obsd. (ESI⁺) [(M+H)⁺]: 510.1.

### Step 2: Preparation of 2-[2-[(1-aminocyclopropyl)methoxy]-4-(trifluoromethyl)phenyl]-8-chloro-chromen-4-one

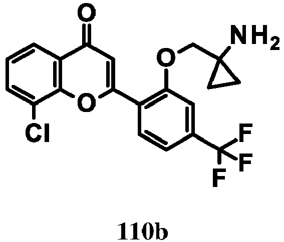

Compound tert-butyl N-[1-[[2-(8-chloro-4-oxo-chromen-2-yl)-5-(trifluoromethyl)phenoxy]methyl]cyclopropyl]carbamate (200 mg, 0.392 mmol) was dissolved in the DCM (5 mL), followed by adding TFA (1 mL, 13 mmol) dropwise. The reaction was stirred at room temperature for 2 hours. Then, the reaction mixture was concentrated *in vacuo* to give 2-[2-[(1-aminocyclopropyl)methoxy]-4-(trifluoromethyl)phenyl]-8-chloro-chromen-4-one (130 mg, yield: 80.9%) as a yellow foam. MS obsd. (ESI⁺) [(M+H)⁺]: 410.1.

### Step 3: Preparation of ethyl 2-[[1-[[2-(8-chloro-4-oxo-chromen-2-yl)-5-(trifluoromethyl)phenoxy]methyl]cyclopropyl]amino]-2-oxo-acetate

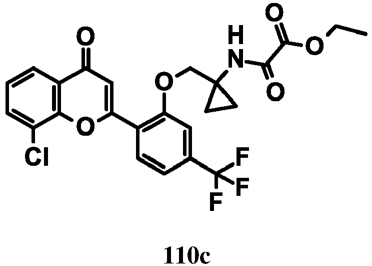

To a solution of 2-[2-[(1-aminocyclopropyl)methoxy]-4-(trifluoromethyl)phenyl]-8-chloro-chromen-4-one (150 mg, 0.366 mmol) and DIPEA (128 µL, 0.732 mmol) in DCM (5 mL) was added ethyl 2-chloro-2-oxoacetate (46 µL, 0.403 mmol, as the **"TAIL"** in Table 6) at 0 °C. The reaction was stirred at room temperature for 2 hours. After the reaction was completed, the reaction was adjusted to pH~4 by addition of 4N HCl and then extracted with EtOAc (50 mL) three times. The combined organic layer was washed with brine, dried over anhydrous Na₂SO₄ and concentrated *in vacuo* to give ethyl 2-[[1-[[2-(8-chloro-4-oxo-chromen-2-yl)-5-(trifluoromethyl)phenoxy]methyl]cyclopropyl]amino]-2-oxo-acetate (142 mg, yield: 76.13%) as a light yellow foam. MS obsd. (ESI⁺) [(M+H)⁺]: 510.1.

### Step 4: Preparation of 2-[[1-[[2-(8-chloro-4-oxo-chromen-2-yl)-5-(trifluoromethyl)phenoxy]methyl]cyclopropyl]amino]-2-oxo-acetic acid

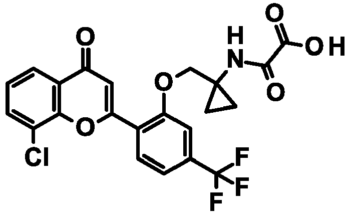

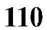

To a solution of ethyl 2-[[1-[[2-(8-chloro-4-oxo-chromen-2-yl)-5-(trifluoromethyl)phenoxy]methyl]cyclopropyl]amino]-2-oxo-acetate (100 mg, 196 µmol) in THF (5 mL) and water (5 mL) was added LiOH (28.2 mg, 1.18 mmol). The mixture was stirred at room temperature for 2 hours. After the reaction was completed, the reaction was adjusted to pH~4 by addition of 4N HCl and extracted with EtOAc (50 mL) three times. The combined organic layer was washed with brine, dried over anhydrous Na₂SO₄ and concentrated *in vacuo* to give the crude product. The crude product was purified by Pre-HPLC to give 2-[[1-[[2-(8-chloro-4-oxo-chromen-2-yl)-5-(trifluoromethyl)phenoxy]methyl]cyclopropyl]amino]-2-oxo-acetic acid (25 mg, yield: 23.8%) as a yellow foam. MS obsd. (ESI+) [(M+H)+]: 482.1.

**Example 110:** ¹H NMR (DMSO-*d*₆, 400MHz) δ ppm 9.05-9.12 (m, 1H), 8.07-8.15 (m, 1H), 7.97-8.07 (m, 2H), 7.47-7.63 (m, 3H), 7.05-7.15 (m, 1H), 4.33-4.44 (m, 2H), 0.82-0.99 (m, 4H).

The following compounds **111** to **125** were prepared in analogy to the procedure described for the preparation of Example **110,** replacing 8-chloro-2-[2-hydroxy-4-(trifluoromethyl)phenyl]chromen-4-one with **"CORE",** tert-butyl (1-(bromomethyl)cyclopropyl)carbamate with **"LINKER"** in **Step 1,** replacing ethyl 2-chloro-2-oxoacetate with **"TAIL"** in **Step 3,** by the reagent indicated in Table 6.

**Table 6: Compounds synthesis and characterization**

| Example No. | Compounds Name and Structure | **CORE, LINKER** and **TAIL** | ¹H NMR and (ESI⁺) |
|---|---|---|---|
| **111** | **2-[2-[2-(8-chloro-4-oxo-chromen-2-yl)-5-methoxy-phenoxy]ethylamino]-2-oxo-acetic acid** | **CORE: Int-10** | ¹H NMR (DMSO-*d*₆, 400MHz) δ ppm 13.89 (s, 1H), 9.01 (s, 1H), 8.00-7.93 (m, 3H), 7.47 (t, *J* = 7.9 Hz, 1H), 7.00 (s, 1H), 6.82 (d, *J* = 11.9 Hz, 2H), 4.30 (t, *J* = 6.0 Hz, 2H), 3.88 (s, 3H), 3.58-3.55 (m, 2H). MS obsd. (ESI⁺) [(M+H)⁺]: 418.1. |
| | | **LINKER:** tert-butyl N-(2-bromoethyl)car bamate | |
| | 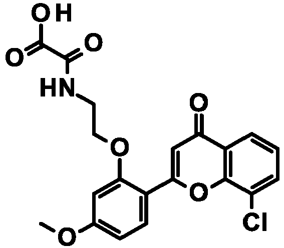 | **TAIL:** ethyl 2-chloro-2-oxo-acetate | |
| **112** | **2-[2-[2-(8-chloro-4-oxo-chromen-2-yl)-3-fluoro-5-methyl-phenoxy]ethylamino] - 2-oxo-acetic acid** | **CORE: Int-18** | ¹H NMR (DMSO-*d*₆, 400MHz) δ ppm 13.64-13.88 (m, 1H), 8.88 (br t, *J* = 5.6 Hz, 1H), 8.00 (dd, *J* = 14.9, 7.3 Hz, 2H), 7.51 (t, *J* = 7.9 Hz, 1H), 6.96-7.02 (m, 1H), 6.82-6.92 (m, 1H), 6.51-6.60 (m, 1H), 4.13-4.22 (m, 2H), 3.41-3.50 (m, 2H), 2.39 (s, 3H). MS obsd. (ESI⁺) [(M+H)⁺]: 420.0. |
| | | **LINKER:** tert-butyl N-(2-bromoethyl)car bamate | |
| | 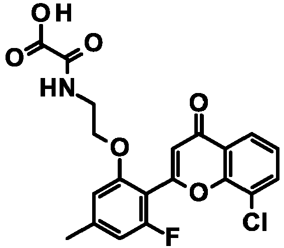 | **TAIL:** ethyl 2-chloro-2-oxo-acetate | |
| **113** | **2-[2-[5-bromo-2-(8-chloro-4-oxo-chromen-2-yl)phenoxy]ethylamino]-2-oxo-acetic acid** | **CORE: Int-4** | ¹H NMR (DMSO-*d*₆, 400MHz) δ ppm 13.75-13.93 (m, 1H), 9.01 (br t, *J* = 5.4 Hz, 1H), 7.99 (dd, *J* = 7.9, 2.4 Hz, 2H), 7.87 (d, *J* = 8.4 Hz, 1H), 7.56 (d, *J =* 1.0 Hz, 1H), 7.37-7.53 (m, 2H), 7.01 (s, 1H), 4.34 (br t, *J* = 5.6 Hz, 2H), 3.56 (q, *J* = 5.7 Hz, 2H). MS obsd. (ESI⁺) [(M+H)⁺]: 466.0. |
| | | **LINKER:** tert-butyl N-(2-bromoethyl)car bamate | |
| | 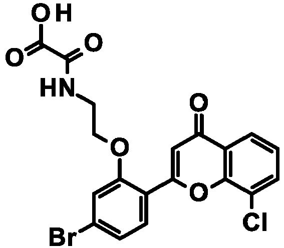 | **TAIL:** ethyl 2-chloro-2-oxo-acetate | |
| **114** | **2-[2-[2-(8-chloro-4-oxo-chromen-2-yl)-4,5-dimethoxy-phenoxy]ethylamino]-2-oxo-acetic acid** | **CORE: Int-8** | ¹H NMR (DMSO-*d*₆, 400MHz) δ ppm 8.98 (br s, 1H), 7.97 (br d, *J* = 7.5 Hz, 2H), 7.57 (s, 1H), 7.39-7.50 (m, 1H), 7.06 (s, 1H), 6.92 (s, 1H), 4.30 (br t, *J =* 5.4 Hz, 2H), 3.91 (s, 3H), 3.81 (s, 3H), 3.57 (t, *J* = 5.9 Hz, 2H). MS obsd. (ESI⁺) [(M+H)⁺]: 448.0. |
| | | **LINKER:** tert-butyl N-(2-bromoethyl)car bamate | |
| | 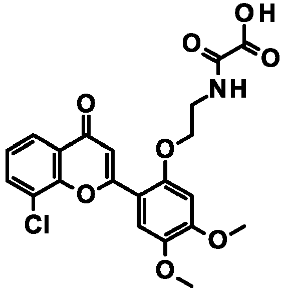 | **TAIL:** ethyl 2-chloro-2-oxo-acetate | |
| **115** | **2-[[2-[2-(8-chloro-4-oxo-chromen-2-yl)-5-(trifluoromethyl)phenoxy] - 1,1-dimethyl-ethyl]amino]-2-oxo-acetic acid** | **CORE: Int-1** | ¹H NMR (DMSO-*d*₆, 400MHz) δ ppm 8.24-8.33 (m, 1H), 8.07-8.15 (m, 1H), 7.96-8.06 (m, 2H), 7.47-7.63 (m, 3H), 6.97-7.08 (m, 1H), 4.32-4.43 (m, 2H), 1.41 (s, 6H). MS obsd. (ESI⁺) [(M+H)⁺]: 484.1. |
| | | **LINKER:** tert-butyl N-(2-bromo-1,1-dimethylethyl)carbamat e | |
| | 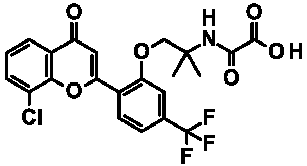 | **TAIL:** ethyl 2-chloro-2-oxo-acetate | |
| **116** | **2-[3-[2-(8-chloro-6-methoxy-4-oxo-chromen-2-yl)-5-(trifluoromethyl)phenoxy]pr opylamino]-2-oxo-acetic acid** | **CORE: Int-16** | ¹H NMR (DMSO-*d*₆, 400MHz) δ ppm 13.60-13.89 (m, 1H), 8.95 (br s, 1H), 8.10 (d, *J* = 8.3 Hz, 1H), 7.70 (d, *J* = 2.9 Hz, 1H), 7.53-7.62 (m, 2H), 7.42 (d, *J =* 3.1 Hz, 1H), 7.08 (s, 1H), 4.20-4.31 (m, 2H), 3.82-3.95 (m, 3H), 3.26-3.31 (m, 2H), 1.92-2.02 (m, 2H). MS obsd. (ESI⁺) [(M+H)⁺]: 500.1. |
| | | **LINKER:** tert-butyl N-(3-bromopropyl)c arbamate | |
| | 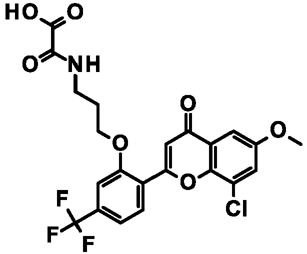 | **TAIL:** ethyl 2-chloro-2-oxo-acetate | |
| **117** | 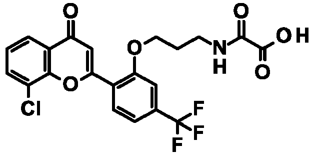 **2-[3-[2-(8-chloro-4-oxo-chromen-2-yl)-5-(trifluoromethyl)phenoxy]pr opylamino]-2-oxo-acetic acid** | **CORE: Int-1** | ¹H NMR (DMSO-*d*₆, 400MHz) δ ppm 8.89 (s, 1H), 8.11 (d, *J =* 8.1 Hz, 1H), 8.00 (d, *J* = 7.9 Hz, 2H), 7.64 -7.41 (m, 3H), 7.13 (s, 1H), 4.25 (t, *J* = 6.1 Hz, 2H), 3.30 (d, *J* = 6.2 Hz, 2H), 2.09-1.89 (m, 2H). MS obsd. (ESI⁺) [(M+H)⁺]: 470.1. |
| | | **LINKER:** tert-butyl N-(3-bromopropyl)c arbamate | |
| | | **TAIL:** ethyl 2-chloro-2-oxo-acetate | |
| **118** | **2-[2-[2-(8-chloro-4-oxo-chromen-2-yl)-3-methoxy-5-methyl-phenoxy]ethylamino] -2-oxo-acetic acid** | **CORE: Int-19** | ¹H NMR (DMSO-*d*₆, 400MHz) δ ppm 8.70 (br s, 1H), 7.72 (dd, *J* = 7.9, 1.1 Hz, 1H), 7.55 (dd, *J* = 7.5, 1.2 Hz, 1H), 7.39 (s, 1H), 7.30 (t, *J* = 7.8 Hz, 1H), 7.14 (s, 1H), 6.75 (s, 1H), 3.96-4.03 (m, 2H), 3.90 (s, 3H), 3.32-3.45 (m, 2H), 2.46 (s, 3H). MS obsd. (ESI⁺) [(M+H)⁺]: 432.1. |
| | | **LINKER:** tert-butyl N-(2-bromoethyl)car bamate **TAIL:** ethyl 2-chloro-2-oxo-acetate | |
| | 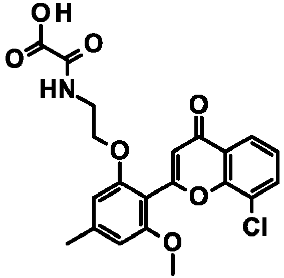 | | |
| **119** | **2-[2-[2-(8-chloro-4-oxo-chromen-2-yl)-5-(trifluoromethyl)phenoxy]eth ylamino]-2-oxo-acetic acid** | **CORE: Int-1** | ¹H NMR (DMSO-*d*₆, 400MHz) δ ppm 13.54-13.88 (m, 1H), 9.02 (t, *J* = 5.6 Hz, 1H), 8.12 (d, *J =* 7.8 Hz, 1H), 8.01 (dq, *J* = 7.9, 1.7 Hz, 2H), 7.45-7.66 (m, 3H), 7.05 (s, 1H), 4.41 (t, *J* = 5.9 Hz, 2H), 3.51-3.63 (m, 2H). MS obsd. (ESI⁺) [(M+H)⁺]: 456.1. |
| | | **LINKER:** tert-butyl N-(2-bromoethyl)carbamate | |
| | 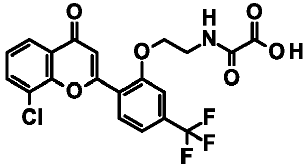 | **TAIL:** ethyl 2-chloro-2-oxo-acetate | |
| **120** | **2-[3-[2-(8-chloro-4-oxo-chromen-2-yl)-5-methyl-phenoxy]propylamino]-2-oxo-acetic acid** | **CORE: Int-2** | ¹H NMR (DMSO-*d*₆, 400MHz) δ ppm 8.91-9.01 (m, 1H), 7.95-8.03 (m, 2H), 7.79-7.91 (m, 1H), 7.44-7.53 (m, 1H), 7.06-7.12 (m, 2H), 6.98-7.06 (m, 1H), 4.08-4.21 (m, 2H), 3.30-3.35 (m, 2H), 2.43 (s, 3H), 1.95-2.07 (m, 2H). MS obsd. (ESI⁺) [(M+H)⁺]: 416.1. |
| | | **LINKER:** tert-butyl N-(3-bromopropyl)carbamate | |
| | 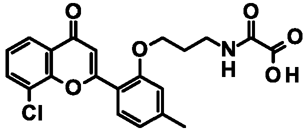 | **TAIL:** ethyl 2-chloro-2-oxo-acetate | |
| **121** | **2-[2-[2-(8-chloro-4-oxo-chromen-2-yl)-5-methyl-phenoxy]ethylamino]-2-oxo-acetic acid** | **CORE: Int-2** | ¹H NMR (DMSO-*d*₆, 400MHz) δ ppm 8.94-9.09 (m, 1H), 7.94-8.04 (m, 2H), 7.83-7.91 (m, 1H), 7.44-7.55 (m, 1H), 7.12-7.20 (m, 1H), 6.96-7.07 (m, 2H), 4.24-4.33 (m, 2H), 3.55-3.65 (m, 2H), 2.44 (s, 3H). MS obsd. (ESI⁺) [(M+H)⁺]: 402.1. |
| | | **LINKER:** tert-butyl N-(2-bromoethyl)car bamate | |
| | | **TAIL:** ethyl 2-chloro-2-oxo-acetate | |
| | 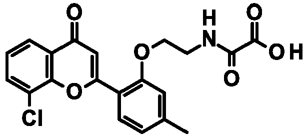 | | |
| **122** | **2-[2-[4-bromo-2-(8-chloro-4-oxo-chromen-2-yl)-5-methoxy-phenoxy]ethylamino]-2-oxo-acetic acid** | **CORE: Int-11** | ¹H NMR (DMSO-*d*₆, 400MHz) δ ppm 13.88 (s, 1H), 9.08 (s, 1H), 8.12 (s, 1H), 8.03-7.91 (m, 2H), 7.48 (t, *J* = 7.8 Hz, 1H), 7.00 (d, *J* = 5.4 Hz, 2H), 4.39 (t, *J* = 5.8 Hz, 2H), 4.00 (s, 3H), 3.60 (d, *J* = 5.7 Hz, 2H). MS obsd. (ESI⁺) [(M+H)⁺]: 496.0. |
| | | **LINKER:** tert-butyl N-(2-bromoethyl)car bamate | |
| | 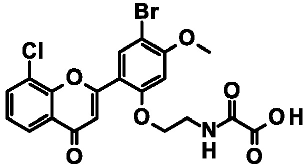 | **TAIL:** ethyl 2-chloro-2-oxo-acetate | |
| **123** | **2-[2-[2-(8-chloro-4-oxo-chromen-2-yl)-5-(trifluoromethyl)phenoxy]eth ylamino]acetic acid** | **CORE: Int-1** | ¹H NMR (DMSO-*d*₆, 400MHz) δ ppm 9.17 (br s, 1H), 8.02-8.12 (m, 3H), 7.63 (d, *J* =8.2 Hz, 1H), 7.62 (s, 1H), 7.54 (t, *J* =7.9 Hz, 1H), 7.07 (s, 1H), 4.57 (t, *J* =5.0 Hz, 2H), 3.95 (s, 2H), 3.43-3.54 (m, 2H). MS obsd. (ESI⁺) [(M+H)⁺]: 442.3. |
| | | **LINKER:** tert-butyl N-(2-bromoethyl)car bamate | |
| | 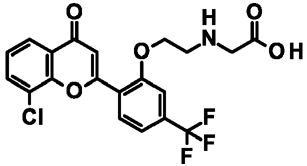 | **TAIL:** methyl 2-bromoacetate | |
| **124** | **3-[2-[2-(8-chloro-4-oxo-chromen-2-yl)-5-(trifluoromethyl)phenoxy]eth ylamino]propanoic acid** | **CORE: Int-1** | ¹H NMR (DMSO-*d*₆, 400MHz) δ ppm 8.10-8.17 (m, 1H), 8.02 (d, *J* =8.2 Hz, 2H), 7.49-7.61 (m, 3H), 7.19 (s, 1H), 4.35 (t, *J* =5.3 Hz, 2H), 3.04 (m, 2H), 2.83 (t, *J* =6.7 Hz, 3H), 2.22-2.45 (m, 2H). MS obsd. (ESI⁺) [(M+H)⁺]: 456.3. |
| | | **AMINE:** tert-butyl N-(2-bromoethyl)car bamate | |
| | 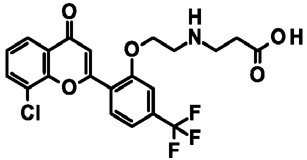 | **TAIL:** methyl 3-bromopropano ate | |
| **125** | **2-[3-[2-(8-chloro-4-oxo-chromen-2-yl)-5-(trifluoromethyl)phenoxy]pr opylamino**]**acetic acid** | **CORE: Int-1** **LINKER:** tert-butyl N-(3-bromopropyl)c arbamate **TAIL:** methyl 2-bromoacetate | ¹H NMR (DMSO-*d*₆, 400MHz) δ ppm 8.05-8.02 (m, 3H), 7.49-7.61 (m, 3H), 7.03 (s, 1H), 4.33 (t, *J* =6.3 Hz, 2H), 3.13 (m, 2H), 2.95 (t, *J* =7.4 Hz, 2H), 2.11 (m, 2H). MS obsd. (ESI⁺) [(M+H)⁺]: 456.3. |
| | 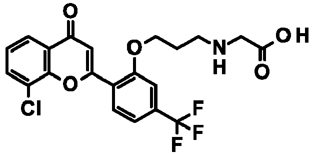 | | |
| | | | |

### Example 126:

### 2-oxo-2-[(3S)-3-[2-(8-chloro-4-oxo-chromen-2-yl)-5-(trifluoromethyl)phenoxy]pyrrolidin-1-yl]acetic acid

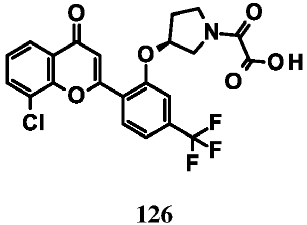

### Step 1: Preparation of tert-butyl (3S)-3-[2-(8-chloro-4-oxo-chromen-2-yl)-5-(trifluoromethyl)phenoxy]pyrrolidine-1-carboxylate

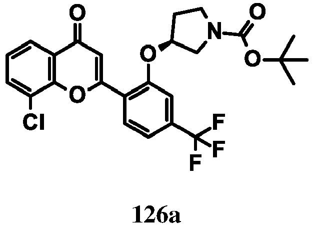

To a solution of 8-chloro-2-[2-hydroxy-4-(trifluoromethyl)phenyl]chromen-4-one (300 mg, 881 µmol, as the **"CORE"** in Table 7) and tert-butyl (S)-3-((methylsulfonyl)oxy)pyrrolidine-1-carboxylate (421 mg, 1.59 mmol, CAS registry number: 127423-61-4, Vendor: Bide Pharmatech, Catalog number: BD265247, as the **"CYC"** in Table) in DMF (4 mL) was added K₂CO₃ (609 mg, 4.4 mmol). The reaction mixture was stirred at 80 °C overnight. After the reaction went completed, EtOAc and water were poured into the reaction mixture. The aqueous layer was extracted with EtOAc (20 mL) three times, and the organic layer was combined, dried over anhydrous Na₂SO₄, filtered and concentrated *in vacuo* to afford tert-butyl (3S)-3-[2-(8-chloro-4-oxo-chromen-2-yl)-5-(trifluoromethyl)phenoxy]pyrrolidine-1-carboxylate (450 mg, yield: 100%) as a yellow solid. MS obsd. (ESI⁺) [(M+H)⁺]: 510.1.

### Step 2: Preparation of 8-chloro-2-[2-[(3S)-pyrrolidin-3-yl]oxy-4-(trifluoromethyl)phenyl]chromen-4-one

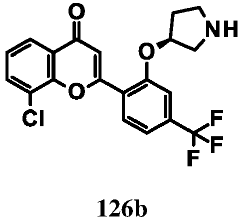

To a solution of tert-butyl (3S)-3-[2-(8-chloro-4-oxo-chromen-2-yl)-5-(trifluoromethyl)phenoxy]pyrrolidine-1-carboxylate (450 mg, 883 µmol) in DCM (5 mL) was added TFA (10 mL, 130 mmol). The reaction was stirred at room temperature for 2 hours. After the reaction was completed, the reaction was adjusted to pH~4 by addition of 4N HCl and then extracted with EtOAc (50 mL) three times. The combined organic layer was washed with brine, dried over anhydrous Na₂SO₄ and concentrated *in vacuo* to give 8-chloro-2-[2-[(3S)-pyrrolidin-3-yl]oxy-4-(trifluoromethyl)phenyl]chromen-4-one (350 mg, yield: 96.8%) as a light yellow foam. MS obsd. (ESI⁺) [(M+H)⁺]: 410.1.

### Step 3: Preparation of ethyl 2-oxo-2-[(3S)-3-[2-(8-chloro-4-oxo-chromen-2-yl)-5-(trifluoromethyl)phenoxy]pyrrolidin-1-yl]acetate

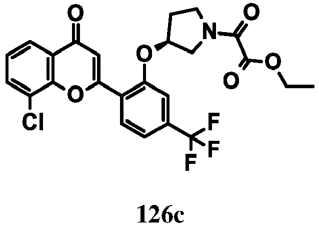

To a solution of (3S)-3-[2-(8-chloro-4-oxo-chromen-2-yl)-5-(trifluoromethyl)phenoxy]pyrrolidine-1-carboxylate (300 mg, 732 µmol) and DIPEA (256 µL, 1.46 mmol) in DCM (5 mL) was added ethyl 2-chloro-2-oxoacetate (91.6 µL, 805 µmol, as the **"TAIL"** in Table 7) at 0 °C. The reaction was stirred at room temperature for 2 hours. After the reaction was completed, the reaction was adjusted to pH~4 by addition of 4N HCl and then extracted with EtOAc (50 mL) three times. The combined organic layer was washed with brine, dried over anhydrous Na₂SO₄ and concentrated *in vacuo* to give ethyl 2-oxo-2-[(3S)-3-[2-(8-chloro-4-oxo-chromen-2-yl)-5-(trifluoromethyl)phenoxy]pyrrolidin-1-yl]acetate (300 mg, yield: 80.4%) as a light yellow foam. MS obsd. (ESI⁺) [(M+H)⁺]: 510.1.

### Step 4: Preparation of 2-oxo-2-[(3S)-3-[2-(8-chloro-4-oxo-chromen-2-yl)-5-(trifluoromethyl)phenoxy]pyrrolidin-1-yl]acetic acid

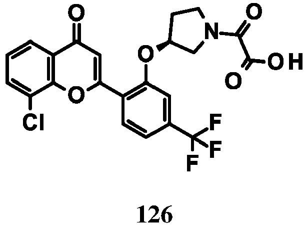

To a solution of ethyl 2-oxo-2-[(3S)-3-[2-(8-chloro-4-oxo-chromen-2-yl)-5-(trifluoromethyl)phenoxy]pyrrolidin-1-yl]acetate (300 mg, 588 µmol) in THF (5 mL) and water (5 mL) was added LiOH (84.5 mg, 3.53 mmol). The reaction mixture was stirred at room temperature for 2 hours. After the reaction was completed, the reaction was adjusted to pH~4 by addition of 4N HCl and then extracted with EtOAc (50 mL) three times. The combined organic layer was washed with brine, dried over anhydrous Na₂SO₄ and concentrated *in vacuo* to give the crude product, which was purified by Pre-HPLC to give 2-oxo-2-[(3S)-3-[2-(8-chloro-4-oxo-chromen-2-yl)-5-(trifluoromethyl)phenoxy]pyrrolidin-1-yl]acetic acid (200 mg, yield: 67%) as a yellow foam. MS obsd. (ESI⁺) [(M+H)⁺]: 482.1.

**Example 126:** ¹H NMR (DMSO-*d*₆, 400MHz) δ ppm 8.06-8.15 (m, 1H), 7.96-8.05 (m, 2H), 7.65-7.70 (m, 1H), 7.57-7.64 (m, 1H), 7.46-7.55 (m, 1H), 6.89-6.97 (m, 1H), 5.42-5.53 (m, 1H), 3.90-4.00 (m, 1H), 3.72-3.83 (m, 1H), 3.60-3.68 (m, 1H), 3.36-3.48 (m, 1H), 2.12-2.36 (m, 2H).

The following compounds **127** to **133** were prepared in analogy to the procedure described for the preparation of Example **126,** replacing 8-chloro-2-[2-hydroxy-4-(trifluoromethyl)phenyl]chromen-4-one with **"CORE",** and tert-butyl (S)-3-((methylsulfonyl)oxy)pyrrolidine-1-carboxylate with **"CYC"** in **Step 1** and replacing ethyl 2-chloro-2-oxo-acetate with **"TAIL"** in **Step 3,** by the reagent indicated in Table 7.

**Table 7: Compounds synthesis and characterization**

| Example No. | Compounds Name and Structure | **CORE, CYC, TAIL** | ¹H NMR and (ESI⁺) |
|---|---|---|---|
| **127** | **2-[3-[2-(8-chloro-4-oxo-chromen-2-yl)-5-(trifluoromethyl)phenoxy]aze tidin-1-yl]-2-oxo-acetic acid** | **CORE: Int-1** | ¹H NMR (DMSO-*d*₆, 400MHz) δ ppm 8.11-8.19 (m, 1H), 7.99-8.08 (m, 2H), 7.57-7.66 (m, 1H), 7.46-7.57 (m, 1H), 7.22-7.28 (m, 1H), 7.07-7.14 (m, 1H), 5.30-5.42 (m, 1H), 4.68-4.81 (m, 1H), 4.32-4.44 (m, 1H), 4.19-4.28 (m, 1H), 3.78-3.92 (m, 1H). MS obsd. (ESI⁺) [(M+H)⁺]: 468.1. |
| | 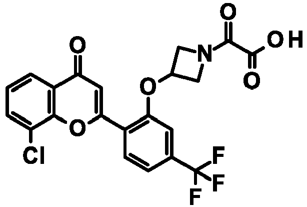 | **CYC:** tert-butyl 3-methylsulfonyloxyazetidine-1-carboxylate (CAS registry number: 141699-58-3, Vendor: Bide Pharmatech, Catalog number: BD51861) | |
| | | **TAIL:** ethyl 2-chloro-2-oxo-acetate | |
| **128** | **3-[[4-[5-bromo-2-(8-chloro-4-oxo-chromen-2-yl)phenoxy]-1-piperidyl]methyl]benzoic acid** | **CORE: Int-4** | ¹H NMR (DMSO-*d*₆, 400MHz) δ ppm 12.93-13.43 (m, 1H), 7.96-8.22 (m, 3H), 7.73-7.95 (m, 2H), 7.61-7.72 (m, 2H), 7.32-7.60 (m, 3H), 6.96 (s, 1H), 4.77-5.11 (m, 1H), 4.29-4.56 (m, 2H), 3.46-3.64 (m, 2H), 2.88-3.20 (m, 2H), 2.17-2.38 (m, 2H), 1.74-2.15(m, 2H). MS obsd. (ESI⁺) [(M+H)⁺]: 568.1. |
| | 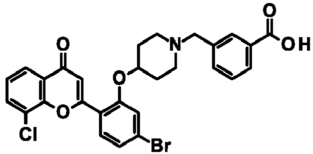 | **CYC:** tert-butyl 4-methylsulfonyloxypiperidine-1-carboxylate (CAS registry number: 141699-59-4, Vendor: Bide Pharmatech, Catalog number: BD13505) | |
| | | **TAIL:** methyl 3-(bromomethyl)benzo ate | |
| **129** | **2-[4-[2-(8-chloro-4-oxo-chromen-2-yl)-5-(trifluoromethyl)phenoxy]-1-piperidyl]-2-oxo-acetic acid** | **CORE: Int-1** | ¹H NMR (DMSO-*d*₆, 400MHz) δ ppm 8.07-8.14 (m, 1H), 7.98-8.06 (m, 2H), 7.64-7.70 (m, 1H), 7.47-7.58 (m, 2H), 7.01-7.07 (m, 1H), 5.04-5.15 (m, 1H), 3.41-3.61 (m, 2H), 3.21-3.30 (m, 2H), 1.86-2.03 (m, 2H), 1.51-1.78 (m, 2H). MS obsd. (ESI⁺) [(M+H)⁺]: 496.1. |
| | | **CYC:** tert-butyl 4-methylsulfonyloxypi peridine-1-carboxylate | |
| | | **TAIL:** ethyl 2-chloro-2-oxo-acetate | |
| | 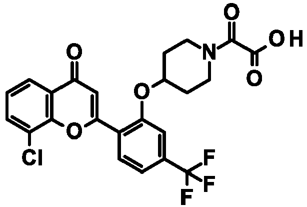 | | |
| **130** | **2-oxo-2-[ (3R)-3-[2-(8-chloro-4-oxo-chromen-2-yl)-5-(trifluoromethyl)phenoxy]py rrolidin-1-yl]acetic acid** | **CORE: Int-1** | ¹H NMR (DMSO-*d*₆, 400MHz) δ ppm 8.07-8.15 (m, 1H), 7.97-8.05 (m, 2H), 7.65-7.72 (m, 1H), 7.57-7.64 (m, 1H), 7.47-7.56 (m, 1H), 6.88-7.00 (m, 1H), 5.40-5.55 (m, 1H), 3.69-3.84 (m, 2H), 3.56-3.68 (m, 2H), 2.12-2.39 (m, 2H). MS obsd. (ESI⁺) [(M+H)⁺]: 482.1. |
| | | **CYC:** tert-butyl (R)-3-((methylsulfonyl)ox y)pyrrolidine-1-carboxylate | |
| | 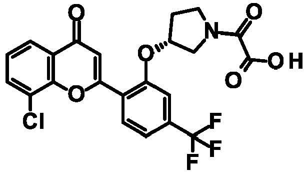 | **TAIL:** ethyl 2-chloro-2-oxo-acetate | |
| **131** | **3-[4-[5-bromo-2-(8-chloro-4-oxo-chromen-2-yl)phenoxy]-1-piperidyl]cyclobutanecarbox ylic acid** | **CORE: Int-4** | ¹H NMR (DMSO-*d*₆, 400MHz) δ ppm 11.57-12.62 (m, 1H), 7.99-8.13 (m, 2H), 7.79-7.93 (m, 1H), 7.41-7.74 (m, 3H), 6.90-7.02 (m, 1H), 4.78-5.13 (m, 1H), 3.43-4.02 (m, 3H), 2.74-3.12 (m, 3H), 2.12-2.41 (m, 4H), 1.92-2.11 (m, 1H), 1.66-1.91 (m, 1H), 1.00-1.51 (m, 2H). MS obsd. (ESI⁺) [(M+H)⁺]: 532.0. |
| | | **CYC:** tert-butyl 4-methylsulfonyloxypi peridine-1-carboxylate | |
| | 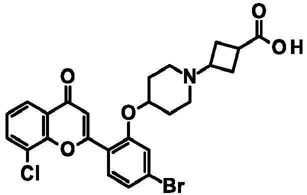 | **TAIL:** methyl 3-chlorocyclobutaneca rboxylate | |
| **132** | **2-[(3S)-3-[2-(8-chloro-4-oxo-chromen-2-yl)-5-methyl-phenoxy]pyrrolidin-1-yl]-2-oxo-acetic acid** | **CORE: Int-2** | ¹H NMR (DMSO-*d*₆, 400MHz) δ ppm 7.94-8.02 (m, 2H), 7.79-7.89 (m, 1H), 7.44-7.55 (m, 1H), 7.15-7.23 (m, 1H), 6.97-7.08 (m, 1H), 6.86-6.94 (m, 1H), 5.22-5.36 (m, 1H), 3.54-3.98 (m, 4H), 2.44 (s, 3H), 2.13-2.36 (m, 2H). MS obsd. (ESI⁺) [(M+H)⁺]: 428.1. |
| | | **CYC:** tert-butyl (S)-3-((methylsulfonyl)oxy)pyrrolidine-1-carboxylate **TAIL:** ethyl 2-chloro-2-oxo-acetate | |
| | 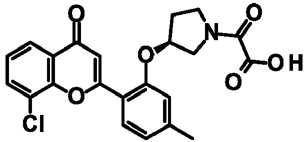 | | |
| | | | |
| **133** | **2-[[3-[2-(8-chloro-4-oxo-chromen-2-yl)-5-(trifluoromethyl)phenoxy]cyc lobutyl]amino]-2-oxo-acetic acid** | **CORE: Int-1** | ¹H NMR (DMSO-*d*₆, 400MHz) δ ppm 7.41 (d, *J* =8.2 Hz, 1H), 7.28 (dd, *J* =8.1, 1.6 Hz, 1H), 7.06 (dd, *J* =7.8, 1.5 Hz, 1H), 6.89 (s, 1H), 6.64 (t, *J* =7.9 Hz, 2H), 6.52 (s, 1H), 6.40 (s, 1H), 3.94 (m, 1H), 3.36 (br t, *J* =7.9 Hz, 1H), 2.25 (m, 2H), 1.49-1.66 (m, 2H). MS obsd. (ESI⁺) [(M+H)⁺]: 482.0. |
| | | **CYC:** [3-(tert-butoxycarbonylamin o)cyclobutyl] methane sulfo nate | |
| | 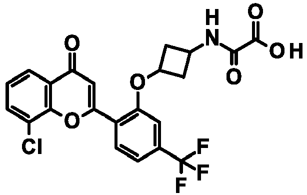 | **TAIL:** ethyl 2-chloro-2-oxo-acetate | |

### Example 134: 2-[3-[2-(8-chloro-4-oxo-chromen-2-yl)-5-(trifluoromethyl)phenoxy]propylmethyl-amino]acetic acid

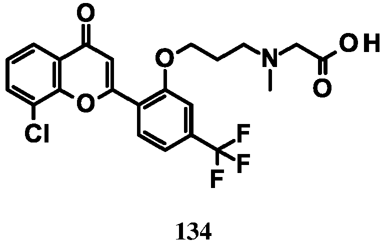

To a solution of 2-[3-[2-(8-chloro-4-oxo-chromen-2-yl)-5-(trifluoromethyl)phenoxy]propylamino]acetic acid (200 mg, 439 µmol, Example **125)** in THF (9 mL) was added formaldehyde (2 mL, 26.9 mmol). The reaction mixture was stirred for 30 minutes and then added sodium cyanoborohydride (138 mg, 2.19 mmol). After stirring for 2 hours, the reaction mixture was diluted with water (30 mL), and the aqueous layer was extracted with EtOAc (10 mL) three times. The combined organic layer was washed with brine, dried over anhydrous Na₂SO₄, filtered and concentrated *in vacuo* to give the crude product, which was purified by prep-HPLC to afford 2-[3-[2-(8-chloro-4-oxo-chromen-2-yl)-5-(trifluoromethyl)phenoxy]propyl-methyl-amino]acetic acid (66 mg, yield: 30.7%). MS obsd. (ESI⁺) [(M+H)⁺]: 470.4.

**Example 134:** ¹H NMR (DMSO-*d*₆, 400MHz) δ ppm 8.12 (d, *J* =7.7 Hz, 1H), 8.04 (dd, *J* =7.9, 1.2 Hz, 1H), 7.38-7.69 (m, 4H), 7.06 (s, 1H), 4.35 (t, *J* =6.1 Hz, 2H), 3.97 (s, 2H), 3.13-3.25 (m, 2H), 2.79 (s, 3H), 2.15-2.29 (m, 2H), 1.06-1.27 (m, 3H).

### Example 135: 2-[2-[2-(8-chloro-4-oxo-chromen-2-yl)-5-methyl-phenoxy]ethyl-cyclopropyl-amino]-2-oxo-acetic acid

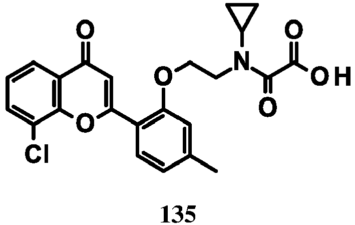

### Step 1: Preparation of 2-[2-(2-bromoethoxy)-4-methyl-phenyl]-8-chloro-chromen-4-one

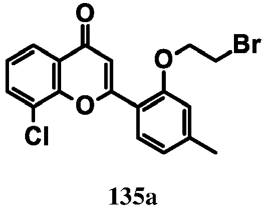

To a solution of 8-chloro-2-(2-hydroxy-4-methyl-phenyl)chromen-4-one (500 mg, 1.74 mmol) and 1,2-dibromoethane (750 mg, 3.99 mmol) in DMF (18 mL) was added K₂CO₃ (1.21 g, 8.72 mmol). The reaction mixture was stirred at 80 °C overnight. After the reaction was completed, the reaction mixture was partitioned between EtOAc (10 mL) and water (30 mL). The organic layer was separated out and the aqueous phase was extracted with EtOAc (30 mL) twice. The combined organic layer was concentrated *in vacuo* and the residue was purified by ISCO (eluting with EtOAc: PE = 0 to 30%) to give 2-[2-(2-bromoethoxy)-4-methyl-phenyl]-8-chloro-chromen-4-one (416 mg, yield: 60.6%) as a yellow solid. MS obsd. (ESI⁺) [(M+H)⁺]: 394.0.

### Step 2: Preparation of 8-chloro-2-[2-[2-(cyclopropylamino)ethoxy]-4-methylphenyl]chromen-4-one

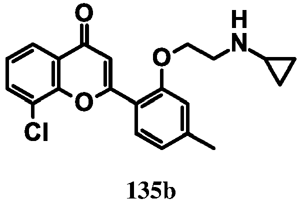

To a solution of 2-[2-(2-bromoethoxy)-4-methyl-phenyl]-8-chloro-chromen-4-one (400 mg, 1.02 mmol) in DMF (10 mL) was added cyclopropanamine (174 mg, 3.05 mmol) and potassium carbonate (702 mg, 5.08 mmol). The reaction was stirred at room temperature overnight. Then, the reaction mixture was quenched by adding water (50 mL), and extracted with EtOAc (30 mL) three times. The combined organic layer was washed with brine, dried over anhydrous Na₂SO₄, filtered and concentrated *in vacuo* to give 8-chloro-2-[2-[2-(cyclopropylamino)ethoxy]-4-methyl-phenyl]chromen-4-one (376 mg, yield: 100%). MS obsd. (ESI⁺) [(M+H)⁺]: 370.0.

### Step 3: Preparation of 2-[2-[2-(8-chloro-4-oxo-chromen-2-yl)-5-methyl-phenoxy]ethyl-cyclopropyl-amino]-2-oxo-acetic acid

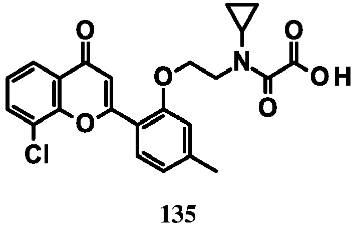

To a solution of 8-chloro-2-[2-[2-(cyclopropylamino)ethoxy]-4-methyl-phenyl]chromen-4-one (0.175 g, 473 µmol) in DCM (5 mL) cooled in the ice-water bath was added DIPEA (100 µl, 573 µmol). Then, oxalyl chloride (0.5 mL, 5.71 mmol) was slowly injected into the reaction mixture. The reaction was allowed to warm to room temperature and kept stirring overnight. After the starting material was consumed, the reaction mixture was partitioned between EtOAc (10 mL) and water (20 mL). The aqueous layer was extracted with EtOAc (20 mL) twice, and the combined organic layer was washed with brine, dried over Na₂SO₄, filtered and concentrated *in vacuo* to give the crude product, which was purified by prep-HPLC to give 2-[2-[2-(8-chloro-4-oxo-chromen-2-yl)-5-methyl-phenoxy]ethyl-cyclopropyl-amino]-2-oxo-acetic acid (4.3 mg, yield: 1.75%). MS obsd. (ESI⁺) [(M+H)⁺]: 442.1.

**Example 135:** ¹H NMR (METHANOL-*d*₆, 400MHz) δ ppm 9.10 (s, 1H), 7.76-8.18 (m, 4H), 7.46 (t, *J* =7.9 Hz, 2H), 7.28 (s, 1H), 4.69-4.80 (m, 2H), 4.45-4.56 (m, 2H), 2.46 (s, 3H), 1.26-1.40 (m, 1H), 0.73-0.97 (m, 4H).

### Example 136: 8-chloro-2-[2-[2-[ethylsulfamoyl(methyl)amino]ethoxy]-4-methyl-phenyl]-4-oxo-chromene

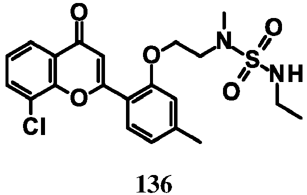

### Step 1: Preparation of tert-butyl N-[2-[2-(8-chloro-4-oxo-chromen-2-yl)-5-methyl-phenoxy] ethyl] carbamate

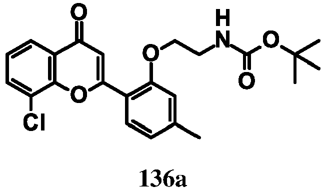

To a solution of 8-chloro-2-(2-hydroxy-4-methyl-phenyl)chromen-4-one (500 mg, 1.74 mmol) and tert-butyl (3-bromopropyl)carbamate (415 mg, 1.74 mmol) in DMF (18 mL) was added K₂CO₃ (1.21 g, 8.72 mmol) and the mixture was stirred at 50 °C overnight. After the reaction was completed, the reaction mixture was partitioned between EtOAc (20 mL) and water (30 mL). The organic layer was separated out and the aqueous phase was extracted with EtOAc (30 mL) twice. The combined organic layer was concentrated *in vacuo* and the residue was purified by ISCO (eluting with EtOAc : PE = 0 to 30%) to give tert-butyl N-[3-[2-(8-chloro-4-oxo-chromen-2-yl)-5-methyl-phenoxy]propyl]carbamate (500 mg, yield: 66.7%) as a yellow solid. MS obsd. (ESI⁺) [(M+H)⁺]: 430.1.

### Step 2: Preparation of tert-butyl N-[2-[2-(8-chloro-4-oxo-chromen-2-yl)-5-methyl-phenoxy]ethyl]-N-methyl-carbamate

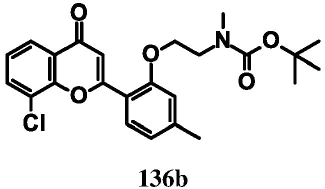

To a solution of N-[2-[2-(8-chloro-4-oxo-chromen-2-yl)-5-methyl-phenoxy]ethyl]carbamate (200 mg, 465 µmol) in DMF (5 mL) was added Mel (116 µl, 1.86 mmol). The reaction was cooled in the ice-water bath, and followed by adding NaH (46.5 mg, 1.16 mmol). The reaction was allowed to warm to room temperature and stirred for 2 hours. Then, the reaction was quenched by adding water (20 mL), and the aqueous layer was extracted with EtOAc (10 mL) three times. The organic layer was combined, washed with brine, dried over anhydrous Na₂SO₄, filtered and concentrated *in vacuo* to give N-[2-[2-(8-chloro-4-oxo-chromen-2-yl)-5-methyl-phenoxy]ethyl]-N-methyl-carbamate (200 mg, yield: 96.8%).

### Step 3: Preparation of 8-chloro-2-[4-methyl-2-[2-(methylamino)ethoxy]phenyl]chromen-4-one

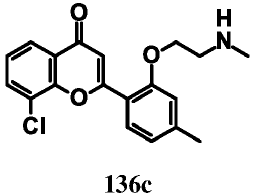

To a solution of N-[2-[2-(8-chloro-4-oxo-chromen-2-yl)-5-methyl-phenoxy]ethyl]-N-methyl-carbamate (300 mg, 676 µmol) in DCM (2 mL) was added TFA (2 mL, 26 mmol). The reaction was stirred at room temperature and went complete after 2 hours. Then, the reaction mixture was concentrated *in vacuo* and azeotroped with toluene twice to give 8-chloro-2-[4-methyl-2-[2-(methylamino)ethoxy]phenyl]chromen-4-one ( 230 mg, yield: 99%) as a yellow oil. MS obsd. (ESI⁺) [(M+H)⁺]: 344.1.

### Step 4: Preparation of 8-chloro-2-[2-[2-[ethylsulfamoyl(methyl)amino]ethoxy]-4-methylphenyl] -4-oxo-chromene

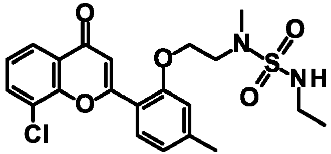

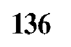

A solution of 8-chloro-2-[4-methyl-2-[2-(methylamino)ethoxy]phenyl]chromen-4-one (120 mg, 349 µmol), DIPEA (61 µl, 349 µmol) and ethylsulfamoyl chloride (50.1 mg, 349 µmol) in DCM (4 mL) was stirred at room temperature overnight. After completion, the reaction was quenched by adding 1N HCl. The residue was then partitioned between EtOAc (10 mL) and water (20 mL). The organic layer was combined, washed with brine, dried over anhydrous Na₂SO₄, filtered and concentrated *in vacuo* to give the crude product, which was purified by prep-HPLC to afford 8-chloro-2-[2-[2-[ethylsulfamoyl(methyl)amino]ethoxy]-4-methyl-phenyl]-4-oxo-chromene (3.3 mg, yield: 2%) as a white solid. MS obsd. (ESI⁺) [(M+H)⁺]: 451.7.

**Example 136:** ¹H NMR (DMSO-*d*₆, 400MHz) δ ppm 8.00 (d, *J* =7.8 Hz, 2H), 7.85 (d, *J* =7.9 Hz, 1H), 7.49 (t, *J* =7.9 Hz, 1H), 7.10-7.23 (m, 2H), 7.05 (s, 2H), 4.31 (t, *J* =5.6 Hz, 2H), 3.50 (t, *J* =5.7 Hz, 2H), 2.84 (dd, *J* =7.2, 5.7 Hz, 2H), 2.77 (s, 3H), 2.41 (s, 3H), 0.98 (t, *J* =7.2 Hz, 3H).

### Example 137: ethyl 2-[2-[2-(8-chloro-4-oxo-chromen-2-yl)-5-methyl-phenoxy]ethyl-methyl-amino]-2-oxo-acetate

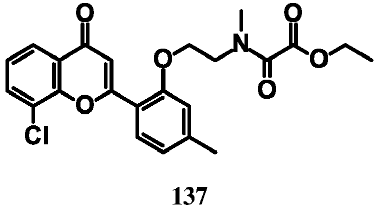

**137** was prepared in analogy to the procedure described for the preparation of compound **136** by using ethyl 2-chloro-2-oxo-acetate as the starting material instead of ethylsulfamoyl chloride in **Step 4.** MS obsd. (ESI⁺) [(M+H)⁺]: 444.0.

**Example 137:** ¹H NMR (DMSO-*d*₆, 400MHz) δ ppm 7.99 (d, *J* =7.8 Hz, 1H), 7.81 (dd, *J* = 10.0, 8.1 Hz, 1H), 7.49 (t, *J* =7.9 Hz, 1H), 7.16 (d, *J* =9.2 Hz, 1H), 7.03 (d, *J* =7.8 Hz, 1H), 6.84-7.00 (m, 1H), 4.34 (br d, *J* =4.9 Hz, 2H), 4.04-4.17 (m, 2H), 3.68-3.83 (m, 2H), 3.17 (d, *J* =4.9 Hz, 3H), 2.40 (s, 3H), 1.06-1.27 (m, 3H).

### Example 138: ethyl 2-[3-[2-(8-chloro-4-oxo-chromen-2-yl)-5-(trifluoromethyl)phenoxy]propyl-ethylsulfonyl-amino]-2-oxo-acetate

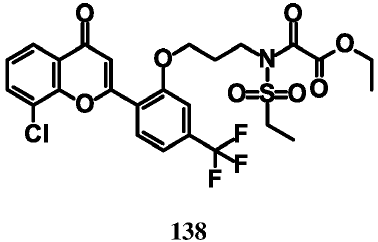

### Step 1: Preparation of N-[3-[2-(8-chloro-4-oxo-chromen-2-yl)-5-(trifluoromethyl)phenoxy]propyl]ethanesulfonamide

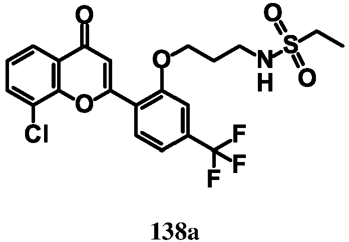

To a solution of 2-[2-(3-bromopropoxy)-4-(trifluoromethyl)phenyl]-8-chloro-chromen-4-one (200 mg, 435 µmol, **062a**) in DCM was added TEA (303 µl, 2.18 mmol) and ethanesulfonamide (238 mg, 2.18 mmol) dropwise. The reaction was stirred at room temperature overnight. After the reaction was complete, the reaction mixture was quenched by adding 1N HCl, and then partitioned between DCM (10 mL) and water (20 mL). The aqueous layer was extracted with EtOAc (10 mL) twice. The combined organic layer was washed with brine, dried over anhydrous Na₂SO₄, filtered and concentrated *in vacuo* to give N-[3-[2-(8-chloro-4-oxo-chromen-2-yl)-5-(trifluoromethyl)phenoxy]propyl]ethanesulfonamide (100 mg, yield: 46.9%) as a yellow solid. MS obsd. (ESI⁺) [(M+H)⁺]: 490.5.

### Step 2: Preparation of 2-[3-[2-(8-chloro-4-oxo-chromen-2-yl)-5-(trifluoromethyl)phenoxy]propyl-ethylsulfonyl-amino]-2-oxo-acetate

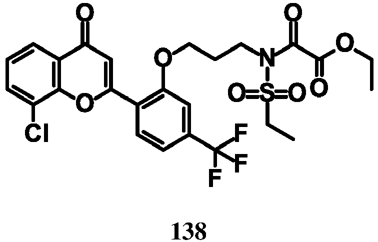

Compound N-[3-[2-(8-chloro-4-oxo-chromen-2-yl)-5-(trifluoromethyl)phenoxy]propyl]ethanesulfonamide (100 mg, 204 µmol) was dissolved in the DMF (2 mL), followed by adding ethyl 2-chloro-2-oxo-acetate (36 µL, 320 µmol) and DIPEA (56 µL, 320 µmol). The reaction was stirred at room temperature. After the reaction was completed, the reaction mixture was partitioned between ethyl acetate (10 mL) and water (20 mL). The combined organic layer was washed with brine, dried over anhydrous sodium sulfate, filtered and concentrated *in vacuo* to give 2-[3-[2-(8-chloro-4-oxo-chromen-2-yl)-5-(trifluoromethyl)phenoxy]propyl-ethylsulfonyl-amino]-2-oxo-acetate (100 mg, yield: 81.5%). MS obsd. (ESI⁺) [(M+H)⁺]: 590.0.

**Example 138:** ¹H NMR (DMSO-*d*₆, 400MHz) δ ppm 7.94-8.17 (m, 3H), 7.44-7.64 (m, 3H), 7.02 (s, 1H), 4.17-4.49 (m, 4H), 3.77-3.97 (m, 2H), 3.59 (d, *J* =7.3 Hz, 2H), 2.03-2.21 (m, 2H), 1.11-1.40 (m, 6H).

### Example 139: 3-[3-[2-(8-chloro-4-oxo-chromen-2-yl)-5-(trifluoromethyl)phenoxy]propyl-cyclopropylsulfonyl-amino]cyclobutanecarboxylic acid

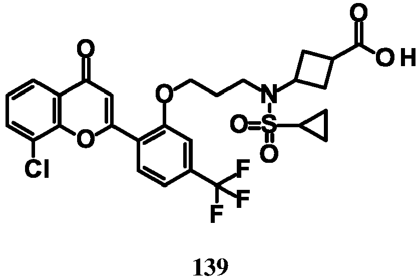

### Step 1: Preparation of methyl 3-[3-[2-(8-chloro-4-oxo-chromen-2-yl)-5-(trifluoromethyl)phenoxy]propylamino]cyclobutanecarboxylate

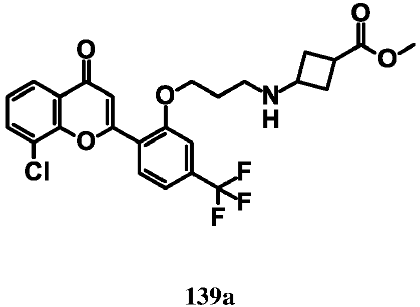

To a solution of 2-[2-(3-bromopropoxy)-4-(trifluoromethyl)phenyl]-8-chloro-chromen-4-one (200 mg, 433 µmol) and methyl 3-aminocyclobutane-1-carboxylate (280 mg, 2.17 mmol, as the "**TAIL1**" in Table 8) in DMF (5 mL) was added NaHCO₃ (364 mg, 4.33 mmol). The mixture was stirred at 50 °C overnight. After the reaction was completed, the reaction mixture was diluted with EtOAc and partitioned between EtOAc (30 mL) and water (30 mL). The organic layer was separated out and the aqueous phase was extracted with EtOAc (30 mL) twice. The combined organic layer was concentrated *in vacuo* to give methyl 3-[3-[2-(8-chloro-4-oxo-chromen-2-yl)-5-(trifluoromethyl)phenoxy]propylamino]cyclobutanecarboxylate (100 mg, yield: 45.3%) as a yellow solid.

### Preparation of 3-[3-[2-(8-chloro-4-oxo-chromen-2-yl)-5-(trifluoromethyl)phenoxy]propylamino]cyclobutanecarboxylic acid

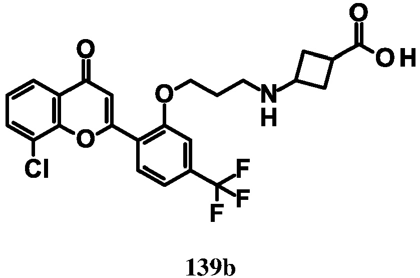

To the solution of methyl 3-[3-[2-(8-chloro-4-oxo-chromen-2-yl)-5-(trifluoromethyl)phenoxy]propylamino]cyclobutanecarboxylate (100 mg, 0.196 mmol) in mixed solvent of MeOH (5 mL), THF (5 mL) and water (2 mL) was added LiOH (23.5 mg, 0.981 mmol). The reaction mixture was stirred at room temperature till completion. Then, the reaction mixture was neutralized by acetic acid and then concentrated *in vacuo.* The crude material was purified by prep-HPLC to give 3-[3-[2-(8-chloro-4-oxo-chromen-2-yl)-5-(trifluoromethyl)phenoxy]propylamino]cyclobutanecarboxylic acid (65 mg, yield: 63.5%) as a white solid. (ESI⁺) [(M+H)]⁺: 495.9.

### Step 3: Preparation of 3-[3-[2-(8-chloro-4-oxo-chromen-2-yl)-5-(trifluoromethyl)phenoxy]propyl-cyclopropylsulfonyl-amino]cyclobutanecarboxylic acid

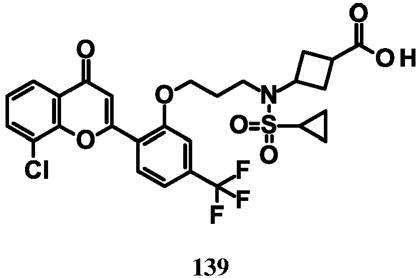

To a solution of 3-[3-[2-(8-chloro-4-oxo-chromen-2-yl)-5-(trifluoromethyl)phenoxy]propylamino]cyclobutanecarboxylic acid (100 mg, 202 µmol, as the **"TAIL2"** in Table 8) and TEA (84.3 µl, 605 µmol) in DCM (15 mL) was added cyclopropanesulfonyl chloride (85 mg, 605 µmol). The reaction mixture was stirred at room temperature. After the starting material was consumed, the reaction mixture was adjusted to pH = 4 with HOAc, and then concentrated *in vacuo* to give the crude product. The residue was purifed by prep-HPLC to give 3-[3-[2-(8-chloro-4-oxo-chromen-2-yl)-5-(trifluoromethyl)phenoxy]propyl-cyclopropylsulfonyl-amino]cyclobutanecarboxylic acid (3.6 mg, yield: 2.83%). MS obsd. (ESI⁺) [(M+H)⁺]: 600.5.

**Example 139:** 11.79-12.26 (m, 1H), 7.94-8.16 (m, 3H), 7.41-7.66 (m, 3H), 7.07 (s, 1H), 4.26-4.35 (m, 2H), 4.00-4.11 (m, 2H), 2.61-2.73 (m, 3H), 2.13-2.33 (m, 4H), 1.91-2.12 (m, 2H), 0.80-0.95 (m, 4H).

The following compounds **140** to **145** were prepared in analogy to the procedure described for the preparation of Example **139,** replacing methyl 3-aminocyclobutane-1-carboxylate with "**TAIL1**" in **Step 1** and replacing cyclopropanesulfonyl chloride with **"TAIL2"** in **Step 3,** by the reagent indicated in Table 8.

**Table 8: Compounds synthesis and characterization**

| Example No. | Compounds Name and Structure | **TAIL1** and **TAIL2** | ¹H NMR and (ESI⁺) |
|---|---|---|---|
| **140** | **2-**[**carboxymethyl-[3-[2-(8-chloro-4-oxo-chromen-2-yl)-5-(trifluoromethyl)phenoxy]pr opyl]amino]acetic acid** | **TAIL1:** methyl 2-bromoacetate | ¹H NMR (METHANOL-*d*₄, 400MHz) δ ppm 8.26 (d, *J* = 7.8 Hz, 1H), 8.14 (dd, *J* = 8.0, 1.5 Hz, 1H), 7.94 (dd, *J* = 7.8, 1.5 Hz, 1H), 7.42-7.61 (m, 3H), 7.27 (s, 1H), 4.38 (t, *J* = 5.7 Hz, 2H), 4.28 (s, 4H), 3.57-3.72 (m, 2H), 2.44 (br dd, *J* = 10.9, 5.5 Hz, 2H). MS obsd. (ESI⁺) [(M+H)⁺]: 514.1. |
| | | **TAIL2:** methyl 2-bromoacetate | |
| | 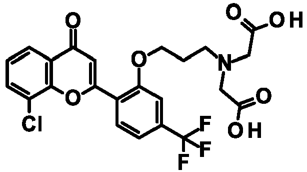 | | |
| **141** | **cis-3-[benzenesulfonyl-[3-[2-(8-chloro-4-oxo-chromen-2-yl)-5-(trifluoromethyl)phenoxy]pr opyl]amino]cyclobutanecarb oxylic acid** | **TAIL1:** methyl 3-chlorocyclobut anecarboxylate | ¹H NMR (DMSO-*d*₆, 400MHz) δ ppm 11.83-12.21 (m, 1H), 8.11 (d, *J* = 8.1 Hz, 1H), 8.02 (ddd, *J* = 11.3, 7.9, 1.5 Hz, 2H), 7.69-7.77 (m, 2H), 7.60-7.69 (m, 1H), 7.42-7.60 (m, 5H), 7.08 (s, 1H), 4.32 (t, *J* = 5.8 Hz, 2H), 3.89-4.05 (m, 1H), 3.14-3.26 (m, 2H), 2.54-2.65 (m, 1H), 1.84-2.35 (m, 4H), 1.14-1.57 (m, 2H). MS obsd. (ESI⁺) [(M+H)⁺]: 636.2. |
| | 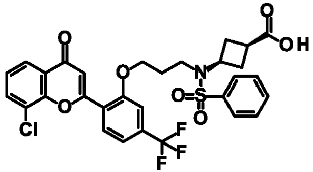 | **TAIL2:**benzenesulfonyl chloride | |
| **142** | **trans-3-[benzenesulfonyl-[3-[2-(8-chloro-4-oxo-chromen-2-yl)-5-(trifluoromethyl)phenoxy]pr opyl]amino]cyclobutanecarb oxylic acid** | **TAIL1:** cis-methyl 3-chlorocyclobut anecarboxylate | ¹H NMR (DMSO-*d*₆, 400MHz) δ ppm 12.05-12.34 (m, 1H), 8.10 (d, *J* = 8.1 Hz, 1H), 7.96-8.07 (m, 2H), 7.59-7.73 (m, 3H), 7.46-7.58 (m, 5H), 6.98-7.08 (m, 1H), 4.23-4.42 (m, 3H), 3.20-3.28 (m, 2H), 2.57-2.66 (m, 1H), 1.95-2.24 (m, 4H), 1.19-1.53 (m, 2H). MS obsd. (ESI⁺) [(M+H)⁺]: 636.2. |
| | 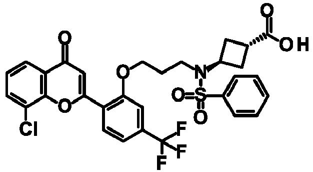 | **TAIL2:**benzenesulfonyl chloride | |
| **143** | **2-[benzenesulfonyl-[3-[2-(8-chloro-4-oxo-chromen-2-yl)-5-(trifluoromethyl)phenoxy]pr opyl]amino]acetic acid** | **TAIL1:** trans- methyl 2-bromoacetate | ¹H NMR (DMSO-*d*₆, 400MHz) δ ppm 8.01-8.13 (m, 3H), 7.70 (d, *J* = 7.7 Hz, 2H), 7.54 (q, *J* =7.9 Hz, 3H), 7.41-7.47 (m, 3H), 7.06 (s, 1H), 4.24 (t, *J* =5.9 Hz, 2H), 3.98 (s, 2H), 3.25-3.33 (m, 2H), 2.00 (m, 2H). MS obsd. (ESI⁺) [(M+H)⁺]: 596.3. |
| | | **TAIL2:** benzenesulfon yl chloride | |
| | 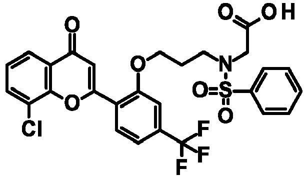 | | |
| **144** | **2-[acetyl-[3-[2-(8-chloro-4-oxo-chromen-2-yl)-5-(trifluoromethyl)phenoxy]pr opyl]amino]acetic acid** | **TAIL1:** methyl 2-bromoacetate | ¹H NMR (DMSO-*d*₆, 400MHz) δ ppm 8.07-8.22 (m, 1H), 7.95-8.07 (m, 2H), 7.39-7.67 (m, 3H), 6.97-7.18 (m, 1H), 4.32 (m, 2H), 4.24 (m, 2H), 4.09 (s, 2H), 3.90 (s, 3H), 1.94-2.13 (m, 2H). MS obsd. (ESI⁺) [(M+H)⁺]: 498.4. |
| | 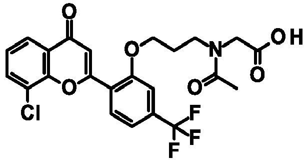 | **TAIL2:** acetyl chloride | |
| **145** | **2-[3-[2-(8-chloro-4-oxo-chromen-2-yl)-5-(trifluoromethyl)phenoxy]pr opyl-cyclopropylsulfonyl-amino]acetic acid** | **TAIL1:** methyl 2-bromoacetate | ¹H NMR (DMSO-*d*₆, 400MHz) δ ppm 8.11 (d, *J* = 8.1 Hz, 1H), 8.03 (d, *J* = 7.8 Hz, 2H), 7.43-7.69 (m, 3H), 7.06 (s, 1H), 4.33 (t, *J* = 5.9 Hz, 2H), 3.99 (s, 2H), 3.38-3.50 (m, 2H), 2.60-2.73 (m, 1H), 2.07 (br t, *J* = 6.1 Hz, 2H), 0.73-0.94 (m, 4H). MS obsd. (ESI⁺) [(M+H)⁺]: 560.1. |
| | 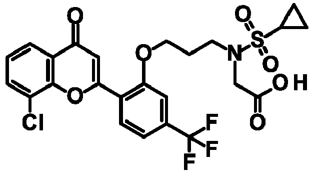 | **TAIL2:**cyclopropanesulfonyl chloride | |

### Example 146: N'-[2-[2-(8-chloro-4-oxo-chromen-2-yl)-5-(trifluoromethyl)phenoxy]ethyl]oxamide

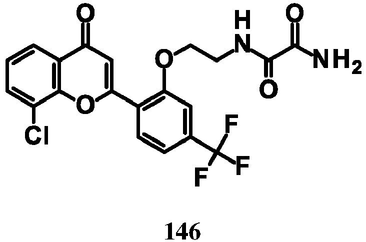

To a flask charged with 2-[2-[2-(8-chloro-4-oxo-chromen-2-yl)-5-(trifluoromethyl)phenoxy]ethylamino]-2-oxo-acetic acid (200 mg, 0.440 mmol, Example **119)** was added NH₃/MeOH (4.0 mL, 0.440 mmol) and then the reaction was stirred at 55 °C for 1 hour. After the starting material was consumed, the reaction mixture was concentrated *in vacuo* to give the crude compound, which was recrystallized in MeOH to afford N'-[2-[2-(8-chloro-4-oxo-chromen-2-yl)-5-(trifluoromethyl)phenoxy]ethyl]oxamide (116 mg, yield: 57.4%) as a white solid. MS obsd. [(M+H)⁺] (ESI⁺): 455.0.

**Example 146:** ¹H NMR (400 MHz, DMSO-d₆): δ ppm 8.89 (br t, *J* = 5.8 Hz, 1H), 8.13 (d, *J =* 8.1 Hz, 1H), 7.96-8.05 (m, 3H), 7.77 (br s, 1H), 7.47-7.64 (m, 3H), 7.04 (s, 1H), 4.40 (t, *J* = 5.9 Hz, 2H), 3.58 (q, *J* = 6.0 Hz, 2H).

### Example 147: N'-[2-[2-(8-chloro-4-oxo-chromen-2-yl)-5-methoxy-phenoxy]ethyl]oxamide

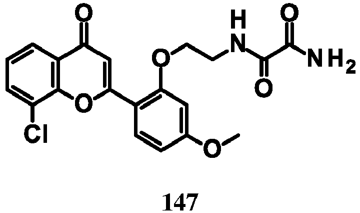

The Example **147** was prepared in analogy to the procedure described for the preparation of **146,** replacing 2-[2-[2-(8-chloro-4-oxo-chromen-2-yl)-5-(trifluoromethyl)phenoxy]ethylamino]-2-oxo-acetic acid with 2-[2-[2-(8-chloro-4-oxo-chromen-2-yl)-5-methoxy-phenoxy]ethylamino]-2-oxo-acetic acid (**111**) as starting material. MS obsd. (ESI⁺) [(M+H)⁺]: 417.0.

**Example 147:** ¹H NMR (400 MHz, DMSO-d6): δ ppm 8.89 (t, *J* = 5.4 Hz, 1H), 8.07 (s, 1H), 7.96 (dd, *J* = 7.8, 5.1 Hz, 3H), 7.79 (s, 1H), 7.47 (t, *J* = 7.8 Hz, 1H), 6.98 (s, 1H), 6.87-6.75 (m, 2H), 4.29 (t, *J* = 5.5 Hz, 2H), 3.88 (s, 3H), 3.59 (dd, *J* = 11.3, 5.4 Hz, 2H).

### Example 148: N-[2-[2-(8-chloro-4-oxo-chromen-2-yl)-5-(trifluoromethyl)phenoxy]ethyl]-N'-cyclopropyl-oxamide

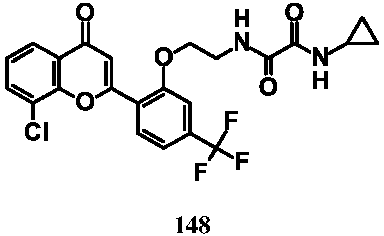

To a solution of ethyl 2-[2-[2-(8-chloro-4-oxo-chromen-2-yl)-5-(trifluoromethyl)phenoxy]ethylamino]-2-oxo-acetate (100 mg, 0.21 mmol, Example **101**) in DCM (20 mL) was added cyclopropylamine (0.02 mL, 0.320 mmol) and titanium tetrachloride (80.75 mg, 0.430 mmol) at 0 °C under N₂. Then, the reaction was heated to 110 °C and stirred for 2 hours. After the starting material was consumed, the reaction mixture was cooled to room temperature and then poured into ice-water (20 mL). The aqueous layer was extracted with EtOAc (50 mL) three times. The combined organic layer was washed with brine, dried over anhydrous Na₂SO₄, filtered and concentrated *in vacuo* to give the crude residue, which was purified by prep-HPLC to afford N-[2-[2-(8-chloro-4-oxo-chromen-2-yl)-5-(trifluoromethyl)phenoxy]ethyl]-N'-cyclopropyl-oxamide (41.3 mg, yield: 39.1%) as a white solid. MS obsd. (ESI⁺)[(M+H)⁺]: 495.1.

**Example 148:** ¹H NMR (400 MHz, DMSO-d6): δ ppm 8.90 (t, *J* = 5.9 Hz, 1H), 8.66 (d, *J* = 5.3 Hz, 1H), 8.12 (d, *J* = 7.9 Hz, 1H), 8.02 (dq, *J* = 7.9, 1.4 Hz, 2H), 7.62 (s, 1H), 7.46-7.60 (m, 2H), 6.95-7.05 (m, 1H), 4.40 (t, *J* = 5.9 Hz, 2H), 3.58 (q, *J* = 6.0 Hz, 2H), 2.64-2.73 (m, 1H), 0.54-0.64 (m, 4H).

### Example 149: N-[3-[2-(8-chloro-4-oxo-chromen-2-yl)-5-(trifluoromethyl)phenoxy]propyl]-N'-cyclopropylsulfonyl-oxamide

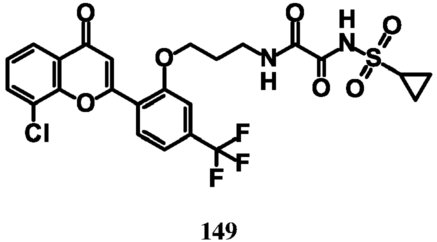

The Example **149** was prepared in analogy to the procedure described for the preparation of **148,** replacing ethyl 2-[2-[2-(8-chloro-4-oxo-chromen-2-yl)-5-(trifluoromethyl)phenoxy]ethylamino]-2-oxo-acetate (Example **101**) with ethyl 2-[3-[2-(8-chloro-4-oxo-chromen-2-yl)-5-(trifluoromethyl)phenoxy]propylamino]-2-oxo-acetate (**100**) and cyclopropanesulfonamide with cyclopropylamine as starting material. MS obsd. (ESI⁺) [(M+H)⁺]: 573.1.

**Example 149:** ¹H NMR (400 MHz, DMSO-d₆): δ ppm 12.20 (s, 1H), 9.16 (t, *J* = 5.7 Hz, 1H), 8.12 (d, *J* = 8.0 Hz, 1H), 8.03 (ddd, *J* = 8.0, 3.2, 1.5 Hz, 2H), 7.54 (dt, *J* = 15.8, 8.1 Hz, 3H), 7.12 (s, 1H), 4.28 (t, *J* = 6.1 Hz, 2H), 3.38-3.34 (m, 2H), 3.00- 2.90 (m, 1H), 2.01 (p, *J* = 6.0 Hz, 2H), 1.16-0.98 (m, 4H).

### Example 150: N-[2-[2-(8-chloro-4-oxo-chromen-2-yl)-5-methyl-phenoxy]ethyl]-2-[(3S)-3-hydroxypyrrolidin-1-yl]-2-oxo-acetamide

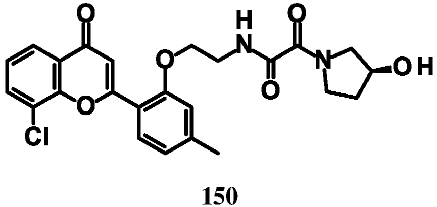

The Example **150** was prepared in analogy to the procedure described for the preparation of **148,** replacing ethyl 2-[2-[2-(8-chloro-4-oxo-chromen-2-yl)-5-(trifluoromethyl)phenoxy]ethylamino]-2-oxo-acetate (Example **101**) with ethyl 2-[2-[2-(8-chloro-4-oxo-chromen-2-yl)-5-methyl-phenoxy]ethylamino]-2-oxo-acetate (Example **109**) and cyclopropanesulfonamide with (3S)-pyrrolidin-3-ol as starting material. MS obsd. (ESI⁺) [(M+H)⁺]: 471.2.

**Example 150:** ¹H NMR (METHANOL-*d*₄, 400MHz) δ ppm 8.80-8.97 (m, 1H), 7.83-8.16 (m, 3H), 7.47 (br t, *J* =7.6 Hz, 1H), 7.26 (s, 1H), 7.09 (s, 1H), 7.03 (br d, *J* =7.6 Hz, 1H), 4.31-4.50 (m, 4H), 3.71-3.93 (m, 4H), 3.44-3.64 (m, 2H), 2.45 (s, 3H), 1.82-2.08 (m, 1H).

### Example 151: (2S)-2-[2-[2-(8-chloro-4-oxo-chromen-2-yl)-5-methyl-phenoxy]ethylcarbamoylamino]propanoic acid

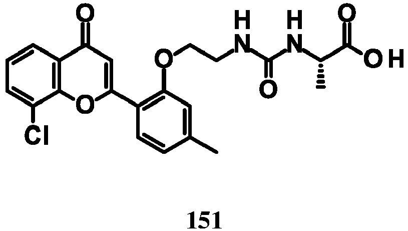

### Step: 1 Preparation of 2-[2-(2-aminoethoxy)-4-methyl-phenyl]-8-chloro-chromen-4-one

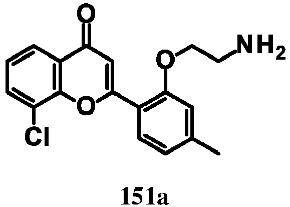

The compounds **151a** was prepared in analogy to the procedure described for the preparation of **095b,** using 8-chloro-2-(2-hydroxy-4-methyl-phenyl)chromen-4-one (as the **"CORE"** in Table 9) and replacing tert-butyl (3-bromopropyl)carbamate with tert-butyl (3-bromoethyl)carbamate as starting material in **Step 1.**

### Step 2: Preparation of methyl (2S)-2-[2-[2-(8-chloro-4-oxo-chromen-2-yl)-5-methyl-phenoxy]ethylcarbamoylamino]propanoate

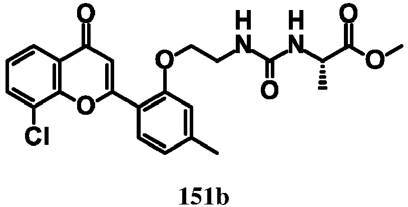

To a solution of 2-[2-(2-aminoethoxy)-4-methyl-phenyl]-8-chloro-chromen-4-one (50 mg, 152 µmol) and methyl (S)-2-isocyanatopropanoate (40 mg, 303 µmol, as the **"TAIL"** in Table 9) in DCM (1.5 mL) was added DIPEA (56 µl, 323 µmol). The reaction was stirred at room temperature for 2 hours. After the reaction was completed, the reaction residue was concentrated *in vacuo* to afford methyl (2S)-2-[2-[2-(8-chloro-4-oxo-chromen-2-yl)-5-methyl-phenoxy]ethylcarbamoylamino]propanoate (69 mg, yield: 99.2%). MS obsd. (ESI⁺) [(M+H)⁺]: 459.2.

### Step 3: Preparation of (2S)-2-[2-[2-(8-chloro-4-oxo-chromen-2-yl)-5-methyl-phenoxy]ethylcarbamoylamino]propanoic acid

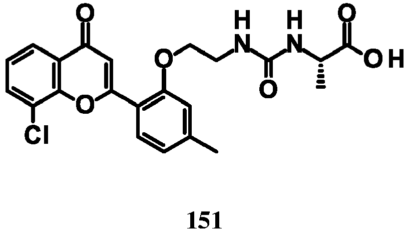

To a solution of methyl (2S)-2-[2-[2-(8-chloro-4-oxo-chromen-2-yl)-5-methyl-phenoxy]ethylcarbamoylamino]propanoate (70 mg, 153 µmol) in THF (1.5 mL) and water (1.5 mL) was added sodium hydroxide (30.5 mg, 763 µmol). After stirring for 2 hours, the reaction mixture was partitioned between EtOAc (10 mL) and water (20 mL). The aqueous layer was extracted with EtOAc (10 mL) and combined organic layer was washed with brine, dried over anhydrous Na₂SO₄, filtered and concentrated *in vacuo* to give a yellow oil, which was purified by prep-HPLC to afford (2S)-2-[2-[2-(8-chloro-4-oxo-chromen-2-yl)-5-methyl-phenoxy]ethylcarbamoylamino]propanoic acid (7.1 mg, 10.3%).

**Example 151:** MS obsd. (ESI⁺) [(M+H)⁺]: 445.3. ¹H NMR (DMSO-*d*₆, 400MHz) δ ppm 8.00 (dd, J=7.9, 2.3 Hz, 2H), 7.89 (d, J=7.9 Hz, 1H), 7.49 (t, J=7.8 Hz, 1H), 7.14 (s, 1H), 7.08 (s, 1H), 7.02 (d, J=8.7 Hz, 1H), 6.26-6.39 (m, 2H), 4.14 (s, 2H), 4.06 (s, 1H), 2.40 (s, 3H), 1.20 (d, J=7.2 Hz, 3H).

The following compounds **152** to **155** were prepared in analogy to the procedure described for the preparation of Example **151,** replacing 8-chloro-2-(2-hydroxy-4-methyl-phenyl)chromen-4-one with **"CORE"** and replacing methyl (S)-2-isocyanato-3-methylbutanoate with **"TAIL"** in **Step 2,** by the reagent indicated in Table 9.

**Table 9: Compounds synthesis and characterization**

| Example No. | Compounds Name and Structure | **CORE** and **TAIL** | ¹H NMR and (ESI⁺) |
|---|---|---|---|
| **152** | **(5S)-3-[2-[2-(8-chloro-4-oxo-chromen-2-yl)-5-methyl-phenoxy]ethyl]-5-isopropyl-imidazolidine-2,4-dione** | **CORE: Int-2** | ¹H NMR (DMSO-*d*₆, 400MHz) δ ppm 8.22 (s, 1H), 7.92-8.04 (m, 2H), 7.80 (d, J=7.9 Hz, 1H), 7.48 (t, J=7.9 Hz, 1H), 7.14 (s, 1H), 7.01 (d, J=7.9 Hz, 1H), 6.87 (s, 1H), 4.35 (s, 2H), 3.90 (dd, J=3.7, 1.1 Hz, 1H), 3.79 (s, 2H), 3.17 (s, 3H), 1.84-1.97 (m, 1H), 0.83 (d, J=7.0 Hz, 3H), 0.64 (d, J=6.7 Hz, 3H). MS obsd. (ESI⁺) [(M+H)⁺]: 455.3. |
| | 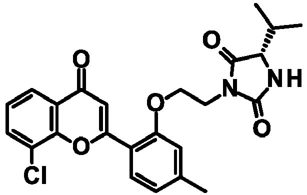 | **TAIL:** methyl(S)-2-isocyanato-3-methylbutanoate | |
| **153** | **(5S)-3-[2-[2-(8-chloro-4-oxo-chromen-2-yl)-5-(trifluoromethyl)phenoxy]eth yl]-5-isopropyl-imidazolidine-2,4-dione** | **CORE: Int-1** | ¹H NMR (DMSO-*d*₆, 400MHz) δ ppm 8.21 (s, 1H), 7.89-8.11 (m, 3H), 7.61 (s, 1H), 7.38-7.59 (m, 2H), 6.90 (s, 1H), 4.45 (t, J=5.5 Hz, 2H), 3.84 (dd, J=3.6, 1.2 Hz, 1H), 3.73-3.83 (m, 2H), 1.76-1.96 (m, 1H), 0.80 (d, J=7.0 Hz, 3H), 0.60 (d, J=6.8 Hz, 3H). MS obsd. (ESI⁺) [(M+H)⁺]: 509.1. |
| | | **TAIL:** methyl (S)-2-isocyanato-3-methylbutanoa te | |
| | 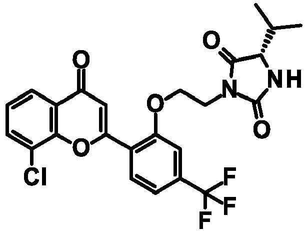 | | |
| **154** | **2-[2-[2-(8-chloro-4-oxo-chromen-2-yl)-5-methyl-phenoxy]ethylcarbamoylami no]acetic acid** | **CORE: Int-2** | ¹H NMR (DMSO-*d*₆, 400MHz) δ ppm 8.00 (m, 2H), 7.88 (m, 1H), 7.42-7.61 (m, 1H), 7.14 (m, 1H), 7.08 (s, 1H), 7.03 (m, 1H), 6.42 (m, 1H), 6.29 (m, 1H), 4.11-4.20 (m, 2H), 3.92 (m, 2H), 3.70 (m, J=5.5 Hz, 2H), 2.39 (s, 3H). MS obsd. (ESI⁺) [(M+H)⁺]: 431.0. |
| | 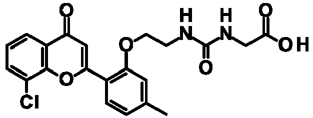 | **TAIL:** ethyl 2-isocyanatoacetate | |
| **155** | **(5S)-3-[2-[2-(8-chloro-4-oxo-chromen-2-yl)-5-(trifluoromethyl)phenoxy]eth yl]-5-methyl-imidazolidine-2,4-dione** | **CORE: Int-1** | ¹H NMR (DMSO-*d*₆, 400MHz) δ ppm 8.06-8.22 (m, 2H), 8.02 (d, J=7.8 Hz, 2H), 7.32-7.66 (m, 3H), 6.92 (s, 1H), 4.47 (t, J=5.6 Hz, 2H), 4.02 (dd, J=7.0, 1.1 Hz, 1H), 3.76 (t, J=5.5 Hz, 2H), 1.11 (d, J=7.0 Hz, 3H). MS obsd. (ESI⁺) [(M+H)⁺]: 481.0. |
| | 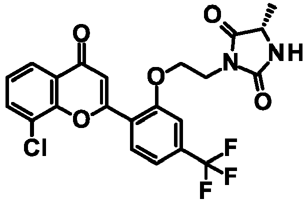 | **TAIL:** methyl(S)-2-isocyanatopropanoate | |

### Example 156: 1-[3-[2-(8-chloro-4-oxo-chromen-2-yl)-5-methyl-phenoxy]propyl]-3-(p-tolylsulfonyl)urea

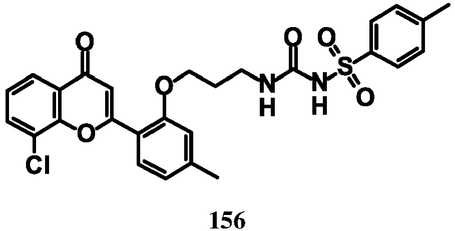

A solution of 2-[2-(3-aminopropoxy)-4-methyl-phenyl]-8-chloro-chromen-4-one (100 mg, 291 µmol) in DCM (5.82 mL) was cooled in the ice-water bath. Into the stirring solution was slowly added 4-methylbenzenesulfonyl isocyanate (88.6 µl, 582 µmol). The reaction was allowed to warm to room temperature and stirred overnight. After the reaction went complete, the reaction mixture was concentrated *in vacuo* to give a yellow solid, which was purified by prep-HPLC and afforded 1-[3-[2-(8-chloro-4-oxo-chromen-2-yl)-5-methyl-phenoxy]propyl]-3-(p-tolylsulfonyl)urea (56 mg, yield: 33.8%) as a light yellow solid. MS obsd. (ESI⁺) [(M+H)⁺]: 541.2.

**Example 156:** ¹H NMR (DMSO-*d*₆, 400MHz) δ ppm 7.98 (dd, *J* =7.9, 1.4 Hz, 2H), 7.86 (d, *J* =8.3 Hz, 1H), 7.76 (d, *J* =8.3 Hz, 2H), 7.48 (t, *J* =7.8 Hz, 1H), 7.36 (d, *J* =7.9 Hz, 2H), 7.02 (s, 3H), 6.68 (br t, *J* =5.6 Hz, 1H), 4.04 (t, *J* =6.4 Hz, 2H), 3.07-3.21 (m, 2H), 2.39 (s, 3H), 2.35 (s, 3H), 2.07 (s, 1H), 1.88 (br t, *J* =6.5 Hz, 2H).

### Example 157:

### 3-[2-(8-chloro-4-oxo-chromen-2-yl)-5-(trifluoromethyl)phenoxy]propylurea

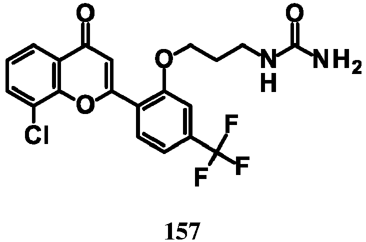

### Step 1: Preparation of 2-[2-(3-aminopropoxy)-4-(trifluoromethyl)phenyl]-8-chloro-chromen-4-one

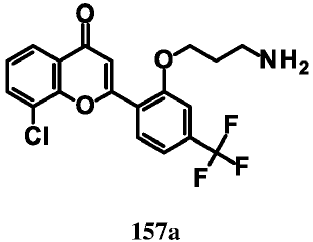

The compound **157a** was prepared in analogy to the procedure described for the preparation of **095b,** replacing tert-butyl (3-bromoethyl)carbamate with tert-butyl (3-bromopropyl)carbamate and replacing 8-chloro-2-(2-hydroxy-4-methyl-phenyl)chromen-4-one with 8-chloro-2-[2-hydroxy-4-(trifluoromethyl)phenyl]chromen-4-one as starting material in **Step 1.** (ESI⁺) [(M+H)⁺]: 398.2.

### Step 2: Preparation of 3-[2-(8-chloro-4-oxo-chromen-2-yl)-5-(trifluoromethyl)phenoxy]propylurea

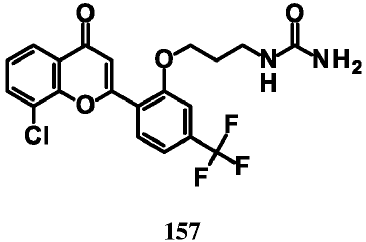

To a solution of 2-[2-(3-aminopropoxy)-4-(trifluoromethyl)phenyl]-8-chloro-chromen-4-one (0.18 g, 453 µmol) in DCM (4mL) was added isocyanatotrimethylsilane (73.6 µl, 543 µmol). The reaction mixture was stirred at room temperature for 1 day. Then the reaction was concentrated *in vacuo* to give the crude product, which was purified by prep-HPLC to give 3-[2-(8-chloro-4-oxo-chromen-2-yl)-5-(trifluoromethyl)phenoxy]propylurea (19.7 mg, yield: 9.38%) as a white solid. MS obsd. (ESI⁺) [(M+H)⁺]: 441.5.

**Example 157:** ¹H NMR (DMSO-*d*₆, 400MHz) δ ppm 8.14 (d, *J* =7.8 Hz, 1H), 8.03 (d, *J* =7.7 Hz, 2H), 7.45-7.66 (m, 3H), 7.11 (s, 1H), 6.09 (br t, *J* =5.7 Hz, 1H), 5.40 (s, 2H), 4.18-4.35 (m, 2H), 3.14 (br d, *J* =6.1 Hz, 2H), 1.90 (m, 2H).

### Example 158: 1-[2-[2-(8-chloro-4-oxo-chromen-2-yl)-5-(trifluoromethyl)phenoxy]ethyl]-3-(p-tolylsulfonyl)urea

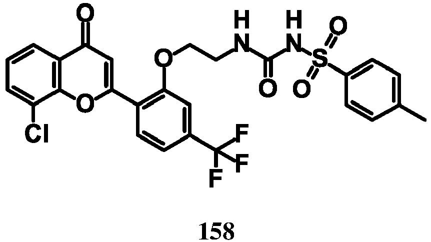

### Step 1: Preparation of 2-[2-(2-aminoethoxy)-4-(trifluoromethyl)phenyl]-8-chloro-chromen-4-one

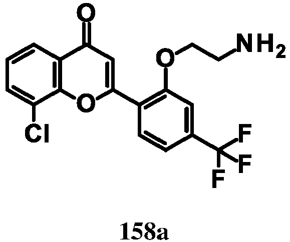

The compound **158a** was prepared in analogy to the procedure described for the preparation of **157a,** replacing tert-butyl (3-bromopropyl)carbamate with tert-butyl (3-bromoethyl)carbamate as starting material. (ESI⁺) [(M+H)⁺]: 384.1.

### Step 2: Preparation of 1-[2-[2-(8-chloro-4-oxo-chromen-2-yl)-5-(trifluoromethyl)phenoxy]ethyl]-3-(p-tolylsulfonyl)urea

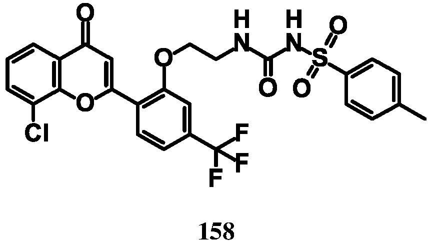

The compound **158** was prepared in analogy to the procedure described for the preparation of **158,** replacing 2-[2-(3-aminopropoxy)-4-methyl-phenyl]-8-chloro-chromen-4-one with 2-[2-(2-aminoethoxy)-4-(trifluoromethyl)phenyl]-8-chloro-chromen-4-one as starting material. MS obsd. (ESI⁺) [(M+H)⁺]: 581.2.

**Example 158:** ¹H NMR (DMSO-*d*₆, 400MHz) δ ppm 8.12 (d, *J* =7.9 Hz, 1H), 7.94-8.07 (m, 2H), 7.71 (d, *J* =8.2 Hz, 1H), 7.45-7.67 (m, 3H), 7.29 (d, *J* =7.9 Hz, 2H), 7.03 (s, 1H), 6.67 (br t, *J* =5.4 Hz, 1H), 4.24 (t, *J* =5.6 Hz, 2H), 3.44 (br d, *J* =5.6 Hz, 2H), 2.32 (s, 3H).

### Example 159: 8-chloro-4-oxo-2-[2-[2-(sulfamoylamino)ethoxy]-4-(trifluoromethyl)phenyl]chromene

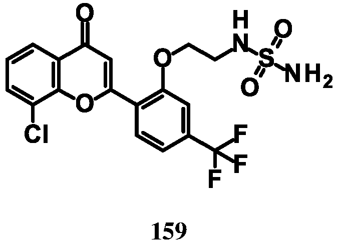

### Step 1: Preparation of tert-butyl N-[2-[2-(8-chloro-4-oxo-chromen-2-yl)-5-(trifluoromethyl)phenoxy]ethylsulfamoyl]carbamate

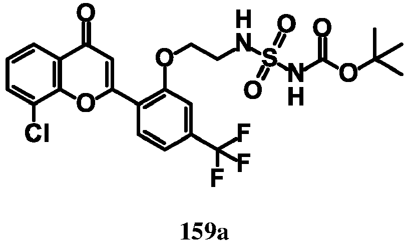

To a solution of 2-[2-(2-aminoethoxy)-4-(trifluoromethyl)phenyl]-8-chloro-chromen-4-one (200 mg, 447 µmol) in DMF (5 mL) was added K₂CO₃ (309 mg, 2.23 mmol) and tert-butyl N-chlorosulfonylcarbamate (193 mg, 983 µmol). The reaction was stirred at 70 °C overnight After the starting material was consumed, the reaction was cooled to room temperature and quenched by water (20 mL). The aqueous layer was extracted with EtOAc (10 mL) twice, and the organic layer was combined, washed with water and brine, dried over anhydrous Na₂SO₄, filtered and concentrated *in vacuo* to give tert-butyl N-[2-[2-(8-chloro-4-oxo-chromen-2-yl)-5-(trifluoromethyl)phenoxy]ethylsulfamoyl]carbamate (252 mg, 100%) as brown oil. MS obsd. (ESI⁺) [(M+H)⁺]: 563.2.

### Step 2: Preparation of 8-chloro-4-oxo-2-[2-[2-(sulfamoylamino)ethoxy]-4-(trifluoromethyl)phenyl]chromene

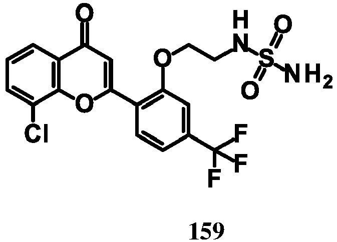

To a solution of tert-butyl N-[2-[2-(8-chloro-4-oxo-chromen-2-yl)-5-(trifluoromethyl)phenoxy]ethylsulfamoyl]carbamate (275 mg, 489 µmol) in DCM (6 mL) was added TFA (3 mL, 489 µmol) dropwise. The reaction was stirred for 2 hours and then the reaction was concentrated *in vacuo* to give the crude product, which was purified by prep-HPLC to afford 8-chloro-4-oxo-2-[2-[2-(sulfamoylamino)ethoxy]-4-(trifluoromethyl)phenyl]chromene (12.4 mg, yield: 5.37%) as a white solid. MS obsd. (ESI⁺) [(M+H)⁺]: 462.9.

**Example 159:** ¹H NMR (DMSO-*d*₆, 400MHz) δ ppm 8.17 (d, *J* =7.9 Hz, 1H), 8.04 (dd, *J* =7.9, 0.9 Hz, 2H), 7.45-7.70 (m, 3H), 7.28 (s, 1H), 6.84-6.94 (m, 1H), 6.67 (s, 2H), 4.37 (t, *J* =5.7 Hz, 2H), 3.46-3.55 (m, 2H).

### Example 160: 8-chloro-4-oxo-2-[2-[3-(sulfamoylamino)propoxy]-4-(trifluoromethyl)phenyl]chromene

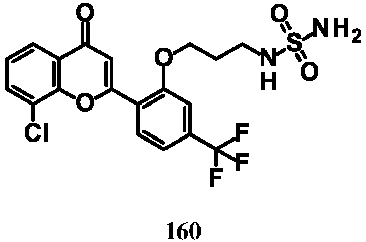

The Example **160** was prepared in analogy to the procedure described for the preparation of **159,** replacing 2-[2-(2-aminoethoxy)-4-(trifluoromethyl)phenyl]-8-chloro-chromen-4-one with 2-[2-(3-aminopropoxy)-4-(trifluoromethyl)phenyl]-8-chloro-chromen-4-one as starting material. MS obsd. (ESI⁺) [(M+H)⁺]: 476.9.

**Example 160:** ¹H NMR (DMSO-*d*₆, 400MHz) δ ppm 8.15 (d, *J* =7.8 Hz, 1H), 8.03 (dd, *J* =7.9, 1.8 Hz, 2H), 7.54 (d, *J* =16.3 Hz, 3H), 7.12 (s, 1H), 6.62-6.74 (m, 1H), 6.53 (s, 2H), 4.33 (s, 2H), 3.08 (d, *J* =6.2 Hz, 2H), 1.98-2.08 (m, 2H).

**Example 161: 8-chloro-2-[4-methyl-2-[2-(sulfamoylamino)ethoxy]phenyl]-4-oxo-chromene**

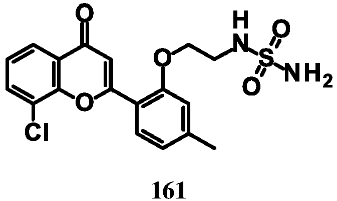

The compound **161** was prepared in analogy to the procedure described for the preparation of **159,** replacing 2-[2-(2-aminoethoxy)-4-(trifluoromethyl)phenyl]-8-chloro-chromen-4-one with 2-[2-(2-aminoethoxy)-4-methyl-phenyl]-8-chloro-chromen-4-one as starting material. MS obsd. (ESI⁺) [(M+H)⁺]: 410.1.

**Example 161:** ¹H NMR (DMSO-*d*₆, 400MHz) δ ppm 7.95-8.10 (m, 4H), 7.51 (t, *J* =7.9 Hz, 1H), 7.36 (s, 1H), 7.03 (s, 1H), 4.35 (br t, *J* =4.8 Hz, 2H), 3.27 (m, 2H), 2.45 (s, 3H).

### Example 162 2-[3-[2-[2-(8-chloro-4-oxo-chromen-2-yl)-5-(trifluoromethyl)phenoxy]ethyl]-2-oxo-imidazolidin-1-yl]-2-oxo-acetic acid

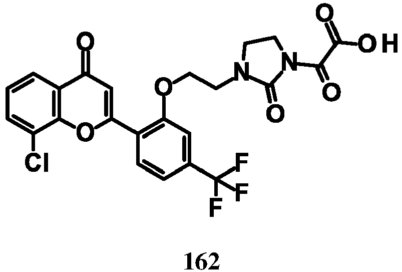

### Step 1: Preparation of 1-(2-chloroethyl)-3-[2-[2-(8-chloro-4-oxo-chromen-2-yl)-5-(trifluoromethyl)phenoxy]ethyl]urea

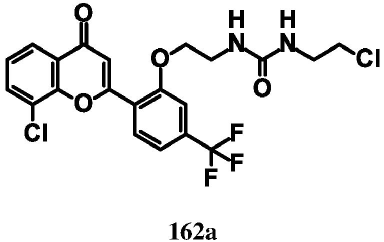

To a solution of 2-[2-(2-aminoethoxy)-4-(trifluoromethyl)phenyl]-8-chloro-chromen-4-one (110 mg, 287 µmol) in DCM (5 ml) was added 1-chloro-2-isocyanatoethane (36.7 µL, 430 µmol) and DIPEA (50.1 µL, 287 µmol). The mixture was stirred at room temperature overnight. After stirring for 2 hours, the reaction mixture was concentrated *in vacuo* to give 1-(2-chloroethyl)-3-[2-[2-(8-chloro-4-oxo-chromen-2-yl)-5-(trifluoromethyl)phenoxy]ethyl]urea (140 mg, yield: 100%). MS obsd. (ESI⁺) [(M+H)⁺]: 489.2.

### Step 2: Preparation of 1-[2-[2-(8-chloro-4-oxo-chromen-2-yl)-5-(trifluoromethyl)phenoxy]ethyl]imidazolidin-2-one

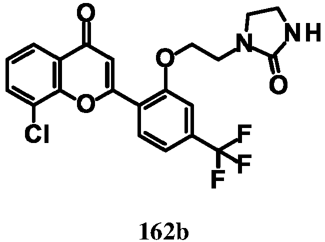

To a solution of 1-(2-chloroethyl)-3-[2-[2-(8-chloro-4-oxo-chromen-2-yl)-5-(trifluoromethyl)phenoxy]ethyl]urea (140 mg, 287 µmol) in THF (5 mL) was added KOH (80.4 mg, 1.43 mmol). The reaction was stirred at 80 °C overnight. After the starting material was consumed, the reaction was quenched by adding water (20 mL). The aqueous layer was extracted with EtOAc (10 mL) three times. The organic layer was combined, washed with brine, dried over anhydrous sodium sulfate, filtered and concentrated *in vacuo* to give 1-[2-[2-(8-chloro-4-oxo-chromen-2-yl)-5-(trifluoromethyl)phenoxy]ethyl]imidazolidin-2-one (0.126 g, yield: 97.1%) as a light yellow solid. MS obsd. (ESI⁺) [(M+H)⁺]: 453.0.

### Step 3: Preparation of 2-[3-[2-[2-(8-chloro-4-oxo-chromen-2-yl)-5-(trifluoromethyl)phenoxy]ethyl]-2-oxo-imidazolidin-1-yl]-2-oxo-acetic acid

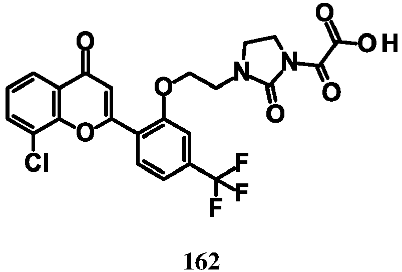

To a solution of 1-[2-[2-(8-chloro-4-oxo-chromen-2-yl)-5-(trifluoromethyl)phenoxy]ethyl]imidazolidin-2-one (100 mg, 221 µmol) in DCM (5 mL) was added oxalyl dichloride (56.1 mg, 442 µmol) and DIPEA (38.6 µL, 221 µmol). The reaction was stirred at room temperature for 1 hour and the starting material was completely converted. Then, into the reaction was added water and the reaction mixture was stirred for another 1 hour. The reaction was partitioned between DCM (10 mL) and water (20 mL). The organic layer was combined, washed with brine, dried over anhydrous sodium sulfate, filtered and concentrated *in vacuo* to give yellow oil crude. The crude product was purified by prep-HPLC to give 2-[3-[2-[2-(8-chloro-4-oxo-chromen-2-yl)-5-(trifluoromethyl)phenoxy]ethyl]-2-oxo-imidazolidin-1-yl]-2-oxo-acetic acid (11.5 mg, yield: 8.93%) as a white solid. MS obsd. (ESI⁺) [(M+H)⁺]: 525.0.

**Example 162:** ¹H NMR (DMSO-*d*₆, 400MHz) δ ppm 8.12 (d, *J* =8.1 Hz, 1H), 8.02 (d, *J* =8.1 Hz, 2H), 7.30-7.71 (m, 3H), 7.07 (s, 1H), 4.47 (t, *J* =5.2 Hz, 2H), 3.63-3.72 (m, 4H), 3.54-3.62 (m, 2H).

### Example 163: (3S)-1-[3-[2-(8-chloro-4-oxo-chromen-2-yl)-5-(trifluoromethyl)phenoxy]-2-hydroxy-propyl]pyrrolidine-3-carboxylic acid

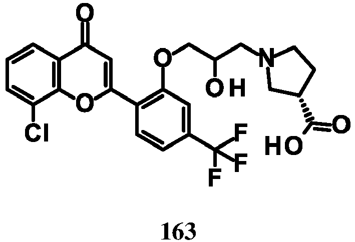

### Step 1: Preparation of 8-chloro-2-[2-(oxiran-2-ylmethoxy)-4-(trifluoromethyl)phenyl]chromen-4-one

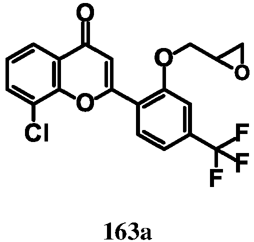

To a solution of 8-chloro-2-[2-hydroxy-4-(trifluoromethyl)phenyl]chromen-4-one (400 mg, 1.17 mmol) and 2-(chloromethyl)oxirane (435 mg, 4.7 mmol) in DMF was added K₂CO₃ (162 mg, 1.17 mmol). The reaction mixture was stirred at 50 °C overnight. After the reaction was complete, the mixture was partitioned between EtOAc (10 mL) and water (30 mL). The organic layer was concentrated *in vacuo* to give 8-chloro-2-[2-(oxiran-2-ylmethoxy)-4-(trifluoromethyl)phenyl]chromen-4-one (460 mg, yield: 98.7%) as a white solid. MS obsd. (ESI⁺) [(M+H)⁺]: 397.1.

### Step 2: Preparation of methyl (3S)-1-[3-[2-(8-chloro-4-oxo-chromen-2-yl)-5-(trifluoromethyl)phenoxy]-2-hydroxy-propyl]pyrrolidine-3-carboxylate

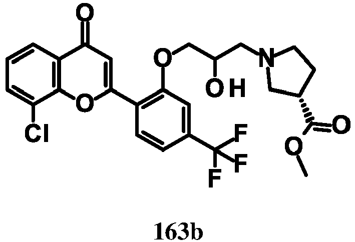

A mixture of 8-chloro-2-[2-(oxiran-2-ylmethoxy)-4-(trifluoromethyl)phenyl]chromen-4-one (80 mg, 202 µmol), methyl (S)-pyrrolidine-3-carboxylate (104 mg, 807 µmol) and K₂CO₃ (27.9 mg, 202 µmol) was stirred at 50 °C. After the reaction was complete, the reaction mixture was concentrated *in vacuo* to give methyl (3S)-1-[3-[2-(8-chloro-4-oxo-chromen-2-yl)-5-(trifluoromethyl)phenoxy]-2-hydroxy-propyl]pyrrolidine-3-carboxylate (80mg, yield 75.4%). MS obsd. (ESI⁺) [(M+H)⁺]: 526.1.

### Step 3: Preparation of (3S)-1-[3-[2-(8-chloro-4-oxo-chromen-2-yl)-5-(trifluoromethyl)phenoxy]-2-hydroxy-propyl]pyrrolidine-3-carboxylic acid

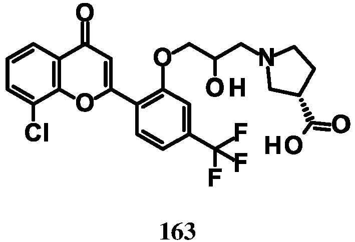

To a solution of methyl (3S)-1-[3-[2-(8-chloro-4-oxo-chromen-2-yl)-5-(trifluoromethyl)phenoxy]-2-hydroxy-propyl]pyrrolidine-3-carboxylate (60 mg, 114 µmol) in the mixed solvent of DMF (5 mL) and MeOH (2 mL) was added LiOH (2.73 mg, 114 µmol). The reaction mixture was stirred at room temperature overnight. After the reaction was complete, the reaction mixture was acidified with HOAc and then the resulting solution was purified by prep-HPLC to afford (3S)-1-[3-[2-(8-chloro-4-oxo-chromen-2-yl)-5-(trifluoromethyl)phenoxy]-2-hydroxy-propyl]pyrrolidine-3-carboxylic acid (13 mg, yield: 21.1%) as a white powder. MS obsd. (ESI⁺) [(M+H)⁺]: 511.8.

**Example 163:** ¹H NMR (DMSO-*d*₆, 400MHz) δ ppm 8.14 (d, *J* = 8.1 Hz, 1H), 8.04 (d, *J* = 7.9 Hz, 2H), 7.58-7.67 (m, 2H), 7.47-7.56 (m, 1H), 7.18 (d, *J* = 4.6 Hz, 1H), 6.07 (br dd, *J* = 10.0, 4.1 Hz, 1H), 4.25-4.42 (m, 3H), 3.75-3.88 (m, 1H), 3.54-3.70 (m, 1H), 3.38-3.47 (m, 1H), 3.27 (br d, *J* = 3.3 Hz, 2H), 3.08-3.20 (m, 1H), 2.76-2.83 (m, 1H), 1.96-2.29 (m, 2H).

### Example 164:

### (3S)-1-[3-[5-bromo-2-(8-chloro-4-oxo-chromen-2-yl)phenoxy]-2-hydroxypropyl]pyrrolidine-3-carboxylic acid

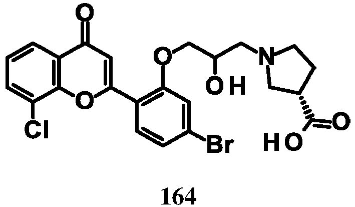

The compound **164** was prepared in analogy to the procedure described for the preparation of **163,** replacing 8-chloro-2-[2-hydroxy-4-(trifluoromethyl)phenyl]chromen-4-one with 2-(4-bromo-2-hydroxy-phenyl)-8-chloro-chromen-4-one as starting material in **Step 1.** MS obsd. (ESI⁺) [(M+H)⁺]: 522.1.

**Example 164:** ¹H NMR (DMSO-*d*₆, 400MHz) δ ppm 12.56-13.11 (m, 1H), 8.02 (d, *J* = 7.8 Hz, 2H), 7.88 (d, *J* = 8.4 Hz, 1H), 7.37-7.64 (m, 3H), 7.12 (d, *J* = 3.1 Hz, 1H), 5.90-6.15 (m, 1H), 4.13-4.39 (m, 4H), 3.72-3.93 (m, 2H), 3.56-3.70 (m, 2H), 3.10-3.21 (m, 1H), 1.85-2.27 (m,3H).

### Example 165: (3R)-1-[3-[5-bromo-2-(8-chloro-4-oxo-chromen-2-yl)phenoxy]-2-hydroxypropyl]pyrrolidine-3-carboxylic acid

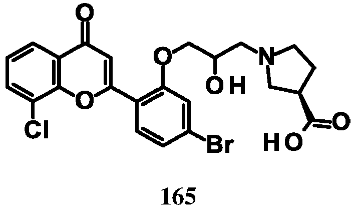

The compound **165** was prepared in analogy to the procedure described for the preparation of **164,** replacing 8-chloro-2-[2-hydroxy-4-(trifluoromethyl)phenyl]chromen-4-one with 2-(4-bromo-2-hydroxy-phenyl)-8-chloro-chromen-4-one in **Step 1** and methyl (R)-pyrrolidine-3-carboxylate with methyl (S)-pyrrolidine-3-carboxylate in **Step 2** as starting material. MS obsd. (ESI⁺) [(M+H)⁺]: 522.0.

**Example 165:** ¹H NMR (DMSO-*d*₆, 400MHz) δ ppm 12.73-13.22 (m, 1H), 8.03 (dd, *J* = 7.8, 1.2 Hz, 2H), 7.89 (d, *J* = 8.4 Hz, 1H), 7.37-7.62 (m, 3H), 7.13 (dd, *J* = 3.0, 1.3 Hz, 1H), 5.97-6.14 (m, 1H), 4.13-4.43 (m, 4H), 3.75-3.96 (m, 2H), 3.59-3.72 (m, 2H), 3.06-3.23 (m, 1H), 1.95-2.41 (m, 3H).

### Example 166: 2-[[3-[2-(8-chloro-4-oxo-chromen-2-yl)-5-(trifluoromethyl)phenoxy]-2-hydroxy-propyl]amino]-2-oxo-acetic acid

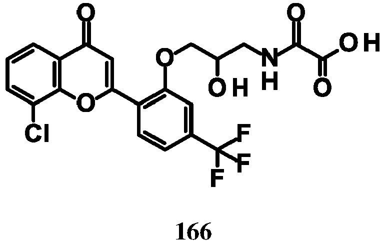

### Step 1: Preparation of 8-chloro-2-[2-[2-hydroxy-3-[(4-methoxyphenyl)methylamino]propoxy]-4-(trifluoromethyl)phenyl]chromen-4-one

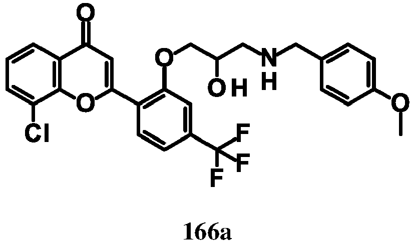

To a solution of 4-aminomethyl-anisole (0.26 mL, 2.02 mmol) in ethanol (8.86 mL) was added K₂CO₃ (1.38 g, 10.1 mmol) and 8-chloro-2-[2-(oxiran-2-ylmethoxy)-4-(trifluoromethyl)phenyl]chromen-4-one (800 mg, 2.02 mmol) under N₂. Then, the reaction was heated and to 50 °C and kept stirring at this temperature for 16 hours. After the starting material was consumed, the reaction mixture was concentrated *in vacuo.* The crude product was purified by flash column (eluting with EtOAc: Isohexane = 60%) to give 8-chloro-2-[2-[2-hydroxy-3-[(4-methoxyphenyl)methylamino]propoxy]-4-(trifluoromethyl)phenyl]chromen-4-one (800 mg, yield: 74.3%) as a white solid. MS obsd. (ESI⁺)[(M+H)⁺]: 534.0.

### Step 2: Preparation of 2-[2-(3-amino-2-hydroxy-propoxy)-4-(trifluoromethyl)phenyl]-8-chloro-chromen-4-one

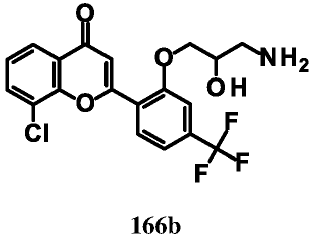

A solution of 8-chloro-2-[2-[2-hydroxy-3-[(4-methoxyphenyl)methylamino]propoxy]-4-(trifluoromethyl)phenyl]chromen-4-one (800 mg, 1.5 mmol) and TFA (10.0 mL, 1.5 mmol) in DCM was stirred at 100 °C under N₂ for 8 hours. After the reaction was consumed, the reaction mixture was concentrated *in vacuo* to give 2-[2-(3-amino-2-hydroxy-propoxy)-4-(trifluoromethyl)phenyl]-8-chloro-chromen-4-one (300 mg, yield: 48.4%) as colorless oil. MS obsd. (ESI⁺)[(M+H)⁺]: 414.0.

### Step 3: Preparation of ethyl 2-[[3-[2-(8-chloro-4-oxo-chromen-2-yl)-5-(trifluoromethyl)phenoxy]-2-hydroxy-propyl]amino]-2-oxo-acetate

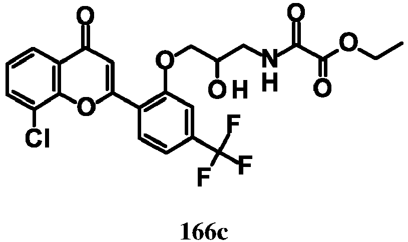

To a solution of 2-[2-(3-amino-2-hydroxy-propoxy)-4-(trifluoromethyl)phenyl]-8-chloro-chromen-4-one (110 mg, 0.270 mmol) in DCM (5.5 mL) was added TEA (0.07 mL, 0.530 mmol) and ethyl oxalyl chloride (43.56 mg, 0.320 mmol) at 0 °C. Then, the reaction was stirred at room temperature for 16 hours. After the reaction was completed, the reaction mixture was quenched with water (100 mL) and the aqueous layer was extracted with DCM (50 mL) three times. The combined organic layer was washed with brine (50 mL) twice, dried over anhydrous MgSO₄, filtered and concentrated *in vacuo* to give the crude product, which was purified by flash column chromatography (EtOAc: PE = 50%) to afford ethyl 2-[[3-[2-(8-chloro-4-oxo-chromen-2-yl)-5-(trifluoromethyl)phenoxy]-2-hydroxy-propyl]amino]-2-oxo-acetate (80 mg, yield: 58.6%) as a light yellow solid. MS obsd. (ESI⁺)[(M+H)⁺]: 514.0.

### Step 4: Preparation of 2-[[3-[2-(8-chloro-4-oxo-chromen-2-yl)-5-(trifluoromethyl)phenoxy]-2-hydroxy-propyl]amino]-2-oxo-acetic acid

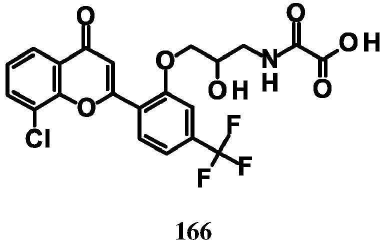

To a solution of ethyl 2-[[3-[2-(8-chloro-4-oxo-chromen-2-yl)-5-(trifluoromethyl)phenoxy]-2-hydroxy-propyl]amino]-2-oxo-acetate (116.81 mg, 0.230 mmol) in THF (1.2 mL) and water (1.2 mL) was added lithium hydroxide (19 mg, 0.450 mmol). Then the mixture was stirred at room temperature for 2 hours. After the reaction was completed, the reaction mixture was adjusted to pH 6 with 1N HCl. The resulting residue was concentrated *in vacuo* to give the crude product, which was purified by prep-HPLC to afford 2-[[3-[2-(8-chloro-4-oxo-chromen-2-yl)-5-(trifluoromethyl)phenoxy]-2-hydroxy-propyl]amino]-2-oxo-acetic acid (51 mg, yield: 43.9%) as a white solid. MS obsd. (ESI⁺)[(M+H)⁺]: 486.0.

**Example 166:** 13.69-13.92 (m, 1H), 8.82 (br t, *J* = 5.9 Hz, 1H), 8.16 (d, *J* = 8.1 Hz, 1H), 8.02 (d, *J* = 7.7 Hz, 2H), 7.46-7.65 (m, 3H), 7.29 (s, 1H), 5.31-5.50 (m, 1H), 4.22-4.31 (m, 1H), 4.10-4.19 (m, 1H), 3.97-4.07 (m, 1H), 3.40-3.52 (m, 2H).

### Example 167: 2-[2-[2-(8-chloro-4-oxo-chromen-2-yl)-5-(trifluoromethyl)phenoxy]ethylamino]acetamide

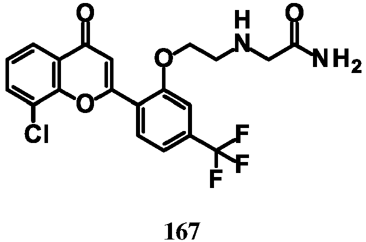

### Step 1: Preparation of ethyl 2-[2-[2-(8-chloro-4-oxo-chromen-2-yl)-5-(trifluoromethyl)phenoxy]ethylamino]acetate

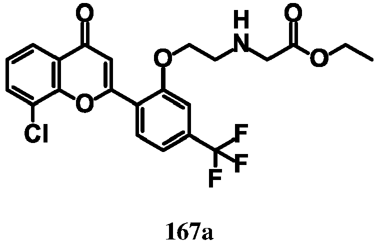

To a solution of 2-[2-(2-aminoethoxy)-4-(trifluoromethyl)phenyl]-8-chloro-chromen-4-one (400 mg, 1.04 mmol) and ethyl glyoxalate (213 mg, 1.04 mmol) in methanol (16 mL) was added sodium cyanoborohydride (196.5 mg, 3.13 mmol). The reaction mixture was stirred at 30 °C for 6 hours and then quenched with water (2 mL). The resulting mixture was concentrated *in vacuo* to give the crude product, which was purified by flash chromatography (eluting with EtOAc: PE = 20%) to give ethyl 2-[2-[2-(8-chloro-4-oxo-chromen-2-yl)-5-(trifluoromethyl)phenoxy]ethylamino]acetate (203 mg, yield: 41. 5%) as a light yellow solid. MS obsd. (ESI⁺)[(M+H)⁺]: 470.1.

### Step 2: Preparation of 2-[2-[2-(8-chloro-4-oxo-chromen-2-yl)-5-(trifluoromethyl)phenoxy]ethylamino]acetic acid

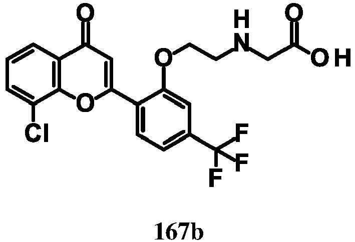

To a solution of ethyl 2-[2-[2-(8-chloro-4-oxo-chromen-2-yl)-5-(trifluoromethyl)phenoxy]ethylamino]acetate (170 mg, 0.360 mmol) in methanol was added hydrochloric acid (15 mL, 180 mmol). The reaction mixture was stirred at 100 °C for 6 hours and then concentrated *in vacuo.* The resulting residue was triturated with EtOAc (10 mL) and filtered to afford 2-[2-[2-(8-chloro-4-oxo-chromen-2-yl)-5-(trifluoromethyl)phenoxy]ethylamino]acetic acid (150 mg, yield: 93.8%) as a white solid. MS obsd. (ESI⁺)[(M+H)⁺]: 442.1.

### Step 3: Preparation of 2-[2-[2-(8-chloro-4-oxo-chromen-2-yl)-5-(trifluoromethyl)phenoxy]ethylamino]acetamide

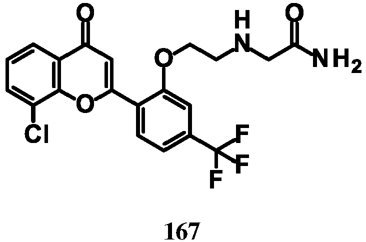

To a solution of 2-[2-[2-(8-chloro-4-oxo-chromen-2-yl)-5-(trifluoromethyl)phenoxy]ethylamino]acetic acid (150 mg, 0.340 mmol), DIPEA (0.24 mL, 1.36 mmol), and HATU (258.2 mg, 0.680 mmol) in DCM (5 mL) was added ammonium chloride (36 mg, 0.680 mmol). The reaction mixture was stirred at 30 °C for 12 hours. After the reaction was completed, the reaction mixture was quenched with water (1 mL) and concentrated *in vacuo* to give the crude product, which was purified by prep-HPLC to afford 2-[2-[2-(8-chloro-4-oxo-chromen-2-yl)-5-(trifluoromethyl)phenoxy]ethylamino]acetamide (12.5 mg, yield: 8.1%) as a white solid. MS obsd. (ESI⁺): 441.1

**Example 167:** ¹H NMR (400 MHz, DMSO-d₆): *δ* ppm 8.15 (d, *J* = 7.9 Hz, 1H), 7.96-8.07 (m, 2H), 7.45-7.63 (m, 3H), 7.24 (s, 2H), 7.01 (br s, 1H), 4.33 (t, *J* = 5.4 Hz, 2H), 3.04-3.16 (m, 2H), 2.95 (t, *J* = 5.3 Hz, 2H).

### Example 168: 2-[2-[2-(8-chloro-4-oxo-chromen-2-yl)-5-(trifluoromethyl)phenoxy]ethylamino]propanamide

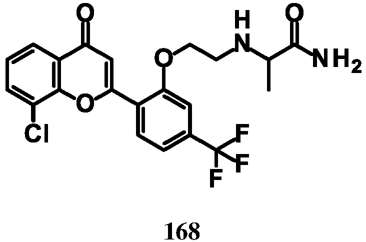

The compound **168** was prepared in analogy to the procedure described for the preparation of **168,** replacing ethyl glyoxalate with ethyl pyruvate as starting material in **Step 1.** MS obsd. (ESI⁺) [(M+H)⁺]: 455.1.

**Example 168:** ¹H NMR (DMSO-*d₆*, 400MHz) δ ppm 8.13-8.19 (m, 1H), 7.97-8.05 (m, 2H), 7.48-7.61 (m, 3H), 7.26-7.33 (m, 2H), 6.95 (br s, 1H), 4.29-4.37 (m, 2H), 3.03-3.14 (m, 1H), 2.77-2.99 (m, 2H), 1.13 (d, *J* = 6.7 Hz, 3H).

### Example 169: 1-[3-[5-bromo-2-(8-chloro-4-oxo-chromen-2-yl)phenoxy]propyl]-5-oxo-pyrrolidine-3-carboxylic acid

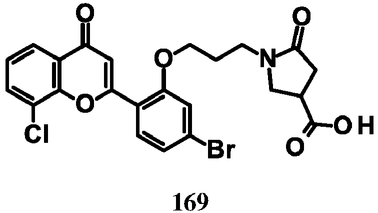

### Step 1: Preparation of methyl 1-[3-[5-bromo-2-(8-chloro-4-oxo-chromen-2-yl)phenoxy]propyl]-5-oxo-pyrrolidine-3-carboxylate

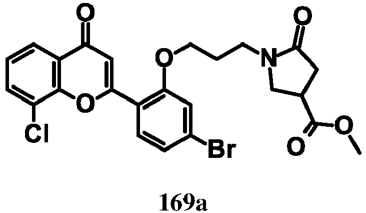

To a solution of 2-[2-(3-aminopropoxy)-4-bromo-phenyl]-8-chloro-chromen-4-one (300 mg, 0.670 mmol) in methanol (6 mL) were added TEA (0.09 mL, 0.670 mmol) and dimethyl itaconate (106.59 mg, 0.670 mmol). The reaction mixture was stirred at 60 °C for 20 hours and then quenched with water (50 mL). The aqueous layer was extracted with EtOAc (50 mL) three times. The organic layer was combined, washed with brine, dried over anhydrous sodium sulfate and concentrated *in vacuo.* The resulting residue was purified by flash chromatography (EtOAc: PE = 50%) to give methyl 1-[3-[5-bromo-2-(8-chloro-4-oxo-chromen-2-yl)phenoxy]propyl]-5-oxo-pyrrolidine-3-carboxylate (270 mg, yield: 68.9%) as a yellow solid. MS obsd. (ESI⁺) [(M+H)⁺]: 534.0.

### Step 2: Preparation of 1-[3-[5-bromo-2-(8-chloro-4-oxo-chromen-2-yl)phenoxy]propyl]-5-oxo-pyrrolidine-3-carboxylic acid

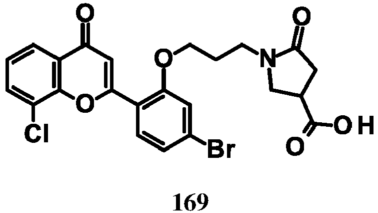

To a solution of methyl 1-[3-[5-bromo-2-(8-chloro-4-oxo-chromen-2-yl)phenoxy]propyl]-5-oxo-pyrrolidine-3-carboxylate (270 mg, 0.500 mmol) in THF (2 mL) and water (2 mL) was added lithium hydroxide (18.14 mg, 0.760 mmol). The mixture was stirred at room temperature for 2 hours. After the reaction was completed, the reaction mixture was added water (20 mL) and EtOAc (20 mL) to form a suspension. The suspension was filtered and the filter cake was washed with EtOAc (50 mL) to afford 1-[3-[5-bromo-2-(8-chloro-4-oxo-chromen-2-yl)phenoxy]propyl]-5-oxo-pyrrolidine-3-carboxylic acid (86 mg, yield: 32.4%) as a yellow solid. MS obsd. (ESI⁺) [(M+H)⁺]: 520.1.

**Example 169:** ¹H NMR (400 MHz, DMSO) *δ* ppm: 12.61 (br s, 1H), 8.00 (d, *J* = 7.8 Hz, 2H), 7.87 (d, *J* = 8.3 Hz, 1H), 7.38-7.54 (m, 3H), 7.05-7.15 (m, 1H), 4.17 (t, *J* = 6.2 Hz, 2H), 3.53-3.62 (m, 1H), 3.45-3.52 (m, 1H), 3.34-3.38 (m, 2H), 3.09-3.23 (m, 1H), 2.33-2.47 (m, 2H), 1.91-2.08 (m, 2H).

Similar flavone compounds, 7-hydroxy-2-(2-hydroxyphenyl)chromen-4-one (compound **F-1**) in patent WO2015061294 for treating HBV infection as STING agonist and 6-methoxy-2-(2-methoxyphenyl)chromen-4-one (compound **F-2**) disclosed in patent WO 2001003681 for treating infections, were chosen as reference compounds in present invention.

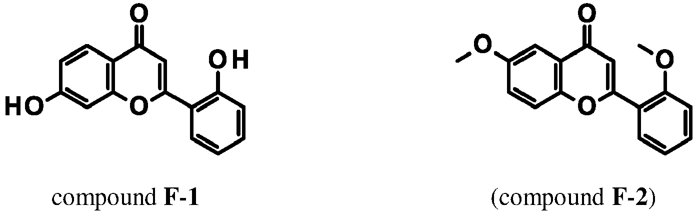

### BIOLOGICAL EXAMPLES

### BIO-Example 1: Engineered HepDES19 primary screen assay

The assay was employed to screen for cccDNA inhibitors. HepDES19 is a cccDNA-producing cell line. In this cell line, HBeAg in the cell culture supernatant as surrogate marker, as HBeAg production depends on cccDNA level and activity. HepDES19 is an engineered cell line which contains a 1.1 unit length HBV genome, and pgRNA transcription from the transgene is controlled by Tetracycline (Tet). In the absence of Tet, pgRNA transcription will be induced, but HBV e antigen (HBeAg) could not be produced from this pgRNA due to very short leader sequence before the HBeAg start codon and the start codon is disrupted. Only after cccDNA is formed, the missing leader sequence and start codon mutation would be restored from the 3'-terminal redundancy of pgRNA, and then HBeAg could be synthesized. Therefore, HBeAg could be used as a surrogate marker for cccDNA (Zhou, T. et al., Antiviral Res. (2006), 72(2), 116-124; Guo, H. et al., J. Virol. (2007), 81(22), 12472-12484).

HepDES19 cells were seeded at 2×10⁶ cells per T150 flask and cultured with the culture medium (Dulbecco's Modified Eagle Medium: Nutrient Mixture F-12 [DMEM-F12, Gibco Cat. 11320-82], 10% Fetal Bovine Serum [FBS, Clontech Cat. 631101], 0.1mM Non-Essential Amino Acids Solution [NEAA, Gibco Cat. 11140-050], 50µg/mL Penicillin-Streptomycin [PS, Invitrogen Cat. 15140-163], 500 µg/mL Geneticin [G418, Invitrogen Cat. 10131-027]) containing 3µg/mL Tet (Sigma, Cat. 87128) for 5 days. Cells were then seeded at 4×10⁶ cells per T150 in the same culture medium as described above in the absence of Tet for 8 days. Cells were then harvested and frozen at density of 2×10⁶ cells per mL. For compound testing, the frozen cells were thawed and seeded into 96-well plates at a density of 6 ×10⁴ cells per well. At 24hours after seeding, half log serial dilutions of compounds made with Dimethyl sulfoxide (DMSO, Sigma, Cat. D2650) were further diluted with the same culture medium as described above before they were added to the cells to reach desired final compound concentrations and 1% DMSO concentration. Plates were then incubated at 37°C for another 5 days before measurement of HBeAg level and cell viability. Intracellular HBeAg level were measured with enzyme-linked immunosorbent assay (ELISA) kit (Shanghai Kehua Diagnostic Medical Products Co., Ltd). Cell viability was assessed using Cell Counting Kit-8 (DonJindo, Cat. CK04-20). IC₅₀ values were derived from the dose-response curve using 4 parameter logistic curve fit method.

The compounds of the present invention were tested for their capacity to inhibit extracellular HBeAg level as described herein. The compounds of this invention were found to have IC₅₀ below 50 µM. Particular compounds of formula (I) were found to have IC₅₀ below 5.0 µM. Results of HepDES19 primary screen assay are given in Table BIO1.

**Table BIO1: Activity data in HepDES19 primary screen assay**

| **Example No.** | **IC₅₀ (µM)** | **Example No.** | **IC₅₀ (µM)** | **Example No.** | **IC₅₀ (µM)** |
|---|---|---|---|---|---|
| **001** | 0.35 | **002** | 0.25 | **003** | 2.41 |
| **004** | 2.02 | **005** | 8.93 | **006** | 4.68 |
| **007** | 9.50 | **008** | 0.17 | **009** | 7.47 |
| **010** | 0.22 | **011** | 3.61 | **012** | 0.11 |
| **013** | 4.23 | **014** | 0.37 | **015** | 7.85 |
| **016** | 0.89 | **017** | 0.34 | **018** | 0.41 |
| **019** | 0.57 | **020** | 0.87 | **021** | 0.18 |
| **022** | 1.31 | **023** | 0.13 | **024** | 1.04 |
| **025** | 0.79 | **026** | 0.46 | **027** | 0.09 |
| **029** | 1.85 | **030** | 1.33 | **031** | 0.17 |
| **032** | 0.65 | **033** | 1.09 | **034** | 1.12 |
| **035** | 2.38 | **036** | 2.03 | **037** | 4.12 |
| **038** | 7.24 | **039** | 3.48 | **040** | 9.06 |
| **041** | 0.03 | **042** | 0.05 | **043** | 1.42 |
| **044** | 4.75 | **045** | 6.50 | **046** | 4.37 |
| **047** | 0.99 | **048** | 6.68 | **049** | 2.81 |
| **050** | 6.93 | **051** | 7.86 | **052** | 0.08 |
| **053** | 0.10 | **054** | 0.24 | **055** | 0.98 |
| **056** | 1.00 | **057** | 1.50 | **058** | 3.41 |
| **059** | 4.68 | **060** | 4.87 | **061** | 5.71 |
| **062** | 2.02 | **063** | 4.32 | **064** | 0.08 |
| **065** | 7.21 | **066** | 2.79 | **067** | 8.40 |
| **068** | 5.51 | **069** | 1.77 | **070** | 1.01 |
| **071** | 1.03 | **072** | 2.89 | **073** | 0.14 |
| **074** | 1.03 | **075** | 0.11 | **076** | 1.64 |
| **077** | 0.05 | **078** | 7.80 | **079** | 0.78 |
| **080** | 3.14 | **081** | 7.11 | **082** | 0.24 |
| **083** | 0.56 | **084** | 0.59 | **085** | 1.08 |
| **086** | 3.22 | **087** | 5.24 | **088** | 0.27 |
| **089** | 1.94 | **090** | 1.30 | **091** | 2.83 |
| **092** | 0.22 | **093** | 5.98 | **094** | 9.79 |
| **095** | 9.30 | **096** | 0.13 | **097** | 1.16 |
| **098** | 4.34 | **099** | 6.47 | **100** | 3.84 |
| **101** | 1.08 | **102** | 2.91 | **103** | 3.66 |
| **104** | 9.65 | **105** | 1.90 | **106** | 1.10 |
| **107** | 2.71 | **108** | 1.14 | **109** | 4.11 |
| **110** | 3.76 | **111** | 0.18 | **112** | 0.49 |
| **113** | 0.49 | **114** | 0.87 | **115** | 1.04 |
| **116** | 1.80 | **117** | 2.16 | **118** | 1.98 |
| **119** | 3.43 | **120** | 2.96 | **121** | 4.25 |
| **122** | 7.72 | **123** | 5.11 | **124** | 5.88 |
| **125** | 2.03 | **126** | 0.91 | **127** | 0.08 |
| **128** | 0.27 | **129** | 4.75 | **130** | 4.82 |
| **131** | 5.86 | **132** | 0.35 | **133** | 4.57 |
| **134** | 8.17 | **135** | 0.21 | **136** | 0.58 |
| **137** | 3.40 | **138** | 4.56 | **139** | 10.20 |
| **140** | 0.19 | **141** | 0.35 | **142** | 0.74 |
| **143** | 2.42 | **144** | 11.40 | **145** | 14.30 |
| **146** | 0.33 | **147** | 1.20 | **148** | 1.21 |
| **149** | 8.79 | **150** | 12.20 | **151** | 0.63 |
| **152** | 0.48 | **153** | 0.83 | **154** | 1.43 |
| **155** | 5.11 | **156** | 4.14 | **157** | 4.15 |
| **158** | 8.35 | **159** | 0.83 | **160** | 2.43 |
| **161** | 4.74 | **162** | 0.08 | **163** | 2.76 |
| **164** | 2.96 | **165** | 3.19 | **166** | 1.57 |
| **167** | 3.30 | **168** | 1.14 | **169** | 0.99 |
| **F-1** | >50 | **F-2** | >50 | | |

### BIO-Example 2: Cryopreserved primary human hepatocytes (PHH) assay

This assay is used to confirm the anti-HBV effect of the compounds in HBV PHH infection assay. Cryopreserved PHH (BioreclamationIVT, Lot YJM) was thawed at 37°C and gently transferred into pre-warmed InVitroGRO HT medium(BioreclamationIVT, Cat. S03317). The mixture was centrifuged at 70 relative centrifugal force (RCF) for 3 minutes at RT, and the supernatant was discarded. Pre-warmed InVitroGRO CP medium (BioreclamationIVT, Cat# S03316) was added to the cell pellet to gently re-suspend cells. The cells were seeded at the density of 5.8 × 10⁴ cells per well to collagen I coated 96-well plate (Gibco, Cat. A1142803) with the InVitroGRO CP medium. All plates were incubated at 37°C with 5% CO₂ and 85% humidity.

At 20 hours after plating, the medium was changed to PHH culture medium (Dulbecco's Modified Eagle Medium (DMEM)/F12 (1:1) (Gibco, Cat. 11320-033), 10% fetal bovine serum (Gibco Cat. 10099141), 100 U/mL penicillin, 100 µg/mL streptomycin (Gibco, Cat. 151401-122), 5 ng/mL human epidermal growth factor (Invitrogen Cat. PHG0311L), 20 ng/mL dexamethasone (Sigma, Cat. D4902) and 250 ng/mL human recombinant insulin (Gibco, Cat. 12585-014)). And the cells were incubated at 37 °C with 5% CO₂ and 85% humidity for 4 hours. The medium was then changed to pre-warmed PHH culture medium containing 4% polyethylene glycol (PEG) MW8000 (Sigma, Cat. P1458-50ML) and 1% DMSO (Sigma, Cat. D2650). 5.8 × 10⁶ genomic equivalents of HBV were added into the medium.

At 24 hours post-infection, the cells were gently washed with PBS and refreshed with PHH culture medium supplemented with 1% DMSO, and 0.25mg/mL Matrix gel (Corning, Cat. 356237) at 200µL per well. All plates were immediately placed in at 37°C CO₂ incubator.

24 hours later, serial dilutions of compounds made with DMSO were further diluted with the same culture medium (PHH culture medium supplemented with 1% DMSO and 0.25mg/mL Matrix gel as described above) before they were added to the cells to reach desired final compound concentrations and 1% DMSO concentration. The medium containing the compounds were refreshed every three days.

At 9 days post-compound treatment, extracellular HBsAg level were measured with Chemiluminescence Immuno Assay (CLIA) kit (Autobio, HBsAg Quantitative CLIA). Extracellular HBV DNA was extracted by MagNA Pure 96 system (Roche) and then determined by quantitative PCR with the following primers and probe:
HBV-Forward Primer (SEQ ID NO: 1): AAGAAAAACCCCGCCTGTAA (5' to 3');
HBV-Reverse Primer (SEQ ID NO: 2): CCTGTTCTGACTACTGCCTCTCC (5' to 3');
HBV-Probe: 5'+ tetramethylrhodamine + SEQ ID NO: 3 + black hole quencher 2-3', wherein SEQ ID NO: 3 is CCTGATGTGATGTTCTCCATGTTCAGC.
HBsAg IC₅₀ and HBV DNA IC₅₀ values were derived from the dose-response curve using 4 parameter logistic curve fit method. The compounds of formula (I) have HBsAg IC₅₀ <20 µM, particularly <1 µM; and HBV DNA IC₅₀ < 50 µM. Results of Cryopreserved PHH assay are given in TableBIO2.

**Table BIO2: HBsAg IC₅₀ data in Cryopreserved PHH assay**

| **Example No.** | **HBsAg IC50 (µM)** | **Example No.** | **HBsAg IC50 (µM)** | **Example No.** | **HBsAg IC50 (µM)** |
|---|---|---|---|---|---|
| **001** | 5.43 | **002** | 4.01 | **005** | 8.34 |
| **006** | 3.82 | **007** | 4.95 | **013** | 7.35 |
| **014** | 5.08 | **015** | 9.75 | **016** | 7.64 |
| **017** | 3.42 | **018** | 5.13 | **019** | 7.13 |
| **020** | 8.36 | **022** | 6.80 | **023** | 4.95 |
| **025** | 7.63 | **027** | 5.85 | **041** | 5.48 |
| **042** | 7.30 | **047** | 2.83 | **049** | 7.81 |
| **052** | 5.82 | **064** | 6.41 | **066** | 9.03 |
| **073** | 7.47 | **075** | 3.57 | **076** | 7.95 |
| **077** | 2.18 | **080** | 4.02 | **082** | 7.56 |
| **084** | 3.48 | **087** | 7.84 | **088** | 7.80 |
| **089** | 5.81 | **093** | 6.13 | **098** | 6.34 |
| **101** | 1.41 | **116** | 3.41 | **117** | 1.90 |
| **119** | 0.67 | **125** | 3.19 | **126** | 4.21 |
| **131** | 5.05 | **133** | 2.80 | **146** | 7.08 |
| **157** | 3.40 | **160** | 10.00 | **164** | 9.66 |
| **165** | 9.98 | **166** | 3.03 | **167** | 7.94 |

## Claims

1. A compound of the formula (I), 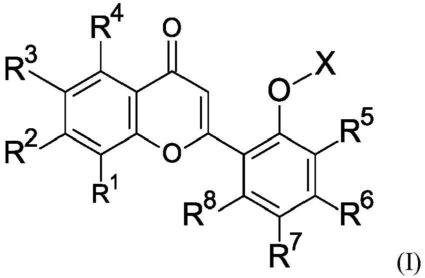 wherein
R¹ is halogen;
R² is selected from H, OH, halogen, C₁₋₆alkyl, haloC₁₋₆alkyl and C₁₋₆alkoxy;
R³ is selected from H, OH, halogen, C₁₋₆alkyl, haloC₁₋₆alkyl and C₁₋₆alkoxy;
R⁴ is selected from H, OH, halogen, C₁₋₆alkyl, haloC₁₋₆alkyl and C₁₋₆alkoxy;
R⁵ is selected from H, OH, halogen, C₁₋₆alkyl, haloC₁₋₆alkyl and C₁₋₆alkoxy;
R⁶ is selected from H, OH, halogen, C₁₋₆alkyl, haloC₁₋₆alkyl and C₁₋₆alkoxy;
R⁷ is selected from H, OH, halogen, C₁₋₆alkyl, haloC₁₋₆alkyl and C₁₋₆alkoxy;
R⁸ is selected from H, OH, halogen, C₁₋₆alkyl, haloC₁₋₆alkyl and C₁₋₆alkoxy;
X is 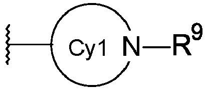 or -L-Y; wherein
Cyl is N-containing heterocyclyl;
R⁹ is selected from C₃₋₇cycloalkylsulfonyl, carboxycarbonyl, carboxyphenylC₁₋₆alkyl and carboxyC₃₋₇cycloalkyl;
L is selected from C₁₋₆alkyl, C₁₋₆alkoxyC₁₋₆alkyl, C₃₋₇cycloalkyl, C₃₋₇cycloalkylC₁₋₆alkyl and C₁₋₆alkylheterocyclylC₁₋₆alkyl; wherein C₁₋₆alkyl is unsubstituted or substituted by OH;
Y is -NR¹⁰R¹¹ or 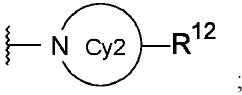 wherein
Cy2 is N-containing heterocyclyl; wherein N-containing heterocyclyl is unsubstituted or substituted by one or two or three substituents independently selected from halogen, C₁₋₆alkyl and OH;
R¹⁰ is selected from H, C₁₋₆alkyl, C₃₋₇cycloalkyl, C₁₋₆alkylsulfonyl, C₃₋₇cycloalkylsulfonyl, carboxyC₁₋₆alkyl, phenylsulfonyl and C₁₋₆alkylcarbonyl;
R¹¹ is selected from carboxyC₃₋₇cycloalkyl, carboxyC₁₋₆alkyl, carboxycarbonyl, carboxyheterocyclyl, carboxyC₃₋₇cycloalkylC₁₋₆alkyl, heterocyclyl, C₁₋₆alkylsulfonylC₁₋₆alkyl, aminosulfonylC₁₋₆alkyl, C₃₋₇cycloalkylsulfonyl, C₁₋₆alkoxycarbonylcarbonyl, phenylsulfonyl, C₁₋₆alkylcarbonyl, C₁₋₆alkoxycarbonyl, C₁₋₆alkylaminosulfonyl, aminocarbonylcarbonyl, C₃₋₇cycloalkylaminocarbonylcarbonyl, C₃₋₇cycloalkylsulfonylaminocarbonylcarbonyl, hydroxyheterocyclylcarbonylcarbonyl, carboxyC₁₋₆alkylaminocarbonyl, C₁₋₆alkylphenylsulfonylaminocarbonyl, aminocarbonyl, aminosulfonyl and aminocarbonylC₁₋₆alkyl;
R¹² is selected from H, carboxy, carboxyC₁₋₆alkyl, C₁₋₆alkylsulfonylaminocarbonyl, C₃₋₇cycloalkylsulfonylaminocarbonyl, C₁₋₆alkyl(C₁₋₆alkylsulfonyl)amino, C₁₋₆alkylsulfonyl, C₁₋₆alkylcarbonyl, C₁₋₆alkylsulfinyl, C₁₋₆alkylsulfonimidoyl, aminocarbonyl, carboxycarbonyl and heterocyclyl;
or a pharmaceutically acceptable salt thereof.

2. A compound according to claim 1, wherein
R¹ is halogen;
R² is H;
R³ is selected from H, halogen and C₁₋₆alkoxy;
R⁴ is selected from H and haloC₁₋₆alkyl;
R⁵ is H;
R⁶ is selected from H, halogen, C₁₋₆alkyl, haloC₁₋₆alkyl and C₁₋₆alkoxy;
R⁷ is selected from H, halogen, C₁₋₆alkyl and C₁₋₆alkoxy;
R⁸ is selected from H, halogen and C₁₋₆alkoxy;
X is 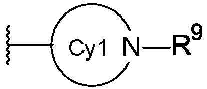 or -L-Y; wherein
Cy1 is selected from piperidyl, pyrrolidinyl and azetidinyl;
R⁹ is selected from C₃₋₇cycloalkylsulfonyl, carboxycarbonyl, carboxyphenylC₁₋₆alkyl and carboxyC₃₋₇cycloalkyl;
L is selected from C₁₋₆alkyl, C₁₋₆alkoxyC₁₋₆alkyl, C₃₋₇cycloalkyl, C₃₋₇cycloalkylC₁₋₆alkyl and C₁₋₆alkyloxetanylC₁₋₆alkyl; wherein C₁₋₆alkyl is unsubstituted or substituted by OH;
Y is -NR¹⁰R¹¹ or 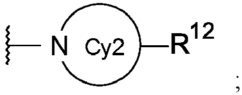 wherein
Cy2 is selected from pyrrolidinyl, morpholinyl, azetidinyl, piperidyl, azabicyclo[3.2.1]octanyl, oxopyrrolidinyl, piperazinyl, thiomorpholinyl, dioxothiazinanyl, oxoimidazolidinyl and dioxoimidazolidinyl; wherein pyrrolidinyl is unsubstituted or substituted by one or two substituents independently selected from halogen, C₁₋₆alkyl and OH;
R¹⁰ is selected from H, C₁₋₆alkyl, C₃₋₇cycloalkyl, C₁₋₆alkylsulfonyl, C₃₋₇cycloalkylsulfonyl, carboxyC₁₋₆alkyl, phenylsulfonyl and C₁₋₆alkylcarbonyl;
R¹¹ is selected from carboxyC₃₋₇cycloalkyl, carboxyC₁₋₆alkyl, carboxycarbonyl, carboxytetrahydropyranyl, carboxyC₃₋₇cycloalkylC₁₋₆alkyl, dioxothiazinanyl, C₁₋₆alkylsulfonylC₁₋₆alkyl, aminosulfonylC₁₋₆alkyl, C₃₋₇cycloalkylsulfonyl, C₁₋₆alkoxycarbonylcarbonyl, phenylsulfonyl, C₁₋₆alkylcarbonyl, C₁₋₆alkoxycarbonyl, C₁₋₆alkylaminosulfonyl, aminocarbonylcarbonyl, C₃₋₇cycloalkylaminocarbonylcarbonyl, C₃₋₇cycloalkylsulfonylaminocarbonylcarbonyl, hydroxypyrrolidinylcarbonylcarbonyl, carboxyC₁₋₆alkylaminocarbonyl, C₁₋₆alkylphenylsulfonylaminocarbonyl, aminocarbonyl, aminosulfonyl and aminocarbonylC₁₋₆alkyl;
R¹² is selected from H, carboxy, carboxyC₁₋₆alkyl, C₁₋₆alkylsulfonylaminocarbonyl, C₃₋₇cycloalkylsulfonylaminocarbonyl, C₁₋₆alkyl(C₁₋₆alkylsulfonyl)amino, C₁₋₆alkylsulfonyl, C₁₋₆alkylcarbonyl, C₁₋₆alkylsulfinyl, C₁₋₆alkylsulfonimidoyl, aminocarbonyl, carboxycarbonyl and 2H-tetrazolyl;
or a pharmaceutically acceptable salt thereof.

3. A compound according to claim 1 or 2, or a pharmaceutically acceptable salt thereof, wherein R³ is selected from H.

4. A compound according to any one of claims 1, 2 and 3, or a pharmaceutically acceptable salt thereof, wherein R⁶ is selected from halogen, C₁₋₆alkyl and haloC₁₋₆alkyl.

5. A compound according to any one of claims 1, 2, 3 and 4, or a pharmaceutically acceptable salt thereof, R⁷ is selected from Hand C₁₋₆alkoxy.

6. A compound according to any one of claims 1, 2, 3, 4 and 5, or a pharmaceutically acceptable salt thereof, wherein X is -L-Y.

7. A compound according to any one of claims 1, 2, 3, 4, 5 and 6, or a pharmaceutically acceptable salt thereof, wherein L is selected from C₁₋₆alkyl and C₁₋₆alkoxyC₁₋₆alkyl; wherein C₁₋₆alkyl is unsubstituted or substituted by OH.

8. A compound according to any one of claims 1, 2, 3, 4, 5, 6 and 7, or a pharmaceutically acceptable salt thereof, wherein Y is 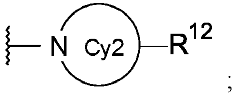 wherein Cy2 is selected from pyrrolidinyl and morpholinyl.

9. A compound according to any one of claims 1, 2, 3, 4, 5, 6 and 7, or a pharmaceutically acceptable salt thereof, wherein Y is -NHR¹¹.

10. A compound according to any one of claims 1 to 2, selected from
1-[3-[5-bromo-2-(8-chloro-4-oxo-chromen-2-yl)phenoxy]propyl]pyrrolidine-3-carboxylic acid;
4-[3-[5-bromo-2-(8-chloro-4-oxo-chromen-2-yl)phenoxy]propyl]morpholine-2-carboxylic acid;
(2R)-1-[3-[5-bromo-2-(8-chloro-4-oxo-chromen-2-yl)phenoxy]propyl]pyrrolidine-2-carboxylic acid;
(2S)-1-[3-[5-bromo-2-(8-chloro-4-oxo-chromen-2-yl)phenoxy]propyl]pyrrolidine-2-carboxylic acid;
(3R)-1-[3-[5-bromo-2-(8-chloro-4-oxo-chromen-2-yl)phenoxy]propyl]pyrrolidine-3-carboxylic acid;
3-[3-[2-(8-chloro-4-oxo-chromen-2-yl)-5-(trifluoromethyl)phenoxypropylamino]cyclobutanecarboxylic acid;
3-[3-[2-(8-chloro-4-oxo-chromen-2-yl)-5-(trifluoromethyl)phenoxy]propylamino]cyclopentanecarboxylic acid;
2-[1-[3-[2-(8-chloro-4-oxo-chromen-2-yl)-5-(trifluoromethyl)phenoxy]propyl]pyrrolidin-3-yl]acetic acid;
(2R)-2-[3-[2-(8-chloro-4-oxo-chromen-2-yl)-5-(trifluoromethyl)phenoxy]propylamino]-3-methyl-butanoic acid;
2-[[3-[[2-(8-chloro-4-oxo-chromen-2-yl)-5-(trifluoromethyl)phenoxy]methyl]oxetan-3-yl]methylamino]-2-oxo-acetic acid;
2-[3-[2-(8-chloro-4-oxo-chromen-2-yl)-5-(trifluoromethyl)phenoxy]propylamino]-4,4-dimethyl-pentanoic acid;
1-[3-[2-(8-chloro-4-oxo-chromen-2-yl)-5-(trifluoromethyl)phenoxy]propyl]pyrrolidine-3-carboxylic acid;
(3R)-1-[3-[2-(8-chloro-4-oxo-chromen-2-yl)-5-(trifluoromethyl)phenoxy]propyl]pyrrolidine-3-carboxylic acid;
(3S)-1-[3-[2-(8-chloro-4-oxo-chromen-2-yl)-5-(trifluoromethyl)phenoxy]propyl]pyrrolidine-3-carboxylic acid;
(3S)-1-[3-[2-(8-chloro-4-oxo-chromen-2-yl)-5-ethoxy-phenoxy]propyl]pyrrolidine-3-carboxylic acid;
(3R)-1-[3-[5-bromo-2-(8-chloro-4-oxo-chromen-2-yl)-4-methoxy-phenoxy]propyl]pyrrolidine-3-carboxylic acid;
(3S)-1-[3-[5-bromo-2-(8-chloro-4-oxo-chromen-2-yl)-4-methoxy-phenoxy]propyl]pyrrolidine-3-carboxylic acid;
4-[3-[2-(8-chloro-4-oxo-chromen-2-yl)-5-(trifluoromethyl)phenoxy]propyl]morpholine-2-carboxylic acid;
1-[2-[5-bromo-2-(8-chloro-4-oxo-chromen-2-yl)phenoxy]ethyl]azetidine-3-carboxylic acid;
1-[2-[5-bromo-2-(8-chloro-4-oxo-chromen-2-yl)phenoxy]ethyl]pyrrolidine-2-carboxylic acid;
1-[2-[5-bromo-2-(8-chloro-4-oxo-chromen-2-yl)phenoxy]ethyl]pyrrolidine-3-carboxylic acid;
1-[2-[5-bromo-2-(8-chloro-4-oxo-chromen-2-yl)phenoxy]ethyl]piperidine-4-carboxylic acid;
4-[2-[5-bromo-2-(8-chloro-4-oxo-chromen-2-yl)phenoxy]ethyl]morpholine-2-carboxylic acid;
(3 S,4S)-1-[2-[5-bromo-2-(8-chloro-4-oxo-chromen-2-yl)phenoxy]ethyl]-4-methyl-pyrrolidine-3-carboxylic acid;
8-[2-[5-bromo-2-(8-chloro-4-oxo-chromen-2-yl)phenoxy]ethyl]-8-azabicyclo[3.2.1]octane-3-carboxylic acid;
3-[2-[5-bromo-2-(8-chloro-4-oxo-chromen-2-yl)phenoxy]ethylamino]bicyclo[1.1.1]pentane-1-carboxylic acid;
1-[2-[5-bromo-2-(8-chloro-4-oxo-chromen-2-yl)phenoxy]ethyl]-4,4-difluoro-pyrrolidine-2-carboxylic acid;
1-[2-[2-(8-chloro-4-oxo-chromen-2-yl)phenoxy]ethyl]piperidine-4-carboxylic acid;
(2R)-1-[2-[2-(8-chloro-4-oxo-chromen-2-yl)phenoxy]ethyl]pyrrolidine-2-carboxylic acid;
1-[2-[2-(8-chloro-4-oxo-chromen-2-yl)phenoxy]ethyl]pyrrolidine-3-carboxylic acid;
(2S)-1-[2-[2-(8-chloro-4-oxo-chromen-2-yl)phenoxy]ethyl]pyrrolidine-2-carboxylic acid;
1-[2-[2-(8-chloro-4-oxo-chromen-2-yl)phenoxy]ethyl]azetidine-3-carboxylic acid;
(3 S)-1-[2-[2-(8-chloro-4-oxo-chromen-2-yl)-5-(trifluoromethyl)phenoxy] ethyl]pyrrolidine-3-carboxylic acid;
4-[2-[2-(8-chloro-4-oxo-chromen-2-yl)-5-(trifluoromethyl)phenoxy]ethyl]morpholine-2-carboxylic acid;
(2R)-2-[2-[2-(8-chloro-4-oxo-chromen-2-yl)-5-(trifluoromethyl)phenoxy]ethylamino]-3-methylbutanoic acid;
(2S)-2-[2-[2-(8-chloro-4-oxo-chromen-2-yl)-5-(trifluoromethyl)phenoxy]ethylamino]-3-methylbutanoic acid;
3-[2-[2-(8-chloro-4-oxo-chromen-2-yl)-5-(trifluoromethyl)phenoxy]ethylamino]cyclobutanecarboxylic acid;
(3R)-1-[2-[2-(8-chloro-4-oxo-chromen-2-yl)-5-isobutoxy-phenoxy]ethyl]pyrrolidine-3-carboxylic acid;
(3S)-1-[2-[2-(8-chloro-4-oxo-chromen-2-yl)-5-isobutoxy-phenoxy]ethyl]pyrrolidine-3-carboxylic acid;
(3R)-1-[2-[5-bromo-2-[8-chloro-4-oxo-5-(trifluoromethyl)chromen-2-yl]phenoxy]ethyl]pyrrolidine-3-carboxylic acid;
(3S)-1-[2-[5-bromo-2-[8-chloro-4-oxo-5-(trifluoromethyl)chromen-2-yl]phenoxy]ethyl]pyrrolidine-3-carboxylic acid;
1-[2-[2-(8-chloro-6-fluoro-4-oxo-chromen-2-yl)phenoxy]ethyl]pyrrolidine-3-carboxylic acid;
(2R)-1-[2-[5-bromo-2-(8-chloro-4-oxo-chromen-2-yl)-4-methyl-phenoxy]ethyl]pyrrolidine-2-carboxylic acid;
(2S)-1-[2-[5-chloro-2-(8-chloro-4-oxo-chromen-2-yl)-4-methyl-phenoxy]ethyl]pyrrolidine-2-carboxylic acid;
3-[3-[5-chloro-2-(8-chloro-4-oxo-chromen-2-yl)-4-methyl-phenoxy]propylamino]cyclobutanecarboxylic acid;
(3R)-1-[2-[2-[5-bromo-2-(8-chloro-4-oxo-chromen-2-yl)phenoxy]ethoxy]ethyl]pyrrolidine-3-carboxylic acid;
(2R)-1-[2-[2-[5-bromo-2-(8-chloro-4-oxo-chromen-2-yl)-4-methyl-phenoxy]ethoxy]ethyl]pyrrolidine-2-carboxylic acid;
4-[2- [2-[5 -bromo-2-(8-chloro-4-oxo-chromen-2-yl)phenoxy] ethoxy] ethyl] morpholine-2-carboxylic acid;
(3R)-1-[2-[2-[2-(8-chloro-4-oxo-chromen-2-yl)-5-methoxy-4-methyl-phenoxy]ethoxy]ethyl]pyrrolidine-3-carboxylic acid;
(3S)-1-[2-[2-[2-(8-chloro-4-oxo-chromen-2-yl)-5-methoxy-4-methyl-phenoxy]ethoxy]ethyl]pyrrolidine-3-carboxylic acid;
4-[2-[5-bromo-2-(8-chloro-4-oxo-chromen-2-yl)phenoxy]ethyl]morpholine-3-carboxylic acid;
3-[2-[2-(8-chloro-4-oxo-chromen-2-yl)-5-methyl-phenoxy]ethylamino]cyclobutanecarboxylic acid;
1-[2-[2-(8-chloro-4-oxo-chromen-2-yl)-5-methyl-phenoxy]ethyl]azetidine-3-carboxylic acid;
cis-4-[2-[5-bromo-2-(8-chloro-4-oxo-chromen-2-yl)phenoxy]ethylamino]cyclohexanecarboxylic acid;
trans-4-[2-[5-bromo-2-(8-chloro-4-oxo-chromen-2-yl)phenoxy]ethylamino]cyclohexanecarboxylic acid;
2-[2-[2-(8-chloro-4-oxo-chromen-2-yl)-5-(trifluoromethyl)phenoxy]ethylamino]propanoic acid;
2-[2-[2-(8-chloro-4-oxo-chromen-2-yl)-5-methyl-phenoxy]ethylamino]-2-methyl-propanoic acid;
3-[3-[2-(8-chloro-4-oxo-chromen-2-yl)-5-methyl-phenoxy]propylamino]cyclobutanecarboxylic acid;
cis-4-[3-[5-bromo-2-(8-chloro-4-oxo-chromen-2-yl)phenoxy]propylamino]tetrahydropyran-2-carboxylic acid;
1-[2-[5-chloro-2-(8-chloro-4-oxo-chromen-2-yl)-4-methyl-phenoxy]ethyl]azetidine-3-carboxylic acid;
3-[3-[2-(8-chloro-4-oxo-chromen-2-yl)-5-(trifluoromethyl)phenoxy]propylamino]butanoic acid;
2-[3-[2-(8-chloro-4-oxo-chromen-2-yl)-5-(trifluoromethyl)phenoxy]propylamino]-3-cyclohexyl-propanoic acid;
(3R)-1-[2-[5-bromo-2-(8-chloro-4-oxo-chromen-2-yl)phenoxy]ethyl]-N-methylsulfonyl-pyrrolidine-3-carboxamide;
(3R)-1-[3-[2-(8-chloro-4-oxo-chromen-2-yl)-5-(trifluoromethyl)phenoxy]propyl]-N-methylsulfonyl-pyrrolidine-3-carboxamide;
(3S)-1-[3-[2-(8-chloro-4-oxo-chromen-2-yl)-5-(trifluoromethyl)phenoxy]propyl]-N-methylsulfonyl-pyrrolidine-3-carboxamide;
(3R)-1-[3-[2-(8-chloro-4-oxo-chromen-2-yl)-5-(trifluoromethyl)phenoxy]propyl]-N-cyclopropylsulfonyl-pyrrolidine-3-carboxamide;
4-[2-[2-(8-chloro-4-oxo-chromen-2-yl)-5-(trifluoromethyl)phenoxy]ethyl]-N-methylsulfonyl-morpholine-2-carboxamide;
(3 S)-1-[2-[5-bromo-2-(8-chloro-4-oxo-chromen-2-yl)phenoxy]ethyl]-N-methylsulfonyl-pyrrolidine-3-carboxamide;
(3R)-1-[2-[2-(8-chloro-4-oxo-chromen-2-yl)-5-(trifluoromethyl)phenoxy]ethyl]-N-methylsulfonyl-pyrrolidine-3-carboxamide;
(3 S)-1-[2-[2-(8-chloro-4-oxo-chromen-2-yl)-5-(trifluoromethyl)phenoxy] ethyl]-N-methylsulfonyl-pyrrolidine-3-carboxamide;
1-[2-[2-(8-chloro-4-oxo-chromen-2-yl)-5-(trifluoromethyl)phenoxy]ethyl]-N-cyclopropylsulfonyl-pyrrolidine-2-carboxamide;
1-[2-[2-(8-chloro-4-oxo-chromen-2-yl)-5-(trifluoromethyl)phenoxy]ethyl]-N-methylsulfonyl-pyrrolidine-2-carboxamide;
1-[2-[5-bromo-2-(8-chloro-4-oxo-chromen-2-yl)phenoxy]ethyl]-N-methylsulfonyl-pyrrolidine-2-carboxamide;
1-[2-[5-bromo-2-(8-chloro-4-oxo-chromen-2-yl)phenoxy]ethyl]pyrrolidin-2-one;
N-[1-[2-[5-bromo-2-(8-chloro-4-oxo-chromen-2-yl)phenoxy]ethyl]pyrrolidin-3-yl]-N-methyl-methanesulfonamide;
8-chloro-2-[2-[3-[(3R,4S)-3,4-dihydroxypyrrolidin-1-yl]propoxy]-4-(trifluoromethyl)phenyl]chromen-4-one;
8-chloro-2-[2-[3-[(1,1-dioxothian-4-yl)amino]propoxy]-4-(trifluoromethyl)phenyl]chromen-4-one;
8-chloro-2-[2-[3-(2-methylsulfonylethylamino)propoxy]-4-(trifluoromethyl)phenyl]chromen-4-one;
2-[3-[2-(8-chloro-4-oxo-chromen-2-yl)-5-(trifluoromethyl)phenoxy]propylamino]ethanesulfonamide;
8-chloro-2-[2-[3-(4-methylsulfonylpiperazin-1-yl)propoxy]-4-(trifluoromethyl)phenyl]chromen-4-one;
2-[2-[2-(4-acetylpiperazin-1-yl)ethoxy]-4-bromo-phenyl]-8-chloro-chromen-4-one;
1-[2-[5-bromo-2-(8-chloro-4-oxo-chromen-2-yl)phenoxy]ethyl]pyrrolidine-3-carboxamide;
2-[4-bromo-2-[2-(3-methylsulfonylpyrrolidin-1-yl)ethoxy]phenyl]-8-chloro-chromen-4-one;
4-[2-[5-bromo-2-(8-chloro-4-oxo-chromen-2-yl)phenoxy]ethyl]thiomorpholine-3-carboxamide;
N-[3-[2-(8-chloro-4-oxo-chromen-2-yl)-5-(trifluoromethyl)phenoxy]-2-hydroxypropyl]cyclopropanesulfonamide;
8-chloro-2-[2-[2-[4-(1H-tetrazol-5-yl)-1-piperidyl]ethoxy]-4-(trifluoromethyl)phenyl]chromen-4-one;
8-chloro-2-[2-[3-(3-methylsulfonylpyrrolidin-1-yl)propoxy]-4-(trifluoromethyl)phenyl]chromen-4-one;
2-[4-bromo-2-[2-(1,1-dioxo-1,4-thiazinan-4-yl)ethoxy]phenyl]-8-chloro-chromen-4-one;
2-[4-bromo-2-[2-(2-morpholinoethoxy)ethoxy]phenyl]-8-chloro-chromen-4-one;
1-[2-[2-(8-chloro-4-oxo-chromen-2-yl)-5-methyl-phenoxy]ethyl]imidazolidin-2-one;
1-[2-[2-(8-chloro-4-oxo-chromen-2-yl)-5-methyl-phenoxy]ethyl]-3-methyl-imidazolidin-2-one;
8-chloro-2-[2-[2-(4-methylsulfinyl-1-piperidyl)ethoxy]-4-(trifluoromethyl)phenyl]chromen-4-one;
8-chloro-2-[2-[2-[4-(methylsulfonimidoyl)-1-piperidyl]ethoxy]-4-(trifluoromethyl)phenyl]chromen-4-one;
ethyl 2-[3-[2-(8-chloro-4-oxo-chromen-2-yl)-5-methyl-phenoxy]propylamino]-2-oxo-acetate;
N-[2-[2-(8-chloro-4-oxo-chromen-2-yl)-5-(trifluoromethyl)phenoxy]ethyl]cyclopropanesulfonamide;
N-[3-[2-(8-chloro-4-oxo-chromen-2-yl)-5-(trifluoromethyl)phenoxy]propyl]benzenesulfonamide;
N-[3-[2-(8-chloro-4-oxo-chromen-2-yl)-5-(trifluoromethyl)phenoxy]propyl]acetamide;
N-[3-[2-(8-chloro-4-oxo-chromen-2-yl)-5-(trifluoromethyl)phenoxy]propyl]propanamide;
ethyl 2-[3-[2-(8-chloro-4-oxo-chromen-2-yl)-5-(trifluoromethyl)phenoxy]propylamino]-2-oxo-acetate;
ethyl 2-[2-[2-(8-chloro-4-oxo-chromen-2-yl)-5-(trifluoromethyl)phenoxy]ethylamino]-2-oxo-acetate;
ethyl 2-[2-[4-bromo-2-(8-chloro-4-oxo-chromen-2-yl)-5-methoxy-phenoxy]ethylamino]-2-oxo-acetate;
ethyl 2-[2-[2-(8-chloro-6-methoxy-4-oxo-chromen-2-yl)-5-methyl-phenoxy]ethylamino]-2-oxo-acetate;
ethyl 2-[2-[2-(8-chloro-4-oxo-chromen-2-yl)-5-methoxy-phenoxy]ethylamino]-2-oxo-acetate;
methyl N-[2-[5-bromo-2-(8-chloro-4-oxo-chromen-2-yl)phenoxy]ethyl]carbamate;
ethyl N-[2-[5-bromo-2-(8-chloro-4-oxo-chromen-2-yl)phenoxy]ethyl]carbamate;
8-chloro-2-[4-methyl-2-[3-(methylsulfamoylamino)propoxy]phenyl]chromen-4-one;
2-[4-bromo-2-[(1-cyclopropylsulfonyl-4-piperidyl)oxy]phenyl]-8-chloro-chromen-4-one;
ethyl 2-[2-[2-(8-chloro-4-oxo-chromen-2-yl)-5-methyl-phenoxy]ethylamino]-2-oxo-acetate;
2-[[1-[[2-(8-chloro-4-oxo-chromen-2-yl)-5-(trifluoromethyl)phenoxy]methyl]cyclopropyl]amino]-2-oxo-acetic acid;
2-[2-[2-(8-chloro-4-oxo-chromen-2-yl)-5-methoxy-phenoxy]ethylamino]-2-oxo-acetic acid;
2-[2-[2-(8-chloro-4-oxo-chromen-2-yl)-3-fluoro-5-methyl-phenoxy]ethylamino]-2-oxo-acetic acid;
2-[2-[5-bromo-2-(8-chloro-4-oxo-chromen-2-yl)phenoxy]ethylamino]-2-oxo-acetic acid;
2-[2-[2-(8-chloro-4-oxo-chromen-2-yl)-4,5-dimethoxy-phenoxy]ethylamino]-2-oxo-acetic acid;
2-[[2-[2-(8-chloro-4-oxo-chromen-2-yl)-5-(trifluoromethyl)phenoxy]-1,1-dimethylethyl]amino]-2-oxo-acetic acid;
2-[3-[2-(8-chloro-6-methoxy-4-oxo-chromen-2-yl)-5-(trifluoromethyl)phenoxy]propylamino]-2-oxo-acetic acid;
2-[3-[2-(8-chloro-4-oxo-chromen-2-yl)-5-(trifluoromethyl)phenoxy]propylamino]-2-oxo-acetic acid;
2-[2-[2-(8-chloro-4-oxo-chromen-2-yl)-3-methoxy-5-methyl-phenoxy]ethylamino]-2-oxo-acetic acid;
2-[2-[2-(8-chloro-4-oxo-chromen-2-yl)-5-(trifluoromethyl)phenoxy]ethylamino]-2-oxo-acetic acid;
2-[3-[2-(8-chloro-4-oxo-chromen-2-yl)-5-methyl-phenoxy]propylamino]-2-oxo-acetic acid;
2-[2-[2-(8-chloro-4-oxo-chromen-2-yl)-5-methyl-phenoxy]ethylamino]-2-oxo-acetic acid;
2-[2-[4-bromo-2-(8-chloro-4-oxo-chromen-2-yl)-5-methoxy-phenoxy]ethylamino]-2-oxo-acetic acid;
2-[2-[2-(8-chloro-4-oxo-chromen-2-yl)-5-(trifluoromethyl)phenoxy]ethylamino]acetic acid;
3-[2-[2-(8-chloro-4-oxo-chromen-2-yl)-5-(trifluoromethyl)phenoxy]ethylamino]propanoic acid;
2-[3-[2-(8-chloro-4-oxo-chromen-2-yl)-5-(trifluoromethyl)phenoxy]propylamino]acetic acid;
2-oxo-2-[(3S)-3-[2-(8-chloro-4-oxo-chromen-2-yl)-5-(trifluoromethyl)phenoxy]pyrrolidin-1-yl]acetic acid;
2-[3-[2-(8-chloro-4-oxo-chromen-2-yl)-5-(trifluoromethyl)phenoxy]azetidin-1-yl]-2-oxo-acetic acid;
3-[[4-[5-bromo-2-(8-chloro-4-oxo-chromen-2-yl)phenoxy]-1-piperidyl]methyl]benzoic acid;
2-[4-[2-(8-chloro-4-oxo-chromen-2-yl)-5-(trifluoromethyl)phenoxy]-1-piperidyl]-2-oxo-acetic acid;
2-oxo-2-[(3R)-3-[2-(8-chloro-4-oxo-chromen-2-yl)-5-(trifluoromethyl)phenoxy]pyrrolidin-1-yl]acetic acid;
3-[4-[5-bromo-2-(8-chloro-4-oxo-chromen-2-yl)phenoxy]-1-piperidyl]cyclobutanecarboxylic acid;
2-[(3S)-3-[2-(8-chloro-4-oxo-chromen-2-yl)-5-methyl-phenoxy]pyrrolidin-1-yl]-2-oxo-acetic acid;
2-[[3-[2-(8-chloro-4-oxo-chromen-2-yl)-5-(trifluoromethyl)phenoxy]cyclobutyl]amino]-2-oxo-acetic acid;
2-[3-[2-(8-chloro-4-oxo-chromen-2-yl)-5-(trifluoromethyl)phenoxy]propyl-methyl-amino]acetic acid;
2-[2-[2-(8-chloro-4-oxo-chromen-2-yl)-5-methyl-phenoxy]ethyl-cyclopropyl-amino]-2-oxo-acetic acid;
8-chloro-2-[2-[2-[ethylsulfamoyl(methyl)amino]ethoxy]-4-methyl-phenyl]-4-oxo-chromene;
ethyl 2-[2-[2-(8-chloro-4-oxo-chromen-2-yl)-5-methyl-phenoxy]ethyl-methyl-amino]-2-oxo-acetate;
ethyl 2-[3-[2-(8-chloro-4-oxo-chromen-2-yl)-5-(trifluoromethyl)phenoxy]propyl-ethylsulfonyl-amino]-2-oxo-acetate;
3-[3-[2-(8-chloro-4-oxo-chromen-2-yl)-5-(trifluoromethyl)phenoxy]propyl-cyclopropylsulfonyl-amino]cyclobutanecarboxylic acid;
2-[carboxymethyl-[3-[2-(8-chloro-4-oxo-chromen-2-yl)-5-(trifluoromethyl)phenoxy]propyl] amino] acetic acid;
cis-3-[benzenesulfonyl-[3-[2-(8-chloro-4-oxo-chromen-2-yl)-5-(trifluoromethyl)phenoxy]propyl]amino]cyclobutanecarboxylic acid;
trans-3-[benzenesulfonyl-[3-[2-(8-chloro-4-oxo-chromen-2-yl)-5-(trifluoromethyl)phenoxy]propyl]amino]cyclobutanecarboxylic acid;
2-[benzenesulfonyl-[3-[2-(8-chloro-4-oxo-chromen-2-yl)-5-(trifluoromethyl)phenoxy]propyl] amino] acetic acid;
2-[acetyl-[3-[2-(8-chloro-4-oxo-chromen-2-yl)-5-(trifluoromethyl)phenoxy]propyl]amino]acetic acid;
2-[3-[2-(8-chloro-4-oxo-chromen-2-yl)-5-(trifluoromethyl)phenoxy]propyl-cyclopropylsulfonyl-amino]acetic acid;
N'-[2-[2-(8-chloro-4-oxo-chromen-2-yl)-5-(trifluoromethyl)phenoxy]ethyl]oxamide;
N'-[2-[2-(8-chloro-4-oxo-chromen-2-yl)-5-methoxy-phenoxy]ethyl]oxamide;
N-[2-[2-(8-chloro-4-oxo-chromen-2-yl)-5-(trifluoromethyl)phenoxy]ethyl]-N'-cyclopropyl-oxamide;
N-[3-[2-(8-chloro-4-oxo-chromen-2-yl)-5-(trifluoromethyl)phenoxy]propyl]-N'-cyclopropylsulfonyl-oxamide;
N-[2-[2-(8-chloro-4-oxo-chromen-2-yl)-5-methyl-phenoxy]ethyl]-2-[(3S)-3-hydroxypyrrolidin-1-yl]-2-oxo-acetamide;
(2S)-2-[2-[2-(8-chloro-4-oxo-chromen-2-yl)-5-methyl-phenoxy]ethylcarbamoylamino]propanoic acid;
(5S)-3-[2-[2-(8-chloro-4-oxo-chromen-2-yl)-5-methyl-phenoxy]ethyl]-5-isopropyl-imidazolidine-2,4-dione;
(5S)-3-[2-[2-(8-chloro-4-oxo-chromen-2-yl)-5-(trifluoromethyl)phenoxy]ethyl]-5-isopropyl-imidazolidine-2,4-dione;
2-[2-[2-(8-chloro-4-oxo-chromen-2-yl)-5-methyl-phenoxy]ethylcarbamoylamino]acetic acid;
(5S)-3-[2-[2-(8-chloro-4-oxo-chromen-2-yl)-5-(trifluoromethyl)phenoxy]ethyl]-5-methylimidazolidine-2,4-dione;
1-[3-[2-(8-chloro-4-oxo-chromen-2-yl)-5-methyl-phenoxy]propyl]-3-(p-tolylsulfonyl)urea;
3-[2-(8-chloro-4-oxo-chromen-2-yl)-5-(trifluoromethyl)phenoxy]propylurea;
1-[2-[2-(8-chloro-4-oxo-chromen-2-yl)-5-(trifluoromethyl)phenoxy]ethyl]-3-(p-tolylsulfonyl)urea;
8-chloro-4-oxo-2-[2-[2-(sulfamoylamino)ethoxy]-4-(trifluoromethyl)phenyl]chromene;
8-chloro-4-oxo-2-[2-[3-(sulfamoylamino)propoxy]-4-(trifluoromethyl)phenyl]chromene;
8-chloro-2-[4-methyl-2-[2-(sulfamoylamino)ethoxy]phenyl]-4-oxo-chromene;
2-[3-[2-[2-(8-chloro-4-oxo-chromen-2-yl)-5-(trifluoromethyl)phenoxy]ethyl]-2-oxo-imidazolidin-1-yl]-2-oxo-acetic acid;
(3S)-1-[3-[2-(8-chloro-4-oxo-chromen-2-yl)-5-(trifluoromethyl)phenoxy]-2-hydroxy-propyl]pyrrolidine-3-carboxylic acid;
(3S)-1-[3-[5-bromo-2-(8-chloro-4-oxo-chromen-2-yl)phenoxy]-2-hydroxy-propyl]pyrrolidine-3-carboxylic acid;
(3R)-1-[3-[5-bromo-2-(8-chloro-4-oxo-chromen-2-yl)phenoxy]-2-hydroxy-propyl]pyrrolidine-3-carboxylic acid;
2-[[3-[2-(8-chloro-4-oxo-chromen-2-yl)-5-(trifluoromethyl)phenoxy]-2-hydroxy-propyl]amino]-2-oxo-acetic acid;
2-[2-[2-(8-chloro-4-oxo-chromen-2-yl)-5-(trifluoromethyl)phenoxy]ethylamino]acetamide;
2-[2-[2-(8-chloro-4-oxo-chromen-2-yl)-5-(trifluoromethyl)phenoxy]ethylamino]propanamide; and
1-[3-[5-bromo-2-(8-chloro-4-oxo-chromen-2-yl)phenoxy]propyl]-5-oxo-pyrrolidine-3-carboxylic acid;
or a pharmaceutically acceptable salt thereof.

11. A compound according to any one of claims 1 to 9, selected from
1-[3-[5-bromo-2-(8-chloro-4-oxo-chromen-2-yl)phenoxy]propyl]pyrrolidine-3-carboxylic acid;
4-[3-[5-bromo-2-(8-chloro-4-oxo-chromen-2-yl)phenoxy]propyl]morpholine-2-carboxylic acid;
3-[3-[2-(8-chloro-4-oxo-chromen-2-yl)-5-(trifluoromethyl)phenoxy]propylamino]cyclobutanecarboxylic acid;
3-[3-[2-(8-chloro-4-oxo-chromen-2-yl)-5-(trifluoromethyl)phenoxy]propylamino]cyclopentanecarboxylic acid;
(3R)-1-[3-[2-(8-chloro-4-oxo-chromen-2-yl)-5-(trifluoromethyl)phenoxy]propyl]pyrrolidine-3-carboxylic acid;
(3S)-1-[3-[2-(8-chloro-4-oxo-chromen-2-yl)-5-(trifluoromethyl)phenoxy]propyl]pyrrolidine-3-carboxylic acid;
(3 S)-1-[3-[5-bromo-2-(8-chloro-4-oxo-chromen-2-yl)-4-methoxy-phenoxy]propyl]pyrrolidine-3-carboxylic acid;
4-[3-[2-(8-chloro-4-oxo-chromen-2-yl)-5-(trifluoromethyl)phenoxy]propyl]morpholine-2-carboxylic acid;
1-[2-[5-bromo-2-(8-chloro-4-oxo-chromen-2-yl)phenoxy]ethyl]pyrrolidine-2-carboxylic acid;
4-[2-[5-bromo-2-(8-chloro-4-oxo-chromen-2-yl)phenoxy]ethyl]morpholine-2-carboxylic acid;
(3R)-1-[2-[5-bromo-2-[8-chloro-4-oxo-5-(trifluoromethyl)chromen-2-yl]phenoxy]ethyl]pyrrolidine-3-carboxylic acid;
(3R)-1-[2-[2-[5-bromo-2-(8-chloro-4-oxo-chromen-2-yl)phenoxy]ethoxy]ethyl]pyrrolidine-3-carboxylic acid;
4-[2-[5-bromo-2-(8-chloro-4-oxo-chromen-2-yl)phenoxy]ethyl]morpholine-3-carboxylic acid;
(3R)-1-[2-[5-bromo-2-(8-chloro-4-oxo-chromen-2-yl)phenoxy]ethyl]-N-methylsulfonyl-pyrrolidine-3-carboxamide;
1-[2-[2-(8-chloro-4-oxo-chromen-2-yl)-5-(trifluoromethyl)phenoxy]ethyl]-N-methylsulfonyl-pyrrolidine-2-carboxamide;
2-[4-bromo-2-[2-(3-methylsulfonylpyrrolidin-1-yl)ethoxy]phenyl]-8-chloro-chromen-4-one;
(2S)-2-[2-[2-(8-chloro-4-oxo-chromen-2-yl)-5-methyl-phenoxy]ethylcarbamoylamino]propanoic acid;
3-[2-(8-chloro-4-oxo-chromen-2-yl)-5-(trifluoromethyl)phenoxy]propylurea;
8-chloro-4-oxo-2-[2-[3-(sulfamoylamino)propoxy]-4-(trifluoromethyl)phenyl]chromene;
(3R)-1-[3-[5-bromo-2-(8-chloro-4-oxo-chromen-2-yl)phenoxy]-2-hydroxy-propyl]pyrrolidine-3-carboxylic acid; and
2-[[3-[2-(8-chloro-4-oxo-chromen-2-yl)-5-(trifluoromethyl)phenoxy]-2-hydroxy-propyl]amino]-2-oxo-acetic acid;
or a pharmaceutically acceptable salt thereof.

12. A process for the preparation of a compound according to any one of claims 1 to 11 comprising at least one of the following steps:
(a) Substitution of a compound of formula (XI), 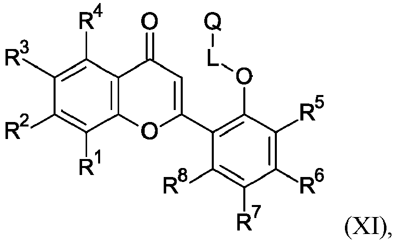 with a compound of formula (X-1), 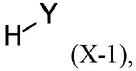 in the presence of a base;
(b) Substitution of a compound of formula (XI) with amine (X-2), 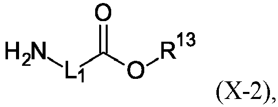 in the presence of a base;
(c) Hydrolysis of a compound of formula (I-2), 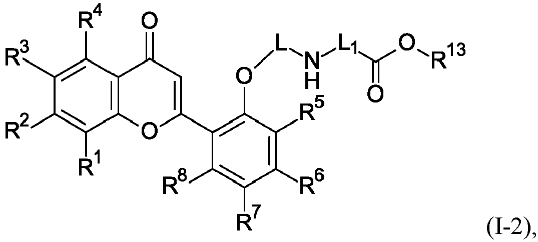 in the presence of a base;
(d) Substitution of a compound of formula (I-2) with a compound of formula (XVII-1), 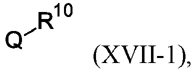 in the presence of a base;
(e) Hydrolysis of a compound of formula (I-4), 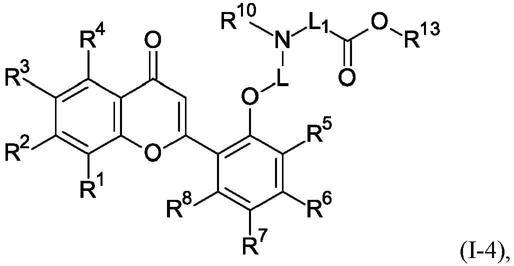 in the presence of a base or a Lewis acid;
(f) Treatment of a compound of formula (I-3), 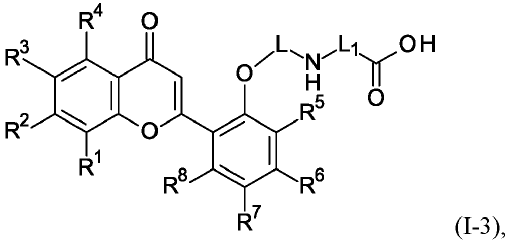 with a compound of formula (XVII-1) in the presence of a base;
(g) Condensation of a compound of formula (I-3), with a compound of formula (X-3), 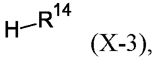 in the presence of a condensation reagent;
(h) Substitution of a compound of formula (XIX), 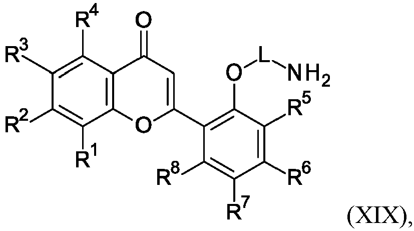 with a compound of formula (XVII-3), 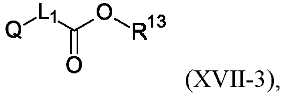 in the presence of a base;
(i) Substitution of a compound of formula (XIX), with a compound of formula (XVII-5), **Q-R¹¹** (XVII-5), in the presence of a base;
(j) Deprotection of a compound of formula (XVI), 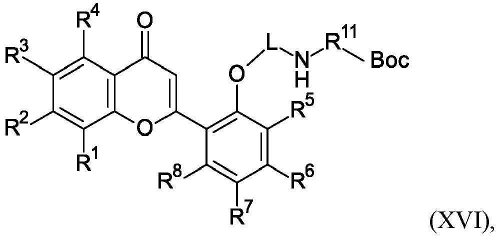 in the presence of a Lewis acid;
(k) Hydrolysis of a compound of formula (XX), 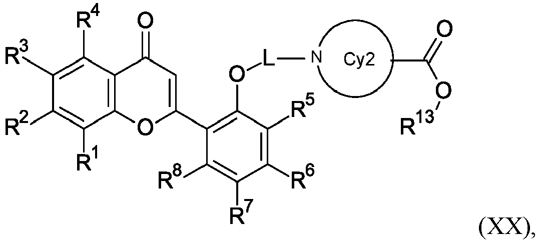 in the presence of a base or a Lewis acid;
(l) Condensation of a compound of formula (I-8), 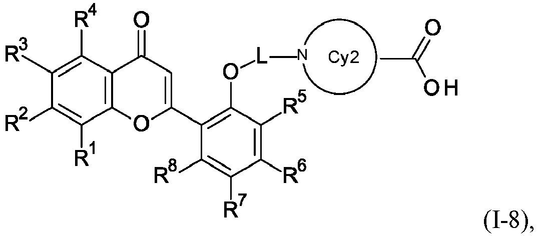 with a compound of formula (X-3), in the presence of a condensation reagent and a base;
(m) Treatment of a compound of formula (XXII), 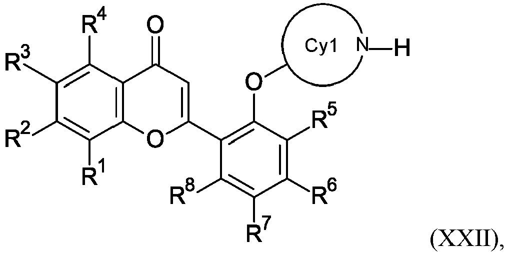 with a compound of formula (XVII-7); 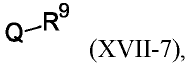 in the presence of a base;
(n) Hydrolysis of a compound of formula (XXIII), 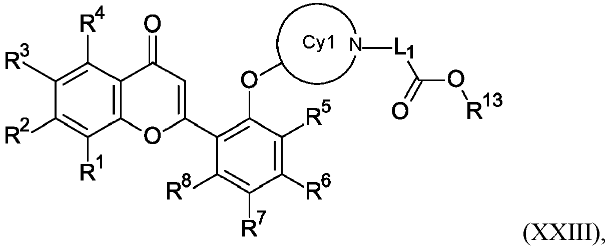 in the presence of a base;
(o) Treatment of a compound of formula (XXXI), 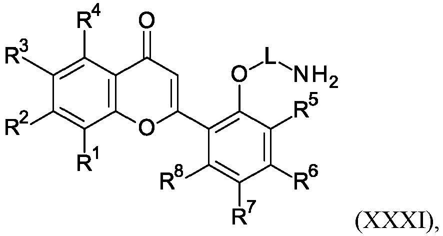 with isocyanato(trimethyl)silane in the presence of a base;
(p) Treatment of a compound of formula (XXXI) with a compound of formula (XXXII-1); 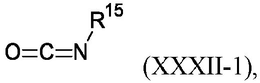 in the presence of a base;
(q) Deprotection of a compounds of formula (XXXIII), 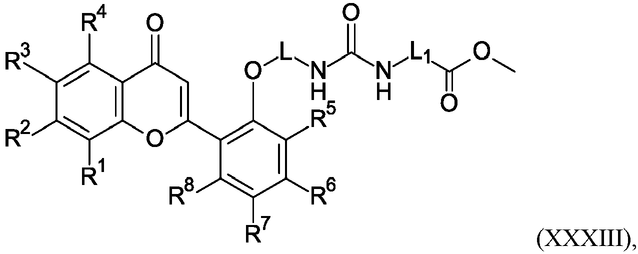 in the presence of a base;
(r) Hydrolysis of a compound of formula (XXXV), 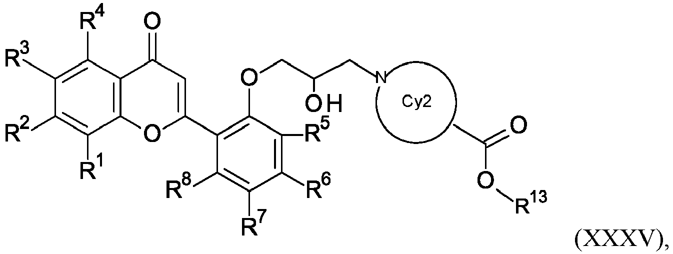 in the presence of a base;
(s) Hydrolysis of a compound of formula (XXXVIII), 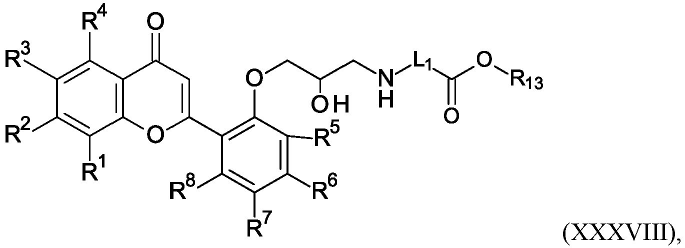 in the presence of a base;
wherein R¹ to R¹², L, Cy1, Cy2 and Y are defined as any one of claims 1 to 19; wherein Q is halogen or Oms; L₁ is C₁₋₆alkyl, carbonyl or C₃₋₇cycloalkyl; R¹³ is C₁₋₆alkyl; R¹⁴ is C₁₋₆alkylsulfonylamino, C₃₋₇cycloalkylsulfonylamino, C₃₋₇cycloalkylamino or hydroxyheterocyclyl; R¹⁵ is C₁₋₆alkylphenylsulfonyl.

13. A compound according to any one of claims 1 to 11 for use as therapeutically active substance.

14. A pharmaceutical composition comprising a compound in accordance with any one of claims 1 to 11 and a therapeutically inert carrier.

15. A compound according to any one of claims 1 to 11 for use in the treatment or prophylaxis of HBV infection.

## Patentansprüche

1. Verbindung der Formel (I) 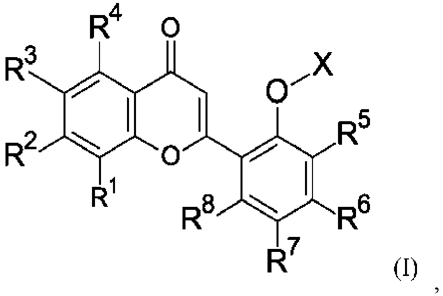 wobei
R¹ Halogen ist;
R² aus H, OH, Halogen, C₁₋₆-Alkyl, Halogen-C₁₋₆-alkyl und C₁₋₆-Alkoxy ausgewählt ist;
R³ aus H, OH, Halogen, C₁₋₆-Alkyl, Halogen-C₁₋₆-alkyl und C₁₋₆-Alkoxy ausgewählt ist;
R⁴ aus H, OH, Halogen, C₁₋₆-Alkyl, Halogen-C₁₋₆-alkyl und C₁₋₆-Alkoxy ausgewählt ist;
R⁵ aus H, OH, Halogen, C₁₋₆-Alkyl, Halogen-C₁₋₆-alkyl und C₁₋₆-Alkoxy ausgewählt ist;
R⁶ aus H, OH, Halogen, C₁₋₆-Alkyl, Halogen-C₁₋₆-alkyl und C₁₋₆-Alkoxy ausgewählt ist;
R⁷ aus H, OH, Halogen, C₁₋₆-Alkyl, Halogen-C₁₋₆-alkyl und C₁₋₆-Alkoxy ausgewählt ist;
R⁸ aus H, OH, Halogen, C₁₋₆-Alkyl, Halogen-C₁₋₆-alkyl und C₁₋₆-Alkoxy ausgewählt ist;
X 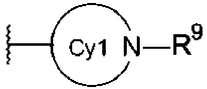 oder -L-Y ist; wobei
Cy1 N-enthaltendes Heterocyclyl ist;
R⁹ aus C₃₋₇-Cycloalkylsulfonyl, Carboxycarbonyl, Carboxyphenyl-C₁₋₆-alkyl und Carboxy-C₃-₇-cycloalkyl ausgewählt ist;
L aus C₁₋₆-Alkyl, C₁₋₆-Alkoxy-C₁₋₆-alkyl, C₃₋₇-Cycloalkyl, C₃₋₇-Cycloalkyl-C₁₋₆-alkyl und C₁₋₆-Alkylheterocyclyl-C₁₋₆-alkyl ausgewählt ist; wobei C₁₋₆-Alkyl unsubstituiert oder mit OH substituiert ist;
Y -NR¹⁰R¹¹ oder 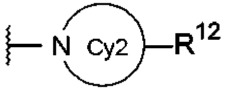 ist; wobei
Cy2 N-enthaltendes Heterocyclyl ist; wobei N-enthaltendes Heterocyclyl unsubstituiert oder mit einem oder zwei oder drei Substituenten substituiert ist, die unabhängig voneinander aus Halogen, C₁₋₆-Alkyl und OH ausgewählt sind;
R¹⁰ aus H, C₁₋₆-Alkyl, C₃₋₇-Cycloalkyl, C₁₋₆-Alkylsulfonyl, C₃₋₇-Cycloalkylsulfonyl, Carboxy-C₁₋₆-alkyl, Phenylsulfonyl und C₁₋₆-Alkylcarbonyl ausgewählt ist;
R¹¹ aus Carboxy-C₃₋₇-cycloalkyl, Carboxy-C₁₋₆-alkyl, Carboxycarbonyl, Carboxyheterocyclyl, Carboxy-C₃₋₇-cycloalkyl-C₁₋₆-alkyl, Heterocyclyl, C₁₋₆-Alkylsulfonyl-C₁₋₆-alkyl, Aminosulfonyl-C₁₋₆-alkyl, C₃₋₇-Cycloalkylsulfonyl, C₁₋₆-Alkoxycarbonylcarbonyl, Phenylsulfonyl, C₁₋₆-Alkylcarbonyl, C₁₋₆-Alkoxycarbonyl, C₁₋₆-Alkylaminosulfonyl, Aminocarbonylcarbonyl, C₃₋₇-Cycloalkylaminocarbonylcarbonyl, C₃₋₇-Cycloalkylsulfonylaminocarbonylcarbonyl, Hydroxyheterocyclylcarbonylcarbonyl, Carboxy-C₁₋₆-alkylaminocarbonyl, C₁₋₆-Alkylphenylsulfonylaminocarbonyl, Aminocarbonyl, Aminosulfonyl und Aminocarbonyl-C₁₋₆-alkyl ausgewählt ist;
R¹² aus H, Carboxy, Carboxy-C₁₋₆-alkyl, C₁₋₆-Alkylsulfonylaminocarbonyl, C₃₋₇-Cycloalkylsulfonylaminocarbonyl, C₁₋₆-Alkyl-(C₁₋₆-alkylsulfonyl)-amino, C₁₋₆-Alkylsulfonyl, C₁₋₆-Alkylcarbonyl, C₁₋₆-Alkylsulfinyl, C₁₋₆-Alkylsulfonimidoyl, Aminocarbonyl, Carboxycarbonyl und Heterocyclyl ausgewählt ist;
oder ein pharmazeutisch unbedenkliches Salz davon.

2. Verbindung nach Anspruch 1, wobei
R¹ Halogen ist;
R² H ist;
R³ aus H, Halogen und C₁₋₆-Alkoxy ausgewählt ist;
R⁴ aus H und Halogen-C₁₋₆-alkyl ausgewählt ist;
R⁵ H ist;
R⁶ aus H, Halogen, C₁₋₆-Alkyl, Halogen-C₁₋₆-alkyl und C₁₋₆-Alkoxy ausgewählt ist;
R⁷ aus H, Halogen, C₁₋₆-Alkyl und C₁₋₆-Alkoxy ausgewählt ist;
R⁸ aus H, Halogen und C₁₋₆-Alkoxy ausgewählt ist;
X 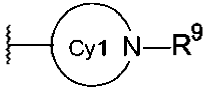 oder -L-Y ist; wobei
Cy1 aus Piperidyl, Pyrrolidinyl und Azetidinyl ausgewählt ist;
R⁹ aus C₃₋₇-Cycloalkylsulfonyl, Carboxycarbonyl, Carboxyphenyl-C₁₋₆-alkyl und Carboxy-C₃₋₇-cycloalkyl ausgewählt ist;
L aus C₁₋₆-Alkyl, C₁₋₆-Alkoxy-C₁₋₆-alkyl, C₃₋₇-Cycloalkyl, C₃₋₇-Cycloalkyl-C₁₋₆-alkyl und C₁₋₆-Alkyloxetanyl-C₁₋₆-alkyl ausgewählt ist; wobei C₁₋₆-Alkyl unsubstituiert oder mit OH substituiert ist;
Y -NR¹⁰R¹¹ oder 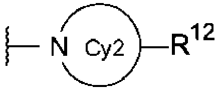 ist; wobei
Cy2 aus Pyrrolidinyl, Morpholinyl, Azetidinyl, Piperidyl, Azabicyclo[3.2.1]octanyl, Oxopyrrolidinyl, Piperazinyl, Thiomorpholinyl, Dioxothiazinanyl, Oxoimidazolidinyl und Dioxoimidazolidinyl ausgewählt ist; wobei Pyrrolidinyl unsubstituiert oder mit einem oder zwei Substituenten substituiert ist, die unabhängig voneinander aus Halogen, C₁₋₆-Alkyl und OH ausgewählt sind;
R¹⁰ aus H, C₁₋₆-Alkyl, C₃₋₇-Cycloalkyl, C₁₋₆-Alkylsulfonyl, C₃₋₇-Cycloalkylsulfonyl, Carboxy-C₁₋₆-alkyl, Phenylsulfonyl und C₁₋₆-Alkylcarbonyl ausgewählt ist;
R¹¹ aus Carboxy-C₃₋₇-cycloalkyl, Carboxy-C₁₋₆-alkyl, Carboxycarbonyl, Carboxytetrahydropyranyl, Carboxy-C₃₋₇-cycloalkyl-C₁₋₆-alkyl, Dioxothiazinanyl, C₁₋₆-Alkylsulfonyl-C₁₋₆-alkyl, Aminosulfonyl-C₁₋₆-alkyl, C₃₋₇-Cycloalkylsulfonyl, C₁₋₆-Alkoxycarbonylcarbonyl, Phenylsulfonyl, C₁₋₆-Alkylcarbonyl, C₁₋₆-Alkoxycarbonyl, C₁₋₆-Alkylaminosulfonyl, Aminocarbonylcarbonyl, C₃₋₇-Cycloalkylaminocarbonylcarbonyl, C₃₋₇-Cycloalkylsulfonylaminocarbonylcarbonyl, Hydroxypyrrolidinylcarbonylcarbonyl, Carboxy-C₁₋₆-alkylaminocarbonyl, C₁₋₆-Alkylphenylsulfonylaminocarbonyl, Aminocarbonyl, Aminosulfonyl und Aminocarbonyl-C₁₋₆-alkyl ausgewählt ist;
R¹² aus H, Carboxy, Carboxy-C₁₋₆-alkyl, C₁₋₆-Alkylsulfonylaminocarbonyl, C₃₋₇-Cycloalkylsulfonylaminocarbonyl, C₁₋₆-Alkyl-(C₁₋₆-alkylsulfonyl)-amino, C₁₋₆-Alkylsulfonyl, C₁₋₆-Alkylcarbonyl, C₁₋₆-Alkylsulfinyl, C₁₋₆-Alkylsulfonimidoyl, Aminocarbonyl, Carboxycarbonyl und 2H-Tetrazolyl ausgewählt ist;
oder ein pharmazeutisch unbedenkliches Salz davon.

3. Verbindung nach Anspruch 1 oder 2 oder ein pharmazeutisch unbedenkliches Salz davon, wobei R³ aus H ausgewählt ist.

4. Verbindung nach einem der Ansprüche 1, 2 und 3 oder ein pharmazeutisch unbedenkliches Salz davon, wobei R⁶ aus Halogen, C₁₋₆-Alkyl und Halogen-C₁₋₆-alkyl ausgewählt ist.

5. Verbindung nach einem der Ansprüche 1, 2, 3 und 4 oder ein pharmazeutisch unbedenkliches Salz davon, wobei R⁷ aus Hund C₁₋₆-Alkoxy ausgewählt ist.

6. Verbindung nach einem der Ansprüche 1, 2, 3, 4 und 5 oder ein pharmazeutisch unbedenkliches Salz davon, wobei X -L-Y ist.

7. Verbindung nach einem der Ansprüche 1, 2, 3, 4, 5 und 6 oder ein pharmazeutisch unbedenkliches Salz davon, wobei L aus C₁₋₆-Alkyl und C₁₋₆-Alkoxy-C₁₋₆-alkyl ausgewählt ist; wobei C₁₋₆-Alkyl unsubstituiert oder mit OH substituiert ist.

8. Verbindung nach einem der Ansprüche 1, 2, 3, 4, 5, 6 und 7 oder ein pharmazeutisch unbedenkliches Salz davon, wobei Y 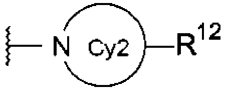 ist; wobei Cy2 aus Pyrrolidinyl und Morpholinyl ausgewählt ist.

9. Verbindung nach einem der Ansprüche 1, 2, 3, 4, 5, 6 und 7 oder ein pharmazeutisch unbedenkliches Salz davon, wobei Y -NHR¹¹ ist.

10. Verbindung nach einem der Ansprüche 1 bis 2, ausgewählt aus
1-[3-[5-Brom-2-(8-chlor-4-oxochromen-2-yl)phenoxy]propyl]pyrrolidin-3-carbonsäure;
4-[3-[5-Brom-2-(8-chlor-4-oxochromen-2-yl)phenoxy]propyl]morpholin-2-carbonsäure;
(2R)-1-[3-[5-Brom-2-(8-chlor-4-oxochromen-2-yl)phenoxy]propyl]pyrrolidin-2-carbonsäure;
(2S)-1-[3-[5-Brom-2-(8-chlor-4-oxochromen-2-yl)phenoxy]propyl]pyrrolidin-2-carbonsäure;
(3R)-1-[3-[5-Brom-2-(8-chlor-4-oxochromen-2-yl)phenoxy]propyl]pyrrolidin-3-carbonsäure;
3 -[3 -[2-(8-Chlor-4-oxochromen-2-yl)-5 - (trifluormethyl)phenoxy]propylamino]cyclobutancarbonsäure;
3 -[3 -[2-(8-Chlor-4-oxochromen-2-yl)-5-(trifluormethyl)phenoxy]propylamino]cyclopentancarbonsäure;
2-[1-[3-[2-(8-Chlor-4-oxochromen-2-yl)-5-(trifluormethyl)phenoxy]propyl]pyrrolidin-3-yl]essigsäure;
(2R)-2-[3-[2-(8-Chlor-4-oxochromen-2-yl)-5-(trifluormethyl)phenoxy]propylamino]-3-methylbutansäure;
2-[[3-[[2-(8-Chlor-4-oxochromen-2-yl)-5-(trifluormethyl)phenoxy]methyl]oxetan-3-yl]methylamino]-2-oxoessigsäure;
2-[3-[2-(8-Chlor-4-oxochromen-2-yl)-5-(trifluormethyl)phenoxy]propylamino]-4,4-dimethylpentansäure;
1-[3-[2-(8-Chlor-4-oxochromen-2-yl)-5-(trifluormethyl)phenoxy]propyl]pyrrolidin-3-carbonsäure;
(3R)-1-[3-[2-(8-Chlor-4-oxochromen-2-yl)-5-(trifluormethyl)phenoxy]propyl]pyrrolidin-3-carbonsäure;
(3 S)-1-[3-[2-(8-Chlor-4-oxochromen-2-yl)-5-(trifluormethyl)phenoxy]propyl]pyrrolidin-3-carbonsäure;
(3 S)-1-[3-[2-(8-Chlor-4-oxochromen-2-yl)-5-ethoxyphenoxy]propyl]pyrrolidin-3-carbonsäure;
(3R)-1-[3-[5-Brom-2-(8-chlor-4-oxochromen-2-yl)-4-methoxyphenoxy]propyl]pyrrolidin-3-carbonsäure;
(3S)-1-[3-[5-Brom-2-(8-chlor-4-oxochromen-2-yl)-4-methoxyphenoxy]propyl]pyrrolidin-3-carbonsäure;
4-[3-[2-(8-Chlor-4-oxochromen-2-yl)-5-(trifluormethyl)phenoxy]propyl]morpholin-2-carbonsäure;
1-[2-[5-Brom-2-(8-chlor-4-oxochromen-2-yl)phenoxy]ethyl]azetidin-3-carbonsäure;
1-[2-[5-Brom-2-(8-chlor-4-oxochromen-2-yl)phenoxy]ethyl]pyrrolidin-2-carbonsäure;
1-[2-[5-Brom-2-(8-chlor-4-oxochromen-2-yl)phenoxy]ethyl]pyrrolidin-3-carbonsäure;
1-[2-[5-Brom-2-(8-chlor-4-oxochromen-2-yl)phenoxy]ethyl]piperidin-4-carbonsäure;
4-[2-[5-Brom-2-(8-chlor-4-oxochromen-2-yl)phenoxy]ethyl]morpholin-2-carbonsäure;
(3 S,4S)-1-[2-[5-Brom-2-(8-chlor-4-oxochromen-2-yl)phenoxy]ethyl]-4-methylpyrrolidin-3-carbonsäure;
8-[2-[5-Brom-2-(8-chlor-4-oxochromen-2-yl)phenoxy]ethyl]-8-azabicyclo[3.2.1]octan-3-carbonsäure;
3-[2-[5-Brom-2-(8-chlor-4-oxochromen-2-yl)phenoxy]ethylamino]bicyclo[1. 1.1]pentan-1-carbonsäure;
1-[2-[5-Brom-2-(8-chlor-4-oxochromen-2-yl)phenoxy]ethyl]-4,4-difluorpyrrolidin-2-carbonsäure;
1-[2-[2-(8-Chlor-4-oxochromen-2-yl)phenoxy]ethyl]piperidin-4-carbonsäure;
(2R)-1-[2-[2-(8-Chlor-4-oxochromen-2-yl)phenoxy]ethyl]pyrrolidin-2-carbonsäure;
1-[2-[2-(8-Chlor-4-oxochromen-2-yl)phenoxy]ethyl]pyrrolidin-3-carbonsäure;
(2S)-1-[2-[2-(8-Chlor-4-oxochromen-2-yl)phenoxy]ethyl]pyrrolidin-2-carbonsäure;
1-[2-[2-(8-Chlor-4-oxochromen-2-yl)phenoxy]ethyl]azetidin-3-carbonsäure;
(3 S)-1-[2-[2-(8-Chlor-4-oxochromen-2-yl)-5-(trifluormethyl)phenoxy]ethyl]pyrrolidin-3-carbonsäure;
4-[2-[2-(8-Chlor-4-oxochromen-2-yl)-5-(trifluormethyl)phenoxy]ethyl]morpholin-2-carbonsäure;
(2R)-2-[2-[2-(8-Chlor-4-oxochromen-2-yl)-5-(trifluormethyl)phenoxy]ethylamino]-3-methylbutansäure;
(2S)-2-[2-[2-(8-Chlor-4-oxochromen-2-yl)-5-(trifluormethyl)phenoxy]ethylamino]-3-methylbutansäure;
3-[2-[2-(8-Chlor-4-oxochromen-2-yl)-5-(trifluormethyl)phenoxy] ethylamino] cyclobutancarbonsäure;
(3R)-1-[2-[2-(8-Chlor-4-oxochromen-2-yl)-5-isobutoxyphenoxy]ethyl]pyrrolidin-3-carbonsäure;
(3 S)-1-[2-[2-(8-Chlor-4-oxochromen-2-yl)-5-isobutoxyphenoxy]ethyl]pyrrolidin-3-carbonsäure;
(3R)-1-[2-[5-Brom-2-[8-chlor-4-oxo-5-(trifluormethyl)chromen-2-yl]phenoxy]ethyl]pyrrolidin-3-carbonsäure;
(3 S)-1-[2-[5-Brom-2-[8-chlor-4-oxo-5-(trifluormethyl)chromen-2-yl]phenoxy]ethyl]pyrrolidin-3-carbonsäure;
1-[2-[2-(8-Chlor-6-fluor-4-oxochromen-2-yl)phenoxy]ethyl]pyrrolidin-3-carbonsäure;
(2R)-1-[2-[5-Brom-2-(8-chlor-4-oxochromen-2-yl)-4-methylphenoxy]ethyl]pyrrolidin-2-carbonsäure;
(2S)-1-[2-[5-Chlor-2-(8-chlor-4-oxochromen-2-yl)-4-methylphenoxy]ethyl]pyrrolidin-2-carbonsäure;
3-[3-[5-Chlor-2-(8-chlor-4-oxochromen-2-yl)-4-methylphenoxy]propylamino]cyclobutancarbonsäure;
(3R)-1-[2-[2-[5-Brom-2-(8-chlor-4-oxochromen-2-yl)phenoxy]ethoxy]ethyl]pyrrolidin-3-carbonsäure;
(2R)-1-[2-[2-[5-Brom-2-(8-chlor-4-oxochromen-2-yl)-4-methylphenoxy] ethoxy] ethyl]pyrroli din-2-carbonsäure;
4-[2-[2-[5-Brom-2-(8-chlor-4-oxochromen-2-yl)phenoxy]ethoxy]ethyl]morpholin-2-carbonsäure;
(3R)-1-[2-[2-[2-(8-Chlor-4-oxochromen-2-yl)-5-methoxy-4-methylphenoxy]ethoxy]ethyl]pyrrolidin-3-carbonsäure;
(3 S)-1-[2-[2-[2-(8-Chlor-4-oxochromen-2-yl)-5-methoxy-4-methylphenoxy]ethoxy]ethyl]pyrrolidin-3-carbonsäure;
4-[2-[5-Brom-2-(8-chlor-4-oxochromen-2-yl)phenoxy]ethyl]morpholin-3-carbonsäure;
3-[2-[2-(8-Chlor-4-oxochromen-2-yl)-5-methylphenoxy]ethylamino]cyclobutancarbonsäure;
1-[2-[2-(8-Chlor-4-oxochromen-2-yl)-5-methylphenoxy]ethyl]azetidin-3-carbonsäure;
cis-4-[2-[5-Brom-2-(8-chlor-4-oxochromen-2-yl)phenoxy]ethylamino]cyclohexancarbonsäure;
trans-4-[2-[5-Brom-2-(8-chlor-4-oxochromen-2-yl)phenoxy]ethylamino]cyclohexancarbonsäure;
2-[2-[2-(8-Chlor-4-oxochromen-2-yl)-5-(trifluormethyl)phenoxy]ethylamino]propansäure;
2-[2-[2-(8-Chlor-4-oxochromen-2-yl)-5-methylphenoxy]ethylamino]-2-methylpropansäure;
3-[3-[2-(8-Chlor-4-oxochromen-2-yl)-5-methylphenoxy]propylamino]cyclobutancarbonsäure;
cis-4-[3-[5-Brom-2-(8-chlor-4-oxochromen-2-yl)phenoxy]propylamino]tetrahydropyran-2-carbonsäure;
1-[2-[5-Chlor-2-(8-chlor-4-oxochromen-2-yl)-4-methylphenoxy]ethyl]azetidin-3-carbonsäure;
3-[3-[2-(8-Chlor-4-oxochromen-2-yl)-5-(trifluormethyl)phenoxy]propylamino]butansäure;
2-[3-[2-(8-Chlor-4-oxochromen-2-yl)-5-(trifluormethyl)phenoxy]propylamino]-3-cyclohexylpropansäure;
(3R)-1-[2-[5-Brom-2-(8-chlor-4-oxochromen-2-yl)phenoxy]ethyl]-N-methylsulfonylpyrrolidin-3-carboxamid;
(3R)-1-[3-[2-(8-Chlor-4-oxochromen-2-yl)-5-(trifluormethyl)phenoxy]propyl]-N-methylsulfonylpyrrolidin-3-carboxamid;
(3 S)-1-[3-[2-(8-Chlor-4-oxochromen-2-yl)-5-(trifluormethyl)phenoxy]propyl]-N-methylsulfonylpyrrolidin-3-carboxamid;
(3R)-1-[3-[2-(8-Chlor-4-oxochromen-2-yl)-5-(trifluormethyl)phenoxy]propyl]-N-cyclopropylsulfonylpyrrolidin-3-carboxamid;
4-[2-[2-(8-Chlor-4-oxochromen-2-yl)-5-(trifluormethyl)phenoxy]ethyl]-N-methylsulfonylmorpholin-2-carboxamid;
(3 S)-1-[2-[5-Brom-2-(8-chlor-4-oxochromen-2-yl)phenoxy]ethyl]-N-methylsulfonylpyrrolidin-3-carboxamid;
(3R)-1-[2-[2-(8-Chlor-4-oxochromen-2-yl)-5-(trifluormethyl)phenoxy]ethyl]-N-methylsulfonylpyrrolidin-3-carboxamid;
(3 S)-1-[2-[2-(8-Chlor-4-oxochromen-2-yl)-5-(trifluormethyl)phenoxy]ethyl]-N-methylsulfonylpyrrolidin-3-carboxamid;
1-[2-[2-(8-Chlor-4-oxochromen-2-yl)-5-(trifluormethyl)phenoxy]ethyl]-N-cyclopropylsulfonylpyrrolidin-2-carboxamid;
1-[2-[2-(8-Chlor-4-oxochromen-2-yl)-5-(trifluormethyl)phenoxy]ethyl]-N-methylsulfonylpyrrolidin-2-carboxamid;
1-[2-[5-Brom-2-(8-chlor-4-oxochromen-2-yl)phenoxy]ethyl]-N-methylsulfonylpyrrolidin-2-carboxamid;
1-[2-[5-Brom-2-(8-chlor-4-oxochromen-2-yl)phenoxy]ethyl]pyrrolidin-2-on;
N-[1-[2-[5-Brom-2-(8-chlor-4-oxochromen-2-yl)phenoxy]ethyl]pyrrolidin-3-yl]-N-methylmethansulfonamid;
8-Chlor-2-[2-[3-[(3R,4S)-3,4-dihydroxypyrrolidin-1-yl]propoxy]-4-(trifluormethyl)phenyl]chromen-4-on;
8-Chlor-2-[2-[3-[(1,1-dioxothian-4-yl)amino]propoxy]-4-(trifluormethyl)phenyl]chromen-4-on;
8-Chlor-2-[2-[3-(2-methylsulfonylethylamino)propoxy]-4-(trifluormethyl)phenyl]chromen-4-on;
2-[3-[2-(8-Chlor-4-oxochromen-2-yl)-5-(trifluormethyl)phenoxy]propylamino]ethansulfonamid;
8-Chlor-2-[2-[3-(4-methylsulfonylpiperazin-1-yl)propoxy]-4-(trifluormethyl)phenyl]chromen-4-on;
2-[2-[2-(4-Acetylpiperazin-1-yl)ethoxy]-4-bromphenyl]-8-chlorchromen-4-on;
1-[2-[5-Brom-2-(8-chlor-4-oxochromen-2-yl)phenoxy]ethyl]pyrrolidin-3-carboxamid;
2-[4-Brom-2-[2-(3-methylsulfonylpyrrolidin-1-yl)ethoxy]phenyl]-8-chlorchromen-4-on;
4-[2-[5-Brom-2-(8-chlor-4-oxochromen-2-yl)phenoxy]ethyl]thiomorpholin-3-carboxamid;
N-[3-[2-(8-Chlor-4-oxochromen-2-yl)-5-(trifluormethyl)phenoxy]-2-hydroxypropyl]cyclopropansulfonamid;
8-Chlor-2-[2-[2-[4-(1H-tetrazol-5-yl)-1-piperidyl]ethoxy]-4-(trifluormethyl)phenyl]chromen-4-on;
8-Chlor-2-[2-[3-(3-methylsulfonylpyrrolidin-1-yl)propoxy]-4-(trifluormethyl)phenyl]chromen-4-on;
2-[4-Brom-2-[2-(1,1-dioxo-1,4-thiazinan-4-yl)ethoxy]phenyl]-8-chlorchromen-4-on;
2-[4-Brom-2-[2-(2-morpholinoethoxy)ethoxy]phenyl]-8-chlorchromen-4-on;
1-[2-[2-(8-Chlor-4-oxochromen-2-yl)-5-methylphenoxy]ethyl]imidazolidin-2-on;
1-[2-[2-(8-Chlor-4-oxochromen-2-yl)-5-methylphenoxy]ethyl]-3-methylimidazolidin-2-on;
8-Chlor-2-[2-[2-(4-methylsulfinyl- 1 -piperidyl)ethoxy] -4-(trifluormethyl)phenyl] chromen-4-on;
8-Chlor-2-[2-[2-[4-(methylsulfonimidoyl)-1-piperidyl]ethoxy]-4-(trifluormethyl)phenyl]chromen-4-on;
Ethyl-2-[3-[2-(8-chlor-4-oxochromen-2-yl)-5-methylphenoxy]propylamino]-2-oxoacetat;
N-[2-[2-(8-Chlor-4-oxochromen-2-yl)-5-(trifluormethyl)phenoxy] ethyl cyclopropansulfonamid;
N-[3-[2-(8-Chlor-4-oxochromen-2-yl)-5-(trifluormethyl)phenoxy]propyl]benzolsulfonamid;
N-[3-[2-(8-Chlor-4-oxochromen-2-yl)-5-(trifluormethyl)phenoxy]propyl]acetamid;
N-[3-[2-(8-Chlor-4-oxochromen-2-yl)-5-(trifluormethyl)phenoxy]propyl]propanamid;
Ethyl-2-[3-[2-(8-chlor-4-oxochromen-2-yl)-5-(trifluormethyl)phenoxy]propylamino]-2-oxoacetat;
Ethyl-2-[2-[2-(8-chlor-4-oxochromen-2-yl)-5-(trifluormethyl)phenoxy]ethylamino]-2-oxoacetat;
Ethyl-2-[2-[4-brom-2-(8-chlor-4-oxochromen-2-yl)-5-methoxyphenoxy]ethylamino]-2-oxoacetat;
Ethyl-2-[2-[2-(8-chlor-6-methoxy-4-oxochromen-2-yl)-5-methylphenoxy]ethylamino]-2-oxoacetat;
Ethyl-2-[2-[2-(8-chlor-4-oxochromen-2-yl)-5-methoxyphenoxy]ethylamino]-2-oxoacetat;
Methyl-N-[2-[5-brom-2-(8-chlor-4-oxochromen-2-yl)phenoxy]ethyl]carbamat;
Ethyl-N-[2-[5-brom-2-(8-chlor-4-oxochromen-2-yl)phenoxy]ethyl]carbamat;
8-Chlor-2-[4-methyl-2-[3-(methylsulfamoylamino)propoxy]phenyl]chromen-4-on;
2-[4-Brom-2-[(1-cyclopropylsulfonyl-4-piperidyl)oxy]phenyl]-8-chlorchromen-4-on;
Ethyl-2-[2-[2-(8-chlor-4-oxochromen-2-yl)-5-methylphenoxy]ethylamino]-2-oxoacetat;
2-[[1-[[2-(8-Chlor-4-oxochromen-2-yl)-5-(trifluormethyl)phenoxy]methyl]cyclopropyl]amino]-2-oxoessigsäure;
2-[2-[2-(8-Chlor-4-oxochromen-2-yl)-5-methoxyphenoxy]ethylamino]-2-oxoessigsäure;
2-[2-[2-(8-Chlor-4-oxochromen-2-yl)-3-fluor-5-methylphenoxy]ethylamino]-2-oxoessigsäure;
2-[2-[5-Brom-2-(8-chlor-4-oxochromen-2-yl)phenoxy]ethylamino]-2-oxoessigsäure;
2-[2-[2-(8-Chlor-4-oxochromen-2-yl)-4,5-dimethoxyphenoxy]ethylamino]-2-oxoessigsäure;
2-[[2-[2-(8-Chlor-4-oxochromen-2-yl)-5-(trifluormethyl)phenoxy]-1,1-dimethylethyl]amino]-2-oxoessigsäure;
2-[3-[2-(8-Chlor-6-methoxy-4-oxochromen-2-yl)-5-(trifluormethyl)phenoxy]propylamino]-2-oxoessigsäure;
2-[3-[2-(8-Chlor-4-oxochromen-2-yl)-5-(trifluormethyl)phenoxy]propylamino]-2-oxoessigsäure;
2-[2-[2-(8-Chlor-4-oxochromen-2-yl)-3-methoxy-5-methylphenoxy]ethylamino]-2-oxoessigsäure;
2-[2-[2-(8-Chlor-4-oxochromen-2-yl)-5-(trifluormethyl)phenoxy]ethylamino]-2-oxoessigsäure;
2-[3-[2-(8-Chlor-4-oxochromen-2-yl)-5-methylphenoxy]propylamino]-2-oxoessigsäure;
2-[2-[2-(8-Chlor-4-oxochromen-2-yl)-5-methylphenoxy]ethylamino]-2-oxoessigsäure;
2-[2-[4-Brom-2-(8-chlor-4-oxochromen-2-yl)-5-methoxyphenoxy]ethylamino]-2-oxoessigsäure;
2-[2-[2-(8-Chlor-4-oxochromen-2-yl)-5-(trifluormethyl)phenoxy]ethylamino]essigsäure;
3-[2-[2-(8-Chlor-4-oxochromen-2-yl)-5-(trifluormethyl)phenoxy]ethylamino]propansäure;
2-[3-[2-(8-Chlor-4-oxochromen-2-yl)-5-(trifluormethyl)phenoxy]propylamino]essigsäure;
2-Oxo-2-[(3 S)-3-[2-(8-chlor-4-oxochromen-2-yl)-5-(trifluormethyl)phenoxy]pyrrolidin-1-yl]essigsäure;
2-[3-[2-(8-Chlor-4-oxochromen-2-yl)-5-(trifluormethyl)phenoxy]azetidin-1-yl]-2-oxoessigsäure;
3-[[4-[5-Brom-2-(8-chlor-4-oxochromen-2-yl)phenoxy]-1-piperidyl]methyl]benzoesäure;
2-[4-[2-(8-Chlor-4-oxochromen-2-yl)-5-(trifluormethyl)phenoxy]-1-piperidyl]-2-oxoessigsäure;
2-Oxo-2-[(3R)-3-[2-(8-chlor-4-oxochromen-2-yl)-5-(trifluormethyl)phenoxy]pyrrolidin-1-yl]essigsäure;
3-[4-[5-Brom-2-(8-chlor-4-oxochromen-2-yl)phenoxy]-1-piperidyl]cyclobutancarbonsäure;
2-[(3 S)-3-[2-(8-Chlor-4-oxochromen-2-yl)-5-methylphenoxy]pyrrolidin-1-yl]-2-oxoessigsäure;
2-[[3-[2-(8-Chlor-4-oxochromen-2-yl)-5-(trifluormethyl)phenoxy]cyclobutyl]amino]-2-oxoessigsäure;
2-[3-[2-(8-Chlor-4-oxochromen-2-yl)-5-(trifluormethyl)phenoxy]propylmethylamino]essigsäure;
2-[2-[2-(8-Chlor-4-oxochromen-2-yl)-5-methylphenoxy]ethylcyclopropylamino]-2-oxoessigsäure;
8-Chlor-2-[2-[2-[ethylsulfamoyl(methyl)amino]ethoxy]-4-methylphenyl]-4-oxochromen;
Ethyl-2-[2-[2-(8-chlor-4-oxochromen-2-yl)-5-methylphenoxy]ethylmethylamino]-2-oxoacetat;
Ethyl-2-[3-[2-(8-chlor-4-oxochromen-2-yl)-5-(trifluormethyl)phenoxy]propylethylsulfonylamino]-2-oxoacetat;
3 -[3 -[2-(8-Chlor-4-oxochromen-2-yl)-5 - (trifluormethyl)phenoxy]propylcyclopropylsulfonylamino]cyclobutancarbonsäure;
2-[Carboxymethyl-[3-[2-(8-chlor-4-oxochromen-2-yl)-5-(trifluormethyl)phenoxy]propyl]amino]essigsäure;
cis-3-[Benzolsulfonyl-[3-[2-(8-chlor-4-oxochromen-2-yl)-5-(trifluormethyl)phenoxy]propyl]amino]cyclobutancarbonsäure;
trans-3-[Benzolsulfonyl-[3-[2-(8-chlor-4-oxochromen-2-yl)-5-(trifluormethyl)phenoxy]propyl]amino]cyclobutancarbonsäure;
2-[Benzolsulfonyl-[3-[2-(8-chlor-4-oxochromen-2-yl)-5-(trifluormethyl)phenoxy]propyl]amino]essigsäure;
2-[Acetyl-[3-[2-(8-chlor-4-oxochromen-2-yl)-5-(trifluormethyl)phenoxy]propyl]amino]essigsäure;
2-[3-[2-(8-Chlor-4-oxochromen-2-yl)-5-(trifluormethyl)phenoxy]propylcyclopropylsulfonylamino]essigsäure;
N'-[2-[2-(8-Chlor-4-oxochromen-2-yl)-5-(trifluormethyl)phenoxy]ethyl]oxamid;
N'-[2-[2-(8-Chlor-4-oxochromen-2-yl)-5-methoxyphenoxy]ethyl]oxamid;
N-[2-[2-(8-Chlor-4-oxochromen-2-yl)-5-(trifluormethyl)phenoxy]ethyl]-N'-cyclopropyloxamid;
N-[3-[2-(8-Chlor-4-oxochromen-2-yl)-5-(trifluormethyl)phenoxy]propyl]-N'-cyclopropylsulfonyloxamid;
N-[2-[2-(8-Chlor-4-oxochromen-2-yl)-5-methylphenoxy]ethyl]-2-[(3S)-3-hydroxypyrrolidin-1-yl]-2-oxoacetamid;
(2S)-2-[2-[2-(8-Chlor-4-oxochromen-2-yl)-5-methylphenoxy]ethylcarbamoylamino]propansäure;
(5S)-3-[2-[2-(8-Chlor-4-oxochromen-2-yl)-5-methylphenoxy]ethyl]-5-isopropylimidazolidin-2,4-dion;
(5S)-3-[2-[2-(8-Chlor-4-oxochromen-2-yl)-5-(trifluormethyl)phenoxy]ethyl]-5-isopropylimidazolidin-2,4-dion;
2-[2-[2-(8-Chlor-4-oxochromen-2-yl)-5-methylphenoxy]ethylcarbamoylamino]essigsäure;
(5S)-3-[2-[2-(8-Chlor-4-oxochromen-2-yl)-5-(trifluormethyl)phenoxy]ethyl]-5-methylimidazolidin-2,4-dion;
1-[3-[2-(8-Chlor-4-oxochromen-2-yl)-5-methylphenoxy]propyl]-3-(p-tolylsulfonyl)harnstoff;
3-[2-(8-Chlor-4-oxochromen-2-yl)-5-(trifluormethyl)phenoxy]propylharnstoff;
1-[2-[2-(8-Chlor-4-oxochromen-2-yl)-5-(trifluormethyl)phenoxy]ethyl]-3-(p-tolylsulfonyl)hamstoff;
8-Chlor-4-oxo-2-[2-[2-(sulfamoylamino)ethoxy]-4-(trifluormethyl)phenyl]chromen;
8-Chlor-4-oxo-2-[2-[3-(sulfamoylamino)propoxy]-4-(trifluormethyl)phenyl]chromen;
8-Chlor-2-[4-methyl-2-[2-(sulfamoylamino)ethoxy]phenyl]-4-oxochromen;
2-[3-[2-[2-(8-Chlor-4-oxochromen-2-yl)-5-(trifluormethyl)phenoxy]ethyl]-2-oxoimidazolidin-1-yl]-2-oxoessigsäure;
(3 S)-1-[3-[2-(8-Chlor-4-oxochromen-2-yl)-5-(trifluormethyl)phenoxy]-2-hydroxypropyl]pyrrolidin-3-carbonsäure;
(3S)-1-[3-[5-Brom-2-(8-chlor-4-oxochromen-2-yl)phenoxy]-2-hydroxypropyl]pyrrolidin-3-carbonsäure;
(3R)-1-[3-[5-Brom-2-(8-chlor-4-oxochromen-2-yl)phenoxy]-2-hydroxypropyl]pyrrolidin-3-carbonsäure;
2-[[3-[2-(8-Chlor-4-oxochromen-2-yl)-5-(trifluormethyl)phenoxy]-2-hydroxypropyl]amino]-2-oxoessigsäure;
2-[2-[2-(8-Chlor-4-oxochromen-2-yl)-5-(trifluormethyl)phenoxy]ethylamino]acetamid;
2-[2-[2-(8-Chlor-4-oxochromen-2-yl)-5-(trifluormethyl)phenoxy]ethylamino]propanamid; und
1-[3-[5-Brom-2-(8-chlor-4-oxochromen-2-yl)phenoxy]propyl]-5-oxopyrrolidin-3-carbonsäure;
oder ein pharmazeutisch unbedenkliches Salz davon.

11. Verbindung nach einem der Ansprüche 1 bis 9, ausgewählt aus
1-[3-[5-Brom-2-(8-chlor-4-oxochromen-2-yl)phenoxy]propyl]pyrrolidin-3-carbonsäure;
4-[3-[5-Brom-2-(8-chlor-4-oxochromen-2-yl)phenoxy]propyl]morpholin-2-carbonsäure;
3 -[3 -[2-(8-Chlor-4-oxochromen-2-yl)-5 - (trifluormethyl)phenoxy]propylamino]cyclobutancarbonsäure;
3 -[3 -[2-(8-Chlor-4-oxochromen-2-yl)-5-(trifluormethyl)phenoxy]propylamino]cyclopentancarbonsäure;
(3R)-1-[3-[2-(8-Chlor-4-oxochromen-2-yl)-5-(trifluormethyl)phenoxy]propyl]pyrrolidin-3-carbonsäure;
(3 S)-1-[3-[2-(8-Chlor-4-oxochromen-2-yl)-5-(trifluormethyl)phenoxy]propyl]pyrrolidin-3-carbonsäure;
(3 S)-1-[3-[5-Brom-2-(8-chlor-4-oxochromen-2-yl)-4-methoxyphenoxy]propyl]pyrrolidin-3-carbonsäure;
4-[3-[2-(8-Chlor-4-oxochromen-2-yl)-5-(trifluormethyl)phenoxy]propyl]morpholin-2-carbonsäure;
1-[2-[5-Brom-2-(8-chlor-4-oxochromen-2-yl)phenoxy]ethyl]pyrrolidin-2-carbonsäure;
4-[2-[5-Brom-2-(8-chlor-4-oxochromen-2-yl)phenoxy]ethyl]morpholin-2-carbonsäure;
(3R)-1-[2-[5-Brom-2-[8-chlor-4-oxo-5-(trifluormethyl)chromen-2-yl]phenoxy]ethyl]pyrrolidin-3-carbonsäure;
(3R)-1-[2-[2-[5-Brom-2-(8-chlor-4-oxochromen-2-yl)phenoxy]ethoxy]ethyl]pyrrolidin-3-carbonsäure;
4-[2-[5-Brom-2-(8-chlor-4-oxochromen-2-yl)phenoxy]ethyl]morpholin-3-carbonsäure;
(3R)-1-[2-[5-Brom-2-(8-chlor-4-oxochromen-2-yl)phenoxy]ethyl]-N-methylsulfonylpyrrolidin-3-carboxamid;
1-[2-[2-(8-Chlor-4-oxochromen-2-yl)-5-(trifluormethyl)phenoxy]ethyl]-N-methylsulfonylpyrrolidin-2-carboxamid;
2-[4-Brom-2-[2-(3-methylsulfonylpyrrolidin-1-yl)ethoxy]phenyl]-8-chlorchromen-4-on;
(2S)-2-[2-[2-(8-Chlor-4-oxochromen-2-yl)-5-methylphenoxy]ethylcarbamoylamino]propansäure;
3-[2-(8-Chlor-4-oxochromen-2-yl)-5-(trifluormethyl)phenoxy]propylharnstoff;
8-Chlor-4-oxo-2-[2-[3-(sulfamoylamino)propoxy]-4-(trifluormethyl)phenyl]chromen;
(3R)-1-[3-[5-Brom-2-(8-chlor-4-oxochromen-2-yl)phenoxy]-2-hydroxypropyl]pyrrolidin-3-carbonsäure; und
2-[[3-[2-(8-Chlor-4-oxochromen-2-yl)-5-(trifluormethyl)phenoxy]-2-hydroxypropyl]amino]-2-oxoessigsäure;
oder ein pharmazeutisch unbedenkliches Salz davon.

12. Verfahren zur Herstellung einer Verbindung nach einem der Ansprüche 1 bis 11, umfassend mindestens einen der folgenden Schritte:
(a) Substitution einer Verbindung der Formel (XI) 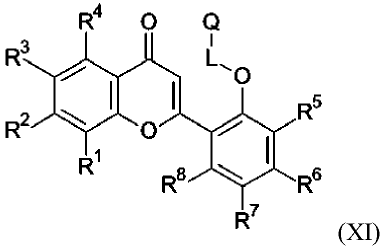 mit einer Verbindung der Formel (X-1) 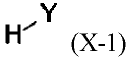 in der Gegenwart einer Base;
(b) Substitution einer Verbindung der Formel (XI) mit einem Amin (X-2) 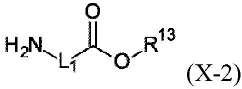 in der Gegenwart einer Base;
(c) Hydrolyse einer Verbindung der Formel (I-2) 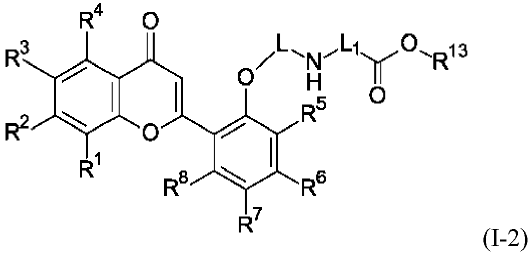 in der Gegenwart einer Base;
(d) Substitution einer Verbindung der Formel (I-2) mit einer Verbindung der Formel (XVII-1) 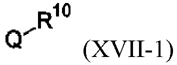 in der Gegenwart einer Base;
(e) Hydrolyse einer Verbindung der Formel (I-4) 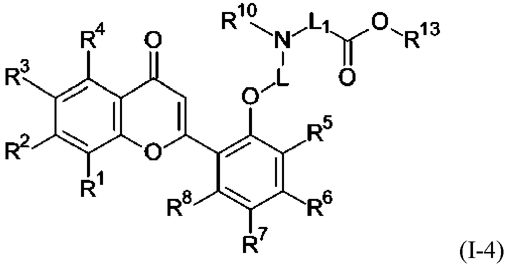 in der Gegenwart einer Base oder einer Lewis-Säure;
(f) Behandlung einer Verbindung der Formel (I-3) 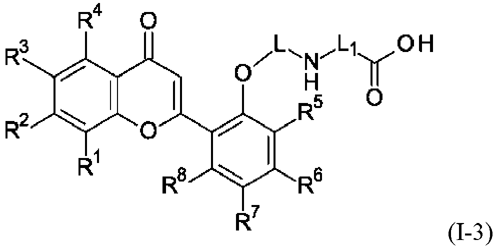 mit einer Verbindung der Formel (XVII-1) in der Gegenwart einer Base;
(g) Kondensation einer Verbindung der Formel (I-3) mit einer Verbindung der Formel (X-3) 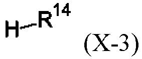 in der Gegenwart eines Kondensationsreagens;
(h) Substitution einer Verbindung der Formel (XIX) 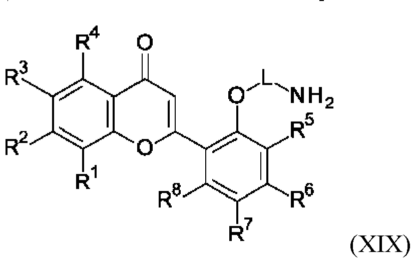 mit einer Verbindung der Formel (XVII-3) 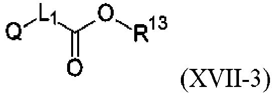 in der Gegenwart einer Base;
(i) Substitution einer Verbindung der Formel (XIX) mit einer Verbindung der Formel (XVII-5) **Q-R¹¹** (XVII-5) in der Gegenwart einer Base;
(j) Entschützung einer Verbindung der Formel (XVI) 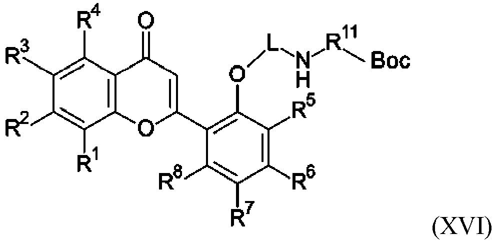 in der Gegenwart einer Lewis-Säure;
(k) Hydrolyse einer Verbindung der Formel (XX) 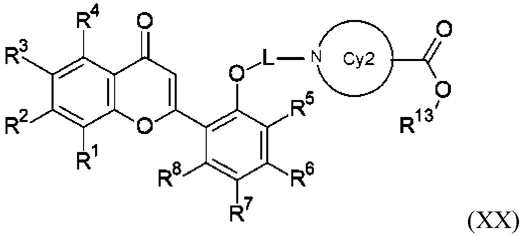 in der Gegenwart einer Base oder einer Lewis-Säure;
(l) Kondensation einer Verbindung der Formel (I-8) 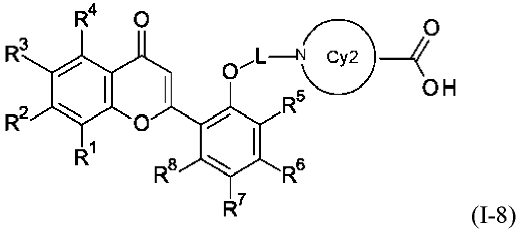 mit einer Verbindung der Formel (X-3) in der Gegenwart eines Kondensationsreagens und einer Base;
(m) Behandlung einer Verbindung der Formel (XXII) 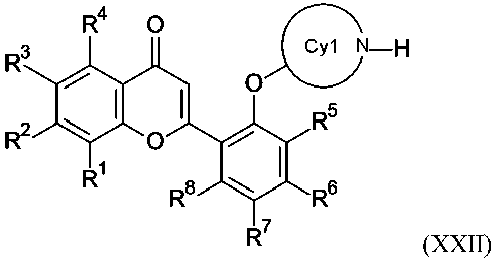 mit einer Verbindung der Formel (XVII-7) 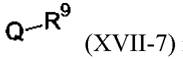 in der Gegenwart einer Base;
(n) Hydrolyse einer Verbindung der Formel (XXIII) 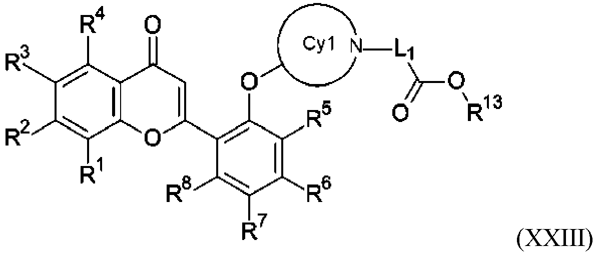 in der Gegenwart einer Base;
(o) Behandlung einer Verbindung der Formel (XXXI) 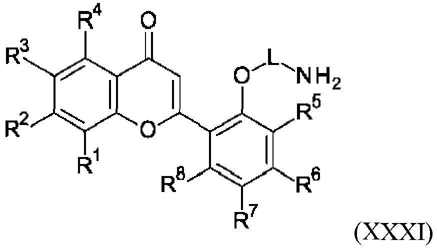 mit Isocyanato(trimethyl)silan in der Gegenwart einer Base;
(p) Behandlung einer Verbindung der Formel (XXXI) mit einer Verbindung der Formel (XXXII-1) 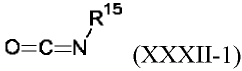 in der Gegenwart einer Base;
(q) Entschützung einer Verbindung der Formel (XXXIII) 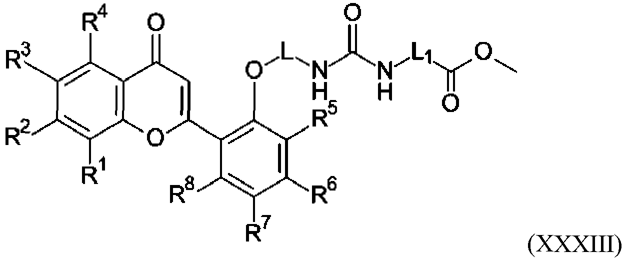 in der Gegenwart einer Base;
(r) Hydrolyse einer Verbindung der Formel (XXXV) 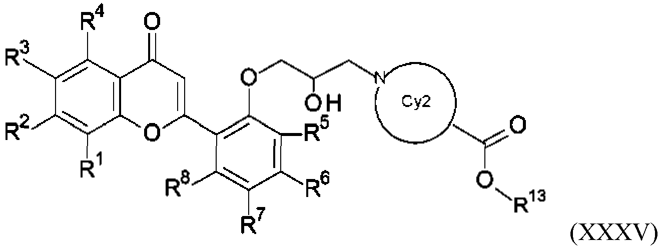 in der Gegenwart einer Base;
(s) Hydrolyse einer Verbindung der Formel (XXXVIII) 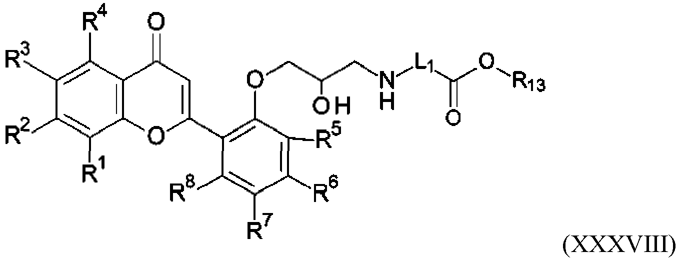 in der Gegenwart einer Base;
wobei R¹ bis R¹², L, Cy1, Cy2 und Y wie in einem der Ansprüche 1 bis 19 definiert sind; wobei Q Halogen oder Oms ist; L₁ C₁₋₆-Alkyl, Carbonyl oder C₃₋₇-Cycloalkyl ist; R¹³ C₁₋₆-Alkyl ist; R¹⁴ C₁₋₆-Alkylsulfonylamino, C₃₋₇-Cycloalkylsulfonylamino, C₃₋₇-Cycloalkylamino oder Hydroxyheterocyclyl ist; R¹⁵ C₁₋₆-Alkylphenylsulfonyl ist.

13. Verbindung nach einem der Ansprüche 1 bis 11 zur Verwendung als therapeutisch wirksame Substanz.

14. Pharmazeutische Zusammensetzung, umfassend eine Verbindung nach einem der Ansprüche 1 bis 11 und einen therapeutisch inerten Träger.

15. Verbindung nach einem der Ansprüche 1 bis 11 zur Verwendung bei der Behandlung oder Prophylaxe einer HBV-Infektion.

## Revendications

1. Composé de formule (I) 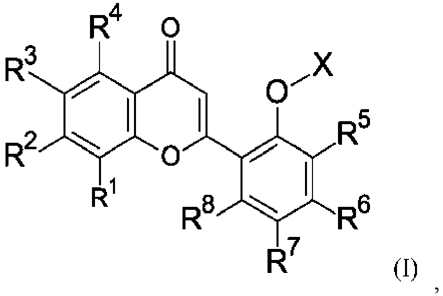 dans lequel
R¹ représente un halogène ;
R² est choisi parmi H, OH, un halogène, un alkyle en C₁₋₆, un halogénoalkyle en C₁₋₆ et un alcoxy en C₁₋₆ ;
R³ est choisi parmi H, OH, un halogène, un alkyle en C₁₋₆, un halogénoalkyle en C₁₋₆ et un alcoxy en C₁₋₆ ;
R⁴ est choisi parmi H, OH, un halogène, un alkyle en C₁₋₆, un halogénoalkyle en C₁₋₆ et un alcoxy en C₁₋₆ ;
R⁵ est choisi parmi H, OH, un halogène, un alkyle en C₁₋₆, un halogénoalkyle en C₁₋₆ et un alcoxy en C₁₋₆ ;
R⁶ est choisi parmi H, OH, un halogène, un alkyle en C₁₋₆, un halogénoalkyle en C₁₋₆ et un alcoxy en C₁₋₆ ;
R⁷ est choisi parmi H, OH, un halogène, un alkyle en C₁₋₆, un halogénoalkyle en C₁₋₆ et un alcoxy en C₁₋₆ ;
R⁸ est choisi parmi H, OH, un halogène, un alkyle en C₁₋₆, un halogénoalkyle en C₁₋₆ et un alcoxy en C₁₋₆ ;
X représente 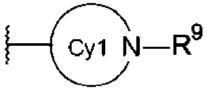 ou -L-Y ; dans lequel
Cy1 représente un hétérocyclyle contenant N ;
R⁹ est choisi parmi un cycloalkyle en C₃₋₇-sulfonyle, un carboxycarbonyle, un carboxyphénylalkyle en C₁₋₆ et un carboxycycloalkyle en C₃₋₇ ;
L est choisi parmi un alkyle en C₁₋₆, un alcoxy en C₁₋₆-alkyle en C₁₋₆, un cycloalkyle en C₃₋₇, un cycloalkyle en C₃₋₇-alkyle en C₁₋₆ et un alkyle en C₁₋₆-hétérocyclylalkyle en C₁₋₆; dans lequel l'alkyle en C₁₋₆ est non substitué ou substitué par OH ;
Y représente -NR¹⁰R¹¹ ou 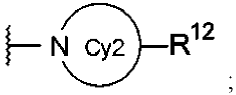 dans lequel
Cy2 représente un hétérocyclyle contenant N ; dans lequel l'hétérocyclyle contenant N est non substitué ou substitué par un ou deux ou trois substituants indépendamment choisis parmi un halogène, un alkyle en C₁₋₆ et OH ;
R¹⁰ est choisi parmi H, un alkyle en C₁₋₆, un cycloalkyle en C₃₋₇, un alkyle en C₁₋₆-sulfonyle, un cycloalkyle en C₃₋₇-sulfonyle, un carboxyalkyle en C₁₋₆, un phénylsulfonyle et un alkyle en C₁₋₆-carbonyle ;
R¹¹ est choisi parmi un carboxycycloalkyle en C₃₋₇, un carboxyalkyle en C₁₋₆, un carboxycarbonyle, un carboxyhétérocyclyle, un carboxycycloalkyle en C₃₋₇-alkyle en C₁₋₆, un hétérocyclyle, un alkyle en C₁₋₆-sulfonylalkyle en C₁₋₆, un aminosulfonylalkyle en C₁₋₆, un cycloalkyle en C₃₋₇-sulfonyle, un alcoxy en C₁₋₆-carbonylcarbonyle, un phénylsulfonyle, un alkyle en C₁₋₆-carbonyle, un alcoxy en C₁₋₆-carbonyle, un alkyle en C₁₋₆-aminosulfonyle, un aminocarbonylcarbonyle, un cycloalkyle en C₃₋₇-aminocarbonylcarbonyle, un cycloalkyle en C₃₋₇-sulfonylaminocarbonylcarbonyle, un hydroxyhétérocyclylcarbonylcarbonyle, un carboxyalkyle en C₁₋₆-aminocarbonyle, un alkyle en C₁₋₆-phénylsulfonylaminocarbonyle, un aminocarbonyle, un aminosulfonyle et un aminocarbonylalkyle en C₁₋₆ ;
R¹² est choisi parmi H, un carboxy, un carboxyalkyle en C₁₋₆, un alkyle en C₁₋₆-sulfonylaminocarbonyle, un cycloalkyle en C₃₋₇-sulfonylaminocarbonyle, un alkyle en C₁₋₆(alkyle en C₁₋₆-sulfonyl)amino, un alkyle en C₁₋₆-sulfonyle, un alkyle en C₁₋₆-carbonyle, un alkyle en C₁₋₆-sulfinyle, un alkyle en C₁₋₆-sulfonimidoyle, un aminocarbonyle, un carboxycarbonyle et un hétérocyclyle ;
ou un sel pharmaceutiquement acceptable de celui-ci.

2. Composé selon la revendication 1, dans lequel
R¹ représente un halogène ;
R² représente H ;
R³ est choisi parmi H, un halogène et un alcoxy en C₁₋₆ ;
R⁴ est choisi parmi H et un halogénoalkyle en C₁₋₆ ;
R⁵ représente H ;
R⁶ est choisi parmi H, un halogène, un alkyle en C₁₋₆, un halogénoalkyle en C₁₋₆ et un alcoxy en C₁₋₆ ;
R⁷ est choisi parmi H, un halogène, un alkyle en C₁₋₆ et un alcoxy en C₁₋₆ ;
R⁸ est choisi parmi H, un halogène et un alcoxy en C₁₋₆ ;
X représente 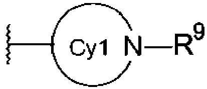 ou -L-Y ; dans lequel
Cy1 est choisi parmi un pipéridyle, un pyrrolidinyle et un azétidinyle ;
R⁹ est choisi parmi un cycloalkyle en C₃₋₇-sulfonyle, un carboxycarbonyle, un carboxyphénylalkyle en C₁₋₆ et un carboxycycloalkyle en C₃₋₇ ;
L est choisi parmi un alkyle en C₁₋₆, un alcoxy en C₁₋₆-alkyle en C₁₋₆, un cycloalkyle en C₃₋₇, un cycloalkyle en C₃₋₇-alkyle en C₁₋₆ et un alkyle en C₁₋₆-oxétanylalkyle en C₁₋₆ ; dans lequel l'alkyle en C₁₋₆ est non substitué ou substitué par OH ;
Y représente -NR¹⁰R¹¹ ou 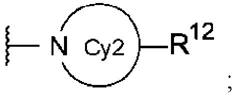 dans lequel
Cy2 est choisi parmi un pyrrolidinyle, un morpholinyle, un azétidinyle, un pipéridyle, un azabicyclo[3.2.1]octanyle, un oxopyrrolidinyle, un pipérazinyle, un thiomorpholinyle, un dioxothiazinanyle, un oxoimidazolidinyle et un dioxoimidazolidinyle ; dans lequel le pyrrolidinyle est non substitué ou substitué par un ou deux substituants indépendamment choisis parmi un halogène, un alkyle en C₁₋₆ et OH ;
R¹⁰ est choisi parmi H, un alkyle en C₁₋₆, un cycloalkyle en C₃₋₇, un alkyle en C₁₋₆-sulfonyle, un cycloalkyle en C₃₋₇-sulfonyle, un carboxyalkyle en C₁₋₆, un phénylsulfonyle et un alkyle en C₁₋₆-carbonyle ;
R¹¹ est choisi parmi un carboxycycloalkyle en C₃₋₇, un carboxyalkyle en C₁₋₆, un carboxycarbonyle, un carboxytétrahydropyranyle, un carboxycycloalkyle en C₃₋₇-alkyle en C₁₋₆, un dioxothiazinanyle, un alkyle en C₁₋₆-sulfonylalkyle en C₁₋₆, un aminosulfonylalkyle en C₁₋₆, un cycloalkyle en C₃₋₇-sulfonyle, un alcoxy en C₁₋₆-carbonylcarbonyle, un phénylsulfonyle, un alkyle en C₁₋₆-carbonyle, un alcoxy en C₁₋₆-carbonyle, un alkyle en C₁₋₆-aminosulfonyle, un aminocarbonylcarbonyle, un cycloalkyle en C₃₋₇-aminocarbonylcarbonyle, un cycloalkyle en C₃₋₇-sulfonylaminocarbonylcarbonyle, un hydroxypyrrolidinylcarbonylcarbonyle, un carboxyalkyle en C₁₋₆-aminocarbonyle, un alkyle en C₁₋₆-phénylsulfonylaminocarbonyle, un aminocarbonyle, un aminosulfonyle et un aminocarbonylalkyle en C₁₋₆ ;
R¹² est choisi parmi H, un carboxy, un carboxyalkyle en C₁₋₆, un alkyle en C₁₋₆-sulfonylaminocarbonyle, un cycloalkyle en C₃₋₇-sulfonylaminocarbonyle, un alkyle en C₁₋₆(alkyle en C₁₋₆-sulfonyl)amino, un alkyle en C₁₋₆-sulfonyle, un alkyle en C₁₋₆-carbonyle, un alkyle en C₁₋₆-sulfinyle, un alkyle en C₁₋₆-sulfonimidoyle, un aminocarbonyle, un carboxycarbonyle et un 2H-tétrazolyle ;
ou un sel pharmaceutiquement acceptable de celui-ci.

3. Composé selon la revendication 1 ou 2, ou un sel pharmaceutiquement acceptable de celui-ci, dans lequel R³ est choisi parmi H.

4. Composé selon l'une quelconque des revendications 1, 2 et 3, ou un sel pharmaceutiquement acceptable de celui-ci, dans lequel R⁶ est choisi parmi un halogène, un alkyle en C₁₋₆ et un halogénoalkyle en C₁₋₆.

5. Composé selon l'une quelconque des revendications 1, 2, 3 et 4, ou un sel pharmaceutiquement acceptable de celui-ci, dans lequel R⁷ est choisi parmi H et un alcoxy en Cl-6.

6. Composé selon l'une quelconque des revendications 1, 2, 3, 4 et 5, ou un sel pharmaceutiquement acceptable de celui-ci, dans lequel X représente -L-Y.

7. Composé selon l'une quelconque des revendications 1, 2, 3, 4, 5 et 6, ou un sel pharmaceutiquement acceptable de celui-ci, dans lequel L est choisi parmi un alkyle en C₁₋₆ et un alcoxy en C₁₋₆-alkyle en C₁₋₆ ; dans lequel l'alkyle en C₁₋₆ est non substitué ou substitué par OH.

8. Composé selon l'une quelconque des revendications 1, 2, 3, 4, 5, 6 et 7, ou un sel pharmaceutiquement acceptable de celui-ci, dans lequel Y représente 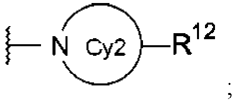 dans lequel Cy2 est choisi parmi un pyrrolidinyle et un morpholinyle.

9. Composé selon l'une quelconque des revendications 1, 2, 3, 4, 5, 6 et 7, ou un sel pharmaceutiquement acceptable de celui-ci, dans lequel Y représente -NHR¹¹.

10. Composé selon l'une quelconque des revendications 1 à 2, choisi parmi l'acide
1-[3-[5-bromo-2-(8-chloro-4-oxo-chromén-2-yl)phénoxy]propyl]pyrrolidine-3-carboxylique ; l'acide
4-[3-[5-bromo-2-(8-chloro-4-oxo-chromén-2-yl)phénoxy]propyl]morpholine-2-carboxylique ; l'acide
(2R)-1-[3-[5-bromo-2-(8-chloro-4-oxo-chromén-2-yl)phénoxy]propyl]pyrrolidine-2-carboxyli que ;
l'acide (2S)-1-[3-[5-bromo-2-(8-chloro-4-oxo-chromén-2-yl)phénoxy]propyl]pyrrolidine-2-carboxyli que ;
l'acide (3R)-1-[3-[5-bromo-2-(8-chloro-4-oxo-chromén-2-yl)phénoxy]propyl]pyrrolidine-3-carboxyli que ;
l'acide 3-[3-[2-(8-chloro-4-oxo-chromén-2-yl)-5-(trifluorométhyl)phénoxy]propylamino]cyclobutane carboxylique ;
l'acide 3-[3-[2-(8-chloro-4-oxo-chromén-2-yl)-5-(trifluorométhyl)phénoxy]propylamino]cyclopentan ecarboxylique ;
l'acide 2-[1-[3-[2-(8-chloro-4-oxo-chromén-2-yl)-5-(trifluorométhyl)phénoxy]propyl]pyrrolidin-3-yl] acétique ;
l'acide (2R)-2-[3-[2-(8-chloro-4-oxo-chromén-2-yl)-5-(trifluorométhyl)phénoxy]propylamino]-3-mét hyl-butanoïque ;
l'acide 2-[[3-[[2-(8-chloro-4-oxo-chromén-2-yl)-5-(trifluorométhyl)phénoxy]méthyl]oxétan-3-yl]mét hylamino]-2-oxo-acétique ;
l'acide 2-[3-[2-(8-chloro-4-oxo-chromén-2-yl)-5-(trifluorométhyl)phénoxy]propylamino]-4,4-diméth yl-pentanoïque ;
l'acide 1-[3-[2-(8-chloro-4-oxo-chromén-2-yl)-5-(trifluorométhyl)phénoxy]propyl]pyrrolidine-3-carb oxylique ;
l'acide (3R)-1-[3-[2-(8-chloro-4-oxo-chromén-2-yl)-5-(trifluorométhyl)phénoxy]propyl]pyrrolidine-3 -carboxylique ;
l'acide (3S)-1-[3-[2-(8-chloro-4-oxo-chromén-2-yl)-5-(trifluorométhyl)phénoxy]propyl]pyrrolidine-3 -carboxylique ;
l'acide (3S)-1-[3-[2-(8-chloro-4-oxo-chromén-2-yl)-5-éthoxy-phénoxy]propyl]pyrrolidine-3-carboxyl ique ;
l'acide (3R)-1-[3-[5-bromo-2-(8-chloro-4-oxo-chromén-2-yl)-4-méthoxy-phénoxy]propyl]pyrrolidine -3-carboxylique ;
l'acide (3S)-1-[3-[5-bromo-2-(8-chloro-4-oxo-chromén-2-yl)-4-méthoxy-phénoxy]propyl]pyrrolidine -3-carboxylique ;
l'acide 4-[3-[2-(8-chloro-4-oxo-chromén-2-yl)-5-(trifluorométhyl)phénoxy]propyl]morpholine-2-car boxylique ;
l'acide 1-[2-[5-bromo-2-(8-chloro-4-oxo-chromén-2-yl)phénoxy]éthyl]azétidine-3-carboxylique ;
l'acide 1-[2-[5-bromo-2-(8-chloro-4-oxo-chromén-2-yl)phénoxy]éthyl]pyrrolidine-2-carboxylique ;
l'acide 1-[2-[5-bromo-2-(8-chloro-4-oxo-chromén-2-yl)phénoxy]éthyl]pyrrolidine-3-carboxylique ;
l'acide 1-[2-[5-bromo-2-(8-chloro-4-oxo-chromén-2-yl)phénoxy]éthyl]pipéridine-4-carboxylique ;
l'acide 4-[2-[5-bromo-2-(8-chloro-4-oxo-chromén-2-yl)phénoxy]éthyl]morpholine-2-carboxylique ;
l'acide (3 S,4S)-1-[2-[5-bromo-2-(8-chloro-4-oxo-chromén-2-yl)phénoxy]éthyl]-4-méthyl-pyrrolidine -3-carboxylique ;
l'acide 8-[2-[5-bromo-2-(8-chloro-4-oxo-chromén-2-yl)phénoxy]éthyl]-8-azabicyclo[3.2.1]octane-3-carboxylique ;
l'acide 3-[2-[5-bromo-2-(8-chloro-4-oxo-chromén-2-yl)phénoxy]éthylamino]bicyclo[1.1.1]pentane-1 -carboxylique ;
l'acide 1-[2-[5-bromo-2-(8-chloro-4-oxo-chromén-2-yl)phénoxy]éthyl]-4,4-difluoro-pyrrolidine-2-ca rboxylique ;
l'acide 1-[2-[2-(8-chloro-4-oxo-chromén-2-yl)phénoxy]éthyl]pipéridine-4-carboxylique ;
l'acide (2R)-1-[2-[2-(8-chloro-4-oxo-chromén-2-yl)phénoxy]éthyl]pyrrolidine-2-carboxylique ;
l'acide 1-[2-[2-(8-chloro-4-oxo-chromén-2-yl)phénoxy]éthyl]pyrrolidine-3-carboxylique ;
l'acide (2S)-1-[2-[2-(8-chloro-4-oxo-chromén-2-yl)phénoxy]éthyl]pyrrolidine-2-carboxylique ;
l'acide 1-[2-[2-(8-chloro-4-oxo-chromén-2-yl)phénoxy]éthyl]azétidine-3-carboxylique ;
l'acide (3 S)-1-[2-[2-(8-chloro-4-oxo-chromén-2-yl)-5-(trifluorométhyl)phénoxy]éthyl]pyrrolidine-3-carboxylique ;
l'acide 4-[2-[2-(8-chloro-4-oxo-chromén-2-yl)-5-(trifluorométhyl)phénoxy]éthyl]morpholine-2-carbo xylique ;
l'acide (2R)-2-[2-[2-(8-chloro-4-oxo-chromén-2-yl)-5-(trifluorométhyl)phénoxy]éthylamino]-3-méth yl-butanoïque ;
l'acide (2S)-2-[2-[2-(8-chloro-4-oxo-chromén-2-yl)-5-(trifluorométhyl)phénoxy]éthylamino]-3-méth yl-butanoïque ;
l'acide 3-[2-[2-(8-chloro-4-oxo-chromén-2-yl)-5-(trifluorométhyl)phénoxy]éthylamino]cyclobutanec arboxylique ;
l'acide (3R)-1-[2-[2-(8-chloro-4-oxo-chromén-2-yl)-5-isobutoxy-phénoxy]éthyl]pyrrolidine-3-carbox ylique ;
l'acide (3S)-1-[2-[2-(8-chloro-4-oxo-chromén-2-yl)-5-isobutoxy-phénoxy]éthyl]pyrrolidine-3-carbox ylique ;
l'acide (3R)-1-[2-[5-bromo-2-[8-chloro-4-oxo-5-(trifluorométhyl)chromén-2-yl]phénoxy]éthyl]pyrrol idine-3-carboxylique ;
l'acide (3S)-1-[2-[5-bromo-2-[8-chloro-4-oxo-5-(trifluorométhyl)chromén-2-yl]phénoxy]éthyl]pyrrol idine-3-carboxylique ;
l'acide 1-[2-[2-(8-chloro-6-fluoro-4-oxo-chromén-2-yl)phénoxy]éthyl]pyrrolidine-3-carboxylique ;
l'acide (2R)-1-[2-[5-bromo-2-(8-chloro-4-oxo-chromén-2-yl)-4-méthyl-phénoxy]éthyl]pyrrolidine-2-carboxylique ;
l'acide (2S)-1-[2-[5-chloro-2-(8-chloro-4-oxo-chromén-2-yl)-4-méthyl-phénoxy]éthyl]pyrrolidine-2-carboxylique ;
l'acide 3-[3-[5-chloro-2-(8-chloro-4-oxo-chromén-2-yl)-4-méthyl-phénoxy]propylamino]cyclobutane carboxylique ;
l'acide (3R)-1-[2-[2-[5-bromo-2-(8-chloro-4-oxo-chromén-2-yl)phénoxy]éthoxy]éthyl]pyrrolidine-3-carboxylique ;
l'acide (2R)-1-[2-[2-[5-bromo-2-(8-chloro-4-oxo-chromén-2-yl)-4-méthyl-phénoxy]éthoxy]éthyl]pyr rolidine-2-carboxylique ;
l'acide 4-[2-[2-[5-bromo-2-(8-chloro-4-oxo-chromén-2-yl)phénoxy]éthoxy]éthyl]morpholine-2-carbo xylique ;
l'acide (3R)-1-[2-[2-[2-(8-chloro-4-oxo-chromén-2-yl)-5-méthoxy-4-méthyl-phénoxy]éthoxy]éthyl]p yrrolidine-3-carboxylique ;
l'acide (3 S)-1-[2-[2-[2-(8-chloro-4-oxo-chromén-2-yl)-5-méthoxy-4-méthyl-phénoxy]éthoxy]éthyl]p yrrolidine-3-carboxylique ;
l'acide 4-[2-[5-bromo-2-(8-chloro-4-oxo-chromén-2-yl)phénoxy]éthyl]morpholine-3-carboxylique ;
l'acide 3-[2-[2-(8-chloro-4-oxo-chromén-2-yl)-5-méthyl-phénoxy]éthylamino]cyclobutanecarboxyliq ue ;
l'acide 1-[2-[2-(8-chloro-4-oxo-chromén-2-yl)-5-méthyl-phénoxy]éthyl]azétidine-3-carboxylique ;
l'acide cis-4-[2-[5-bromo-2-(8-chloro-4-oxo-chromén-2-yl)phénoxy]éthylamino]cyclohexanecarboxy lique ;
l'acide trans-4-[2-[5-bromo-2-(8-chloro-4-oxo-chromén-2-yl)phénoxy]éthylamino]cyclohexanecarbo xylique ;
l'acide 2-[2-[2-(8-chloro-4-oxo-chromén-2-yl)-5-(trifluorométhyl)phénoxy]éthylamino]propanoïque ;
l'acide 2-[2-[2-(8-chloro-4-oxo-chromén-2-yl)-5-méthyl-phénoxy]éthylamino]-2-méthyl-propanoïqu e ;
l'acide 3-[3-[2-(8-chloro-4-oxo-chromén-2-yl)-5-méthyl-phénoxy]propylamino]cyclobutanecarboxyli que ;
l'acide cis-4-[3-[5-bromo-2-(8-chloro-4-oxo-chromén-2-yl)phénoxy]propylamino]tétrahydropyrane-2 -carboxylique ;
l'acide 1-[2-[5-chloro-2-(8-chloro-4-oxo-chromén-2-yl)-4-méthyl-phénoxy]éthyl]azétidine-3-carboxy lique ;
l'acide 3-[3-[2-(8-chloro-4-oxo-chromén-2-yl)-5-(trifluorométhyl)phénoxy]propylamino]butanoïque ;
l'acide 2-[3-[2-(8-chloro-4-oxo-chromén-2-yl)-5-(trifluorométhyl)phénoxy]propylamino]-3-cyclohex yl-propanoïque ;
le (3R)-1-[2-[5-bromo-2-(8-chloro-4-oxo-chromén-2-yl)phénoxy]éthyl]-N-méthylsulfonyl-pyrro lidine-3-carboxamide ;
le (3R)-1-[3-[2-(8-chloro-4-oxo-chromén-2-yl)-5-(trifluorométhyl)phénoxy]propyl]-N-méthylsu lfonyl-pyrrolidine-3-carboxamide ;
le (3S)-1-[3-[2-(8-chloro-4-oxo-chromén-2-yl)-5-(trifluorométhyl)phénoxy]propyl]-N-méthylsul fonyl-pyrrolidine-3-carboxamide ;
le (3R)-1-[3-[2-(8-chloro-4-oxo-chromén-2-yl)-5-(trifluorométhyl)phénoxy]propyl]-N-cyclopro pylsulfonyl-pyrrolidine-3-carboxamide ;
le 4-[2-[2-(8-chloro-4-oxo-chromén-2-yl)-5-(trifluorométhyl)phénoxy]éthyl]-N-méthylsulfonyl-morpholine-2-carboxamide ;
le (3S)-1-[2-[5-bromo-2-(8-chloro-4-oxo-chromén-2-yl)phénoxy]éthyl]-N-méthylsulfonyl-pyrro lidine-3-carboxamide ;
le (3R)-1-[2-[2-(8-chloro-4-oxo-chromén-2-yl)-5-(trifluorométhyl)phénoxy] éthyl]-N-méthylsulf onyl-pyrrolidine-3-carboxamide ;
le (3 S)-1-[2-[2-(8-chloro-4-oxo-chromén-2-yl)-5-(trifluorométhyl)phénoxy]éthyl]-N-méthylsulf onyl-pyrrolidine-3-carboxamide ;
le 1-[2-[2-(8-chloro-4-oxo-chromén-2-yl)-5-(trifluorométhyl)phénoxy]éthyl]-N-cyclopropylsulf onyl-pyrrolidine-2-carboxamide ;
le 1-[2-[2-(8-chloro-4-oxo-chromén-2-yl)-5-(trifluorométhyl)phénoxy]éthyl]-N-méthylsulfonyl-pyrrolidine-2-carboxamide ;
le 1-[2-[5-bromo-2-(8-chloro-4-oxo-chromén-2-yl)phénoxy]éthyl]-N-méthylsulfonyl-pyrrolidine -2-carboxamide ;
la 1-[2-[5-bromo-2-(8-chloro-4-oxo-chromén-2-yl)phénoxy]éthyl]pyrrolidin-2-one ;
le N-[1-[2-[5-bromo-2-(8-chloro-4-oxo-chromén-2-yl)phénoxy]éthyl]pyrrolidin-3-yl]-N-méthyl-méthanesulfonamide ;
la 8-chloro-2-[2-[3-[(3R,4S)-3,4-dihydroxypyrrolidin-1-yl]propoxy]-4-(trifluorométhyl)phényl]c hromén-4-one ;
la 8-chloro-2-[2-[3-[(1,1-dioxothian-4-yl)amino]propoxy]-4-(trifluorométhyl)phényl]chromén-4 -one ;
la 8-chloro-2-[2-[3-(2-méthylsulfonyléthylamino)propoxy]-4-(trifluorométhyl)phényl]chromén-4-one ;
le 2-[3-[2-(8-chloro-4-oxo-chromén-2-yl)-5-(trifluorométhyl)phénoxy]propylamino]éthanesulfo namide ;
la 8-chloro-2-[2-[3-(4-méthylsulfonylpipérazin-1-yl)propoxy]-4-(trifluorométhyl)phényl]chromé n-4-one ;
la 2-[2-[2-(4-acétylpipérazin-1-yl)éthoxy]-4-bromo-phényl]-8-chloro-chromén-4-one ;
le 1-[2-[5-bromo-2-(8-chloro-4-oxo-chromén-2-yl)phénoxy]éthyl]pyrrolidine-3-carboxamide ;
la 2-[4-bromo-2-[2-(3-méthylsulfonylpyrrolidin-1-yl)éthoxy]phényl]-8-chloro-chromén-4-one ;
le 4-[2-[5-bromo-2-(8-chloro-4-oxo-chromén-2-yl)phénoxy]éthyl]thiomorpholine-3-carboxamid e ;
le N-[3-[2-(8-chloro-4-oxo-chromén-2-yl)-5-(trifluorométhyl)phénoxy]-2-hydroxy-propyl]cyclo propanesulfonamide ;
la 8-chloro-2-[2-[2-[4-(1H-tétrazol-5-yl)-1-pipéridyl]éthoxy]-4-(trifluorométhyl)phényl]chromé n-4-one ;
la 8-chloro-2-[2-[3-(3-méthylsulfonylpyrrolidin-1-yl)propoxy]-4-(trifluorométhyl)phényl]chrom én-4-one ;
la 2-[4-bromo-2-[2-(1,1-dioxo-1,4-thiazinan-4-yl)éthoxy]phényl]-8-chloro-chromén-4-one ;
la 2-[4-bromo-2-[2-(2-morpholinoéthoxy)éthoxy]phényl]-8-chloro-chromén-4-one ;
la 1-[2-[2-(8-chloro-4-oxo-chromén-2-yl)-5-méthyl-phénoxy]éthyl]imidazolidin-2-one ;
la 1-[2-[2-(8-chloro-4-oxo-chromén-2-yl)-5-méthyl-phénoxy]éthyl]-3-méthyl-imidazolidin-2-on e ;
la 8-chloro-2-[2-[2-(4-méthylsulfinyl-1-pipéridyl)éthoxy]-4-(trifluorométhyl)phényl]chromén-4-one ;
la 8-chloro-2-[2-[2-[4-(méthylsulfonimidoyl)-1-pipéridyl]éthoxy]-4-(trifluorométhyl)phényl]chr omén-4-one ;
le 2-[3-[2-(8-chloro-4-oxo-chromén-2-yl)-5-méthyl-phénoxy]propylamino]-2-oxo-acétate d'éthyle ;
le N-[2-[2-(8-chloro-4-oxo-chromén-2-yl)-5-(trifluorométhyl)phénoxy]éthyl]cyclopropanesulfo namide ;
le N-[3-[2-(8-chloro-4-oxo-chromén-2-yl)-5-(trifluorométhyl)phénoxy]propyl]benzènesulfonam ide ;
leN-[3-[2-(8-chloro-4-oxo-chromén-2-yl)-5-(trifluorométhyl)phénoxy]propyl]acétamide ;
le N-[3-[2-(8-chloro-4-oxo-chromén-2-yl)-5-(trifluorométhyl)phénoxy]propyl]propanamide ;
le 2-[3-[2-(8-chloro-4-oxo-chromén-2-yl)-5-(trifluorométhyl)phénoxy]propylamino]-2-oxo-acét ate d'éthyle ;
le 2-[2-[2-(8-chloro-4-oxo-chromén-2-yl)-5-(trifluorométhyl)phénoxy]éthylamino]-2-oxo-acétat e d'éthyle ;
le 2-[2-[4-bromo-2-(8-chloro-4-oxo-chromén-2-yl)-5-méthoxy-phénoxy]éthylamino]-2-oxo-acét ate d'éthyle ;
le 2-[2-[2-(8-chloro-6-méthoxy-4-oxo-chromén-2-yl)-5-méthyl-phénoxy]éthylamino]-2-oxo-acé tate d'éthyle ;
le 2-[2-[2-(8-chloro-4-oxo-chromén-2-yl)-5-méthoxy-phénoxy]éthylamino]-2-oxo-acétate d'éthyle ;
le N-[2-[5-bromo-2-(8-chloro-4-oxo-chromén-2-yl)phénoxy]éthyl]carbamate de méthyle ;
le N-[2-[5-bromo-2-(8-chloro-4-oxo-chromén-2-yl)phénoxy]éthyl]carbamate d'éthyle ;
la 8-chloro-2-[4-méthyl-2-[3-(méthylsulfamoylamino)propoxy]phényl]chromén-4-one ;
la 2-[4-bromo-2-[(1-cyclopropylsulfonyl-4-pipéridyl)oxy]phényl]-8-chloro-chromén-4-one ;
le 2-[2-[2-(8-chloro-4-oxo-chromén-2-yl)-5-méthyl-phénoxy]éthylamino]-2-oxo-acétate d'éthyle ;
l'acide 2-[[1-[[2-(8-chloro-4-oxo-chromén-2-yl)-5-(trifluorométhyl)phénoxy]méthyl]cyclopropyl]ami no]-2-oxo-acétique ;
l'acide 2-[2-[2-(8-chloro-4-oxo-chromén-2-yl)-5-méthoxy-phénoxy]éthylamino]-2-oxo-acétique ;
l'acide 2-[2-[2-(8-chloro-4-oxo-chromén-2-yl)-3-fluoro-5-méthyl-phénoxy]éthylamino]-2-oxo-acétiq ue ;
l'acide 2-[2-[5-bromo-2-(8-chloro-4-oxo-chromén-2-yl)phénoxy]éthylamino]-2-oxo-acétique ;
l'acide 2-[2-[2-(8-chloro-4-oxo-chromén-2-yl)-4,5-diméthoxy-phénoxy]éthylamino]-2-oxo-acétique ;
l'acide 2-[[2-[2-(8-chloro-4-oxo-chromén-2-yl)-5-(trifluorométhyl)phénoxy]-1,1-diméthyl-éthyl]ami no]-2-oxo-acétique ;
l'acide 2-[3-[2-(8-chloro-6-méthoxy-4-oxo-chromén-2-yl)-5-(trifluorométhyl)phénoxy]propylamino] -2-oxo-acétique ;
l'acide 2-[3-[2-(8-chloro-4-oxo-chromén-2-yl)-5-(trifluorométhyl)phénoxy]propylamino]-2-oxo-acéti que ;
l'acide 2-[2-[2-(8-chloro-4-oxo-chromén-2-yl)-3-méthoxy-5-méthyl-phénoxy]éthylamino]-2-oxo-acé tique ;
l'acide 2-[2-[2-(8-chloro-4-oxo-chromén-2-yl)-5-(trifluorométhyl)phénoxy]éthylamino]-2-oxo-acétiq ue ;
l'acide 2-[3-[2-(8-chloro-4-oxo-chromén-2-yl)-5-méthyl-phénoxy]propylamino]-2-oxo-acétique ;
l'acide 2-[2-[2-(8-chloro-4-oxo-chromén-2-yl)-5-méthyl-phénoxy]éthylamino]-2-oxo-acétique ;
l'acide 2-[2-[4-bromo-2-(8-chloro-4-oxo-chromén-2-yl)-5-méthoxy-phénoxy]éthylamino]-2-oxo-acét ique ;
l'acide 2-[2-[2-(8-chloro-4-oxo-chromén-2-yl)-5-(trifluorométhyl)phénoxy]éthylamino]acétique ;
l'acide 3-[2-[2-(8-chloro-4-oxo-chromén-2-yl)-5-(trifluorométhyl)phénoxy]éthylamino]propanoïque ;
l'acide 2-[3-[2-(8-chloro-4-oxo-chromén-2-yl)-5-(trifluorométhyl)phénoxy]propylamino]acétique ;
l'acide 2-oxo-2-[(3S)-3-[2-(8-chloro-4-oxo-chromén-2-yl)-5-(trifluorométhyl)phénoxy]pyrrolidin-1-y l]acétique;
l'acide 2-[3-[2-(8-chloro-4-oxo-chromén-2-yl)-5-(trifluorométhyl)phénoxy]azétidin-1-yl]-2-oxo-acéti que ;
l'acide 3-[[4-[5-bromo-2-(8-chloro-4-oxo-chromén-2-yl)phénoxy]-1-pipéridyl]méthyl]benzoïque ;
l'acide 2-[4-[2-(8-chloro-4-oxo-chromén-2-yl)-5-(trifluorométhyl)phénoxy]-1-pipéridyl]-2-oxo-acéti que ;
l'acide 2-oxo-2-[(3R)-3-[2-(8-chloro-4-oxo-chromén-2-yl)-5-(trifluorométhyl)phénoxy]pyrrolidin-1-yl]acétique ;
l'acide 3-[4-[5-bromo-2-(8-chloro-4-oxo-chromén-2-yl)phénoxy]-1-pipéridyl]cyclobutanecarboxyliq ue ;
l'acide 2-[(3S)-3-[2-(8-chloro-4-oxo-chromén-2-yl)-5-méthyl-phénoxy]pyrrolidin-1-yl]-2-oxo-acétiq ue ;
l'acide 2-[[3-[2-(8-chloro-4-oxo-chromén-2-yl)-5-(trifluorométhyl)phénoxy]cyclobutyl]amino]-2-oxo -acétique ;
l'acide 2-[3-[2-(8-chloro-4-oxo-chromén-2-yl)-5-(trifluorométhyl)phénoxy]propyl-méthyl-amino]acé tique ;
l'acide 2-[2-[2-(8-chloro-4-oxo-chromén-2-yl)-5-méthyl-phénoxy]éthyl-cyclopropyl-amino]-2-oxo-a cétique ;
le 8-chloro-2-[2-[2-[éthylsulfamoyl(méthyl)amino]éthoxy]-4-méthyl-phényl]-4-oxo-chromène ;
le 2-[2-[2-(8-chloro-4-oxo-chromén-2-yl)-5-méthyl-phénoxy]éthyl-méthyl-amino]-2-oxo-acétate d'éthyle ;
le 2-[3-[2-(8-chloro-4-oxo-chromén-2-yl)-5-(trifluorométhyl)phénoxy]propyl-éthylsulfonyl-ami no]-2-oxo-acétate d'éthyle ;
l'acide 3-[3-[2-(8-chloro-4-oxo-chromén-2-yl)-5-(trifluorométhyl)phénoxy]propyl-cyclopropylsulfon yl-amino]cyclobutanecarboxylique ;
l'acide 2-[carboxyméthyl-[3-[2-(8-chloro-4-oxo-chromén-2-yl)-5-(trifluorométhyl)phénoxy]propyl]a mino]acétique ;
l'acide cis-3-[benzènesulfonyl-[3-[2-(8-chloro-4-oxo-chromén-2-yl)-5-(trifluorométhyl)phénoxy]pro pyl]amino]cyclobutanecarboxylique ;
l'acide trans-3-[benzènesulfonyl-[3-[2-(8-chloro-4-oxo-chromén-2-yl)-5-(trifluorométhyl)phénoxy]pr opyl]amino]cyclobutanecarboxylique ;
l'acide 2-[benzènesulfonyl-[3-[2-(8-chloro-4-oxo-chromén-2-yl)-5-(trifluorométhyl)phénoxy]propyl] amino]acétique ;
l'acide 2-[acétyl-[3-[2-(8-chloro-4-oxo-chromén-2-yl)-5-(trifluorométhyl)phénoxy]propyl]amino]acé tique ;
l'acide 2-[3-[2-(8-chloro-4-oxo-chromén-2-yl)-5-(trifluorométhyl)phénoxy]propyl-cyclopropylsulfon yl-amino]acétique ;
le N'-[2-[2-(8-chloro-4-oxo-chromén-2-yl)-5-(trifluorométhyl)phénoxy]éthyl]oxamide ;
le N'-[2-[2-(8-chloro-4-oxo-chromén-2-yl)-5-méthoxy-phénoxy]éthyl]oxamide ;
le N-[2-[2-(8-chloro-4-oxo-chromén-2-yl)-5-(trifluorométhyl)phénoxy]éthyl]-N'-cyclopropyl-ox ami de ;
le N-[3-[2-(8-chloro-4-oxo-chromén-2-yl)-5-(trifluorométhyl)phénoxy]propyl]-N'-cyclopropyls ulfonyl-oxamide ;
le N-[2-[2-(8-chloro-4-oxo-chromén-2-yl)-5-méthyl-phénoxy]éthyl]-2-[(3S)-3-hydroxypyrrolidi n-1-yl]-2-oxo-acétamide ;
l'acide (2S)-2-[2-[2-(8-chloro-4-oxo-chromén-2-yl)-5-méthyl-phénoxy]éthylcarbamoylamino]propan oïque ;
la (5S)-3-[2-[2-(8-chloro-4-oxo-chromén-2-yl)-5-méthyl-phénoxy]éthyl]-5-isopropyl-imidazolid ine-2,4-dione ;
la (5S)-3-[2-[2-(8-chloro-4-oxo-chromén-2-yl)-5-(trifluorométhyl)phénoxy]éthyl]-5-isopropyl-i midazolidine-2,4-dione ;
l'acide 2-[2-[2-(8-chloro-4-oxo-chromén-2-yl)-5-méthyl-phénoxy]éthylcarbamoylamino]acétique ;
la (5S)-3-[2-[2-(8-chloro-4-oxo-chromén-2-yl)-5-(trifluorométhyl)phénoxy]éthyl]-5-méthyl-imi dazolidine-2,4-dione ;
la 1-[3-[2-(8-chloro-4-oxo-chromén-2-yl)-5-méthyl-phénoxy]propyl]-3-(p-tolylsulfonyl)urée ;
la 3-[2-(8-chloro-4-oxo-chromén-2-yl)-5-(trifluorométhyl)phénoxy]propylurée ;
la 1-[2-[2-(8-chloro-4-oxo-chromén-2-yl)-5-(trifluorométhyl)phénoxy]éthyl]-3-(p-tolylsulfonyl) urée ;
le 8-chloro-4-oxo-2-[2-[2-(sulfamoylamino)éthoxy]-4-(trifluorométhyl)phényl]chromène ;
le 8-chloro-4-oxo-2-[2-[3-(sulfamoylamino)propoxy]-4-(trifluorométhyl)phényl]chromène ;
le 8-chloro-2-[4-méthyl-2-[2-(sulfamoylamino)éthoxy]phényl]-4-oxo-chromène ;
l'acide 2-[3-[2-[2-(8-chloro-4-oxo-chromén-2-yl)-5-(trifluorométhyl)phénoxy]éthyl]-2-oxo-imidazoli din-1-yl]-2-oxo-acétique ;
l'acide (3S)-1-[3-[2-(8-chloro-4-oxo-chromén-2-yl)-5-(trifluorométhyl)phénoxy]-2-hydroxy-propyl]p yrrolidine-3-carboxylique ;
l'acide (3S)-1-[3-[5-bromo-2-(8-chloro-4-oxo-chromén-2-yl)phénoxy]-2-hydroxy-propyl]pyrrolidine -3-carboxylique ;
l'acide (3R)-1-[3-[5-bromo-2-(8-chloro-4-oxo-chromén-2-yl)phénoxy]-2-hydroxy-propyl]pyrrolidine -3-carboxylique ;
l'acide 2-[[3-[2-(8-chloro-4-oxo-chromén-2-yl)-5-(trifluorométhyl)phénoxy]-2-hydroxy-propyl]amin o]-2-oxo-acétique ;
le 2-[2-[2-(8-chloro-4-oxo-chromén-2-yl)-5-(trifluorométhyl)phénoxy]éthylamino]acétamide ;
le 2-[2-[2-(8-chloro-4-oxo-chromén-2-yl)-5-(trifluorométhyl)phénoxy]éthylamino]propanamide ; et
l'acide 1-[3-[5-bromo-2-(8-chloro-4-oxo-chromén-2-yl)phénoxy]propyl]-5-oxo-pyrrolidine-3-carbox ylique ;
ou un sel pharmaceutiquement acceptable de ceux-ci.

11. Composé selon l'une quelconque des revendications 1 à 9, choisi parmi l'acide
1-[3-[5-bromo-2-(8-chloro-4-oxo-chromén-2-yl)phénoxy]propyl]pyrrolidine-3-carboxylique ; l'acide
4-[3-[5-bromo-2-(8-chloro-4-oxo-chromén-2-yl)phénoxy]propyl]morpholine-2-carboxylique ; l'acide
3-[3-[2-(8-chloro-4-oxo-chromén-2-yl)-5-(trifluorométhyl)phénoxy]propylamino]cyclobutane carboxylique ;
l'acide 3-[3-[2-(8-chloro-4-oxo-chromén-2-yl)-5-(trifluorométhyl)phénoxy]propylamino]cyclopentan ecarboxylique ;
l'acide (3R)-1-[3-[2-(8-chloro-4-oxo-chromén-2-yl)-5-(trifluorométhyl)phénoxy]propyl]pyrrolidine-3 -carboxylique ;
l'acide (3S)-1-[3-[2-(8-chloro-4-oxo-chromén-2-yl)-5-(trifluorométhyl)phénoxy]propyl]pyrrolidine-3 -carboxylique ;
l'acide (3 S)-1-[3-[5-bromo-2-(8-chloro-4-oxo-chromén-2-yl)-4-méthoxy-phénoxy]propyl]pyrrolidine -3-carboxylique ;
l'acide 4-[3-[2-(8-chloro-4-oxo-chromén-2-yl)-5-(trifluorométhyl)phénoxy]propyl]morpholine-2-car boxylique ;
l'acide 1-[2-[5-bromo-2-(8-chloro-4-oxo-chromén-2-yl)phénoxy]éthyl]pyrrolidine-2-carboxylique ;
l'acide 4-[2-[5-bromo-2-(8-chloro-4-oxo-chromén-2-yl)phénoxy]éthyl]morpholine-2-carboxylique ;
l'acide (3R)-1-[2-[5-bromo-2-[8-chloro-4-oxo-5-(trifluorométhyl)chromén-2-yl]phénoxy]éthyl]pyrrol idine-3-carboxylique ;
l'acide (3R)-1-[2-[2-[5-bromo-2-(8-chloro-4-oxo-chromén-2-yl)phénoxy]éthoxy]éthyl]pyrrolidine-3-carboxylique ;
l'acide 4-[2-[5-bromo-2-(8-chloro-4-oxo-chromén-2-yl)phénoxy]éthyl]morpholine-3-carboxylique ;
le (3R)-1-[2-[5-bromo-2-(8-chloro-4-oxo-chromén-2-yl)phénoxy]éthyl]-N-méthylsulfonyl-pyrro lidine-3-carboxamide ;
le 1-[2-[2-(8-chloro-4-oxo-chromén-2-yl)-5-(trifluorométhyl)phénoxy]éthyl]-N-méthylsulfonyl-pyrrolidine-2-carboxamide ;
la 2-[4-bromo-2-[2-(3-méthylsulfonylpyrrolidin-1-yl)éthoxy]phényl]-8-chloro-chromén-4-one ;
l'acide (2S)-2-[2-[2-(8-chloro-4-oxo-chromén-2-yl)-5-méthyl-phénoxy]éthylcarbamoylamino]propan oïque ;
la 3-[2-(8-chloro-4-oxo-chromén-2-yl)-5-(trifluorométhyl)phénoxy]propylurée ;
le 8-chloro-4-oxo-2-[2-[3-(sulfamoylamino)propoxy]-4-(trifluorométhyl)phényl]chromène ;
l'acide (3R)-1-[3-[5-bromo-2-(8-chloro-4-oxo-chromén-2-yl)phénoxy]-2-hydroxy-propyl]pyrrolidine -3-carboxylique ; et
l'acide 2-[[3-[2-(8-chloro-4-oxo-chromén-2-yl)-5-(trifluorométhyl)phénoxy]-2-hydroxy-propyl]amin o]-2-oxo-acétique ;
ou un sel pharmaceutiquement acceptable de ceux-ci.

12. Procédé de préparation d'un composé selon l'une quelconque des revendications 1 à 11 comprenant au moins l'une des étapes suivantes :
(a) substitution d'un composé de formule (XI), 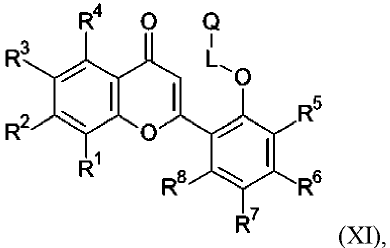 avec un composé de formule (X-1), 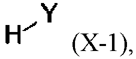 en présence d'une base ;
(b) substitution d'un composé de formule (XI) avec une amine (X-2), 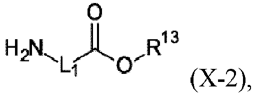 en présence d'une base ;
(c) hydrolyse d'un composé de formule (I-2), 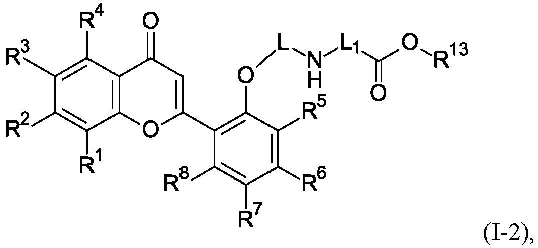 en présence d'une base ;
(d) substitution d'un composé de formule (I-2) avec un composé de formule (XVII-1), 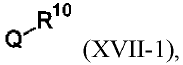 en présence d'une base ;
(e) hydrolyse d'un composé de formule (I-4), 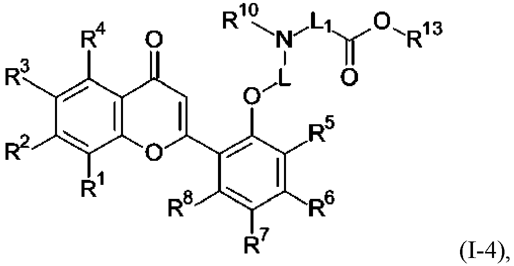 en présence d'une base ou d'un acide de Lewis ;
(f) traitement d'un composé de formule (I-3), 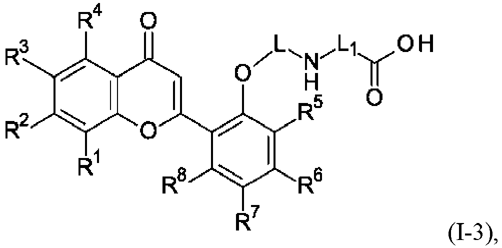 avec un composé de formule (XVII-1) en présence d'une base ;
(g) condensation d'un composé de formule (I-3), avec un composé de formule (X-3), 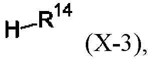 en présence d'un réactif de condensation ;
(h) substitution d'un composé de formule (XIX), 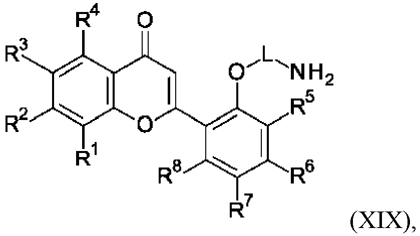 avec un composé de formule (XVII-3), 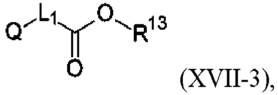 en présence d'une base ;
(i) substitution d'un composé de formule (XIX), avec un composé de formule (XVII-5), Q-R¹¹ (XVII-5), en présence d'une base ;
(j) déprotection d'un composé de formule (XVI), 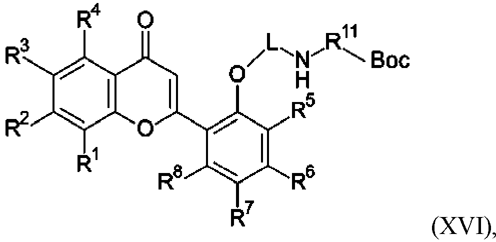 en présence d'un acide de Lewis ;
(k) hydrolyse d'un composé de formule (XX), 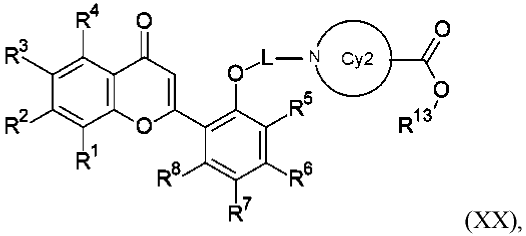 en présence d'une base ou d'un acide de Lewis ;
(l) condensation d'un composé de formule (I-8), 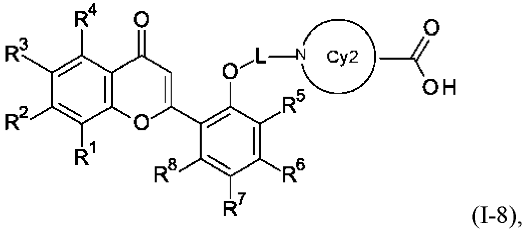 avec un composé de formule (X-3), en présence d'un réactif de condensation et d'une base ;
(m) traitement d'un composé de formule (XXII), 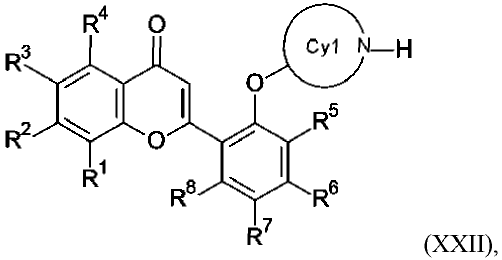 avec un composé de formule (XVII-7) ; 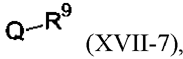 en présence d'une base ;
(n) hydrolyse d'un composé de formule (XXIII), 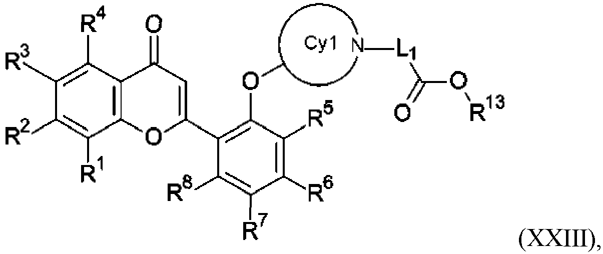 en présence d'une base ;
(o) traitement d'un composé de formule (XXXI), 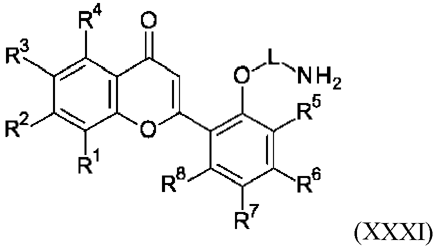 avec de l'isocyanato(triméthyle)silane en présence d'une base ;
(p) traitement d'un composé de formule (XXXI) avec un composé de formule (XXXII-1) ; 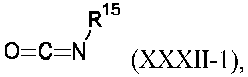 en présence d'une base ;
(q) déprotection d'un composé de formule (XXXIII), 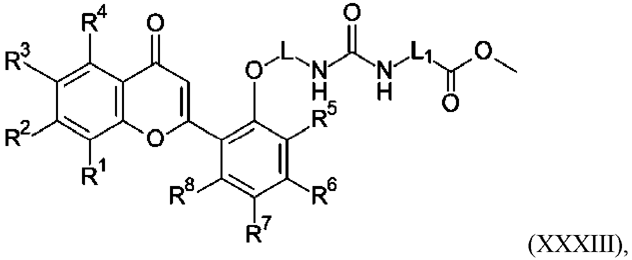 en présence d'une base ;
(r) hydrolyse d'un composé de formule (XXXV), 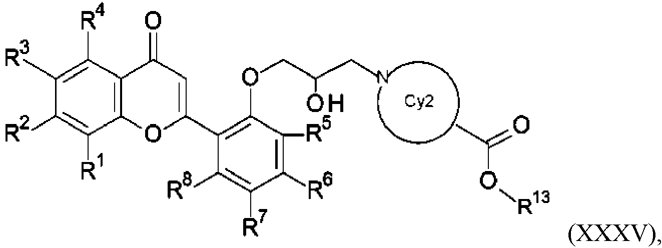 en présence d'une base ;
(s) hydrolyse d'un composé de formule (XXXVIII), 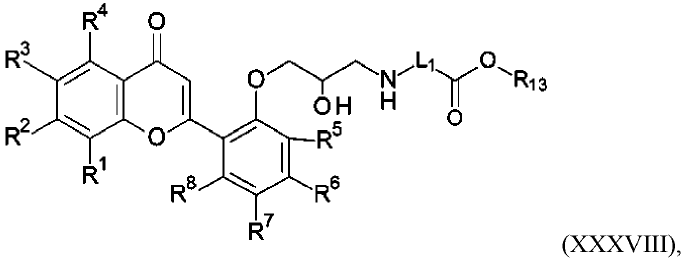 en présence d'une base ; dans lequel R¹ à R¹², L, Cy1, Cy2 et Y sont tels que définis dans l'une quelconque des revendications 1 à 19 ; dans lesquels Q représente un halogène ou Oms ; L₁ représente un alkyle en C₁₋₆, un carbonyle ou un cycloalkyle en C₃₋₇ ; R¹³ représente un alkyle en C₁₋₆ ; R¹⁴ représente un alkyle en C₁₋₆-sulfonylamino, un cycloalkyle en C₃₋₇-sulfonylamino, un cycloalkyle en C₃₋₇-amino ou un hydroxyhétérocyclyle ; R¹⁵ représente un alkyle en C₁₋₆-phénylsulfonyle.

13. Composé selon l'une quelconque des revendications 1 à 11 pour une utilisation comme substance thérapeutiquement active.

14. Composition pharmaceutique comprenant un composé selon l'une quelconque des revendications 1 à 11 et un véhicule thérapeutiquement inerte.

15. Composé selon l'une quelconque des revendications 1 à 11 pour une utilisation dans le traitement ou la prophylaxie d'une infection par le VHB.
